(19) Europäisches Patentamt
European Patent Office
Office européen des brevets

(11) EP 2 325 175 A1

(12) EUROPEAN PATENT APPLICATION
published in accordance with Art. 153(4) EPC

(43) Date of publication:
**25.05.2011 Bulletin 2011/21**

(21) Application number: **09810074.6**

(22) Date of filing: **31.08.2009**

(51) Int Cl.:
*C07D 239/48* (2006.01) *A61K 31/505* (2006.01)
*A61K 31/506* (2006.01) *A61K 31/5377* (2006.01)
*A61K 31/5513* (2006.01) *A61P 37/06* (2006.01)
*C07D 401/12* (2006.01) *C07D 403/12* (2006.01)
*C07D 405/12* (2006.01) *C07D 405/14* (2006.01)
*C07D 409/12* (2006.01) *C07D 453/02* (2006.01)
*C07D 471/08* (2006.01)

(86) International application number:
**PCT/JP2009/065149**

(87) International publication number:
**WO 2010/024430 (04.03.2010 Gazette 2010/09)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **01.09.2008 JP 2008223323**
**20.05.2009 JP 2009121482**

(71) Applicant: **Astellas Pharma Inc.**
**Tokyo 103-8411 (JP)**

(72) Inventors:
- **TANAKA, Akira**
  **Tokyo 103-8411 (JP)**
- **MUKOYOSHI, Koichiro**
  **Tokyo 103-8411 (JP)**
- **KUNIKAWA, Shigeki**
  **Tokyo 103-8411 (JP)**
- **TAKASUNA, Yuji**
  **Tokyo 103-8411 (JP)**
- **MAEDA, Jun**
  **Tokyo 103-8411 (JP)**
- **CHIDA, Noboru**
  **Tokyo 103-8411 (JP)**
- **NAGASHIMA, Shinya**
  **Tokyo 103-8411 (JP)**

(74) Representative: **HOFFMANN EITLE**
**Patent- und Rechtsanwälte**
**Arabellastraße 4**
**81925 München (DE)**

# (54) 2,4-DIAMINOPYRIMIDINE COMPOUND

(57) [Problem]

Provided is a compound which is useful as an active ingredient for a pharmaceutical having a PKCθ inhibition activity, particularly a pharmaceutical composition for inhibiting acute rejection occurring in transplantation.

[Means for Solution]

The present inventors have conducted extensive studies on a compound having a PKCθ inhibition activity, and as a result, they have found that a compound having a structure such as aralkyl and the like on an amino group at the 2-position and also having a structure such as an adamantylalkyl group and the like on an amino group at the 4-position of 2,4-diaminopyrimidine, or a salt thereof has an excellent PKCθ inhibition activity, thereby completing the present invention. The 2,4-diaminopyrimidine compound of the present invention can be used as a PKCθ inhibitor or an inhibitor of acute rejection occurring in transplantation.

EP 2 325 175 A1

## Description

### Technical Field

The present invention relates to a 2,4-diaminopyrimidine compound which is useful as an active ingredient for a pharmaceutical composition, in particular, a pharmaceutical composition for inhibiting acute rejection occurring in transplantation.

### Background Art

Protein kinase C (PKC) is one of the protein kinase families, of which at least ten kinds of isozymes have hitherto been identified and which have been classified into three subfamilies according to differences in the primary structures.

The activation mechanisms of these three subfamilies are greatly different among the subfamilies. A type of PKC which is activated by calcium and diacyl glycerol (DAG) is called a classical PKC (cPKC), and a type of PKC which is activated by DAG but which does not need calcium in this activation is called a novel PKC (nPKC) and a type of PKC which does not need either calcium or DAG is called atypical PKC (aPKC).

Furthermore, each subfamily consists of plural isozymes, cPKC is classified into PKCα, PKCβ and PKCγ, nPKC is classified into PKCδ, PKCε, $PKC_{\eta}$ and PKCθ, and aPKC is classified into PKCξ and PKCλ.
The expression distribution of each isozyme covers a relatively wide range, but the expression of a PKCθ which is one nPKC is restricted to the T lymphocytes and the skeletal muscles. In addition, the phenotype of knockout mice of PKCθ exhibits inhibition of T cell signaling or induction of T cell anergy, and further, from the viewpoint that abnormalities of the skeletal muscles are not observed, PKCθ is promising as a target of an immunosuppressor having few side-effects.

Moreover, since PKCθ is in complementary relationship in the T cell receptor signaling pathway with calcineurin, which is a target molecule of FK506 and cyclosporin A, which have been widely used in current transplantation medication, there is a possibility that combination use of a calcineurin inhibitor and a PKCθ inhibitor will express a synergic immunosuppressive effect.

Therefore, it is considered that if PKCθ is inhibited selectively, an immunosuppressive activity is expressed with a low level of side-effects, and as a transplantation medication, it is promising in regard to the inhibition of acute rejection occurring in transplantation, and also, there is a possiblity that it will be able to express a synergic immunosuppressive activity when used in combination with a calcineurin inhibitor.

In Patent Citation 1, it is reported that a compound represented by the formula (A) inhibits PKCθ and is useful as an immunosuppressor. As a specific compound, a compound having a pyrimidine structure is disclosed, but there is no specific disclosure of the compound of the present invention.

[Chem. 1]

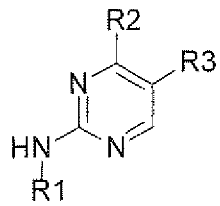

(A)

(R2 in the formula represents

[Chem. 2]

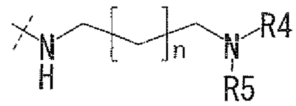
,
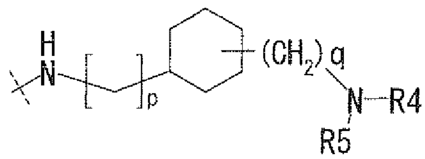
,

or the like. For the other symbols, reference can be made to the publication.)

In Patent Citation 2, it is reported that a compound represented by the formula (B) inhibits PKCθ and is useful as an immunosuppressor. As a specific compound, a compound having a pyrimidine structure is disclosed, but there is no specific disclosure of the compound of the present invention.

[Chem. 3]

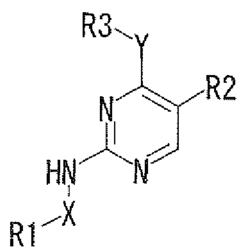
(B)

(R3 in the formula represents

[Chem. 4]

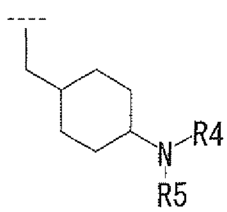
,
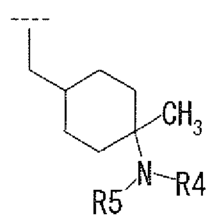
,
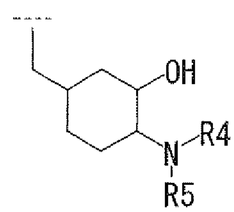
.

For the other symbols, reference can be made to the publication.)

In Patent Citation 3, it is reported that a compound represented by the formula (C) inhibits PKCθ and is useful as an immunosuppressor. As a specific compound, a compound having a pyrimidine structure is disclosed, but there is no specific disclosure of the compound of the present invention.

[Chem. 5]

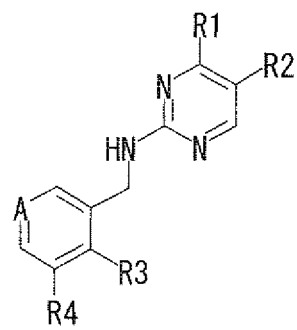

(C)

(R1 in the formula represents

[Chem. 6]

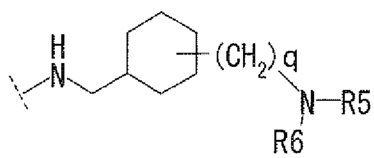

,

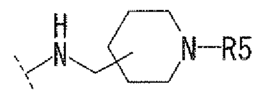

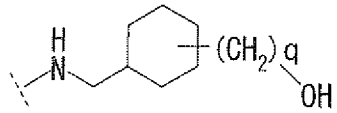

,

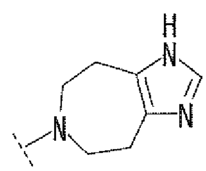

.

For the other symbols, reference can be made to the publication.)

In Patent Citation 4, it is reported that a compound represented by the formula (D) has an inhibition activity against a cyclin-dependent kinase (CDK), a kinase of Aurora B, or the like, and is useful for treatment and prevention of diseases characterized by excessive or abnormal cell growth. As a specific compound, a compound having a pyrimidine structure is disclosed and there is a description that the compound is useful for immunosuppression in organ transplantation, but there is no specific disclosure of the compound of the present invention.

[Chem. 7]

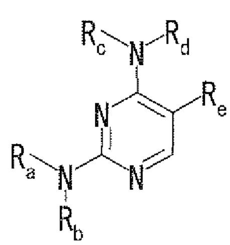

Formula (D)

(For the symbols in the formula, reference can be made to the publication.)

In Patent Citation 5, it is reported that a compound represented by the formula (E) inhibits a polo-like kinase (PLK) and is thus useful for prevention and/or treatment of diseases associated with tumors, neurodegenerative diseases,

and activation of immune systems. As a specific compound, a compound having a pyrimidine structure is disclosed, but there is no specific disclosure of the compound of the present invention. Also, there is neither description of technologies concerning a PKCθ inhibition activity nor description that the compound is useful for inhibition of acute rejection occurring in transplantation.

[Chem. 8]

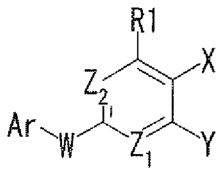

Formula (E)

(For the symbols in the formula, reference can be made to the publication.)

In Patent Citation 6, it is reported that a compound represented by the formula (F) inhibits a G protein-coupled receptor protein 88 (GPR88) and is thus useful for prevention and/or treatment of central nervous system diseases. As a specific compound, a compound having a pyrimidine structure is disclosed, but there is no specific disclosure of the compound of the present invention. Also, there is neither description of technologies concerning a PKCθ inhibition activity nor description that the compound is useful for inhibition of acute rejection occurring in transplantation.

[Chem. 9]

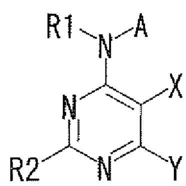

(F)

(R1 in the formula represents hydrogen or the like and A represents a heterocyclic group which may be substituted, heterocyclic alkyl which may be substituted, $C_{3-8}$ cycloalkyl which may be substituted, or the like. For the other symbols, reference can be made to the publication.)

Prior Art Citation

Patent Citation

[Patent Citation 1] Pamphlet of International Publication WO 2004/067516
[Patent Citation 2] Pamphlet of International Publication WO 2006/014482
[Patent Citation 3] Pamphlet of International Publication WO 2007/076247
[Patent Citation 4] Pamphlet of International Publication WO 2003/032997
[Patent Citation 5] Pamphlet of International Publication WO 2004/043936
[Patent Citation 6] Pamphlet of International Publication WO 2004/054617

Disclosure of Invention

Problems to Be Solved by the Invention

It is an object of the present invention to provide a 2,4-diaminopyrimidine compound which is useful as an active ingredient of a pharmaceutical having a PKCθ inhibition activity, particularly, a pharmaceutical composition for

inhibiting acute rejection occurring in transplantation.

Means for Solving the Problem

The present inventors have conducted extensive studies on a compound having a PKCθ inhibition activity, and as a result, they have found that a compound having a structure such as aralkyl and the like on an amino group at the 2-position and also having a structure such as an adamantylalkyl group and the like on an amino group at the 4-position of 2,4-diaminopyrimidine, or a salt thereof has an excellent PUCθ inhibition activity, thereby completing the present invention.
Thus, the present invention relates to a compound of the formula (I) or a pharmaceutically acceptable salt thereof, and a pharmaceutical composition comprising the compound of the formula (I) or a pharmaceutically acceptable salt thereof and a pharmaceutically acceptable excipient.

[Chem. 10]

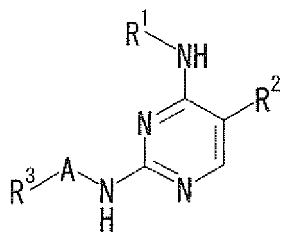

(I)

(the symbols in the formula have the following meanings:

$R^1$ represents any one group selected from the group consisting of:

[Chem. 11]

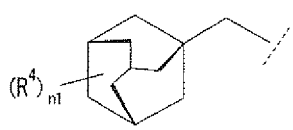
,
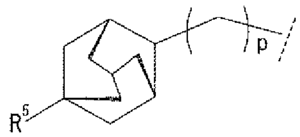
,
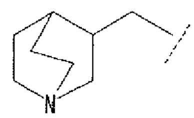
,
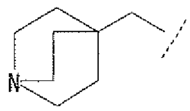
,
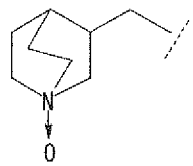
,
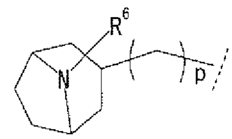
,
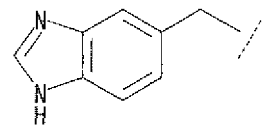
,
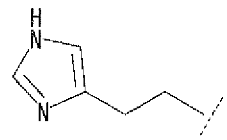
,
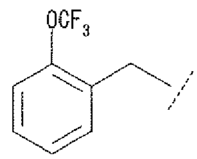
,
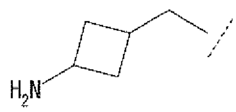
,
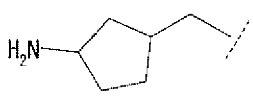
,
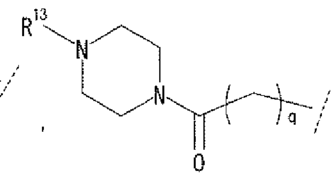
,
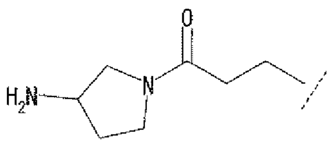
,
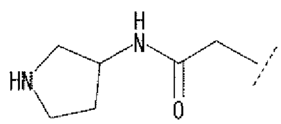
,
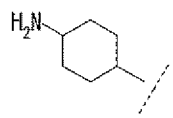
,
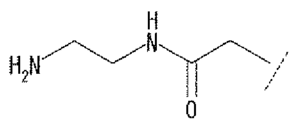
,
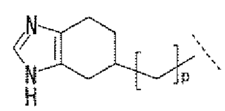
,
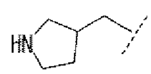
,
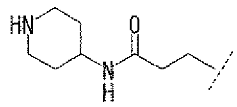
,
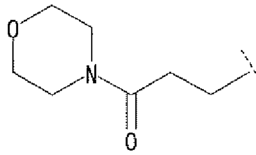
,
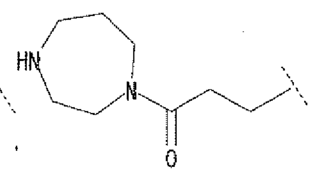
;

$R^4$ represents -OH, amine which may be substituted, or $-CH_2NH_2$;
n1 represents 0 or 1;
$R^5$ represents -OH, ($C_{1-6}$ alkyl which may be substituted with -OH or $-NH_2$), or -CN;
$R^6$ represents -H or $C_{1-6}$ alkyl which may be substituted with aryl;
p represents 0 or 1;
q represents 1, 2, 3, or 4;
$R^{13}$ represents -H or $C_{1-6}$ alkyl;
$R^2$ represents -CN, $-CF_3$, $-NO_2$, or halogen;
A represents a single bond or $C_{1-6}$ alkylene;
$R^3$ represents any one group selected from the group consisting of:

[Chem. 12]

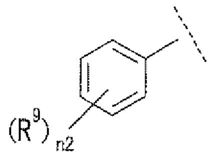 , 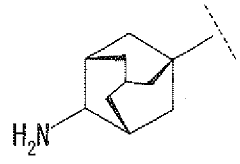 , 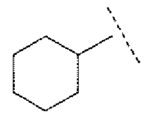 ,

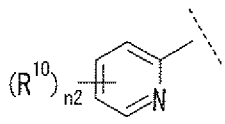 , 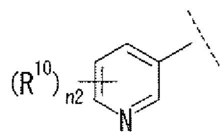 , 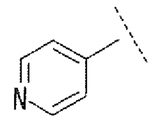 ,

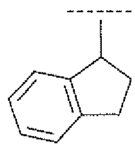 , 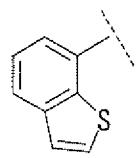 , 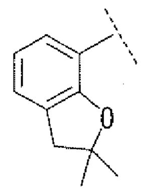 , 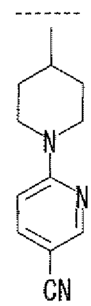

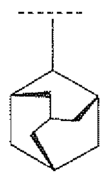 , 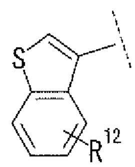 , 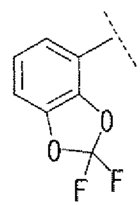 , ;

$R^9$s are the same as or different from each other and represent halogen, $C_{1-6}$ alkyl which may be substituted, -OH, -CN, cycloalkyl, -Q-($C_{1-6}$ alkyl which may be substituted), or aryl which may be substituted;
Q represents -O-, -S-, -SO-, $-SO_2-$, or $-NHSO_2-$;
n2 represents 0, 1, 2, or 3;
$R^{10}$ represents halogen, $C_{1-6}$ alkyl, -CN, -O-($C_{1-6}$ alkyl), -S-($C_{1-6}$ alkyl), -SO-($C_{1-6}$ alkyl), $-SO_2-$($C_{1-6}$ alkyl), -S-(cycloalkyl), or $-OCF_3$; and
$R^{12}$ represents -H or halogen).

In this regard, when a symbol in a certain chemical formula in this specification is used in a different chemical formula, the same symbol has the same meaning, unless otherwise indicated.

In addition, the present invention relates to a pharmaceutical composition comprising the compound of the formula (I) or a pharmaceutically acceptable salt thereof, and a pharmaceutically acceptable excipient, for inhibiting acute rejection occurring in transplantation; i.e., an agent for inhibiting acute rejection occurring in transplantation, comprising the compound of the formula (I) or a pharmaceutically acceptable salt thereof, and a pharmaceutically acceptable excipient.

Moreover, the present invention relates to use of the compound of the formula (I) or a pharmaceutically acceptable salt thereof for the manufacture of an inhibitor of acute rejection occurring in transplantation, and a method for inhibiting acute rejection occurring in transplantation, comprising administering to a patient an effective amount of the compound of the formula (I) or a pharmaceutically acceptable salt thereof.

Effects of the Invention

The compound of the formula (I) or a pharmaceutically acceptable salt thereof has a PKCθ inhibition action and can be used as an inhibitor of acute rejection occurring in transplantation.

Best Mode for Carrying Out the Invention

According to the present invention, the following are provided.

[1]
A compound of the formula (I) or a pharmaceutically acceptable salt thereof:

[Chem. 13]

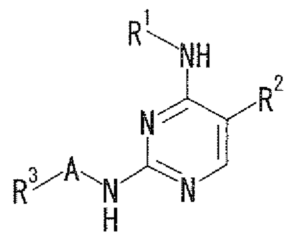

(I)

(the symbols in the formula have the following meanings:

$R^1$ represents any one group selected from the group consisting of:

[Chem. 14]

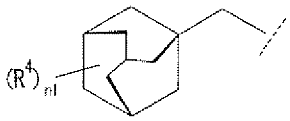, 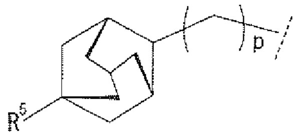, 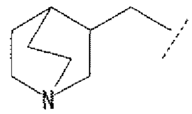,

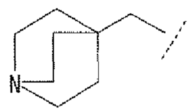, 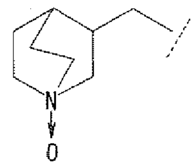, 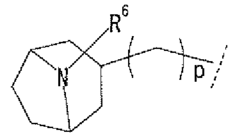,

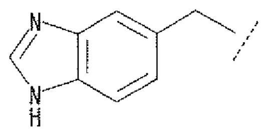, 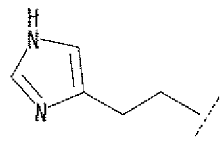, 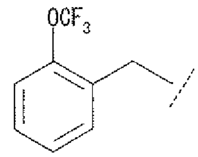,

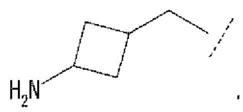, 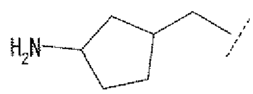, 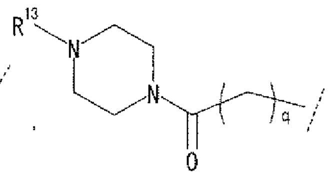,

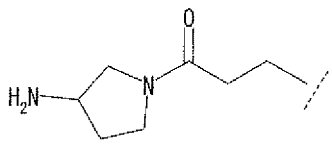, 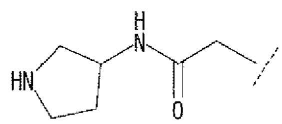, 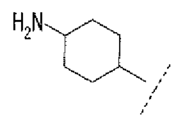,

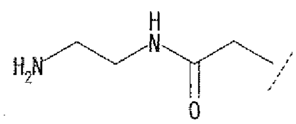, 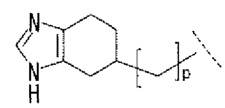, 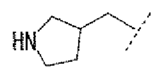,

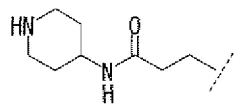, 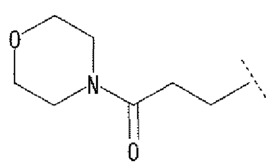, 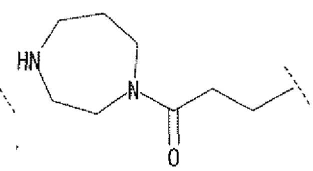;

$R^4$ represents -OH, amine which may be substituted, or $-CH_2NH_2$;
n1 represents 0 or 1;
$R^5$ represents -OH, ($C_{1-6}$ alkyl which may be substituted with -OH or $-NH_2$), or -CN;
$R^6$ represents -H or $C_{1-6}$ alkyl which may be substituted with aryl;
p represents 0 or 1;
q represents 1, 2, 3, or 4;
$R^{13}$ represents -H or $C_{1-6}$ alkyl;
$R^2$ represents -CN, $-CF_3$, $-NO_2$, or halogen;
A represents a single bond or $C_{1-6}$ alkylene;
$R^3$ represents any one group selected from the group consisting of:

[Chem. 15]

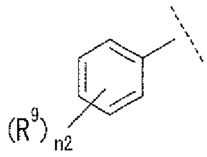,
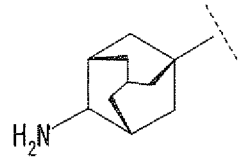,
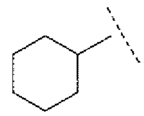,
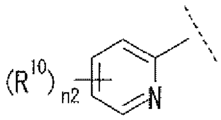,
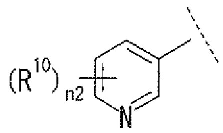,
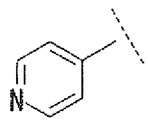,
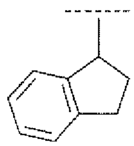,
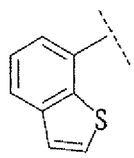,
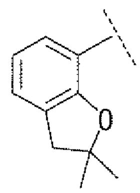,
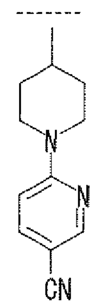
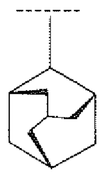,
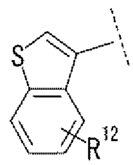,
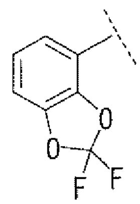,
;

$R^9$s are the same as or different from each other and represent halogen, $C_{1-6}$ alkyl which may be substituted, -OH, -CN, cycloalkyl, -Q-($C_{1-6}$ alkyl which may be substituted), or aryl which may be substituted;
Q represents -O-, -S-, -SO-, $-SO_2-$, or $-NHSO_2-$;
n2 represents 0, 1, 2, or 3;
$R^{10}$ represents halogen, $C_{1-6}$ alkyl, -CN, -O-($C_{1-6}$ alkyl), -S-($C_{1-6}$ alkyl), -SO-($C_{1-6}$ alkyl), $-SO_2$-($C_{1-6}$ alkyl), -S-(cycloalkyl), or $-OCF_3$; and
$R^{12}$ represents -H or halogen).

[2]
The compound or a pharmaceutically acceptable salt thereof described in [1], wherein
$R^4$ is -OH, $-NR^7R^8$, or $-CH_2NH_2$;
$R^7$ and $R^8$ are the same as or different from each other and represent:

(a) -H;
(b) $C_{1-6}$ alkyl, in which the $C_{1-6}$ alkyl may be substituted with at least one group selected from the group consisting of the following 1) to 12):

1) -OH
2) protected -OH
3) halogen
4) -COOH
5) $-CONH_2$
6) oxo
7) aryl
8) heteroaryl
9) cycloalkyl which may be substituted with at least one group selected from the group consisting of -OH, protected -OH, ($C_{1-6}$ alkyl which may be substituted with -OH), halogen, -CN, $-NR_{14}R_{15}$, $-CONR_{14}R_{15}$, $-SO_2NR_{14}R_{15}$, ($C_{1-6}$ alkyl which may be substituted with -OH)-O-, and oxo

10) heterocycloalkyl which may be substituted with -OH or ($C_{1-6}$ alkyl which may be substituted with -OH, $-OCH_3$, -CN, or halogen)
11) (heterocycloalkyl which may be substituted with -OH or $-NH_2$)-CO-, and
12) (heterocycloalkyl)-NH-CO-;

(c) cycloalkyl, in which the cycloalkyl may be substituted with at least one group selected from the group consisting of the following 1) to 6):

1) -OH
2) $-NHR^{11}$
3) halogen
4) oxo
5) $C_{1-6}$ alkyl which may be substituted with -OH, and
6) heterocycloalkyl which may be substituted with (halogen, -OH, $-CH_2OH$, or $-COCH_3$);

(d) heterocycloalkyl, in which the heterocycloalkyl may be substituted with at least one group selected from the group consisting of the following 1) to 11):

1) $C_{1-6}$ alkyl which may be substituted with (-OH, $-OCH_3$, -CN, halogen, or $-CONH_2$)
2) cycloalkyl
3) aryl
4) heterocycloalkyl
5) heterocycloalkyl-CO-
6) $-COCH_3$
7) $-CONH_5$
8) $-COCH_2OH$
9) $-COOCH_2CH_3$
10) $-SO_2CH_3$
11) oxo, and
12) halogen;

(e) acryl;
(f) nicotinoyl; and
(g) $-SO_2CH_3$; or
(h) $R^7$ and $R^8$, together with a nitrogen atom to which they bind, are a nitrogen-containing a heterocycloalkyl which may be substituted with at least one group selected from the group consisting of (-OH, $-NH_2$, -COOH, $-COCH_3$, $-CONH_2$ and $-CH_2OH$);

$R^{11}$ is -H, $C_{1-6}$ alkyl which may be substituted with (halogen or -OH), cycloalkyl which may be substituted with halogen, heterocycloalkyl which may be substituted with $-COCH_3$, or $-COCH_3$; and
$R^{14}$ and $R^{15}$ are the same as or different from each other and are -H, $C_{1-6}$ alkyl, or heterocycloalkyl.

[3]
The compound or a pharmaceutically acceptable salt thereof described in [2],
wherein
$R^1$ is

[Chem. 16]

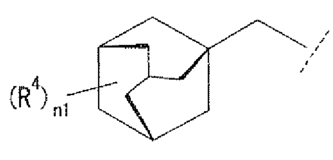

;

and $R^3$ is

[Chem. 17]

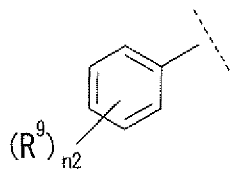

or

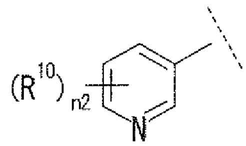

.

[4]

The compound or a pharmaceutically acceptable salt thereof described in [3], wherein

$R^4$ is $-NR^7R^8$;

R and $R^8$ are the same as or different from each other and are

(b) $C_{1-6}$ alkyl, in which the $C_{1-6}$ alkyl may be substituted with at least one group selected from the group consisting of the following 1) to 12):

1) -OH
2) -OH protected with methyl group, or when having two OH groups adjacent to each other, -OH protected with a dimethylmethylene group or a benzylidene group
3) -F
4) -COOH
5) $-CONH_2$
6) oxo
7) phenyl
8) pyridyl
9) cyclohexyl which may be substituted with at least one group selected from the group consisting of -OH and ($C_{1-6}$ alkyl which may be substituted with -OH)
10) (piperidinyl or pyrrolidinyl) which may be substituted with -OH or ($C_{1-6}$ alkyl which may be substituted with -OH, $-OCH_3$, -CN, or -F)
11) (piperazinyl)-CO- or (piperidinyl which may be substituted with -OH or $-NH_2$)-CO-, and
12) (piperidinyl)-NH-CO-; or

(c) cycloalkyl, in which the cycloalkyl may be substituted with at least one group selected from the group consisting of the following 1) to 6):

1) -OH
2)$-NHR^{11}$
3) -F
4) oxo
5) $C_{1-6}$ alkyl which may be substituted with -OH, and
6) (azetidinyl, pyrrolidinyl, piperidinyl, piperazinyl or morpholinyl) which may be substituted with (halogen, -OH, $-CH_2OH$, or $-COCH_3$);

$R^{11}$ is -H;
n1 is 1;
$R^2$ is -CN, $-CF_3$, $-NO_2$, or -F;
A is $C_{1-6}$ alkylene;
$R^9$ is

(i) -F, -Cl, or -Br
(j) $C_{1-6}$ alkyl which may be substituted with -OH or halogen,
(k) -OH,
(l) -CN,

(m) cyclopropyl,
(n) -Q-($C_{1-6}$ alkyl which may be substituted with halogen, -OH, $-OCH_3$, -CN, or $-CONH_2$), or
(o) phenyl which may be substituted with $-CH_2NH_2$; and

n2 is 1.

[5]
The compound or a pharmaceutically acceptable salt thereof described in [4],
wherein
$R^7$ and $R^8$ are the same as or different from each other and are

(b) $C_{1-6}$ alkyl, in which the $C_{1-6}$ alkyl may be substituted with the following groups:

9) cyclohexyl substituted with at least one group selected from the group consisting of -OH, $-CH_3$, and $-CH_2OH$, and
10) piperidinyl which may be substituted with -OH or ($C_{1-6}$ alkyl which may be substituted with -OH, $-OCH_3$, -CN, or -F); or

(c) cycloalkyl, in which the cycloalkyl, may be substituted with at least one group selected from the group consisting of the following 1), 2), and 5):

1)-OH
2) $-NHR^{11}$, and
5) $C_{1-6}$ alkyl which may be substituted with -OH;

$R^{11}$ is-H;
$R^2$ is -CN;
A is methylene;
$R^9$ is
(i) -F, -Cl, or -Br
(j) $C_{1-6}$ alkyl which may be substituted with -OH or -F,
(k) -OH,
(l) -CN,
(m) cyclopropyl,
(n) -Q-($C_{1-6}$ alkyl which may be substituted with halogen, -OH, $-OCH_3$, -CN, or $-CONH_2$), or
(o) phenyl which may be substituted with $-CH_2NH_2$; and

$R^{10}$ is -Cl, $-CH_3$, $-OCH_3$, $-OCH_2CH_3$, $-OCH(CH_2)_2$, $-SCH_3$, $-SCH_2CH_3$, $-SCH(CH_3)_2$, $-SOCH_3$, $-SO_2CH_3$, -S-(cyclopentane), or $-OCF_3$.

[6] A pharmaceutical composition comprising the compound or a pharmaceutically acceptable salt thereof described in [1], and a pharmaceutically acceptable excipient.

[7] A PKCθ inhibitor comprising the compound or a pharmaceutically acceptable salt thereof described in [1].

[8] A pharmaceutical composition for inhibiting acute rejection occurring in transplantation, comprising the compound or a pharmaceutically acceptable salt thereof described in [1].

[9] Use of the compound or a pharmaceutically acceptable salt thereof described in [1] for the manufacture of an inhibitor of acute rejection occurring in transplantation.

[10] A method for inhibiting acute rejection occurring in transplantation, comprising administering to a patient an effective amount of the compound or a pharmaceutically acceptable salt thereof described in [1].

Hereinbelow, the present invention will be described in detail.
In the present specification, the "$C_{1-6}$ alkyl" is linear or branched alkyl having 1 to 6 carbon atoms, and examples thereof include a methyl, ethyl,, n-propyl, isopropyl, n-butyl, isobutyl, sec-butyl, tert-butyl, n-pentyl, n-hexyl, and the like.
In the present specification, the "$C_{1-6}$ alkylene" is linear or branched $C_{1-6}$ alkylene, and examples thereof

include methylene, ethylene, trimethylene, tetramethylene, pentamethylene, hexamethylene, propylene, methylmethylene, ethylethylene, 1,2-dimethylethylene, 1,1,2,2-tetramethylethylene, and the like. In another embodiment, it is $C_{1-4}$ alkylene, in a further embodiment, $C_{1-2}$ alkylene, and in a still further embodiment, methylene or ethylene.

In the present specification, the "halogen" means F, Cl, Br, or I.

In the present specification, the "cycloalkyl" is a $C_{3-10}$ saturated hydrocarbon ring group, which may have a bridge. Examples thereof include cyclopropyl, cyclobutyl, cyclopentyl, cyclohexyl, cycloheptyl, cyclooctyl, adamantyl, and the like. In another embodiment, it is $C_{3-8}$ cycloalkyl, in a further embodiment, $C_{3-6}$ cycloalkyl, and in a still further embodiment, cyclopropyl, cyclobutyl, cyclopentyl, or cyclohexyl.

In the present specification, the "aryl" is a $C_{6-14}$ monocyclic to tricyclic aromatic hydrocarbon ring group, and examples thereof include phenyl and naphthyl, and in another embodiment, phenyl.

In the present specification, the "heterocyclic ring" is a ring group selected from i) a monocyclic 3- to 8-membered heterocyclic ring, and preferably, 5- to 7-membered heterocyclic ring, containing 1 to 4 heteroatoms selected from oxygen, sulfur, and nitrogen, and ii) a bicyclic to tricyclic heterocyclic ring group containing 1 to 5 heteroatoms selected from oxygen, sulfur, and nitrogen, formed by condensation with one or two rings in which the monocyclic heterocyclic ring group is selected from the group consisting of a monocyclic heterocyclic ring group, a benzene ring, $C_{5-8}$ cycloalkane, and $C_{5-8}$ cycloalkene. The ring atom, sulfur or nitrogen, may be oxidized to form an oxide or a dioxide.

Examples of the "heterocyclic ring" include the following embodiments.

(1) Monocyclic saturated heterocyclic ring

(a) those containing 1 to 4 nitrogen atoms, for example, azepanyl, diazepanyl, aziridinyl, azetidinyl, pyrrolidinyl, imidazolylidinyl, piperidyl, pyrazolidinyl, piperazinyl, azocanyl, and the like;
(b) those containing 1 to 3 nitrogen atoms and 1 to 2 sulfur atoms and/or 1 to 2 oxygen atoms, for example, thiomorpholinyl, thiazolidinyl, isothiazolidinyl, oxazolidinyl, morpholinyl, and the like;
(c) those containing 1 to 2 sulfur atoms, for example, tetrahydro-2H-thiopyranyl and the like;
(d) those containing 1 to 2 sulfur atoms and 1 to 2 oxygen atoms, for example, oxathiolanyl and the like; and
(e) those containing 1 to 2 oxygen atoms, for example, oxiranyl, oxetanyl, dioxolanyl, tetrahydrofuranyl, tetrahydro-2H-pyranyl, 1,4-dioxanyl, and the like;

(2) Monocyclic unsaturated heterocyclic group

(a) those containing 1 to 4 nitrogen atoms, for example, pyrrolyl, imidazolyl, pyrazolyl, pyridyl, dihydropyridyl, tetrahydropyridinyl, pyrimidinyl, pyrazinyl, pyridazinyl, triazolyl, tetrazolyl, triazinyl, dihydrotriazinyl, azepinyl, and the like;
(b) those containing 1 to 3 nitrogen atoms and 1 to 2 sulfur atoms and/or 1 to 2 oxygen atoms, for example, thiazolyl, isothiazolyl, thiadiazolyl, dihydrothiazinyl, oxazolyl, isoxazolyl, oxadiazolyl, oxazinyl, and the like;
(c) those containing 1 to 2 sulfur atoms, for example, thienyl, thiepinyl, dihydrodithiopyranyl, dihydrodithionyl, and the like;
(d) those containing 1 to 2 sulfur atoms and 1 to 2 oxygen atoms, for example, dihydroxathiopyranyl and the like; and
(e) those containing 1 to 2 oxygen atoms, for example, furyl, pyranyl, oxepinyl, dioxolyl, and the like;

(3) Condensed polycyclic saturated heterocyclic group

(a) those containing 1 to 5 nitrogen atoms, for example, quinuclidinyl, 7-azabicyclo[2.2.1]heptyl, 3-azabicyclo[3.2.2]nonanyl, and the like;
(b) those containing 1 to 4 nitrogen atoms and 1 to 3 sulfur atoms and/or 1 to 3 oxygen atoms, for example, trithiadiazaindenyl, dioxoloimidazolidinyl, and the like; and
(c) those containing 1 to 3 sulfur atoms and/or 1 to 3 oxygen atoms, for example, 2,6-dioxabicyclo[3.2.2]octo-7-yl, and the like;

(4) Condensed polycyclic unsaturated heterocyclic ring group

(a) those containing 1 to 5 nitrogen atoms, for example, indolyl, isoindolyl, indolinyl, indolidinyl, benzoimidazolyl, dihydrobenzoimidazolyl, tetrahyzorobenzimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, acridinyl, quinoxalinyl, dihydroquinoxalinyl, tetrahydroqunioxalinyl, phthalazinyl, dihydroindazolyl, benzopyrimidinyl, naphthyridinyl, quinazolinyl, cinnolinyl, and the like;
(b) those containing 1 to 4 nitrogen atoms and 1 to 3 sulfur atoms and/or 1 to 3 oxygen atoms, for example, benzothiazolyl, dihydrobenzothiazolyl, benzothiadiazolyl, imidazothiazolyl, imidazothiadiazolyl, benzoxazolyl, dihydrobenzoxazolyl, dihydrobenzoxazinyl, benzoxadiazolyl, benzoisothiazolyl, benzoisoxazolyl, and the like;
(c) those containing 1 to 3 sulfur atoms, for example, benzothienyl, benzodithiopyranyl, dibenzo[b,d]thienyl, and the like;
(d) 1 to 3 sulfur atoms and 1 to 3 oxygen atoms, for example, benzoxathiopyranyl, phenoxadinyl, and the like; and
(e) those containing 1 to 3 oxygen atoms, for example, benzodioxolyl, benzofuranyl, dihydrobenzofuranyl, isobenzofuranyl, chromanyl, chromenyl, dibenzo[b,d]furanyl, methylenedioxyphenyl, ethylenedioxyphenyl, and the like;

etc.

In the present specification, the "heterocycloalkyl" is the monocyclic saturated heterocyclic ring group described in (1) and the condensed polycyclic saturated heterocyclic ring group described in (3) among the above-described "heterocyclic rings", in which a ring atom, sulfur or nitrogen, may be oxidized to form an oxide or a dioxide. In another embodiment, it is the monocyclic saturated heterocyclic ring group described in (1), in which a ring atom, sulfur or nitrogen, may be oxidized to form an oxide or a dioxide, and in a further embodiment, it is azetidinyl, pyrrolidinyl, piperidinyl, piperazinyl, morpholinyl, tetrahydro-2H-thiopyranyl, tetrahydrothiopyranyl dioxide, or tetrahydro-2H-pyranyl.

In the present specification, the "nitrogen-containing heterocycloalkyl" is the Monocyclic saturated heterocyclic ring containing at least one nitrogen atom described in (1) (a) and (b), and the condensed polycyclic saturated heterocyclic ring group containing at least one nitrogen atom described in (3) (a) and (b), among the above-described "heterocyclic rings". In another embodiment, the nitrogen-containing heterocycloalkyl is the monocyclic saturated heterocyclic ring containing at least one nitrogen atom described in (1) (a) and (b), and in a further embodiment, azetidinyl, pyrrolidinyl, piperidyl, piperazinyl, or morpholinyl.

In the present specification, the "heteroaryl" is the heterocyclic ring having an aromaticity among (2) the monocyclic unsaturated heterocyclic ring group and (4) the aromatic heterocyclic ring group among the condensed polycyclic unsaturated heterocyclic ring groups of the above-described "heterocyclic ring". In another embodiment, it is the heterocyclic ring having an aromaticity among (2) the aromatic heterocyclic ring group, (monocyclic heteroaryl), and in a further embodiment, pyridyl.

In the present specification, the expression "which may be substituted" means which is not substituted or which has 1 to 5 substituents, and in another embodiment, which is not substituted or which has 1 to 3 substituents. Further, the expression "(which is) substituted" means which has 1 to 5 substituents, and in another embodiment, which has 1 to 3 substituents. Furthermore, if it has a plurality of substituents, the substituents may be the same as or different from each other.

The "protected -OH" means that the OH group is protected with a protecting group usually used for the protection of a hydroxyl group. In another embodiment, it means being protected with an acyl group, an ether group, a silyl ether group, or an acetal group, and in a further embodiment, it means protection with a methyl group or in the case that two OH groups are adjacent to each other, protection with a dimethylmethylene group or a benzylidene group.

Embodiments regarding the compound (I) of the present invention are shown below.

(1) The compound, wherein $R^1$ is

[Chem. 18]

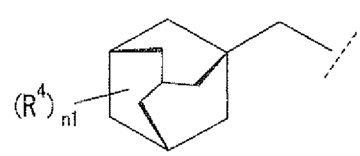

(2) The compound as described in (1), wherein $R^4$ is -OH, $-NR^7R^8$, or $-CH_2NH_2$, and in another embodiment, $-NR^7R^8$.
(3) The compound as described in (2), wherein $R^7$ and $R^8$ are the same as or different from each other and are

(a) -H;

(b) $C_{1-6}$ alkyl, in which the $C_{1-6}$ alkyl may be substituted with at least one group selected from the group consisting of the following 1) to 12):

1) -OH
2) protected -OH, and in another embodiment, -OH protected with a methyl group, or in the case of having two OH groups which are adjacent to each other, -OH protected with a dimethylmethylene group or a benzylidene group
3) halogen, and in another embodiment, -F
4) -COOH
5) $-CONH_2$
6) oxo
7) aryl, and in another embodiment, phenyl
8) heteroaryl, and in another embodiment, pyridyl
9) cycloalkyl which may be substituted with at least one group selected from the group consisting of -OH, protected -OH, ($C_{1-6}$ alkyl which may be substituted with -OH), halogen, -CN, $-NR_{14}R_{15}$, $-CONR_{14}R_{15}$, $-SO_2NR_{14}R_{15}$, ($C_{1-6}$ alkyl which may be substituted with -OH)-O-, and oxo, in another embodiment, cyclohexyl which may be substituted with at least one group selected from the group consisting of -OH and ($C_{1-6}$ alkyl which may be substituted with -OH), and in a further embodiment, cyclohexyl substituted with at least one group selected from the group consisting of -OH, $-CH_3$, and $-CH_2OH$
10) heterocycloalkyl which may be substituted with -OH or ($C_{1-6}$ alkyl which may be substituted with -OH, $-OCH_3$, -CN, or halogen), and in another embodiment, (piperidinyl or pyrrolidinyl) which may be substituted with -OH or ($C_{1-6}$ alkyl which may be substituted with -OH, $-OCH_3$, -CN, or F)
11) (heterocycloalkyl which may be substituted with -OH or $-NH_2$)-CO-, and in another embodiment, (piperazinyl)-CO- or (piperidinyl which may be substituted with -OH or $-NH_2$)-CO-, and
12) (heterocycloalkyl)-NH-CO-, and in another embodiment, (piperidine)-NH-CO-;

(c) cycloalkyl, and in another embodiment, cyclobutyl or cyclohexyl, in which the cycloalkyl may be substituted with at least one group selected from the group consisting of the following 1) to 6):

1) -OH
2) $-NHR^{11}$
3) halogen, and in another embodiment, -F
4) oxo
5) $C_{1-6}$ alkyl which may be substituted with -OH, and in another embodiment, $-CH_3$ or $-CH_2OH$, and
6) heterocycloalkyl which may be substituted with (halogen, -OH, $-CH_2OH$, or $-COCH_3$), and in another embodiment, (azetidinyl, pyrrolidinyl, piperidinyl, piperazinyl, or morpholinyl) which may be substituted with (-F, -OH, $-CH_2OH$ or $-COCH_3$);

(d) heterocycloalkyl, and in another embodiment, azetidinyl, piperidinyl, tetrahydro-2H-pyranyl, or tetrahydro-2H-thiopyranyl, in which the heterocycloalkyl may be substituted with at least one group selected from the group consisting of the following 1) to 11):

1) $C_{1-6}$ alkyl which may be substituted with (-OH, $-OCH_3$, -CN, halogen, or $-CONH_2$), and in another embodiment, $C_{1-6}$ alkyl which may be substituted with (-OH, $-OCH_3$, -CN, -F, or $-CONH_2$)
2) cycloalkyl, and in another embodiment, cyclopropyl
3) aryl, and in another embodiment, phenyl
4) heterocycloalkyl, and in another embodiment, tetrahydro-2H-pyranyl
5) heterocycloalkyl-CO-, and in another embodiment, morpholinyl-CO-
6) $-COCH_3$
7) $-CONH_2$
8) $-COCH_2OH$
9) $-COOCH_2CH_3$
10) $-SO_2CH_3$
11) oxo, and
12) halogen;

(e) aryl, and in another embodiment, phenyl;

(f) nicotinoyl; and
(g) $-SO_2CH_3$; or
(h) $R^7$ and $R^8$, together with a nitrogen atom to which they bind, are a nitrogen-containing heterocycloalkyl which may be substituted with at least one group selected from the group consisting of (-OH, $-NH_2$, -COOH, $-COCH_3$, $-CONH_2$ and-$CH_2OH$), and in another embodiment, (azetidinyl, pyrrolidinyl, piperidinyl, or piperazinyl) which may be substituted with at least one group selected from the group consisting of (-OH, $-NH_2$, -COOH, $-COCH_3$, $-CONH_2$, and-$CH_2OH$).

(4) The compound as described in (3), wherein $R^{11}$ is -H, $C_{1-6}$ alkyl which may be substituted with (halogen or -OH), cycloalkyl which may be substituted with halogen, heterocycloalkyl which may be substituted with $-COCH_3$, or $-COCH_3$, in another embodiment, -H, $C_{1-6}$ alkyl which may be substituted with (-F or -OH), cycloalkyl which may be substituted with -F, heterocycloalkyl which may be substituted with $-COCH_3$, or- $COCH_3$, and in a further embodiment, $C_{1-6}$ alkyl which may be substituted with (-F or -OH), cyclohexyl which may be substituted with -F, tetrahydro-2H-pyranyl, piperidinyl substituted with $-COCH_3$, or $-COCH_3$.
(5) The compound as described in (3), wherein $R^{14}$ and $R^{15}$ are the same as or different from each other and are -H, $C_{1-6}$ alkyl, or heterocycloalkyl, and in another embodiment, -H, methyl, or tetrahydro-2H-pyranyl.
(6) The compound as described in (1), wherein n1 is 1.
(7) The compound, wherein $R^5$ is -OH, $-CH_2OH$, $-CH_2NH_2$, or -CN.
(8) The compound, wherein $R^6$ is -H or $C_{1-6}$ alkyl which may be substituted with aryl, and in another embodiment, -H or $C_{1-6}$ alkyl which may be substituted with phenyl.
(9) The compound, wherein $R^2$ is -CN.
(10) The compound, wherein A is $C_{1-6}$ alkylene, in another embodiment, methylene or ethylene, and in a further embodiment, methylene.
(11) The compound, wherein $R^3$ is

[Chem. 19]

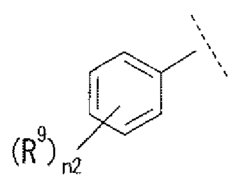
or
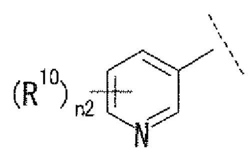
.

(12) The compound as described in (11), wherein $R^9$s are the same as or different from each other and are

(i) halogen, and in another embodiment, -F, -Cl, or -Br;
(j) $C_{1-6}$ alkyl which may be substituted, in another embodiment, $C_{1-6}$ alkyl which may be substituted with -OH or halogen, and in a further embodiment, $C_{1-6}$ alkyl which may be substituted with -OH or -F;
(k) -OH;
(l) -CN;
(m) cycloalkyl, and in another embodiment, cyclopropyl;
(n) -Q-($C_{1-6}$ alkyl which may be substituted), and in another embodiment, -Q-($C_{1-6}$ alkyl which may be substituted with halogen, -OH, $-OCH_3$, -CN, or $-CONH_2$); or
(o) aryl which may be substituted, in another embodiment, aryl which may be substituted with $-CH_2NH_2$, and in a further embodiment, phenyl which may be substituted with $-CH_2NH_2$, and
in a further embodiment, wherein $R^9$ is -Cl, $-O-CF_3$, $-O-CHF_2$, or $-SCH_3$.

(13) The compound as described in (11), wherein n2 is 1.
(14) The compound as described in (11), wherein $R^{10}$ is -Cl, $-CH_3$, $-OCH_3$, $-OCH_2CH_3$, $-OCH(CH_3)_2$, $-SCH_3$, $-SCH_2CH_3$, $-SCH(CH_3)_2$, $-SOCH_3$, $-SO_2CH_3$, -S-(cyclopentane), or $-OCF_3$, and in another embodiment, -Cl, $-CH_3$, $-OCH_3$, or $-SCH_3$.
(15) The compound, wherein $R^{12}$ is -H or -Cl.
(16) The compound, which is a combination of two or more of the groups as described (1) to (15) above.

The compound of the formula (I) may exist in the form of tautomeric properties or geometrical isomers in some

cases, depending on the kind of substituents. In the present specification, the compound shall be described in only one form of isomer, yet the present invention includes other isomers, isolated forms of the isomers, or a mixture thereof.
In addition, the compound of the formula (I) may have asymmetric carbon atoms or axial chirality in some cases, and correspondingly, it may exist in the form of optical isomers. The present invention includes both an isolated form of the optical isomers of the compound of the formula (I) or a mixture thereof.

In addition, the pharmaceutically acceptable prodrugs of the compound represented by the formula (I) are also included in the present invention. The pharmaceutically acceptable prodrug refers to a compound which is converted into the compound of the present invention by solvolysis or under a physiological condition. Examples of the group for forming a prodrug include those as described in Prog. Med., 5, 2157-2161 (1985) or "Iyakuhin no Kaihatsu (Development of Medicines)" (Hirokawa Shoten, 1990), Vol. 7, Bunshi Sekkei (Molecular Design), 163-198.

Furthermore, the compound of the formula (I) refers to a pharmaceutically acceptable salt of the compound of the formula (I), and it may form a salt with an acid or a base, depending on the kind of the substituents. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like, and organic bases such as methylamine, ethylamine, ethanolamine, lysine, ornithine, and the like, salts with various amino acids or amino acid derivatives such as acetylleucine and the like, ammonium salts, and others.

In addition, the present invention also includes various hydrates or solvates, and polymorphic crystal substances of the compound of the formula (I) and a pharmaceutically acceptable salt thereof. Also, the present invention includes compounds labeled with various radioactive or non-radioactive isotopes.

(Preparation Methods)

The compound of the formula (I) and a pharmaceutically acceptable salt thereof can be prepared by applying various known synthesis methods, using the characteristics based on their basic skeletons or the kind of substituents. At this time, depending on the type of the functional groups, it is in some cases effective, from the viewpoint of the preparation techniques, to substitute the functional group with an appropriate protecting group (a group which is capable of being easily converted into the functional group), during the steps from starting materials to intermediates. Examples of such a protective group include those described in "Green's Protective Groups in Organic Synthesis (4th Edition, 2006)", edited by Wuts (P. G. M. Wuts) and Greene (T. W. Green), which may be appropriately selected and used depending on reaction conditions. In these methods, a desired compound can be obtained by introducing the protecting group to carry out the reaction, and then, if desired, removing the protecting group.
In addition, the prodrug of the compound of formula (I) can be prepared by introducing a specific group during the steps from starting materials to intermediates, in the same manner as for the aforementioned protecting groups, or by carrying out the reaction using the obtained compound of formula (I). The reaction can be carried out by applying a method known to a person skilled in the art, such as general esterification, amidation, dehydration, and the like.
Hereinbelow, the representative preparation methods for the compound of formula (I) will be described. Each of the production processes may also be carried out with reference to the References appended in the present description. Further, the preparation methods of the present invention are not limited to the examples as shown below.

Production Process 1

[Chem. 20]

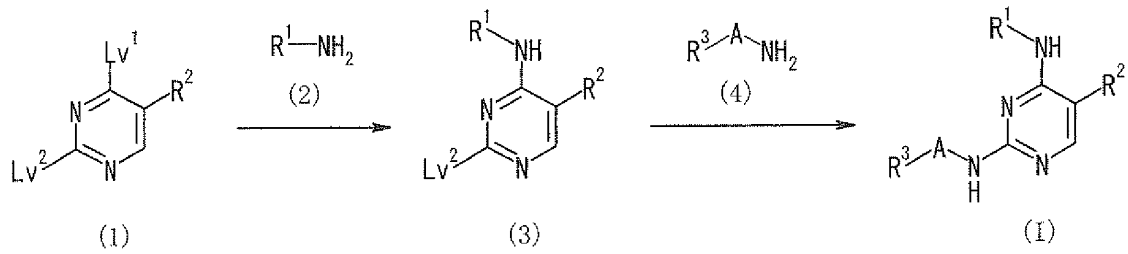

(wherein $Lv^1$ and $Lv^2$ represent a leaving group. The same shall apply hereinafter.)

The present production process is a method in which a compound(1) and an amine compound(2) are subjected to a nucleophilic substitution reaction to prepare a compound (3), and the obtained compound (3) and an amine compound (4) are subjected to a nucleophilic substitution reaction to prepare the compound (I) of the present invention. Here, examples of the leaving group include halogen, a methanesulfonyloxy group, a methylsulfinyl group, a methylsulfonyl group, a p-toluenesulfonyloxy group, and the like.

In this reaction, the compound (1) and the compound (2), or the compound (3) and the compound (4) are used in equivalent amounts or with either thereof in an excess amount, and the mixture is stirred under any temperature condition from cooling to heating with reflux in a solvent which is inert to the reaction or without a solvent, preferably at 0˚C to 80˚C, usually for 0.1 hour to 5 days. The solvent used herein is not particularly limited, but examples thereof include aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, N-methylpyrrolidone, 1,3-dimethyl-2-imidazolidinone, dimethylsulfoxide, ethyl acetate, acetonitrile, and a mixture thereof. It may be advantageous in some cases for the smooth progress of the reaction to carry out the reaction in the presence of organic bases such as triethylamine, N,N-diisopropylethylamine, N-methylmorpholine, and the like, or inorganic bases such as potassium carbonate, sodium carbonate, potassium hydroxide, and the like. In this regard, the compound (2) may be reacted after reacting the compound (1) and the compound (4) first.

[Citations]

"Organic Functional Group Preparations", edited by S. R. Sandler and W. Karo, 2nd Edition, Vol. 1, Academic Press Inc., 1991

"Jikken Kagaku Koza (Courses in Experimental Chemistry) (5th Edition)", edited by The Chemical Society of Japan, Vol. 14 (2005) (Maruzen)

Production Process 2: Other Production Processes

Moreover, several compounds represented by the formula (I) can be prepared from the compound of the formula (I) of the present invention obtained above, by any combination of the processes that can be generally employed by a person skilled in the art, such as well-known amidation, alkylation, reductive amination, reduction of a carbonyl group to a hydroxyl group, and the like. For example, they can be prepared, for example, by the reactions as described below, the methods as described in Examples to be described later, the methods known to a skilled person in the art, or a modified method thereof.

2-1: Amidation

An amide compound can be obtained by subjecting a carboxylic acid compound and an amine compound to amidation.

In this reaction, a carboxylic acid compound and an amine compound are used in equivalent amounts, or with either thereof in an excess amount, and the mixture thereof is stirred at any temperature from under cooling to heating, preferably at a temperature from - 20˚C to 60˚C, usually for 0.1 hour to 5 days, in a solvent which is inert to the reaction, in the presence of a condensing agent. Examples of the solvent as used herein are not particularly limited, and include

aromatic hydrocarbons such as benzene, toluene, xylene, and the like, halogenated hydrocarbons such as dichloromethane, 1,2-dichloroethane, chloroform, and the like, ethers such as diethyl ether, tetrahydrofuran, dioxane, dimethoxyethane, and the like, N,N-dimethylformamide, dimethylsufoxide, ethyl acetate, acetonitrile, water, and a mixture thereof. Examples of the condensing agent include 1-(3-dimethylaminopropyl)-3-ethylcarbodiimide, dicyclohexylcarbodiimide, 1,1'-carbonyldiimidazole, diphenylphosphoric azide, and phosphorus oxychloride, but are not limited thereto. It may be preferable for the reaction in some cases to use an additive (for example, 1-hydroxybenzotriazole). It may be advantageous in some cases for the smooth progress of the reaction to carry out the reaction in the presence of an organic base such as triethylamine, N,N-diisopropylethylamine, N-methylmorpholine, and the like, or an inorganic base such as potassium carbonate, sodium carbonate, potassium hydroxide, and the like.
Further, a method in which the carboxylic acid is converted into a reactive derivative thereof, and then the reactive derivative is reacted with the amine compound may also be used. Examples of the reactive derivative of the carboxylic acid include acid halides obtained by the reaction of a halogenating agent such as phosphorus oxychloride, thionyl chloride, and the like, mixed acid anhydrides obtained by the reaction of isobutyl chloroformate or the like, active esters obtained by the condensation with 1-hydroxybenzotriazole or the like, etc. The reaction of the reactive derivative and the amine compound can be carried out at any temperature from under cooling to heating, preferably at -20˚C to 60˚C, in a solvent which is inert to the reaction, such as halogenated hydrocarbons, aromatic hydrocarbons, ethers, and the like.

[Citations]

"Organic Functional Group Preparations", edited by S. R. Sandler and W. Karo, 2nd Edition, Vol. 1, Academic Press Inc., 1991
"Jikken Kagaku Koza (Courses in Experimental Chemistry) (5th Edition)", edited by The Chemical Society of Japan, Vol. 16 (2005) (Maruzen)

2-2: Alkylation

An alkyl amine compound can be prepared by alkylating the amine compound with a compound having a leaving group.
The alkylation can be carried out by the same method as in Production Process 1.

2-3: Reductive amination

An amine compound can be alkylated by reducing an imine compound which is prepared from a carbonyl compound and a primary or secondary amine compound.
In this reaction, the carbonyl compound and the primary or secondary amine compound are used in equivalent amounts, or with either thereof in an excess amount, and the mixture thereof is stirred at any temperature from under cooling to heating, preferably at a temperature from -45˚C to heating under reflux, and preferably at 0˚C to room temperature, usually for 0.1 hour to 5 days, in a solvent which is inert to the reaction, in the presence of a reducing agent. Examples of the solvent as used herein are not particularly limited, and include alcohols such as methanol, ethanol, and the like, halogenated hydrocarbons such as dichloromethane, 1,2-dichloroethane, chloroform, and the like, ethers such as diethyl ether, tetrahydrofuran, dioxane, dimethoxyethane, and the like, and a mixture thereof. Examples of the reducing agent include sodium cyanoborohydride, sodium triacetoxyborohydride, sodium borohydride, and the like. The reaction may be preferably carried out in the presence of a dehydrating agent such as molecular sieves and the like, or an acid such as acetic acid, hydrochloric acid, titanium (IV) isopropoxide complexes, and the like in some cases. According to the reaction, there may be some cases where an imine is produced by the condensation of the carbonyl compound with the primary or secondary amine compound and it can be isolated as a stable intermediate. In this case, the imine intermediate can be isolated, and then subjected to a reduction reaction, thereby obtaining a desired product. Further, the reaction can be carried out in a solvent such as methanol, ethanol, ethyl acetate, and the like, in the presence or absence of an acid such as acetic acid, hydrochloric acid, and the like, using a reduction catalyst (for example, palladium on carbon, Raney nickel, and the like), instead of treatment with the reducing agent. In this case, it is preferable to carry out the reaction under a hydrogen atmosphere at normal pressure to 50 atmospheres from under cooling to under heating.

[Citations]

"Comprehensive Organic Functional Group Transformations II", edited by A. R. Katritzky and R. J. K. Taylor, Vol. 2, Elsevier Pergamon, 2005,
"Jikken Kagaku Koza (Courses in Experimental Chemistry) (5th Edition)", edited by The Chemical Society of Japan, Vol. 14 (2005) (Maruzen).

2-4: Reduction of Carbonyl Group to Hydroxyl Group

An alcohol compound can be obtained by subjecting a carbonyl compound to reduction.
In this reaction, the carbonyl compound is treated with an equivalent amount or an excess amount of a reducing agent at any temperature from under cooling to heating, preferably at a temperature from -20°C to 80°C, usually for 0.1 hour to 3 days, in a solvent which is inert to the reaction. Examples of the solvent as used herein are not particularly limited, and include ethers such as diethyl ether, tetrahydrofuran, dioxane, dimethoxyethane, and the like, alcohols such as methanol, ethanol, 2-propanol, and the like, aromatic hydrocarbons such as benzene, toluene, xylene, and the like, N, N-dimethylformamide, dimethylsulfoxide, ethyl acetate, and a mixture thereof. As the reducing agent, hydride reducing agents such as sodium borohydride, diisobutylaluminum hydride, and the like, metal reducing agents such as sodium, zinc, iron, and the like, or others described in the following documents are suitably used.

[Citations]

"Reductions in Organic Chemistry, 2nd Ed. (ACS Monograph: 188)" edited by M. Hudlicky, ACS, 1996
"Comprehensive Organic Transformations" edited by R. C. Larock, 2nd ed, VCH Publishers, Inc., 1999
"Oxidation and Reduction in Organic Synthesis (Oxford Chemistry Primers 6)" edited by T. J. Donohoe, Oxford Science Publications, 2000
"Jikken Kagaku Koza (Courses in Experimental Chemistry) (5th Edition)", edited by The Chemical Society of Japan, Vol. 14 (2005) (Maruzen).

(Production Process for Starting Compound)

The starting materials used in the preparation of the compound of the present invention, that is, the amine compound (2) and the amine compound (4) can be prepared, for example, from available well-known compounds, by employing the methods described in Preparation Examples as described later, well-known methods described in "Production Process 2: Other Production Processes", or methods apparent to a skilled person in the art, or modified methods thereof, or the like.
The compound of the formula (I) is isolated and purified as a free compound, pharmaceutically acceptable salts thereof, hydrates, solvates, or polymorphic crystal substances thereof. The pharmaceutically acceptable salt of the compound of the formula (I) can also be prepared in accordance with a conventional method for a salt formation reaction.
Isolation and purification are carried out by employing general chemical operations such as extraction, fractional crystallization, various types of fraction chromatography, and the like.
Various isomers can be separated by selecting an appropriate starting compound or by making use of the difference in the physicochemical properties between isomers. For example, the optical isomer can be derived into a stereochemically pure isomer by means of general optical resolution methods (for example, fractional crystallization for inducing diastereomer salts with optically active bases or acids, chromatography using a chiral column and the like, and others). In addition, the isomers can also be prepared from an appropriate optically active starting compound.
The pharmacological activity of the compound of the formula (I) was confirmed by the following test.

Test Method 1: Measurement of human PKCθ enzyme inhibition activity

The test was carried out using a HTRF$^R$ KinEASE™ S1 kit (CIS bio). To a 384-well plate (CORNING) were put 4 μL of a liquid agent and 3 μL of a mixed liquid of STK Substrate 1-biotin (final 250 nM), and Full-length human PKCθ (Carna Biosciences, final 31 ng/mL), followed by leaving it to stand at room temperature for 30 minutes. Then, 3 μL of an ATP liquid (final 30 μM) was dispensed therein to carry out an enzyme reaction at room temperature for 1 hour. Thereafter, the reaction was stopped by addition of 10 μL of a solution of Sa-XL665 (final 31.25 nM) and an antibody STK-Antibody-Cryptate (finally 800-fold diluted), and the mixture was left to stand at room temperature for 1 hour Fluorescence intensities at 620 nm (Cryptate) and 665 nm (XL665) were measured in Discovery (PACKARD), and with reference to a Vehicle at 0% inhibition and a Blank of 100% inhibition, the inhibition rates and $IC_{50}$ values were calculated.

The test results are shown in Table 1. Ex represents Compound No. of Examples as described later.

[Table 1]

| Ex | $IC_{50}$(nM) | Ex | $IC_{50}$(nM) | Ex | $IC_{50}$(nM) |
|---|---|---|---|---|---|
| 11 | 1.0 | 169 | 0.36 | 302 | 14 |
| 28 | 1.3 | 170 | 110 | 303 | 0.065 |
| 41 | 14 | 178 | 7.6 | 318 | 0.86 |
| 43 | 0.44 | 183 | 29 | 319 | 4.0 |
| 48 | 54 | 187 | 3.8 | 321 | 0.25 |
| 57 | 15 | 189 | 1.7 | 340 | 1.4 |
| 60 | 1.1 | 194 | 6.5 | 341 | 1.7 |
| 70 | 1.9 | 195 | 3.3 | 342 | 0.48 |
| 74 | 0.65 | 196 | 3.9 | 344 | 0.48 |
| 76 | 2.3 | 200 | 0.28 | 348 | 0.41 |
| 107 | 5.4 | 204 | 11 | 357 | 1.2 |
| 115 | 3.5 | 205 | 0.83 | 363 | 0.58 |
| 125 | 89 | 208 | 1.6 | 371 | 0.70 |
| 126 | 100 | 211 | 0.96 | 372 | 1.22 |
| 139 | 13 | 219 | 0.4 | 383 | 1.6 |
| 140 | 2.5 | 240 | 0.064 | 387 | 77 |
| 145 | 1.6 | 246 | 0.31 | 388 | 3.3 |
| 150 | 5.3 | 264 | 1.9 | 390 | 2.1 |
| 156 | 67 | 284 | 0.28 | 401 | 0.65 |
| 165 | 2.3 | 292 | 10 | | |

Test Method 2: Measurement of Human IL-2 Production Inhibition Activity

i) Preparation of Plasmid

The DNA fragments (445 bp) in the Human IL-2 promoter region corresponding to the DNA sequence as described in the database were cloned and inserted into pGL3 basic which is a Vector for Reporter Gene Assay to acquire pGL3-IL2-pro-43.

ii) Maintenance/Passage of Jurkat Cells

Jurkat, Clone E6-1 (ATCC No. TIB-152), which is a human T cell-based culture cell was cultured under the conditions of 37˚C, 5% $CO_2$, and saturated humidity, using 10% FBS RPMI 1640 (Sigma) as a medium, and at a time point of a confluency of about 90%, passage was carried out.

iii) Transfection and Seeding

A cell suspension of a concentration of $2.5 \times 10^7$ cells/mL was preapred using 10% FBS RPMI 1640 (Sigma) by counting the cells using a cell counting plate, and 10 $\mu$g of pGL3-IL2-pro-43 was mixed therewith. Then, 400 $\mu$L of the Jurkat cells prepared at $2.5\times10^7$ cells/mL were added to each of the prepared plasmid mixture and mixed, followed by adding it entirely to Gene Pulsor$^R$ Cuvette (BIO-RAD). By Gene Pulsor$^R$II (BIO-RAD), a plasmid was introduced at 300 V and 975 $\mu$F, and the whole amount of the Jurkat cells having the plasmid introduction completed were gently suspended in 2.5 mL of 10% FBS RPMI 1640. Then, the cells were seeded to a 96-well plate (Coming Coster) at 50 $\mu$L/well, and cultured for about 10 hours under the condition of 37˚C, 5% $CO_2$, and saturated humidity.

iv) Measurement of Human IL-2 Production Inhibition Activity

A drug solution was added respectively at 25 μL/well, and additionally, a mixed liquid obtained by 250-fold dilution of an anti-CD3 antibody, an anti-CD28 antibody (Pharmingen) (all 1000-fold liquid of the final concentration of 1 μg/mL) with 10% FBS RPMI1640 was added respectively thereto at 25 μL/well. The resultant was cultured for about 14 hours under the condition of 37˚C, 5% $CO_2$, and saturated humidity. The assay was performed in duplicate.
A substrate solution supplied by a Bright-Glo™ Luciferase Assay System (Promega) was added respectively at 100 μL/well and mixed gently. A Multilabel Counter (ARVO SX, WALLAC) was set at a reaction temperature: 25˚C, Shaking Duration: 1 sec, and Measurement time: 1 sec, the measurement well of each of the 96-well plates was set up, and a Firefly luciferase activity was measured.

Test Method 3: Measurement of Cytochrome P450 (CYP3A4) Enzyme Inhibition Activity

i) Inhibition Test I (Calculation of Remaining Rate I)

Using a 96-well plate, 2 μM of a substrate (midazolam), 5 μM of a test compound, and human liver microsome (0.1 mg protein/mL) were incubated at 37˚C for 20 minutes in a total amount of 150 μL of a 100 mM phosphate buffer (pH 7.4) containing 0.1 mM EDTA and 1 mM NADPH. Then, the reaction was stopped by adding 130 μL of an aqueous solution containing 80% acetonitrile. Thereafter, the samples were analyzed by LC/MS/MS, and the remaining rates I were calculated using the following equation 1.

(Equation 1)

$$\text{Remaining Rate I } (\%) = \text{Ai, I/ Ao, I} \times 100$$

Ai, I=Amount of produced metabolite after reaction in the presence of the test compound in the inhibition test I
Ao, I= Amount of produced metabolite after reaction in the absence of the test compound in the inhibition test I

ii) Inhibition Test II (Calculation of Remaining Rate II)

Using a 96-well plate, 5 μM of a test compound and human liver microsome (0.1 mg protein/mL) were incubated at 37˚C for 30 minutes in a total amount of 145 μL of a 100 mM phosphate buffer (pH=7.4) containing 0.1 mM EDTA and 1 mM NADPH. Then, 2 μM of midazolam as the substrate was added thereto at a total amount of 150 μL of and incubated at 37˚C for 20 minutes. After the incubation, the reaction was stopped by adding 130 μL of an aqueous solution containing 80% acetonitrile. Thereafter, the samples were analyzed by LC/MS/MS, and the remaining rate II was calculated using the following equation 2.

(Equation 2)

$$\text{Remaining Rate II } (\%) = \text{Ai, II/Ao, II /(Ai, I/Ao, I)} \times 100$$

Ai, II=Amount of produced metabolite after reaction in the presence of the test compound in the inhibition test II
Ao, I= Amount of produced metabolite after reaction in the absence of the test compound in the inhibition test II

The test results are shown in Table 2. Ex represents No. of the Example Compounds as described below.

[Table 2]

| Ex | I(%) | II(%) | Ex | I(%) | II(%) |
|---|---|---|---|---|---|
| 41 | 80 | 102 | 251 | 75 | 85 |
| 43 | 76 | 99 | 252 | 85 | 80 |
| 62 | 87 | 81 | 258 | 88 | 90 |
| 68 | 82 | 90 | 263 | 91 | 87 |
| 95 | 84 | 81 | 266 | 83 | 99 |

(continued)

| Ex | I(%) | II(%) | Ex | I(%) | II(%) |
|---|---|---|---|---|---|
| 107 | 77 | 84 | 281 | 89 | 83 |
| 115 | 75 | 90 | 282 | 81 | 91 |
| 119 | 77 | 80 | 290 | 85 | 88 |
| 169 | 83 | 89 | 303 | 75 | 88 |
| 195 | 88 | 82 | 314 | 85 | 84 |
| 196 | 86 | 87 | 315 | 85 | 86 |
| 200 | 95 | 85 | 316 | 85 | 84 |
| 209 | 79 | 92 | 321 | 90 | 92 |
| 210 | 77 | 94 | 329 | 92 | 95 |
| 212 | 81 | 85 | 339 | 78 | 94 |
| 216 | 92 | 90 | 340 | 91 | 83 |
| 219 | 76 | 94 | 343 | 78 | 91 |
| 220 | 82 | 85 | 344 | 89 | 84 |
| 228 | 79 | 91 | 345 | 94 | 81 |
| 229 | 80 | 95 | 352 | 94 | 81 |
| 232 | 88 | 94 | 370 | 81 | 85 |
| 238 | 75 | 81 | 372 | 78 | 84 |
| 248 | 88 | 82 | 401 | 84 | 89 |
| 249 | 82 | 83 | | | |

As a result of each of the above tests, the compound of the formula (I) has a PKCθ inhibition action and reduction in CYP inhibition, from which it is apparent that the compound is useful for an inhibitor of acute rejection occurring in transplantation, or the like.

A pharmaceutical composition containing one or two or more kinds of the compound of formula (I) or a pharmaceutically acceptable salt thereof as an active ingredient can be prepared in accordance with a generally used method, using a pharmaceutical excipient, a pharmaceutical carrier, or the like, that is generally used in the art.

Administration may be carried out through any mode of oral administration via tablets, pills, capsules, granules, powders, liquid preparations, or the like, or parenteral administration via injections such as intraarticular, intravenous, intramuscular, and the like, suppositories, eye drops, eye ointments, transdermal liquid preparations, ointments, transdermal patches, transmucosal liquid preparations, transmucosal patches, inhalations, and the like.

Regarding solid composition for oral administration, tablets, powders, granules, or the like are used. In such a solid composition, one or more active ingredients are mixed with at least one inactive excipient, for example, lactose, mannitol, glucose, hydroxypropylcellulose, microcrystalline cellulose, starch, polyvinyl pyrrolidone, aluminum magnesium metasilicate, or the like. According to a conventional method, the composition may contain inactive additives, for example, a lubricant such as magnesium stearate and the like, a disintegrator such as sodium carboxymethylstarch and the like, a stabilizer, and a solubilizing agent. As occasion demands, tablets or pills may be coated with a sugar coating, or a gastric or enteric coating agent.

The liquid composition for oral administration includes pharmaceutically acceptable emulsions, solutions, suspensions, syrups, elixirs, or the like, and contains a generally used inert diluent, such as purified water or ethanol. In addition to the inert diluent, the liquid composition may contain adjuvants such as a solubilizing agent, a moisturizing agent, and a suspending agent, a sweetener, a flavor, an aromatic, and an antiseptic.

Injections for parenteral administration include sterile, aqueous or non-aqueous solutions, suspensions, or emulsions. As the aqueous solvent, for example, distilled water for injection or physiological saline is included. Examples of the non-aqueous solvent include propylene glycol, polyethylene glycol, vegetable oils such as olive oil and the like, alcohols such as ethanol and the like, polysorbate 80 (Pharmacopeia), etc. Such a composition may further contain a tonicity agent, an antiseptic, a moistening agent, an emulsifying agent, a dispersing agent, a stabilizer, or a solubilizing

agent. These are sterilized, for example, by filtration through a bacteria-retaining filter, blending with bactericides, or irradiation. In addition, these can also be used by producing a sterile solid composition, and dissolving or suspending it in sterile water or a sterile solvent for injection prior to its use.

Examples of the agent for external use include ointments, plasters, creams, jellies, patches, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.

As the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, and the like, or other forms.

Generally, in the case of oral administration, the daily dose is from about 0.0001 to 100 mg/kg per body weight, administered in one portion or in 2 to 4 divided portions. In the case of intravenous administration, the daily dose is suitably administered from about 0.0001 to 10 mg/kg per body weight, once a day or two or more times a day. In addition, in the case of inhalation, the agent is administered at a dose from about 0.0001 1 to 1 mg/kg per body weight, once a day or two or more times a day. The dose is appropriately decided in response to the individual case by taking the symptoms, the age, the gender, and the like into consideration.

The compound of the formula (I) can be used in combination with various agents for treating or preventing the diseases for which the compound of the formula (I) of the present invention is considered to be effective. The combined preparations may be administered simultaneously, or separately and continuously, or at a desired time interval. The preparations to be co-administered may be a blend or may be prepared individually.

## Examples

Hereinbelow, the preparation methods for the compound of the formula (I) are described in more detail with reference to the Examples. Further, the present invention is not intended to be limited to the compounds described in Examples below. In addition, the production processes for the starting compounds are shown in Preparation Examples. Further, the preparation methods for the compound of the formula (I) are not limited to the specific preparation methods in Examples presented below, but the compound of the formula (I) can be prepared by combinations of the preparation methods, or methods apparent to a skilled person in the art.

Moreover, the following abbreviations are used in some cases in Examples, Preparation Examples, and Tables to be described later.

PEx: Preparation Example No., Ex: Example No., Str: structural formula (a description of, for example, HCl, in the structural formula indicates that the compound is a hydrochloride, and a description of 2HCl indicates that the compound is dihydrochloride), rel: relative configuration (a description of rel under the PEx or Ex No. indicates that steric denotements in the adamantane skeletal portion in the structural formula described in the section of the Str represent relative configuration), Syn: Preparation Method (the numeral alone shows Example No. having the same preparation manner, and when P is prefixed before the number, the numeral shows Preparation Example No. having the same preparation manner), Dat: physicochemical data, NMR1: δ (ppm) 1H-NMR in DMSO-$d_6$, NMR2: δ (ppm) in 1H-NMR in $CDCl_3$, NMR3: δ (ppm) 1H-NMR in $D_2O$, FAB+:FAB-MS (positive ion), ESI+: ESI-MS (positive ion), ESI-: ESI-MS (negative ion), TEA: triethylamine, TFA: trifluoroacetic acid, THF: tetrahydrofuran, DMF: N,N-dimethylformamide, DME: dimethoxyethane, DMI: 1,3-dimethyl-2-imidazolidinone, MeOH: methanol, EtOH: ethanol, EtOAc: ethyl acetate, MeCN: acetonitrile, HOBt: 1-hydroxybenzotriazole, WSC: 3-ethyl-1-(3-dimethylaminopropyl)carbodiimide, DEAD: diethylazodicarboxylate, DIPEA: diisopropylethylamine, MCPBA: m-chloroperbenzoic acid, LAH: lithium aluminum hydride, Pd/C: palladium on carbon, TLC1: TLC analysis (condition: eluting solvent; MeOH/chloroform=1/9, silica gel plate (silica gel 60 F254, Merck)), TLC2: TLC analysis (condition: eluting solvent; hexane/EtOAc=1/1, amino silica gel plate (TLC plate (NH), FUJI SILYSIA)), TLC3: TLC analysis (condition: eluting solvent; EtOAc, amino silica gel plate (TLC plate (NH), FUJI SILYSIA)), HPLC: HPLC analysis, rt: retention time.

Further, there are descriptions of the retention time (HPLC:rt) in HPLC in the physicochemical data, in which the HPLC analysis conditions are as follows.

(Analysis Conditions)

Column: YMC-Pack ODS-AM (S-5 μm, 12 nm) (150x4.6 mm I.D.), Column temperature: 40˚C, Detection method: UV (254 nm), Flow rate: 1 mL/min, Eluent A: acetonitrile, Eluent B: pH 3 buffer (phosphoric acid being added to a 0.05 M aqueous $NaH_2PO_4$ solution to adjust to pH 3)

| Time program: | | | |
|---|---|---|---|
| Time (min) | 0 | 20 | 30 |
| Eluent A (%) | 10 | 60 | 60 |
| Eluent B (%) | 90 | 40 | 40 |

Preparation Example 1

To a solution of rel-[(1R,3S,5S)-5-({[(benzyloxy)carbonyl]amino}methyl)adamantan-2-yl]acetic acid (250 mg) in toluene (3 ml) were sequentially added TEA (127 μl) and diphenylphosphoryl azide (196 μl), followed by stirring at 80˚C for 1 hour. After leaving to be cooled to room temperature, to the mixed reaction liquid were sequentially added copper (I) iodide (69 mg) and tert-butanol (3 ml), followed by stirring at 80˚C for 1 hour. The mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by silica gel flash column chromatography (hexane-EtOAc) to obtain 113 mg of benzyl tert-butyl [(1S,3R,5S)-tricyclo[3.3.1.1$^{3,7}$]decane-1,4-diyl bis(methylene)]bis rel-carbamate.

Preparation Example 2

Under ice-cooling, to a suspension of 60% sodium hydride (oil dispersion, 25.5 mg) in THF (1 ml) was added dropwise triethyl phosphonoacetate (0.128 ml), followed by stirring for 10 minutes. To the mixed reaction liquid was added portionwise benzyl rel-{[(1S,3R,5S)-4-oxoadamantan-1-yl]methyl}carbamate (100 mg) at the same temperature, and the mixed reaction liquid was stirred at room temperature for 1 hour. To the mixed reaction liquid were added EtOAc and water, and the organic layer was collected by separation. The organic layer was sequentially washed with water and saturated brine, and dried over anhydrous sodium sulfate. After the desiccant was removed, the solvent was evaporated under reduced pressure. The obtained residue was purified by silica gel flash column chromatography (hexane-EtOAc) to obtain 120 mg of ethyl rel-(2E)-[(1R,3S,5R)-5-({[(benzyloxy)carbonyl]amino}methyl)tricyclo [3.3.1.1$^{3,7}$]dec-2-ylidene]acetate.

Preparation Example 3

To a solution of tert-butyl rel-{(1R,2S,3S,5S)-5-[({2-[(3-bromobenzyl)amino]-5-cyanopyrimidine-4-yl}amino) methyl]adamantan-2-yl}carbamate (136 mg) in DME (2.7 ml) were added (3-aminomethylphenyl)boronic acid hydrochloride (89.8 mg), tetrakis(triphenylphosphine)palladium (0) (41.5 mg), sodium carbonate (101.6 mg), and water (0.34 ml), followed by stirring at 140˚C for 6 hours under a nitrogen air flow. To the mixed reaction liquid were added (3-aminomethylphenyl)boronic acid hydrochloride (89.8 mg) and a 2 M aqueous sodium carbonate solution (0.479 ml), followed by stirring at 140˚C for additional 4 hours. The mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by amino silica gel flash column chromatography (chloroform-MeOH) to obtain 126.6 mg of tert-butyl rel-[(1R,2S,3S,5S)-5-({[2-({[3'-(aminomethyl)biphenyl-3-yl]methyl}amino)-5-cyanopyrimidin-4-yl] amino}methyl)adamantan-2-yl]carbamate.

Preparation Example 4

To a solution of tert-butyl N-(5-cyano-2-{[2-(trifluoromethoxy)benzyl]amino}pyrimidin-4-yl)glycinate (616 mg) in dichloromethane (6.16 ml) was added trifluoroacetic acid (3.4 ml), followed by stirring at room temperature. After completion of the reaction, to the mixed reaction liquid was added diisopropyl ether. The precipitated solid was collected by filtration, washed with diisopropyl ether, and dried under reduced pressure to obtain 484 mg of N-(5-cyano-2-{ [2-(trifluoromethoxy)benzyl]amino}pyrimidin-4-yl)glycine trifluoroacetate.

Preparation Example 6

To a solution ofmethyl 1H-benzimidazole-5-carboxylate (8.5 g) in THF (85 ml) were added 3,4-dihydro-2H-pyran (5.3 ml) and (1S)-(+)-10-camphorsulfonic acid (1.1 g), followed by heating and refluxing for 24 hours. To the mixed reaction liquid were added 3,4-dihydro-2H-pyran (4.4 ml) and (1S)-(+)-10-camphorsulfonic acid (10.1 g), followed by heating and refluxing for additional 12 hours. The mixed reaction liquid was poured into a mixed liquid of EtOAc and water, and the organic layer was collected by separation. The organic layer was sequentially washed with water and saturated brine, and dried over anhydrous sodium sulfate. After the desiccant was removed, the solvent was evaporated under reduced pressure. The obtained residue was purified by silica gel flash column chromatography (hexane-EtOAc) to obtain a mixture (7.46 g) of methyl 1-(tetrahydro-2H-pyran-2-yl)-1H-benzimidazole-5-carboxylate and methyl 1-(tetrahydro-2H-pyran-2-y1)-1H-benzimidazole-6-carboxylate.

Preparation Example 7

Under ice-cooling, to a solution of tert-butyl rel-[(1R,2S,3S,5S)-5-(aminomethyl)adamantan-2-yl]carbamate (200 mg) and TEA (0.12 ml) in dichloromethane (4 ml) was added benzyl chloroformate (0.11 ml), followed by stirring at room temperature for 4 hours. The reaction liquid was diluted with EtOAc, sequentially washed with 0.1 M hydrochloric acid, water, saturated aqueous sodium bicarbonate, water, and saturated brine, and dried over anhydrous sodium sulfate. After the desiccant was removed, the solvent was evaporated under reduced pressure, and the obtained residue was purified by silica gel column chromatography (hexane-EtOAc) to obtain 295.0 mg of benzyl rel-({(1S,3R,4S,5S)-4-[(tert-butoxycarbonyl)amino]adamantan-1-yl}methyl)carbamate.

Preparation Example 9

Under ice-cooling, to a suspension of rel-1-[(1'R,3'S,5'S)-5'H-spiro[1,3-dioxolane-2,2'-tricyclo[3.3.1.1$^{3,7}$]decan]-5-yl]methanamine (2.15 g) in THF (21.5 ml) were added dropwise benzyl chloroformate (1.92 ml) and a 1M aqueous sodium hydroxide solution (13.5 ml). The mixed reaction liquid was warmed to room temperature, followed by stirring at room temperature for 3 hours. The mixed reaction liquid was diluted with EtOAc and then adjusted to pH 3 with an aqueous sodium hydrogen sulfate solution, and the organic layer was collected by separation. The organic layer was sequentially washed with water and saturated brine, and dried over anhydrous sodium sulfate. After the desiccant was removed, the solvent was evaporated under reduced pressure to obtain 2.66 g of benzyl rel-[(1'R,3'S,5'S)-5'H-spiro[1,3-dioxolane-2,2'-tricyclo[3.3.1.1$^{3,7}$]decan]-5'-ylmethyl]carbamate.

Preparation Example 10

A suspension of {trans-3-[(tert-butoxycarbonyl)amino]cyclobutyl}methyl methanesulfonate (80.7 mg) and sodium azide (93.9 mg) in DMF (0.81 ml) and water (0.081 ml) was stirred at 120˚C for 40 minutes. The reaction liquid was cooled, then diluted with EtOAc, washed with water and saturated brine in this order, and dried over anhydrous sodium sulfate. After the desiccant was removed, the solvent was evaporated under reduced pressure, and the obtained residue was purified by silica gel column chromatography (hexane-EtOAc) to obtain 63.1 mg of tert-butyl [trans-3-(azidomethyl)cyclobutyl]carbamate.

Preparation Example 11

To a solution of tert-butyl [trans-3-(azidomethyl)cyclobutyl]carbamate (270 mg) in MeOH (13.5 ml) was added 10% Pd/C (wetted with 50% water, 81 mg), followed by stirring at room temperature for 40 minutes at a normal pressure under a hydrogen atmosphere. The catalyst was separated by filtration through Celite and washed with MeOH, and then the filtrate was concentrated under reduced pressure. The obtained residue was purified by silica gel column chromatography (chloroform-MeOH-concentrated aqueous ammonia) to obtain 120.5 mg of tert-butyl [trans-3-(aminomethyl)cyclobutyl]carbamate.

Preparation Example 12

To a solution of N-(5-cyano-2-{[2-(trifluoromethoxy)benzyl]amino}pyrimidin-4-yl)glycine trifluoroacetate (30 mg) in DMF (0.9 ml) were sequentially added tert-butyl (2-aminoethyl)carbamate (25.0 mg), HOBt (9.3 mg), and WSC (24.2 mg), followed by stirring at room temperature. After completion of the reaction, the mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue

was purified by preparative silica gel thin layer chromatography (chloroform-MeOH) to obtain 10 mg of tert-butyl (2-{[N-(5-cyano-2-{[2-(trifluoromethoxy)benzyl]amino}pyrimidin-4-yl)glycyl]amino}ethyl)carbamate.

Preparation Example 19

Under ice-cooling, to a solution of benzyl rel-[(1R,2S,3S,5S)-5-(aminomethyl)adamantan-2-yl]carbamate and pyridine (1.4 ml) in dichloromethane (50 ml) were added a solution of trifluoroacetic anhydride (2.5 ml) in dichloromethane (20 ml), followed by stirring at the same temperature for 30 minutes. Pyridine (0.128 ml) and trifluoroacetic anhydride (0.225 ml) were further added thereto, followed by stirring for 30 minutes under ice-cooling. Under ice-cooling, to the mixed reaction liquid was added water, followed by stirring and then dilution with EtOAc, and the organic layer was sequentially washed with water and saturated brine, and dried over anhydrous sodium sulfate. After the desiccant was removed, the solvent was evaporated under reduced pressure to obtain 7.0 g of benzyl rel-[(1R,2S,3S,5S)-5-{[(trifluoroacetyl)amino]methyl}adamantan-2-yl]carbamate.

Preparation Examples 20 and 21

To a solution of rel-(1R,3S,5R,7S)-4-{[(benzyloxy)carbonyl]amino}adamantane-1-carboxylic acid (12 g) in dichloromethane (120 ml) were added oxalyl chloride (4.8 ml), followed by stirring at room temperature. After completion of the reaction, the reaction mixture was concentrated under reduced pressure, and toluene was added thereto, followed by additional concentration under reduced pressure. The obtained residue was dissolved in 1,4-dioxane (12 ml), and added dropwise to 28% aqueous ammonia (110 g) under ice-cooling. The mixed reaction liquid was extracted with EtOAc, and the organic layer was washed with water three times and with saturated brine, and dried over anhydrous sodium sulfate. After the desiccant was removed, the solvent was evaporated under reduced pressure. To the obtained residue was added MeOH to precipitate the solid, which was collected by filtration. The filtrate was concentrated under reduced pressure, and MeOH was used again to precipitate the solid, which was collected by filtration. The filtrate was concentrated under reduced pressure, and MeOH was used several times to precipitate the solid, which was collected by filtration. The obtained solid was dried under reduced pressure to obtain benzyl rel-[(1R,2R,3S,5S)-5-carbamoyladamantan-2yl]carbamate (2.9 g). The filtrate was concentrated under reduced pressure and the obtained residue was purified by silica gel flash column chromatography (hexane-EtOAc) to obtain 1.9 g of benzyl rel-[(1R,2S,3S,5S)-5-carbamoyladamantan-2-yl]carbamate.

Preparation Example 23

To a mixed solution of tert-butyl rel-[(1R,2S,3S,5S)-5-{[(trifluoroacetyl)amino]methyl}adamantan-2-yl]carbamate (4.6 g) in MeOH (46 ml) and water (23 ml) was added potassium carbonate (16.9 g), followed by stirring at room temperature. After completion of the reaction, the mixed reaction liquid was diluted with EtOAc, washed with saturated brine, and dried over anhydrous sodium sulfate. After the desiccant was removed, the solvent was evaporated under reduced pressure to obtain 3.78 g oftert-butyl rel-[(1R,2S,3S,5S)-5-(aminomethyl)adamantan-2-yl]carbamate.

Preparation Example 24

Under ice-cooling, to a solution of benzyl rel-[(1R,2S,3S,5S)-5-carbamoyladamantan-2-yl]carbamate (500 mg) in THF (5.0 ml) was added dropwise a 1.17 M solution of a borane-tetrahydrofuran complex in THF (3.9 ml) under a nitrogen air flow, followed by heating and refluxing for 3 hours. The mixed reaction liquid was ice-cooled, and then water was carefully added dropwise thereinto. Then, the liquid was poured into an aqueous dichloromethane-potassium carbonate solution under stirring. The organic layer was collected by separation, and further extracted with dichloromethane twice. The obtained organic layer was combined and dried over anhydrous sodium sulfate. After the desiccant was removed, the solvent was evaporated under reduced pressure to obtain 530 mg of benzyl rel-[(1R,2S,3S,5S)-5-(aminomethyl)adamantan-2-yl]carbamate.

Preparation Example 27

Under ice-cooling, to a solution of di-tert-butyl iminodicarboxylate (1.88 g) in DMF (28 ml) was added potassium tert-butoxide (970 mg) in small portions, followed by stirring at room temperature for 1 hour. To the reaction mixture was added dropwise a solution of 3-(bromomethyl)-4-chlorophenyl acetate (1.90 g) in DMF (10 ml) under ice-cooling, followed by stirring at room temperature for 2 hours. To the reaction mixture was added water, followed by extraction with EtOAc. The organic layer was sequentially washed with water and saturated brine, and dried over anhydrous magnesium sulfate. After the desiccant was removed, the solvent was evaporated under reduced pressure. The obtained residue was purified

by silica gel column chromatography (hexane-EtOAc) to obtain 2.79 g of 3-{[bis(tert-butoxycarbonyl)amino]methyl}-4-chlorophenyl acetate.

Preparation Example 28

Under an argon atmosphere, to a solution of [2-(benzyloxy)phenyl]methanol (20.2 g) in chloroform (160 ml) was slowly added a solution of thionyl chloride (13.8 ml) in chloroform (40 ml) at room temperature. After stirring at room temperature for 90 minutes, volatile substances were evaporated under reduced pressure to obtain 1-(benzyloxy)-2-(chloromethyl)benzene. Then, under an argon atmosphere, to a solution of di-tert-butyl iminodicarboxylate (41.0 g) in DMF (500 ml) was added potassium tert-butoxide (21.2 g) at room temperature. After stirring at the same temperature for 70 minutes, a solution of 1-(benzyloxy)-2-(chloromethyl)benzene in DMF (60 ml) was added thereto. After stirring at the same temperature for 15 hours, water was added thereto, followed by stirring for additional 90 minutes. The precipitate was collected by filtration, washed with water, and then dried under reduced pressure. The obtained crude product was purified by silica gel column chromatography (chloroform) to obtain 34.5 g of di-tert-butyl [2-(benzyloxy)benzyl]imidodicarbonate.

Preparation Example 29

To a solution of rel-4-({[(1S,3R,4S,5S)-4-aminoadamantan-1-yl]methyl}amino)-2-{[2-(trifluoromethoxy)benzyl]amino}pyrimidine-5-carbonitrile (30 mg) in DMF (0.6 ml) were added DIPEA (22 μl) and ethylbromo acetate (5.8 μl), followed by stirring at 60°C. After completion of the reaction, the mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain ethyl rel-N-[(1R,2S,3S,5S)-5-{[(5-cyano-2-{[2-(trifluoromethoxy)benzyl]amino}pyrimidin-4-yl)amino]methyl}adamantan-2-yl]glycinate(26.1 mg).

Preparation Example 31

To a solution of di-tert-butyl (2-hydroxybenzyl)imidodicarbonate (500 mg) in DMF (5.0 ml) were added 2-bromoacetamide (320 mg), potassium carbonate (641 mg), and potassium iodide (385 mg), followed by stirring at 80°C for 3 hours. After leaving to be cooled to room temperature, water was added thereto, and the precipitated product was collected by filtration to obtain 546 mg of di-tert-butyl [2-(2-amino-2-oxoethoxy)benzyl]imidodicarbonate.

Preparation Example 36

To a mixed solution of tert-butyl (5-formyl-2-nnethoxybenzyl)carbamate (1.0 g) in THF (3.0 ml) and EtOH (6.0 ml) was added sodium borohydride (192.5 mg), followed by stirring at room temperature. After completion of the reaction, to the mixed reaction liquid was added water, followed by extraction with EtOAc, and the organic layer was dried over anhydrous sodium sulfate. After the desiccant was removed, the solvent was evaporated under reduced pressure. The obtained residue was purified by silica gel flash column chromatography (hexane-EtOAc) to obtain 1.09 g oftert-butyl [5-(hydroxymethyl)-2-methoxybenzyl]carbamate.

Preparation Example 37

To a solution of 4-chloro-2-(methylsulfanyl)pyrimidine-5-carbonitrile (2.2 g) in 1,3-dimethylimidazolidin-2-one were added DIPEA (4.13 ml) and tert-butyl rel-[(1R,2S,3S,5S)-5-(aminomethyl)adamantan-2-yl]carbamate (3.99 g), followed by stirring at room temperature. After completion of the reaction, the mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by silica gel flash column chromatography (hexane-EtOAc) to obtain 4.76 g of tert-butyl rel-[(1R,2S,3S,5S)-5-({[5-cyano-2-(methylsulfanyl)pyrimidin-4-yl]amino}methyl)adamantan-2-yl]carbamate.

Preparation Example 54

Under ice-cooling, to a solution of 2,4-dichloropyrimidine-5-carbonitrile (1.00 g) in DMF (15 ml) were added dropwise a solution of 2-(methylthio)benzylamine (881 mg) in DMF (5 ml) and DIPEA (1.2 ml), followed by stirring at the same temperature for 1 hour. A solution of 2-(methylthio)benzylamine (44 mg) in DMF (2 ml) was added thereto, followed by stirring at room temperature for additional 1 hour. To the reaction mixture were added EtOAc and water,

followed by liquid separation. The organic layer was sequentially washed with water and saturated brine, and dried over anhydrous magnesium sulfate. After the desiccant was removed, the solvent was evaporated under reduced pressure, and the obtained residue was purified by silica gel column chromatography (chloroform) to obtain 709 mg of 4-chloro-2-{[2-(methylsulfanyl)benzyl]amino}pyrimidine-5-carbonitrile.

Preparation Examples 100 and 101

To a solution of 2,4-dichloro-5-(trifluoromethyl)pyrimidine (300 mg) in DMF (6.0 ml) which had been cooled in an ice-brine bath were added DIPEA (252.9 μl) and 1-[2-(trifluoromethoxy)phenyl]methanamine (277.5 mg), followed by stirring at -20˚C. After completion of the reaction, the mixed reaction liquid was diluted with EtOAc, and the organic layer was sequentially washed with water and saturated brine, and dried over anhydrous sodium sulfate. After the desiccant was removed, the solvent was evaporated under reduced pressure to obtain 507 mg of a mixture of 4-chloro-N-[2-(trifluoromethoxy)benzyl]-5-(trifluoromethyl)pyrimidin-2-amine and 2-chloro-N-[2-(trifluoromethoxy)benzyl]-5-(trifluoromethyl)pyrimidin-4-amine.

Preparation Examples 102 and 103

To a solution of a mixture (90 mg) of 4-chloro-N-[2-(trifluoromethoxy)benzyl]-5-(trifluoromethyl)pyrimidin-2-amine and 2-chloro-N-[2-(trifluoromethoxy)benzyl]-5-(trifluoromethyl)pyrimidin-4-amine in DMF (1.0 ml) were added DIPEA (84.3 μl) and benzyl rel-[(1R,2S,3S,5S)-5-(aminomethyl)adamantan-2-yl]carbamate (79.9 mg), followed by stirring at room temperature. After completion of the reaction, the mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by silica gel flash column chromatography (hexane-EtOAc) to obtain a crude product of benzyl rel-[(1R,2S,3S,5S)-5-({[2-{[2-(trifluoromethoxy)benzyl]amino}-5-(trifluoromethyl)pyrimidin-4-yl]amino}methyl)adamantan-2-yl]carbamate and a crude product of benzyl rel-[(1R,2S,3S,5S)-S-({[4-{[2-(trifluoromethoxy)benzyl]amino}-5-(trifluoromethyl)pyrimidin-2-yl]amino}methyl)adamantan-2-yl]carbamate. Each of the crude products was further purified by silica gel flash column chromatography (chloroform-MeOH) to obtain benzyl rel-[(1R,2S,3S,5S)-5-({[2-{[2-(trifluoromethoxy)benzyl]amino}-5-(trifluoromethyl)pyrimidin-4-yl]amino}methyl)adamantan-2-yl]carbamate (100 mg) and benzyl rel-[(1R,2S,3S,5S)-5-({[4-{[2-(trifluoromethoxy)benzyl]amino}-5-(trifluoromethyl)pyrimidin-2-yl]amino}methyl)adamantan-2-yl]carbamate (50 mg).

Preparation Example 104

To a solution of benzyl rel-[(1R,2S,3S,5S)-5-({[5-cyano-2-(methylsulfinyl)pyrimidin-4-yl]amino}methyl)adamantan-2-yl]carbamate (50 mg) in 1,3-dimethylimidazolidin-2-one (1.0 ml) were added 3-bromoaniline (0.114 ml) and a 4 M hydrogen chloride dioxane solution (2.6 μl), followed by stirring at 100˚C for 3 hours. After leaving to be cooled to room temperature, to the mixed reaction liquid was added water, and the precipitated solid was collected by filtration, washed with water and hexane, and then dried under reduced pressure to obtain 46 mg of benzyl rel-{(1R,2S,3S,5S)-5-[({2-[(3-bromophenyl)amino]-5-cyanopyrimidine-4-yl}amino)methyl]adamantan-2-yl}carbamate.

Preparation Example 105

To a solution of ethyl rel-[(1R,3S,5S)-5-({[(benzyloxy)carbonyl]amino}methyl)adamantan-2-yl]acetate(300 mg) in MeOH (6.0 ml) was added a 4 M aqueous lithium hydroxide solution (1.2 ml), followed by stirring at 60˚C for 3 hours. The mixed reaction liquid was diluted with EtOAc and then an aqueous potassium hydrogen sulfate solution was added to adjust to pH 2, and the organic layer was collected by separation. The organic layer was sequentially washed with water and saturated brine, and dried over anhydrous sodium sulfate. After the desiccant was removed, the solvent was evaporated under reduced pressure to obtain 264.6 mg of rel-[(1R,3S,5S)-5-({[(benzyloxy)carbonyl]amino}methyl)adamantan-2-yl] acetic acid.

Preparation Example 106

To a mixed solution of methyl rel-(1R,3S,5R,7S)-4-{[(benzyloxy)carbonyl]amino}adamantane-1-carboxylate (15 g) in 1,4-dioxane (75 ml) and MeOH (75 ml) were added a 1M aqueous sodium hydroxide solution (87.4 ml), followed by stirring at 60˚C for 4 hours. The mixed reaction liquid was left to be cooled to room temperature, then adjusted to pH 4 with a 10% aqueous potassium hydrogen sulfate solution, and extracted with EtOAc. The obtained organic layer was washed with saturated brine once and dried over anhydrous magnesium sulfate. After the desiccant was removed, the

solvent was evaporated under reduced pressure to obtain 12 g of rel-(1R,3S,5R,7S)-4-{[(benzyloxy)carbonyl]amino} adamantane-1-carboxylic acid.

Preparation Example 107

Under a nitrogen atmosphere, to a suspension of lithium aluminum hydride (1.2 g) in THF (100 ml) was added dropwise a solution of a mixture (7.0 g) of methyl 1-(tetrahydro-2H-pyran-2-yl)-1H-benzimidazole-5-carboxylate and methyl 1-(tetrahydro-2H-pyran-2-yl)-1H-benzimidazole-6-carboxylate in THF (100 ml) at -10˚C or lower, followed by stirring for 1 hour under ice-cooling. Lithium aluminum hydride (0.8 g) was added in divided portions thereto, followed by stirring for additional 30 minutes under ice-cooling. At the same temperature, water (6.0 ml), a 15% aqueous sodium hydroxide solution (6.0 ml), and water (3.0 ml) were sequentially added thereto, followed by stirring at room temperature for 30 minutes. The insoluble materials were removed by filtration through Celite and the filtrate was concentrated under reduced pressure to obtain 5.48 g of a mixture of [1-(tetrahydro-2H-pyran-2-yl)-1H-benzimidazol-5-yl]methanol and [1-(tetrahydro-2H-pyran-2-yl)-1H-benzimidazol-6-yl]methanol.

Preparation Example 108

Under ice-cooling, to a solution of ethyl rel-(2E)-[(1R,3S,5R)-5-({[(benzyloxy)carbonyl]amino}methyl)tricyclo [3.3.1.1$^{3,7}$]dec-2-ylidene]acetate (350 mg) in MeOH (6.0 ml) was added nickel (II) chloride (23.7 mg) under a nitrogen atmosphere, and sodium borohydride was added portionwise thereto, followed by stirring at the same temperature for 1 hour and at room temperature for 3 hours. To the mixed reaction liquid was added water, followed by extraction with EtOAc. The organic layer was sequentially washed with water and saturated brine, and dried over anhydrous sodium sulfate. After the desiccant was removed, the solvent was evaporated under reduced pressure to obtain 310 mg of ethyl rel-[(1R,3S,5S)-5-({[(benzyloxy)carbonyl]amino}methyl)adamantan-2-yl]acetate.

Preparation Example 109

To a solution of methyl rel-(1R,3S,5R,7S)-4-oxoadamantane-1-carboxylate (500 mg) in dichloromethane (7.5 ml) were sequentially added benzyl amine (0.262 ml) and sodium triacetoxyborohydride (763 mg), followed by stirring at room temperature for 2 hours. To the mixed reaction liquid was added saturated aqueous sodium bicarbonate, followed by stirring and then extraction with dichloromethane. The organic layer was collected by separation. The obtained organic layer was washed with saturated brine and dried over anhydrous magnesium sulfate. After the desiccant was removed, the solvent was evaporated under reduced pressure. The obtained residue was purified by silica gel flash column chromatography (chloroform-MeOH) to obtain 757 mg of methyl rel-(1R,3S,5R,7S)-4-(benzylamino)adamantane-1-carboxylate.

Preparation Example 112, 113

To a solution of benzyl rel-{[(1S,3R,4S,5S)-4-aminoadamantan-1-yl]methyl}carbamate (760 mg) in dichloromethane (22.8 ml) were added 4-{[tert-butyl (dimethyl)silyl]oxy}cyclohexanone (1.22 ml) and sodium triacetoxyborohydride (1.02 g), followed by stirring at room temperature for 4 hours. To the mixed reaction liquid was added saturated aqueous sodium bicarbonate, followed by extraction with EtOAc. The organic layer was sequentially washed with water and saturated brine, and dried over anhydrous sodium sulfate. The desiccant was removed, the solvent was evaporated under reduced pressure, and the obtained residue was purified by amino silica gel column chromatography (hexane-EtOAc) to first elute 674.8 mg of benzyl rel-({(1R,3S,4R,5R)-4-[(cis-4-{[tert-butyl (dimethyl)silyl]oxy}cyclohexyl)amino] adamantan-1-yl}methyl)carbamate and then elute 435.8 mg of benzyl rel-({(1R,3S,4R,SR)-4-[(trans-4-{[tert-butyl(dimethyl)silyl]oxy}cyclohexyl)amino]adamantan-1-yl}methyl)carbamate.
The steric configuration of the obtained product was determined by using the compound (benzyl rel-({(1R,3S,4R,5R)-4-[(trans-4-{[tert-butyl (dimethyl)silyl]oxy}cyclohexyl)amino]adamantan-1-yl}methyl)carbamate) eluted later in amino silica gel column chromatography as a starting material to provide the rel-trans-4-{[(1R,2S,3S,5S)-5-(aminomethyl)adamantan-2-yl]amino}cyclohexanol obtained in Preparation Example 134, which is then used for Example 45, and by confirming that the HPLC retention time (15.1 min) of the obtained product coincided with that in Example 42 (trans-alcohol product).

Preparation Example 117

Under ice-cooling, to a solution of tert-butyl rel-[(1R,2S,3S,5S)-5-({[5-cyano-2-(methylsulfanyl)pyrimidin-4-yl] amino}methyl)adamantan-2-yl]carbamate (4.7 g) in dichloromethane (50 ml) was added 75% MCPBA (contains water)

(2.77 g), followed by stirring at the same temperature. After completion of the reaction, the mixed reaction liquid was diluted with EtOAc, and the organic layer was sequentially washed with saturated aqueous sodium bicarbonate, water, and saturated brine, and dried over anhydrous sodium sulfate. After the desiccant was removed, the solvent was evaporated under reduced pressure. The obtained residue was purified by silica gel flash column chromatography (chloroform-MeOH) to obtain 5.02 g of tert-butyl rel-[(1R,2S,3S,5S)-5-({[5-cyano-2-(methylsulfinyl)pyrimidin-4-yl]amino} methyl)adamantan-2-yl]carbamate.

Preparation Examples 120 and 121

Under ice-cooling, to a solution of tert-butyl rel-[(1R,2S,3S,5S)-5-{[(5-cyano-2-{[2-(methylsulfanyl)benzyl]amino}pyrimidin-4-yl)amino]methyl} adamantan-2-yl]carbamate (147 mg) in dichloromethane (5.0 ml) was added 75% MCPBA (contains water, 69.6 mg), followed by stirring at the same temperature for 1 hour. To the mixed reaction liquid was added saturated aqueous sodium bicarbonate, followed by extraction with EtOAc. The organic layer was sequentially washed with water and saturated brine, and dried over anhydrous sodium sulfate. After the desiccant was removed, the solvent was evaporated under reduced pressure. The obtained residue was purified by silica gel flash column chromatography (starting with hexane-EtOAc and changing to chloroform-MeOH in the middle of the process) to obtain tert-butyl rel-[(1R,2S,3S,5S)-5-{[(5-cyano-2-{[2-(methylsulfinyl)benzyl]amino}pyrimidin-4-yl)amino]methyl}adamantan-2-yl] carbamate (123.9 mg) and tert-butyl rel-[(1R,2S,3S,5S)-5-{[(5-cyano-2-{[2-(methylsulfonyl)benzyl]amino}pyrimidin-4-yl) amino]methyl}adamantan-2-yl]carbamate (28.1mg).

Preparation Example 122

Under ice-cooling, to a suspension of LAH (88 mg) in THF (20 ml) was added tert-butyl 3-cyano-8-azabicyclo [3.2.1] octane-8-carboxylate (550 mg), followed by stirring at room temperature for 4 hours. Under ice-cooling, water was added thereto, followed by extraction with EtOAc, and the organic layer was dried over anhydrous sodium sulfate. After the desiccant was removed, the solvent was evaporated under reduced pressure to obtain 600 mg of tert-butyl 3-(aminomethyl)-8-azabicyclo[3.2.1]octane-8-carboxylate.

Preparation Example 123

To a solution of di-tert-butyl {2-[2-(methoxymethoxy)ethoxy]benzyl}imidodicarbonate (288 mg) in methanol (1.4 ml) was added a 4 M hydrogen chloride dioxane solution (3.5 ml), followed by stirring at room temperature for 2 hours. The solvent was evaporated under reduced pressure to obtain 140 mg of 2-[2-(aminomethyl)phenoxy]ethanol hydrochloride.

Preparation Example 124

Under ice-cooling, to a solution of benzyl rel-({(1S,3R,4S,5S)-4-[(tert-butoxycarbonyl)amino]adamantan-1-yl} methyl)carbamate (295.0 mg) in dichloromethane (3.54 ml) was added trifluoroacetic acid (3.54 ml), followed by stirring at room temperature for 2 hours. The reaction liquid was concentrated under reduced pressure, and then the residue was alkalified by the addition of an aqueous potassium carbonate solution and then extracted with EtOAc. The organic layer was sequentially washed with water and saturated brine, and dried over anhydrous sodium sulfate. After the desiccant was removed, the solvent was evaporated under reduced pressure to obtain 242.8 mg of benzyl rel-{[(1S,3R, 4S,5S)-4-aminoadamantan-1-yl]methyl}carbamate.

Preparation Example 126

To a solution of di-tert-butyl [2-(2-methoxyethoxy)benzyl]imidodicarbonate (341 mg) in 1,4-dioxane (1.7 ml) was added a 4 M hydrogen chloride dioxane solution (3.5 ml) at room temperature, followed by stirring for 2 hours. The solvent was evaporated under reduced pressure to obtain 155 mg of 1-[2-(2-methoxyethoxy)phenyl]methanamine hydrochloride.

Preparation Example 132

To a solution of tert-butyl (2-{4-[(methylsulfonyl)amino]phenyl}ethyl)carbamate (900 mg) in dichloromethane (18 ml) was added trifluoroacetic acid (2.89 ml), followed by stirring at room temperature overnight. The mixed reaction liquid was concentrated under reduced pressure, and toluene was added to the residue, followed by further concentration under reduced pressure. To the obtained residue was added diethyl ether, the precipitated solid was collected by filtration,

washed with diethyl ether, and then dried under reduced pressure. The obtained solid was suspended in EtOH, alkalified by the addition of a 1 M aqueous sodium hydroxide solution, then adjusted to pH 7 with 1 M hydrochloric acid, and extracted with chloroform. The organic layer was combined and dried over anhydrous sodium sulfate. After the desiccant was removed, the solvent was evaporated under reduced pressure to obtain 95 mg of N-[4-(2-aminoethyl)phenyl]methanesulfonamide.

Preparation Example 133

Under ice-cooling, to a solution of benzyl rel-[(1R,2S,3S,5S)-5-{[(trifluoroacetyl)amino]methyl}adamantan-2-yl]carbamate (7.0 g) in EtOH (175 ml) were sequentially added di-tert-butyl dicarbonate (5.58 g), 10% Pd/C (wetted with 50% water, 7.0 g), and cyclohexa-1,4-diene (15.9 ml), followed by stirring at room temperature for 1 hour. The catalyst was removed by filtration, and then the filtrate was concentrated under reduced pressure. The obtained residue was purified by silica gel flash column chromatography (hexane-EtOAc) to obtain 4.67 g of tert-butyl rel-[(1R,2S,3S,5S)-5-{[(trifluoroacetyl)amino]methyl}adamantan-2-yl]carbamate.

Preparation Example 134

To a solution of benzyl rel-({(1R,3S,4R,5R)-4-[(trans-4-hydroxycyclohexyl)amino]adamantan-1-yl}methyl)carbamate (380 mg) in MeOH (11.4 ml) was added 10% Pd/C (wetted with 50% water, 76 mg), followed by stirring at 35˚C for 2.5 hours at a normal pressure under a hydrogen atmosphere. The catalyst was separated by filtration through Celite and washed with MeOH, and then the filtrate was concentrated under reduced pressure to obtain 263.8 mg of rel-trans-4-{[(1R,2S,3S,5S)-5-(aminomethyl)adamantan-2-yl]amino}cyclohexanol.

Preparation Example 137

To a solution of 3-{[bis(tert-butoxycarbonyl)amino]methyl}-4-chlorophenyl acetate (2.79 g) in methanol (56 ml) was added potassium carbonate (1.45 g), followed by stirring at room temperature for 1 hour. To the reaction mixture was added saturated aqueous ammonium chloride solution, and the precipitate was collected by filtration to obtain 2.15 g of di-tert-butyl (2-chloro-5-hydroxybenzyl)imidodicarbonate.

Preparation Example 138

To a mixed solution of rel-4-{[(1'R,3'S,5'S)-5'H-spiro[1,3-dioxolane-2,2'-tricyclo[3.3 .1.1$^{3,7}$]decan]-5'-ylmethyl]amino}-2- {[2-(trifluoromethoxy)benzyl]amino}pyrimidine-5-carbonitrile (385 mg) in THF (23.1 ml) and water (30.8 ml) was added p-toluenesulfonic acid monohydrate (1.42 g), followed by stirring at room temperature overnight. The mixed reaction liquid was concentrated under reduced pressure, and the residue was alkalified by the addition of saturated aqueous sodium bicarbonate, followed by extraction with EtOAc. The organic layer was washed with saturated brine and dried over anhydrous magnesium sulfate. After the desiccant was removed, the solvent was evaporated under reduced pressure. The obtained residue was purified by silica gel flash column chromatography (hexane-EtOAc) to obtain 300 mg of rel-4-({[(1S,3R,5S)-4-oxoadamantan-1-yl]methyl}amino)-2-{[2-(trifluoromethoxy)benzyl]amino}pyrimidine-5-carbonitrile.

Preparation Example 139

To a mixed solution of benzyl rel-[(1'R,3'S,5'S)-5'H-spiro[1,3-dioxolane-2,2'-tricyclo[3.3.1.1$^{3,7}$]decan]-5'-ylmethyl]carbamate (2.5 g) in THF (25 ml) and water (25 ml) was added p-toluenesulfonic acid monohydrate (6.65 g), followed by stirring at room temperature overnight. The mixed reaction liquid was concentrated under reduced pressure, and the residue was alkalified by the addition of saturated aqueous sodium bicarbonate, followed by extraction with EtOAc. The obtained organic layer was washed with saturated brine and dried over anhydrous magnesium sulfate. After the desiccant was removed, the solvent was evaporated under reduced pressure to obtain 2.26 g of benzyl rel-{[(1S,3R,5S)-4-oxoadamantan-1-yl]methyl}carbamate.

Preparation Example 140

To a solution of benzyl rel-({(1R,3S,4R,SR)-4-[(trans-4-{[tert-butyl(dimethyl)silyl]oxy}cyclohexyl)amino]adamantan-1-yl}methyl)carbamate (446 mg) in THF (8.92 ml) was added a solution (2.54 ml) of 1 M tetrabutylammonium fluoride in THF, followed by stirring at 70˚C for 5.5 hours. The solvent was evaporated under reduced pressure, and to the residue was added water, followed by extraction with chloroform. The organic layer was washed with saturated brine

and dried over anhydrous sodium sulfate, the desiccant was then removed, and the solvent was evaporated under reduced pressure. The obtained residue was purified by silica gel column chromatography (starting with chloroform-MeOH and changing to chloroform-MeOH-concentrated aqueous ammonia in the middle of the process) to obtain 385.1 mg of benzyl rel-({(1R,3S,4R,5R)-4-[(trans-4-hydroxycyclohexyl)amino]adamantan-1-yl)methyl)carbamate.

Preparation Example 142

To a mixed solution of a mixture (3.99 g) of 2-{[1-(tetrahydro-21H-pyran-2-yl)-1H-benzimidazol-5-yl]methyl}-1H-isoindole-1,3(2H)-dioneand2-{[1-(tetrahydro-2H-pyran-2-yl)-1H-benzimidazol-6-yl]methyl}-1H-isoindole-1,3(2H)-dione in EtOH (79.8 ml) and THF (79.8 ml) was added hydrazine monohydrate (2.14 ml), followed by heating and refluxing. After completion of the reaction, the insoluble materials were removed by filtration and the filtrate was concentrated under reduced pressure. The obtained residue was diluted with dichloromethane, washed with a 1 M aqueous sodium hydroxide solution, and dried over anhydrous magnesium sulfate. After the desiccant was removed, the solvent was evaporated under reduced pressure. The obtained residue was purified by amino silica gel flash column chromatography (chloroform-MeOH) and then purified by silica gel flash column chromatography (chloroform-MeOH) to obtain 0.42 g of a mixture of 1-[1-(tetrahydro-2H-pyran-2-yl)-1H-benzimidazol-5-yl]methanamine and 1-[1-(tetrahydro-2H-pyran-2-yl)-1H-benzimidazol-6-yl]methanamine.

Preparation Example 143

To a mixed solution of methyl rel-(1R,3S,5R,7S)-4-(benzylamino)adamantane-1-carboxylate (720 mg) in EtOH (7.2 ml) and water (0.72 ml) were added 10% Pd/C (wetted with 50% water, 144 mg) and ammonium formate (455 mg), followed by heating and refluxing for 30 minutes. The mixed reaction liquid was left to be cooled to room temperature, then the catalyst was separated by filtration through Celite, and the filtrate was concentrated under reduced pressure to obtain 482 mg of methyl rel-(1R,3S,5R,7S)-4-aminoadamantane-1-carboxylate.

Preparation Example 144

To a solution of di-tert-butyl [2-(benzyloxy)benzyl]imidodicarbonate (34.5 g) in methanol (170 ml) and THF (170 ml) was added 10% Pd/C (3.5 g), followed by stirring for 14 hours at a normal pressure under a hydrogen atmosphere. The reaction mixture was filtered and the filtrate was concentrated under reduced pressure to obtain 27.0 g of di-tert-butyl (2-hydroxybenzyl)imidodicarbonate.

Preparation Example 145

To a solution of 4-chloro-3-methylphenyl acetate (2.06 g) in carbon tetrachloride (20.6 ml) were added N-bromosuccinimide (1.99 g) and 2,2'-azobis(isobutyronitrile) (366 mg), followed by heating and refluxing for 1 hour. To the reaction liquid was added water, followed by extraction with EtOAc, and the organic layer was washed with saturated aqueous sodium chloride solution, and then dried over anhydrous magnesium sulfate. After the desiccant was removed, the solvent was evaporated under reduced pressure, and the residue was purified by silica gel column chromatography (hexane-EtOAc) to obtain 1.87 g of 3-(bromomethyl)-4-chlorophenyl acetate.

Preparation Example 146

To a solution of tert-butyl (2-methoxybenzyl)carbamate (25.0 g) in MeCN (200 ml) was added N-bromosuccinimide (19.7 g), followed by stirring at room temperature overnight. To the mixed reaction liquid was added N-bromosuccinimide (10.0 g), followed by additionally stirring at room temperature for 8 hours. The mixed reaction liquid was concentrated under reduced pressure, and then the residue was diluted with EtOAc, sequentially washed with water and saturated brine, and dried over anhydrous sodium sulfate. After the desiccant was removed, the solvent was evaporated under reduced pressure. The obtained residue was purified by silica gel flash column chromatography (toluene) to obtain 9.55 g of tert-butyl (5-bromo-2-methoxybenzyl)carbamate.

Preparation Example 147

To a solution of tert-butyl (5-bromo-2-methoxy benzyl)carbamate (5.0 g) in DMF (50 ml) were added bis(triphenylphosphine)palladium (II) dichloride (222 mg), triphenyl phosphine (83 mg), and sodium hydrogen carbonate (1.61 g), followed by heating and stirring at 110˚C at a normal pressure under a carbon monoxide atmosphere. The mixed reaction liquid was diluted with EtOAc, sequentially washed with water, an aqueous sodium carbonate solution, and

saturated brine, and dried over anhydrous sodium sulfate. After the desiccant was removed, the solvent was evaporated under reduced pressure. The obtained residue was purified by silica gel flash column chromatography (hexane-EtOAc) to obtain 1.69 g of tert-butyl (5-formyl-2-methoxybenzyl)carbamate.

Preparation Example 148

Under ice-cooling, to a solution of a mixture (2.0 g) of [1-(tetrahydro-2H-pyran-2-yl)-1H-benzimidazol-5-yl] methanol and [1-(tetrahydro-2H-pyran-2-yl)-1H-benzimidazol-6-yl]methanol in toluene (40 ml) were added succinimide (1.52 g), triphenyl phosphine (2.71 g), and DEAD (1.62 ml), followed by stirring at the same temperature for 3 hours. The mixed reaction liquid was diluted with EtOAc, sequentially washed with water and saturated brine, and dried over anhydrous sodium sulfate. After the desiccant was removed, the solvent was evaporated under reduced pressure, and the obtained residue was purified by silica gel flash column chromatography (chloroform-MeOH) to obtain 4.12 g of a mixture of 2-{[1-(tetrahydro-2H-pyran-2-yl)-1H-benzimidazol-5-yl]methyl}-1H-isoindole-1,3(2H)-dione and 2-{[1-(tetrahydro-2H-pyran-2-yl)-1H-benzimidazol-6-yl]methyl}-1H-isoindole-1,3(2H)-dione.

Preparation Example 149

Under ice-cooling, to a solution of tert-butyl [trans-3-(hydroxymethyl)cyclobutyl]carbamate (60 mg) and TEA (0.066 ml) in dichloromethane (4 ml) was added methanesulfonyl chloride (0.035 ml), followed by stirring at the same temperature for 30 minutes. The reaction liquid was diluted with EtOAc, washed with water and saturated brine in this order, and dried over anhydrous sodium sulfate. The desiccant was removed, the solvent was evaporated under reduced pressure, and the obtained residue was purified by silica gel column chromatography (hexane-EtOAc) to obtain 83.3 mg of {trans-3-[(tert-butoxycarbonyl)amino]cyclobutyl}methyl methanesulfonate.

Preparation Example 150

Under ice-cooling, to a solution of tert-butyl [2-(4-aminophenyl)ethyl]carbamate (1.5 g) in chloroform (15 ml) were sequentially added TEA (0.973 ml) and methanesulfonyl chloride (0.540 ml), followed by stirring at room temperature for 3 hours. TEA (1.326 ml) and mesyl chloride (0.737 ml) were sequentially added thereto, followed by stirring at room temperature for additional 3 hours. The mixed reaction liquid was concentrated under reduced pressure, and the obtained residue was purified by amino silica gel flash column chromatography (hexane-EtOAc) to obtain 1.03 g of tert-butyl (2-{4-[(methylsulfonyl)amino]phenyl}ethyl)carbamate.

Preparation Example 154

Under ice-cooling, to a solution of tert-butyl piperidin-4-ylcarbamate (400 mg) and DIPEA (0.30 ml) in dichloromethane (6 ml) was added chloroacetyl chloride (0.175 ml), followed by stirring at the same temperature for 30 minutes and at room temperature for 2.5 hours. To the reaction mixture were added EtOAc and 0.5 M hydrochloric acid, followed by liquid separation, and then the organic layer was sequentially washed with water, saturated aqueous sodium bicarbonate, water, and saturated brine, and dried over anhydrous sodium sulfate. After the desiccant was removed, the solvent was evaporated under reduced pressure and the residue was purified by silica gel flash column chromatography (hexane-EtOAc) to obtain 545 mg of tert-butyl [1-(chloroacetyl)piperidin-4-yl]carbamate.

Preparation Example 202

To a solution of 6-chloronicotinonitrile (400 mg) and tert-butylpiperidin-4-ylcarbamate (693 mg) in DMF (4.8 ml) was added potassium carbonate (598 mg), followed by stirring at 120˚C for 2 hours. To the reaction mixture were added EtOAc and water, followed by liquid separation, and then the organic layer was sequentially washed with water (three times) and saturated brine, and dried over anhydrous sodium sulfate. After the desiccant was removed, the solvent was evaporated under reduced pressure, and the precipitated powder was collected by filtration. After washing with EtOAc, the powder was dried to obtain 504 mg of tert-butyl [1-(5-cyanopyridin-2-yl)piperidin-4-yl]carbamate.

Preparation Example 238

Under ice-cooling, to a solution of 4-[({(1S,3R,4S,5S)-4-[(3-{[tert-butyldimethyl)silyl]oxypropyl)amino]adamantan-1-yl}methyl)amino]-2- {[2-(methylsulfanyl)benzyl]amino}pyrimidine-5-carbonitrile (65 mg) in MeOH (1.3 mL) were added a 35% aqueous formalin solution (34 mg) and sodium cyanoborohydride (27 mg), followed by stirring at room temperature for 5 hours. To the reaction mixture was added saturated aqueous sodium bicarbonate (5 mL), and the

precipitated white solid was collected by filtration. The obtained solid was dissolved in chloroform and purified by amino silica gel flash column chromatography (hexane-EtOAc) to obtain 60 mg of 4-[({(1S,3R,4S,5S)-4-[(3-[tert-butyldimethyl) silyl]oxypropyl)(methyl)amino]adamantan-1-yl}methyl)amino]-2-{[2-(methylsulfanyl)benzyl]amino}pyrimidine-5-carbonitrile.

Preparation Examples 239 and 240

To a solution of rel-4-({[(1S,3R,4S,5S)-4-aminoadamantan-1-yl]methyl)amino)-2-{[2-(trifluoromethoxy)benzyl] amino}pyrimidine-5-carbonitrile (100 mg) in EtOAc (1.0 ml) were added tert-butyl (4-oxocyclohexyl)carbamate (67.7 mg) and titanium (IV) isopropoxide (250 μl), followed by stirring at room temperature for 20 minutes. Then, to the mixed reaction liquid was added platinum oxide (12 mg), followed by stirring at room temperature for 220 minutes under a hydrogen atmosphere. To the mixed reaction liquid were sequentially added water and EtOAc, followed by stirring, and the insoluble materials were removed by filtration. The filtrate was concentrated under reduced pressure and the residue was extracted with EtOAc. The organic layer was washed with saturated brine and dried over anhydrous sodium sulfate. After the desiccant was removed, the solvent was evaporated under reduced pressure, and the residue was purified by amino silica gel flash column chromatography (hexane-EtOAc) to first elute tert-butyl rel-(cis-4-{[(1R,2S,3S,5S)-5-{[(5-cyano-2-{([2-(trifluoromethoxy) benzyl] amino) pyrimidin-4-yl) amino] methyl} adamantan-2-yl] amino} cyclohexyl) carbamate (86.6 mg), and then elute tert-butyl rel-(trans-4-{[(1R,2S,3S,5S)-5-([(5-cyano-2-{[2-(trifluoromethoxy)benzyl] amino}pyrimidin-4-yl)amino]methyl}adamantan-2-yl]amino}cyclohexyl)carbamate (28.4 mg).

Preparation Example 241

Under ice-cooling, to a solution of (4-methoxypyridin-3-yl)methanol (58 mg) in chloroform (0.6 ml) was added thionyl chloride (0.046 ml), followed by stirring at room temperature for 1 hour. The reaction liquid was concentrated under reduced pressure, and to the reside was added saturated aqueous sodium bicarbonate, followed by extraction with EtOAc. The organic layer was washed with water and saturated brine, and dried over anhydrous magnesium sulfate. After the desiccant was removed, the solvent was evaporated under reduced pressure to obtain 65 mg of 3-(chloromethyl)-4-methoxypyridine.

Preparation Example 245

Under ice-cooling, to a solution of tert-butyl 5-cyano-4,5,6,7-tetrahydro-1H-benzimidazole-1-carboxylate (100 mg) in MeOH (3 mL) were slowly added cobalt (II) chloride hexahydrate (192 mg) and sodium borohydride (61 mg), followed by stirring at room temperature for 1 hour. To the reaction liquid was added 1 M hydrochloric acid (1 mL), and the insoluble materials were removed by filtration. The filtrate was washed with chloroform (10 mL), and to the aqueous layer was added 1 M hydrochloric acid (4 mL), followed by concentration under reduced pressure, to obtain 72 mg of 1-(4,5,6,7-tetrahydro-1H-benzimidazol-5-yl)methylamine dihydrochloride.

Preparation Example 246

To a solution of tert-butyl (2-vinylbenzyl)carbamate (30 mg) in water (0.075 ml) - acetone (0.15 ml) were added a 4% aqueous osmium tetroxide solution (41 mg) and 4-methylmorpholine N-oxide (23 mg) at room temperature. After stirring at the same temperature for 2 hours, a 10% aqueous sodium sulfite solution was added thereto under ice-cooling. The mixture was extracted with EtOAc, and the organic layer was washed with saturated brine and dried over anhydrous magnesium sulfate. After the desiccant was removed, the solvent was evaporated under reduced pressure, and the obtained residue was purified by silica gel flash column chromatography (hexane-EtOAc) to obtain 24 mg of tert-butyl [2-(1,2-dihydroxyethyl)benzyl]carbamate.

Preparation Example 260

Under ice-cooling, to a solution of tert-butyl 5-carbamoyl-4,5,6,7-tetrahydro-1H-benzimidazole-1-carboxylate (500 mg) in THF (5 mL) were added trifluoroacetic anhydride (0.32 mL) and pyridine (0.32 mL), followed by stirring at room temperature for 1 hour. To the reaction liquid was added saturated aqueous sodium bicarbonate (10 mL), followed by extraction with EtOAc (40 mL). The organic layer was washed with water (10 mL) and saturated brine (10 mL), and then dried over anhydrous sodium sulfate. Then, the desiccant was removed and the solvent was evaporated under reduced pressure. The obtained residue was purified by silica gel flash column chromatography (hexane-EtOAc) to obtain 380 mg oftert-butyl 5-cyano-4,5,6,7-tetrahydro-1H-benzimidazole-1-carboxylate.

Preparation Example 261

Under ice-cooling, to a solution of 2-(methylsulfanyl)nicotinonitrile (382 mg) in MeOH (6 ml) was added cobalt (II) chloride hexahydrate (1.69 g), and sodium borohydride (346 mg) was added thereto in small portions at the same temperature. After stirring at room temperature for 3 hours, the precipitate was removed by filtration through Celite. The filtrate was concentrated under reduced pressure, and to the residue was added 1 M hydrochloric acid, followed by washing with chloroform. The aqueous layer was alkalified by the addition of 28% aqueous ammonia and then extracted with chloroform, and the organic layer was dried over anhydrous magnesium sulfate. The solvent was evaporated under reduced pressure to obtain 237 mg of 1-[2-(methylsulfanyl)pyridin-3-yl]methylamine.

Preparation Example 262

Under ice-cooling, to a mixed solution of rel-4-({[(1S,3R,4S,5S)-4-(3-hydroxyazetidin-1-yl)adamantan-1-yl]methyl}amino)-2-{[2-(trifluoromethoxy)benzyl]amino}pyrimidine-5-carbonitrile (50 mg) in THF (2 ml) and toluene (3 ml) were added succinimide (19.8 mg), triphenylphosphine (29.8 mg), and DEAD (17.8 μl), followed by stirring at room temperature. Succinimide (33.4 mg), triphenylphosphine (74.4 mg), DEAD (44.5 μl), THF (2 ml), and toluene (1 ml) were added thereto, followed by further stirring at room temperature. After completion of the reaction, the mixed reaction liquid was diluted with EtOAc, and the organic layer was sequentially washed with water (three times) and saturated brine, and dried over anhydrous sodium sulfate. After the desiccant was removed, the solvent was evaporated under reduced pressure, and the obtained residue was purified by silica gel flash column chromatography (hexane-EtOAc) to obtain 70.7 mg of rel-4-[({(1S,3R,4S,5S)-4-[3-(1,3-dioxo-1,3-dihydro-2H-isoindol-2-yl)azetidin-1-yl]adamantan-1-yl]methyl)amino]-2-{[2-(trifluoromethoxy)benzyl]amino}pyrimidine-5-carbonitrile.

Preparation Example 263

Under ice-cooling, to a suspension of rel-(1S,3R,4S,5S)-4-aminoadamantane-1-carboxylic acid (100 mg) in 1,4-dioxane (0.7 ml) was added a 1M aqueous sodium hydroxide solution (0.62 ml), followed by stirring at the same temperature for 10 minutes for dissolution. Under ice-cooling, a solution of di-tert-butyl dicarbonate (115 mg) in 1,4-dioxane (0.1 ml) was added dropwise thereto, followed by stirring at room temperature for 4 hours. Under ice-cooling, 1 M hydrochloric acid (0.74 ml) was added thereto, followed by extraction with EtOAc, and washing with water (twice) and then with saturated brine. The organic layer was dried over anhydrous sodium sulfate, filtered, and then concentrated under reduced pressure to precipitate crystals. Thus, the crystals were suspended in hexane (3 ml) before dryness, and collected by filtration to obtain 111.1 mg of rel-(1S,3R,4S,5S)-4-[(tert-butoxycarbonyl)amino]adamantane-1-carboxylic acid.

Preparation Example 267

To a suspension of (methoxymethyl)(triphenyl)phosphonium chloride (164.57 g) which had been cooled in a dry ice-acetone bath in THF (500 ml) was added dropwise a solution of n-butyllithium in hexane (concentration 1.65 M, 281.3 ml) at -55˚C or lower under a nitrogen air flow. Then, the mixed reaction liquid was warmed, followed by stirring at room temperature for 1 hour. After stirring, the mixed reaction liquid was cooled under ice, and a solution of 4-hydroxy-4-methylcyclohexanone (20.51 g) in THF (205 ml) was added dropwise thereto. After dropwise addition, the mixed reaction liquid was warmed to room temperature, followed by stirring for 15 hours. To the mixed reaction liquid were sequentially added water and EtOAc, followed by stirring, and then the organic layer was collected by separation. The aqueous layer was further extracted with EtOAc, and the organic layer was combined, washed with saturated brine, and dried over anhydrous sodium sulfate. After the desiccant was removed, the solvent was evaporated under reduced pressure. The residue was purified by silica gel flash column chromatography (hexane-EtOAc) to obtain 4-(methoxymethylene)-1-methylcyclohexanol (21.37 g).

Preparation Example 269

To a solution of tert-butyl rel-[(1R,2S,3S,5S)-5-(azidomethyl)adamantan-2-yl]carbamate (300 mg) in THF (3 ml) was added triphenylphosphine (300 mg), followed by stirring at room temperature for 4 hours. To the reaction mixture was added water (1.8 ml), followed by stirring at room temperature for 2 hours, and then the solvent was evaporated under reduced pressure. The residue was purified by silica gel flash column chromatography (the side products were first eluted with EtOAc alone, and then the eluting solvent was changed to MeOH/chloroform/28% $NH_3$ aq. (1/9/0.1)) to obtain 270 mg of tert-butyl rel-[(1R,2S,3S,5S)-5-(aminomethyl)adamantan-2-yl]carbamate.

Preparation Example 286

To a solution of tert-butyl rel-[(1R,2S,3S,5S)-5-{[(2-chloro-5-fluoropyrimidin-4-yl)amino]methyl}adamantan-2-yl]carbamate (100 mg) and 2-(trifluoromethoxy)benzylamine (280 mg) in DMI (0.8 ml) was added DIPEA (0.127 ml), followed by irradiation with microwaves at 165˚C for 4 hours. The reaction liquid was diluted with EtOAc, sequentially washed with water, saturated aqueous ammonium chloride solution, water, saturated aqueous sodium hydrogen carbonate solution, water, and saturated brine, and dried over anhydrous sodium sulfate. After the desiccant was removed, the solvent was evaporated under reduced pressure. The obtained residue was purified by silica gel flash column chromatography (hexane-EtOAc) to obtain 78.3 mg of tert-butyl rel-[(1R,2S,3S,5S)-5-{[(5-fluoro-2-{[2-(trifluoromethoxy) benzyl]amino}pyrimidin-4-yl)amino]methyl}adamantan-2-yl]carbamate.

Preparation Example 288

Under ice-cooling, to a solution of tert-butyl [(2-chloropyridin-3-yl)methyl]carbamate (450 mg) in DMF (2 ml) were added cyclopentanethiol (0.65 ml) and sodium hydride (about 40% of mineral oil added, 220 mg), followed by stirring at room temperature for 4 hours. Then, the reaction liquid was cooled under ice, and cyclopentanethiol (0.40 ml) and sodium hydride (about 40% of mineral oil added, 140 mg) were added thereto, followed by stirring at room temperature for 2 hours. Again, the reaction liquid was cooled under ice, and saturated aqueous ammonium chloride solution was added thereto, followed by extraction with chloroform. The organic layer was dried over anhydrous sodium sulfate, the desiccant was removed, and then the solvent was evaporated under reduced pressure. The obtained residue was purified by silica gel flash column chromatography (hexane-EtOAc) to obtain 400 mg of tert-butyl {[2-(cyclopentylsulfonyl) pyridin-3-yl]methyl}carbamate.

Preparation Example 289

At room temperature, to a suspension of rel-(1S,3R,4S,5S)-4-[(tert-butoxycarbonyl)amino]adamantane-1-carboxylic acid (99.0 mg) in DME (0.99 ml) was added N-methylmorpholine (0.044 ml) for dissolution. Under ice-cooling, isobutyl chlorocarbonate (0.052 ml) was added dropwise thereto, followed by stirring at the same temperature for 40 minutes. The precipitated white insoluble materials were removed by filtration and washed with DME (0.5 ml). The filtrate was cooled under ice, sodium borohydride (25.3 mg) was added thereto, and then MeOH (0.495 ml) was slowly added dropwise thereto. After stirring at room temperature for 1 hour, the reaction liquid was cooled under ice and diluted with EtOAc. The reaction liquid was acidified by the addition of 1 M hydrochloric acid (1.0 ml), and the organic layer was collected by separation. The organic layer was sequentially washed with water (twice), saturated aqueous sodium bicarbonate, water, and then saturated brine, dried over anhydrous sodium sulfate, filtered, and then concentrated under reduced pressure. The residue was purified by silica gel column chromatography (hexane-EtOAc). A fraction including a desired product was concentrated under reduced pressure, and then the residue was dissolved in EtOAc, and concentrated under reduced pressure to about 0.1 ml. Hexane (1.5 ml) was added portionwise thereto for crystallization, and the crystals were collected by filtration to obtain 77.3 mg of tert-butyl rel-[(1R,2S,3S,5S)-5-(hydroxymethyl)adamantan-2-yl]carbamate.

Preparation Example 294

Rel-(1R,3S,5R,7S)-4-oxoadamantane-1-carboxylic acid (1.0 g) was dissolved in a solution (concentration 8 M, 20 ml) of ammonia in MeOH, and 10% Pd/C (wetted with 50% water, 100 mg) was added thereto, followed by stirring at 25˚C for 10 hours under a hydrogen atmosphere of 3 atm. The product that had been precipitated in a large amount was dissolved in water (20 ml), and the catalyst was removed by filtration through Celite. MeOH was evaporated under reduced pressure, and to the residue was added dropwise acetonitrile (30 ml), followed by stirring at room temperature for 1 hour. The precipitate was collected by filtration, washed with MeCN (10 ml), and then dried under reduced pressure at 45˚C to obtain 982 mg of rel-(1S,3R,5S)-4-aminoadamantane-1-carboxylic acid as a mixture of trans isomer and cis isomer at a ratio of 3.5:1.

Preparation Example 316

Rel-(1S,3R,5S)-4-aminoadamantane-1-carboxylic acid (mixture of trans product and cis product at a ratio of 3.5:1, 100 mg) was suspended in water (4 ml), followed by stirring at 75˚C for 30 minutes. While stirring, the suspension was cooled back to room temperature, and MeCN (4 ml) was slowly added dropwise thereto, followed by stirring at the same temperature for 30 minutes. The precipitate was collected by filtration, washed with acetonitrile (1 ml), and then dried under reduced pressure at 45˚C to obtain 50.0 mg of rel-(1S,3R,4S,5S)-4-aminoadamantane-1-carboxylic acid.

Preparation Example 322, 323

To a solution of 4-(methoxymethylene)-1-methyleyclohexanol (5.0 g) in MeCN (50 ml) were sequentially added water (8.6 ml) and TFA (3.6 ml), followed by stirring at room temperature for 4 hours. The mixed reaction liquid was adjusted to be neutral with saturated aqueous sodium hydrogen carbonate solution, and then extracted with EtOAc four times. The organic layer was combined, washed with saturated brine, and dried over anhydrous magnesium sulfate. After the desiccant was removed, the solvent was evaporated under reduced pressure. The obtained residue was purified by silica gel flash column chromatography (hexane-EtOAc) to first elute cis-4-hydroxy-4-methylcyclohexanecarbaldehyde (2.37 g) and then elute trans-4-hydroxy-4-methylcyclohexanecarbaldehyde (2.7 g).

Each of the Preparation Example compounds was prepared in the same manner as the methods of Preparation Examples above, using each of the corresponding starting materials. The structures, the production processes, and the physicochemical data of the compounds of Preparation Examples are shown in Tables below.

[Table 3]

| PEx | Str | PEx | Str |
|---|---|---|---|
| 1 rel | 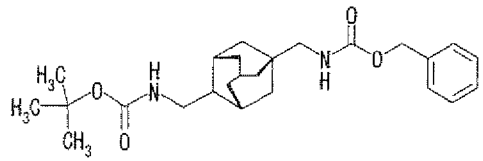 | 2 rel | 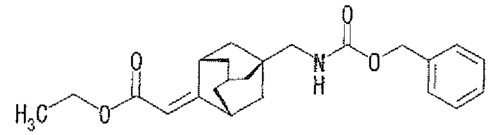 |
| 3 rel | 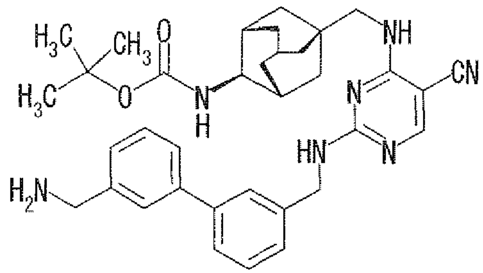 | 4 | 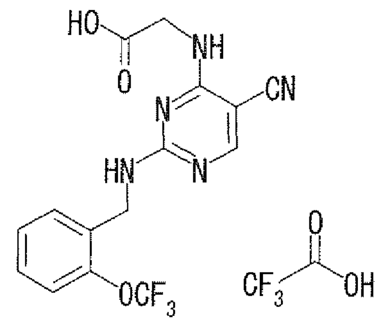 |
| 5 | 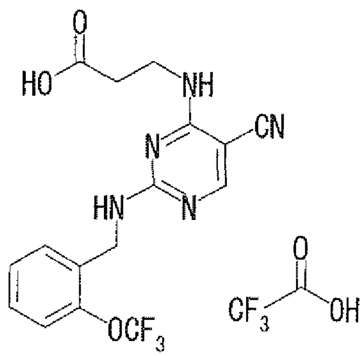 | 6 | 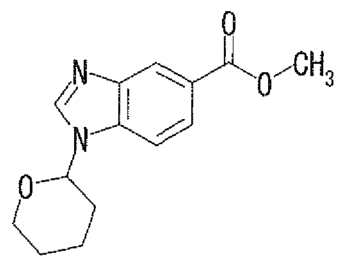 |

(continued)

| PEx | Str | PEx | Str |
|---|---|---|---|
| 7 rel | 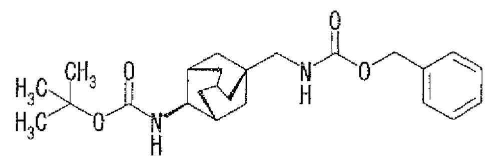 | 8 rel | 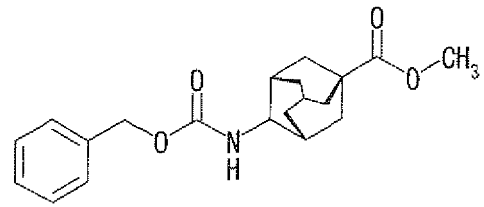 |
| 9 rel | 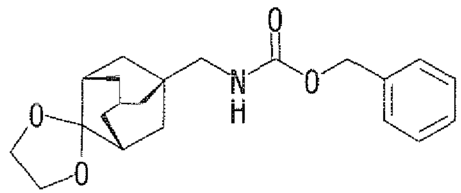 | 10 | 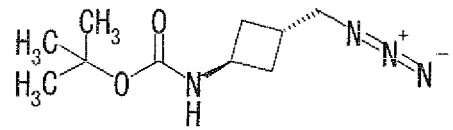 |
| 11 | 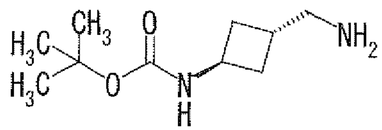 | 12 | 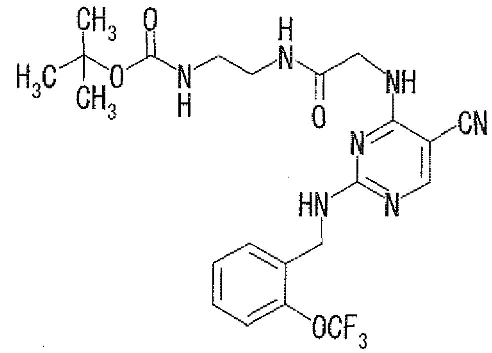 |

[Table 4]

| PEx | Str | PEx | Str |
|---|---|---|---|
| 13 | 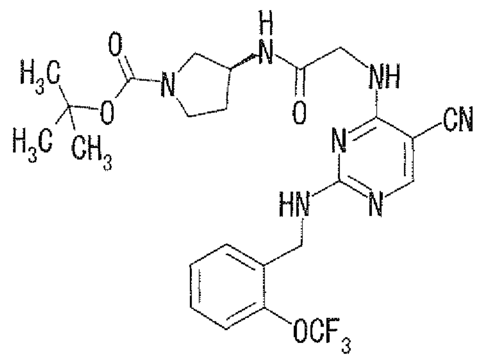 | 14 | 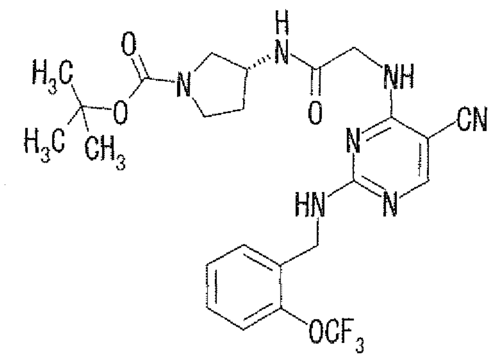 |
| 15 | 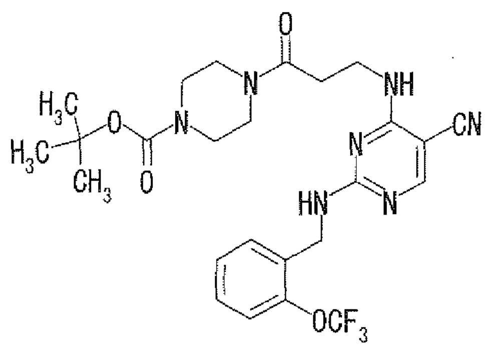 | 16 | 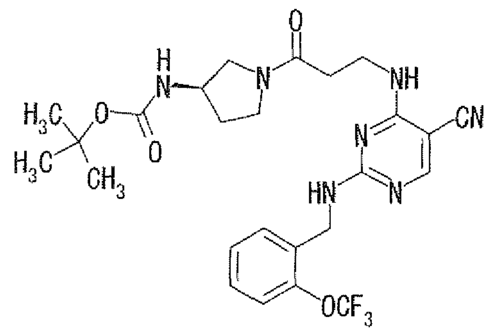 |

(continued)

| PEx | Str | PEx | Str |
|---|---|---|---|
| 17 | 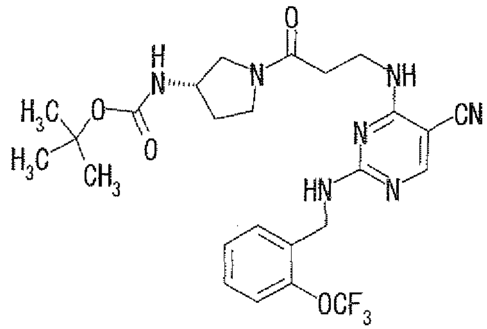 | 18 | 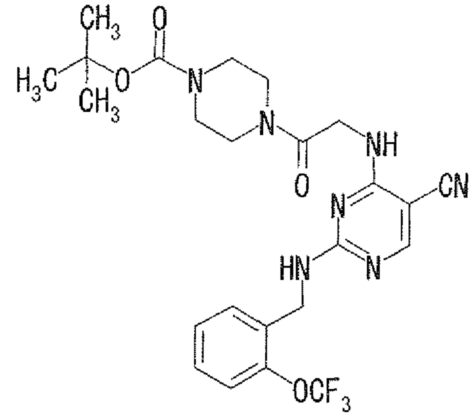 |
| 19 rel | 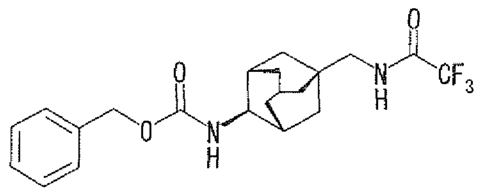 | 20 rel | 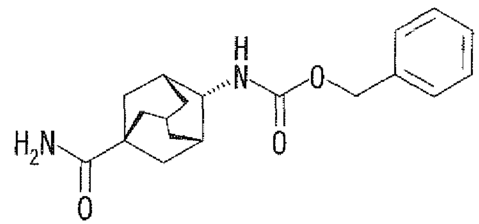 |
| 21 rel | 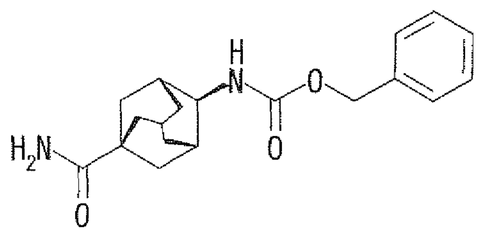 | 22 rel | 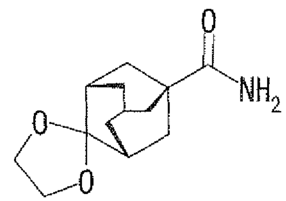 |

[Table 5]

| PEx | Str | PEx | Str |
|---|---|---|---|
| 23 rel | 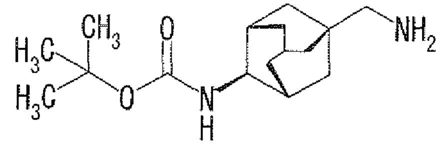 | 24 rel | 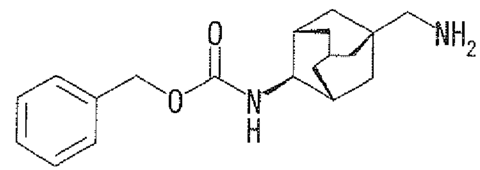 |
| 25 rel | 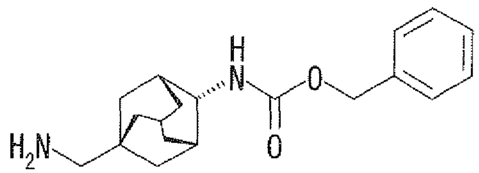 | 26 rel | 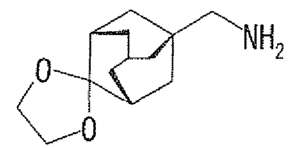 |
| 27 | 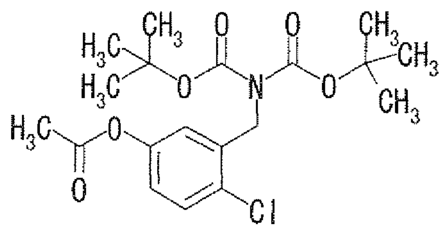 | 28 | 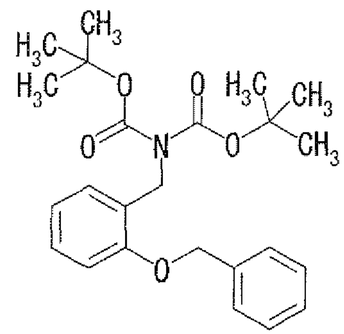 |
| 29 rel | 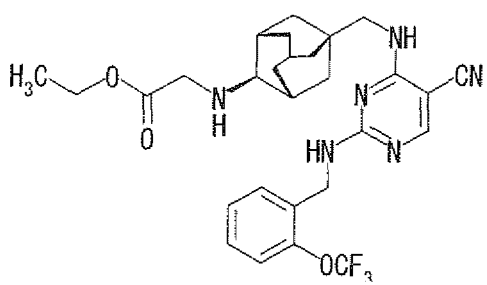 | 30 rel | 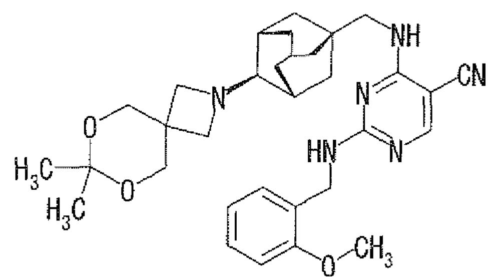 |

(continued)

| PEx | Str | PEx | Str |
|---|---|---|---|
| 31 | 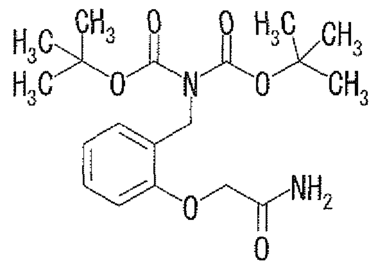 | 32 | 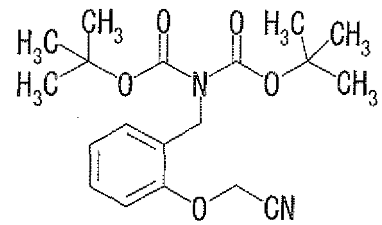 |
| 33 | 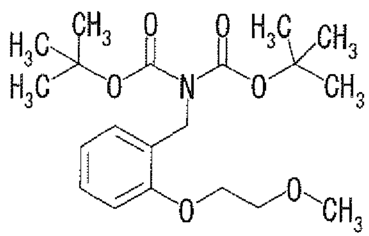 | 34 | 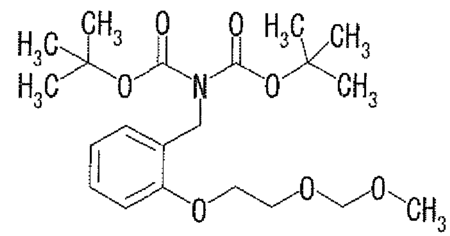 |

[Table 6]

| PEx | Str | PEx | Str |
|---|---|---|---|
| 35 | 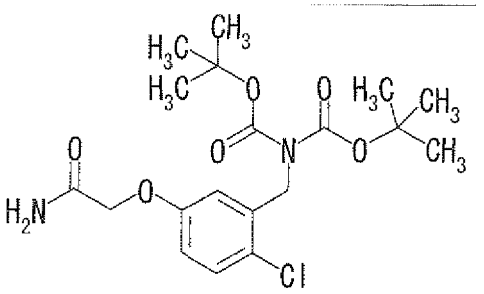 | 36 | 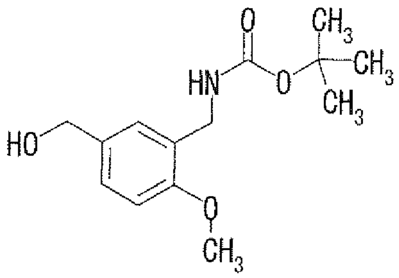 |
| 37 rel | 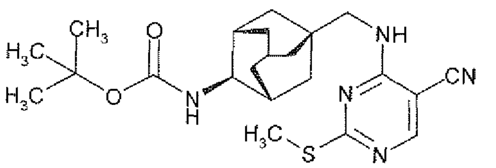 | 38 | 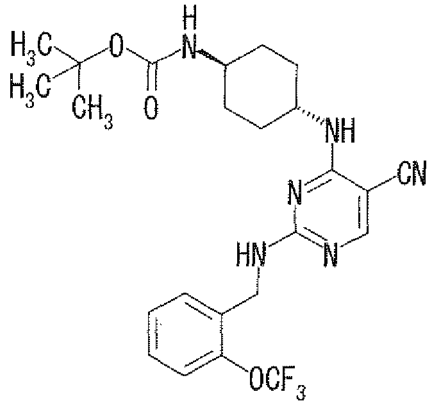 |
| 39 | 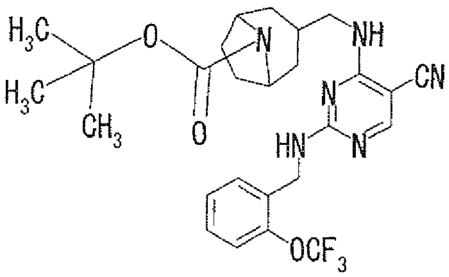 | 40 rel | 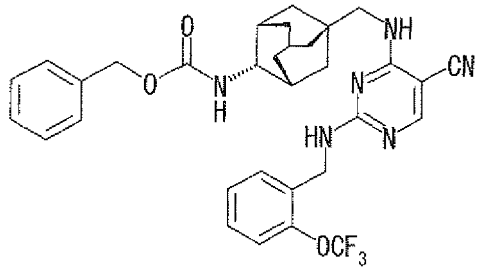 |
| 41 rel | 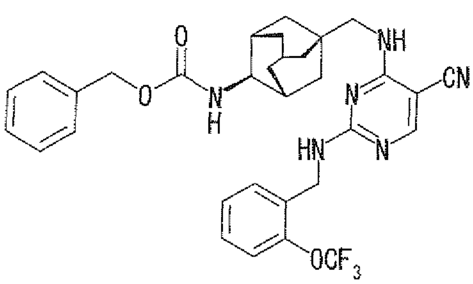 | 42 | 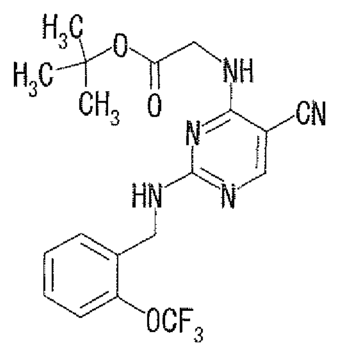 |

[Table 7]

| PEx | Str | PEx | Str |
|---|---|---|---|
| 43 rel | 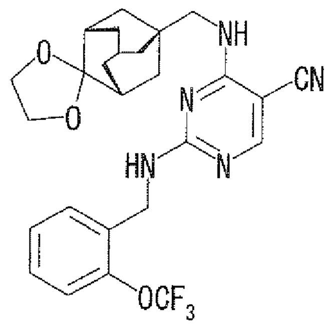 | 44 | 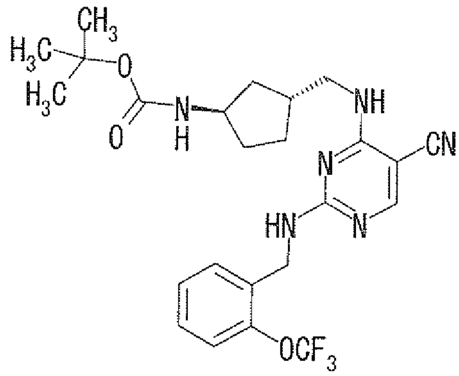 |
| 45 | 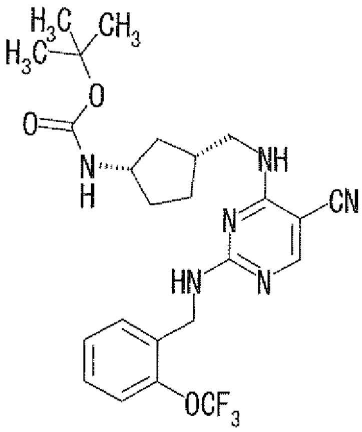 | 46 rel | 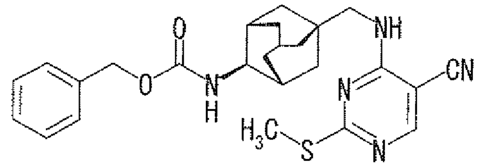 |
| 47 rel | 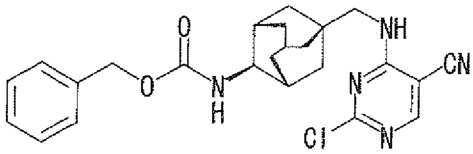 | 48 rel | 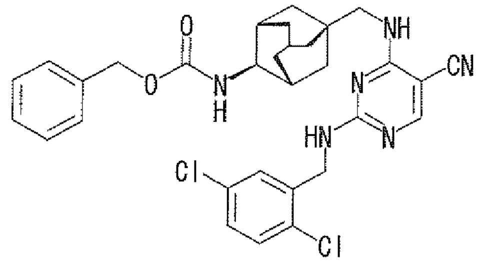 |
| 49 rel | 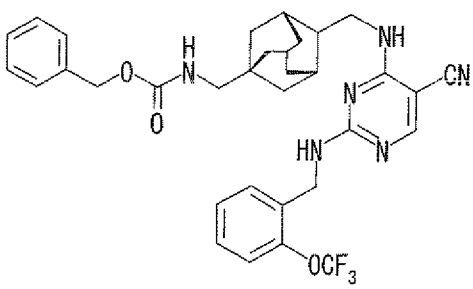 | 50 rel | 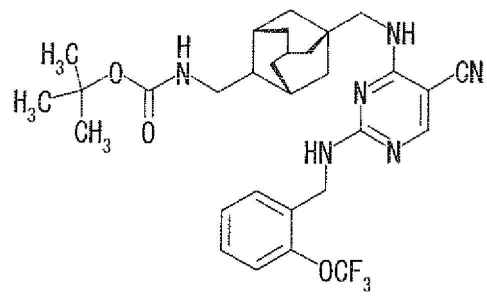 |

[Table 8]

| PEx | Str | PEx | Str |
|---|---|---|---|
| 51 | 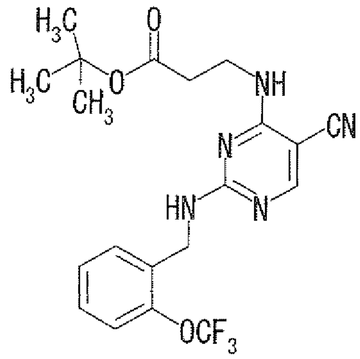 | 52 | 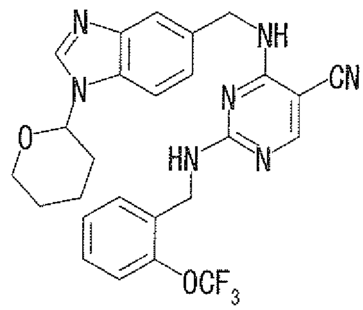 |
| 53 | 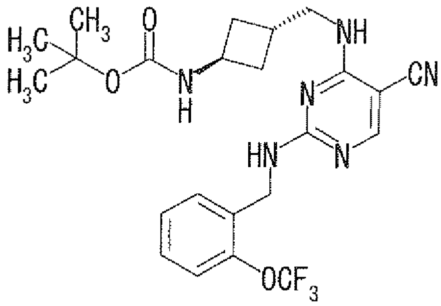 | 54 | 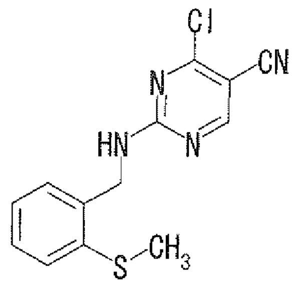 |
| 55 rel | 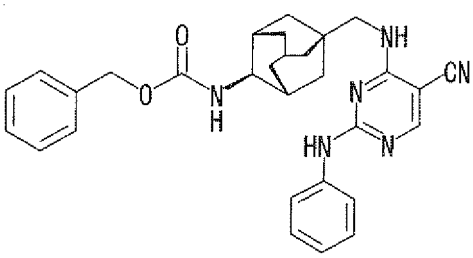 | 56 rel | 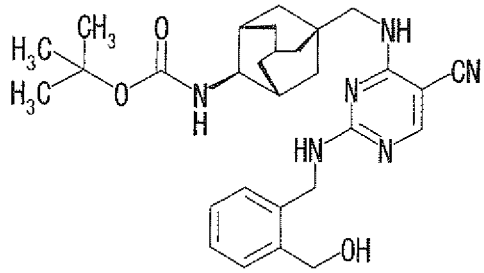 |
| 57 rel | 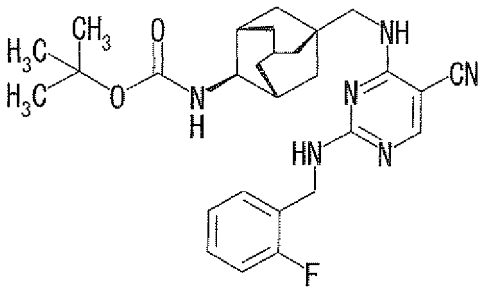 | 58 rel | 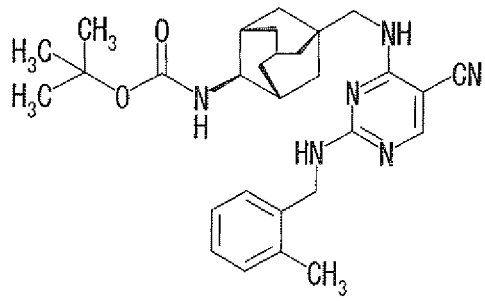 |
| 59 rel | 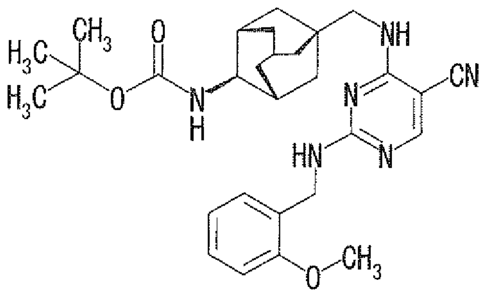 | 60 rel | 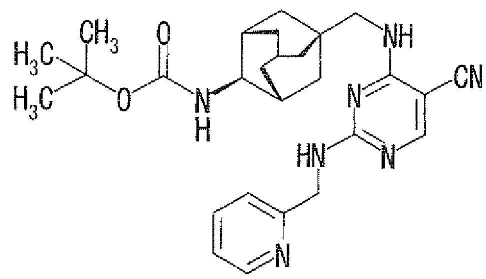 |

[Table 9]

| PEx | Str | PEx | Str |
|---|---|---|---|
| 61 rel | 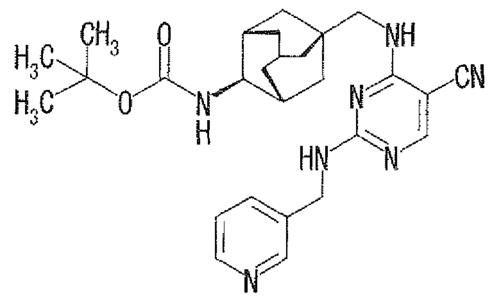 | 62 rel | 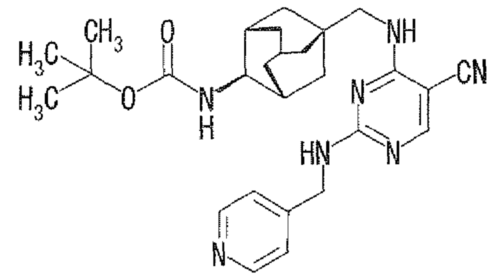 |
| 63 rel | 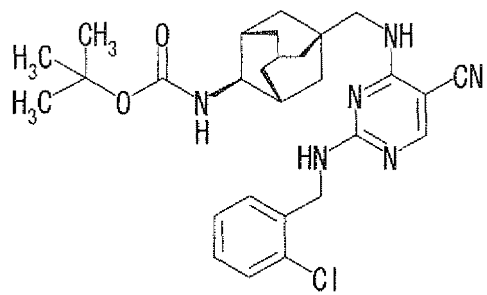 | 64 rel | 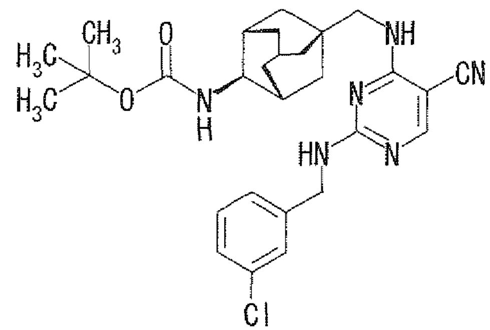 |
| 65 rel | 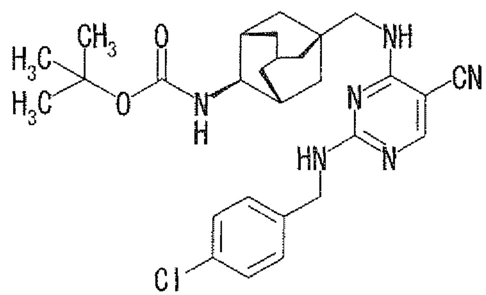 | 66 rel | 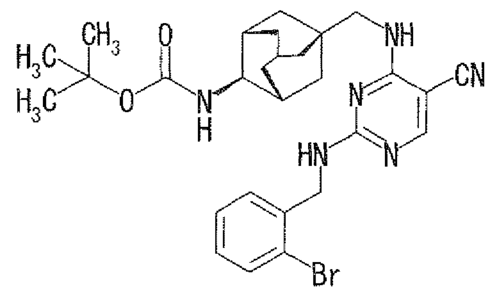 |

(continued)

| PEx | Str | PEx | Str |
|---|---|---|---|
| 67 rel | 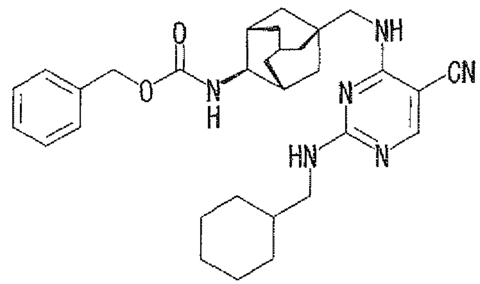 | 68 rel | 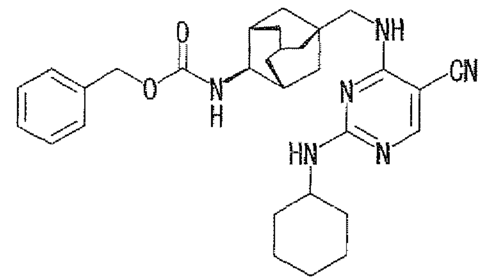 |
| 69 rel | 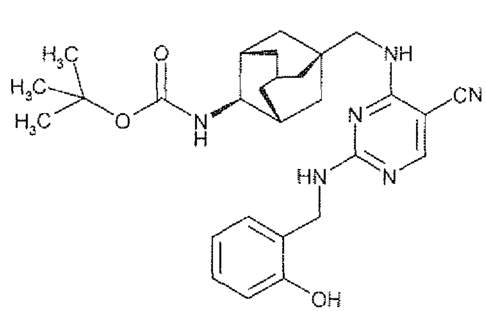 | 70 rel | 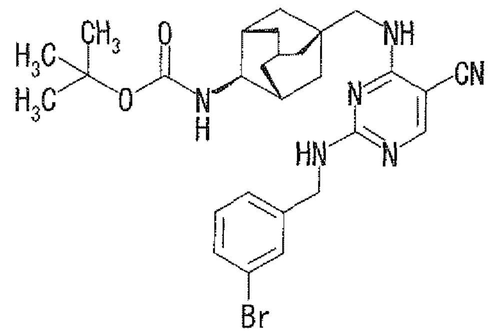 |

[Table 10]

| PEx | Str | PEx | Str |
|---|---|---|---|
| 71 rel | 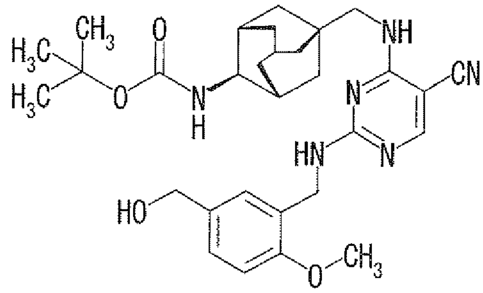 | 72 rel | 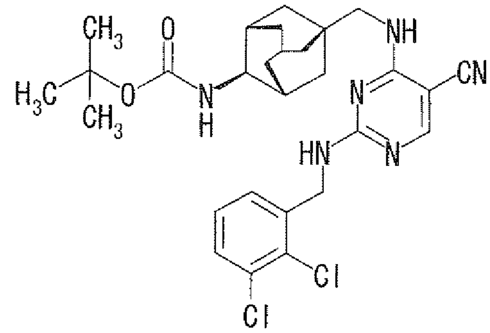 |
| 73 rel | 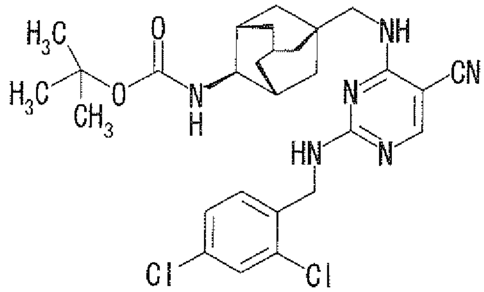 | 74 rel | 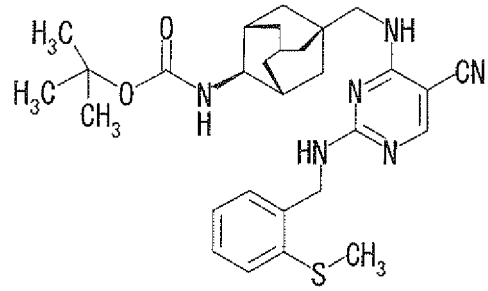 |
| 75 | 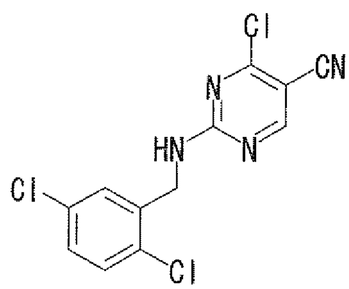 | 76 rel | 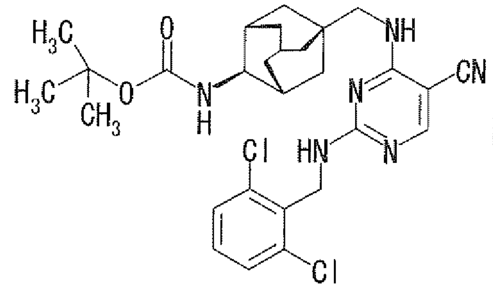 |
| 77 rel | 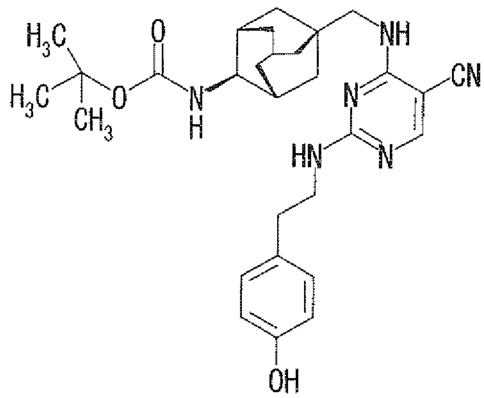 | 78 rel | 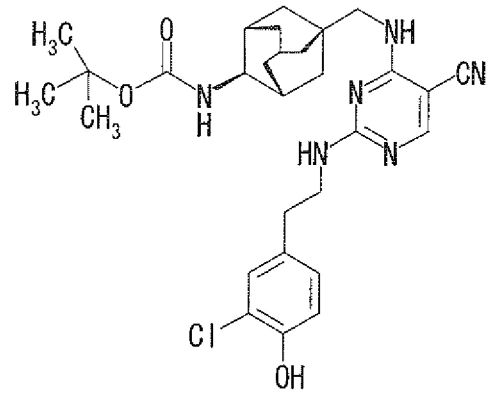 |

[Table 11]

| PEx | Str | PEx | Str |
|---|---|---|---|
| 79 rel | 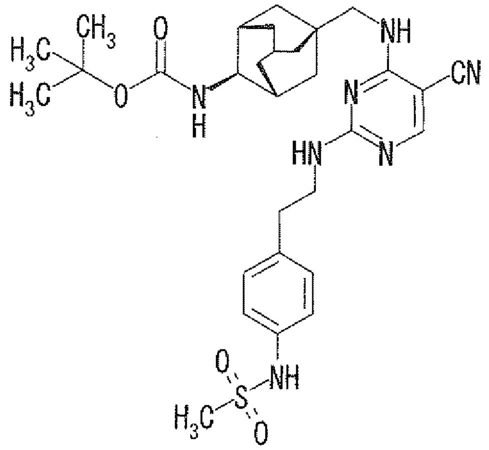 | 80 rel | 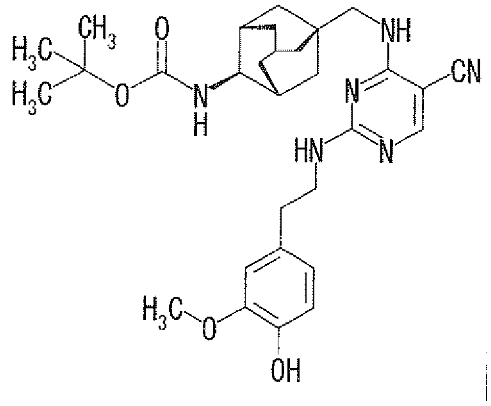 |
| 81 rel | 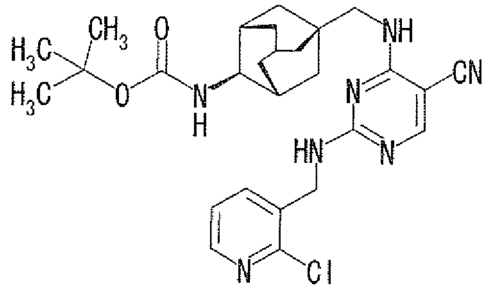 | 82 rel | 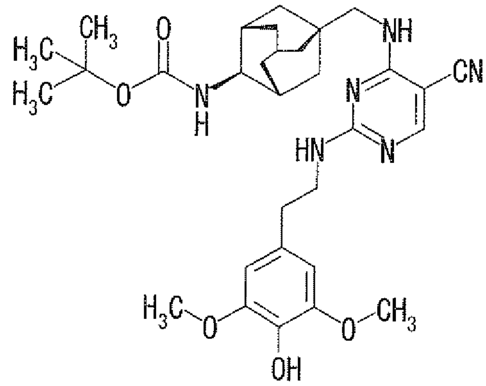 |
| 83 | 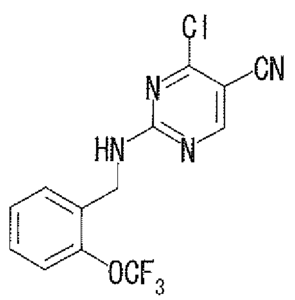 | 84 rel | 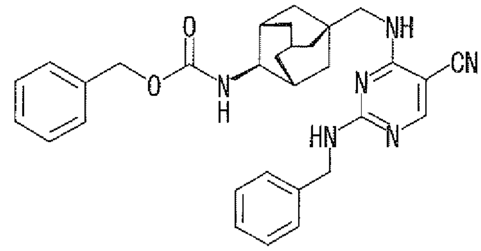 |
| 85 rel | 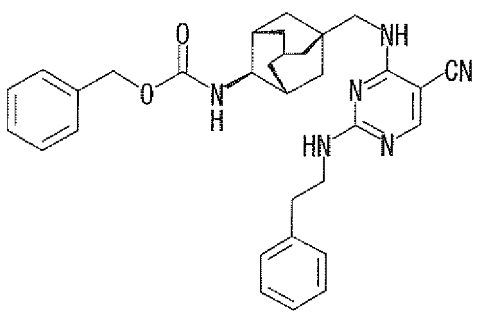 | 86 rel | 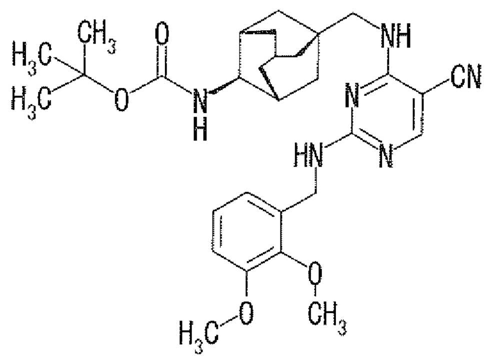 |

[Table 12]

| PEx | Str | PEx | Str |
|---|---|---|---|
| 87 rel | 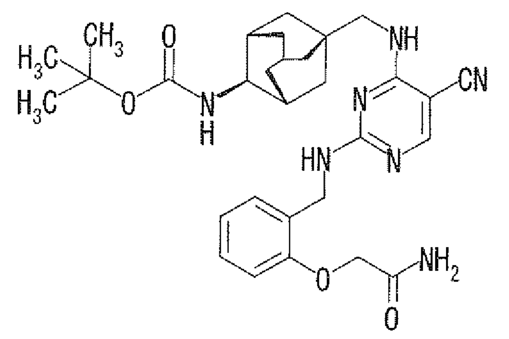 | 88 rel | 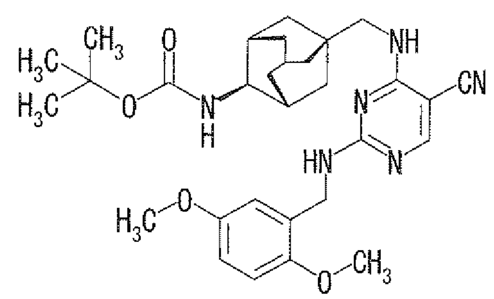 |
| 89 rel | 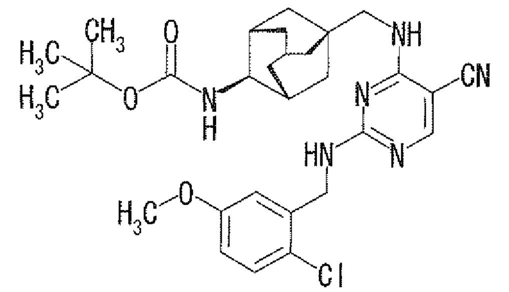 | 90 rel | 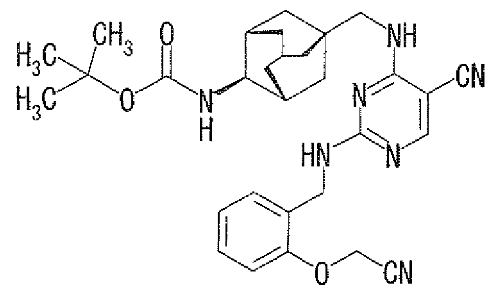 |
| 91 rel | 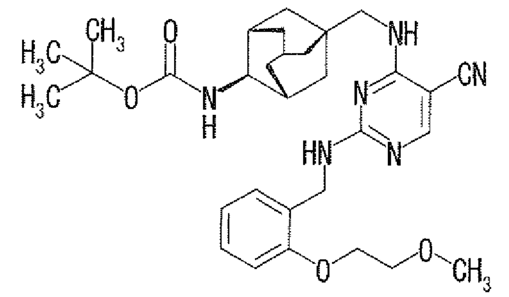 | 92 rel | 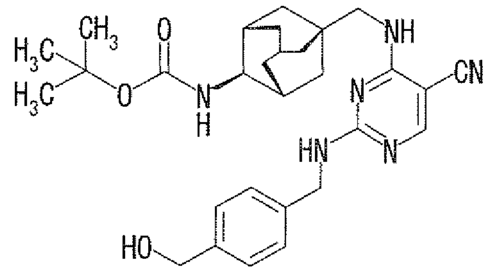 |
| 93 rel | 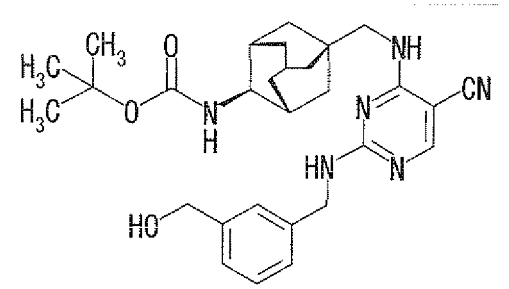 | 94 rel | 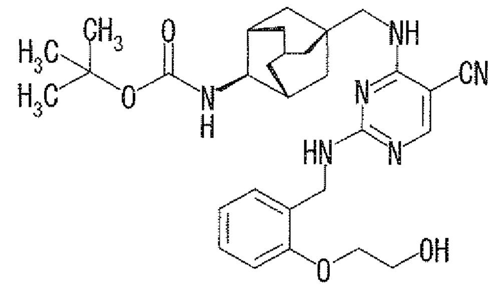 |
| 95 rel | 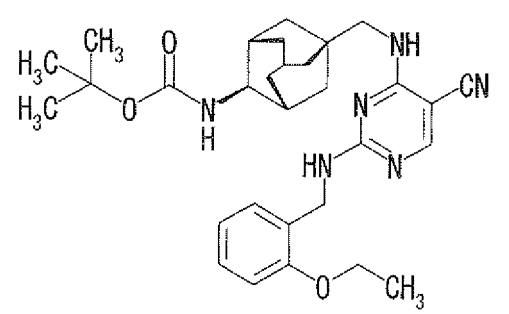 | 96 rel | 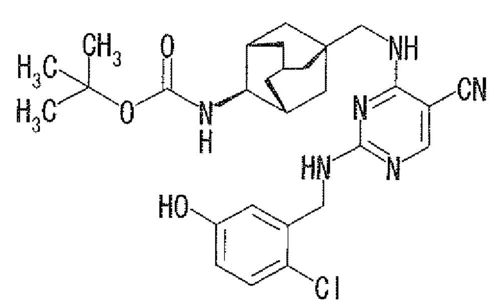 |

[Table 13]

| PEx | Str | PEx | Str |
|---|---|---|---|
| 97 rel | 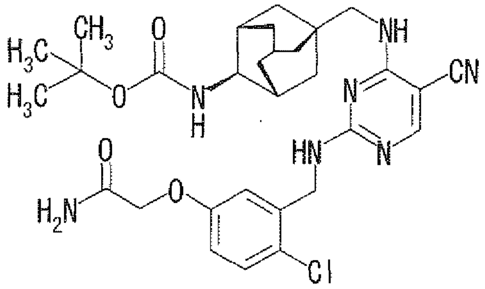 | 98 | 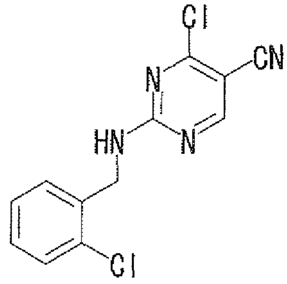 |
| 99 | 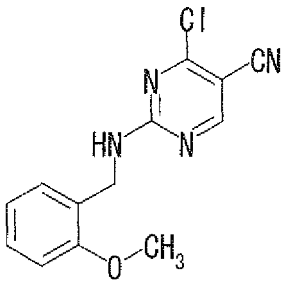 | 100 | 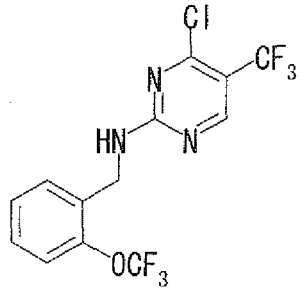 |
| 101 | 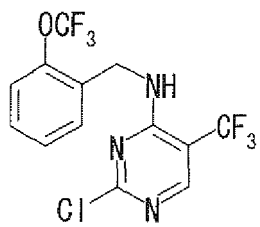 | 102 rel | 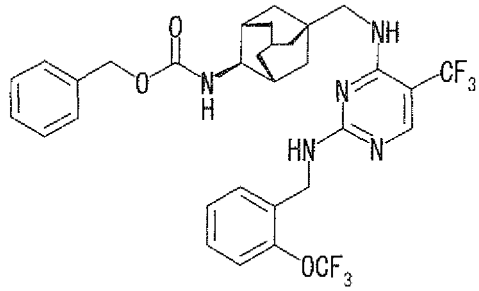 |
| 103 rel | 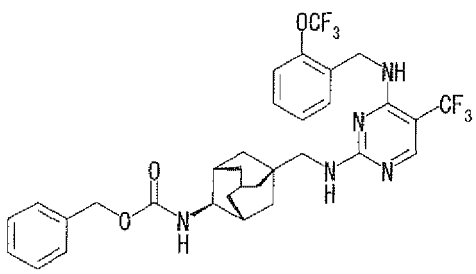 | 104 rel | 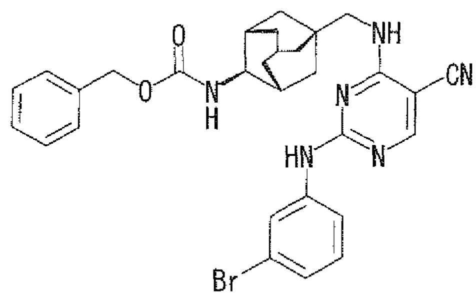 |
| 105 rel | 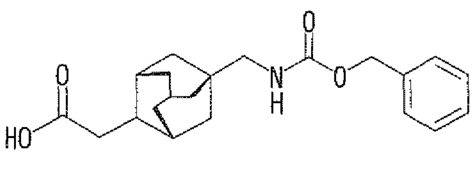 | 106 rel | 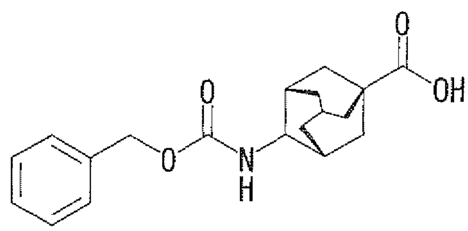 |

[Table 14]

| PEx | Str | PEx | Str |
|---|---|---|---|
| 107 | 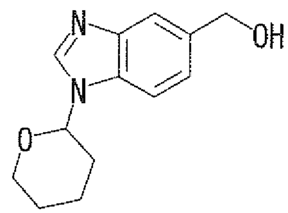 | 108 rel | 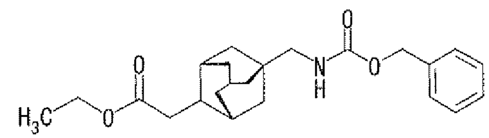 |
| 109 rel | 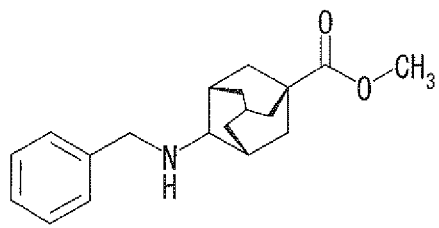 | 110 rel | 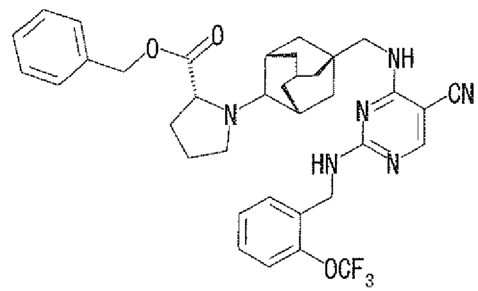 |
| 111 rel | 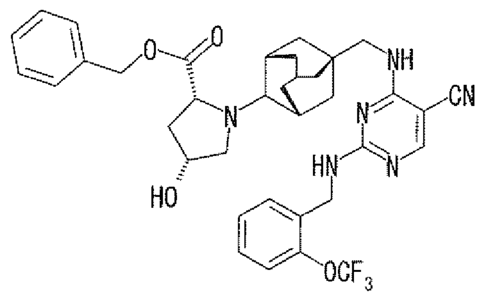 | 112 rel | 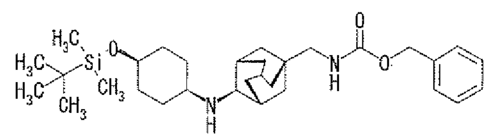 |

(continued)

| PEx | Str | PEx | Str |
|---|---|---|---|
| 113 rel | 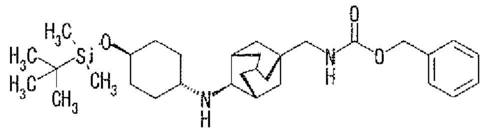 | 114 rel | 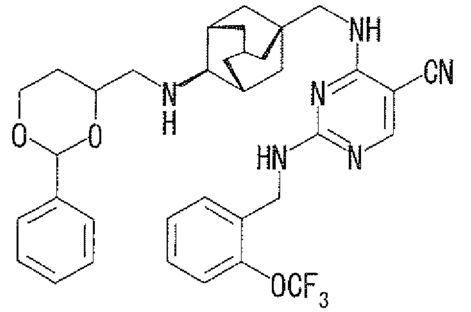 |
| 115 rel | 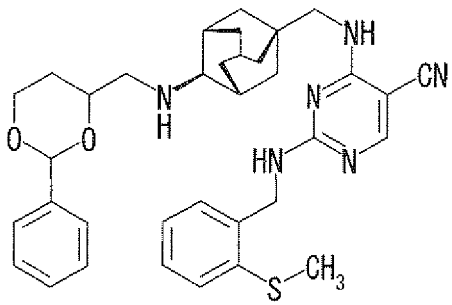 | 116 rel | 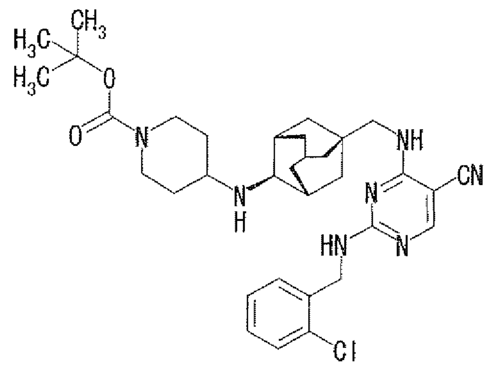 |

[Table 15]

| PEx | Str | PEx | Str |
|---|---|---|---|
| 117 rel | 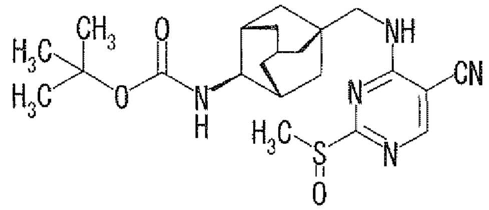 | 118 rel | 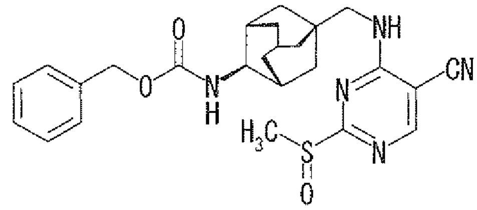 |
| 119 rel | 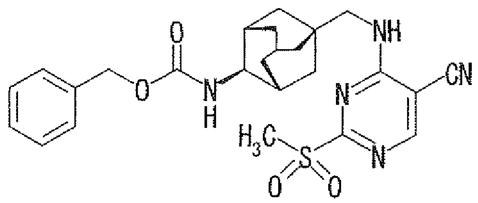 | 120 rel | 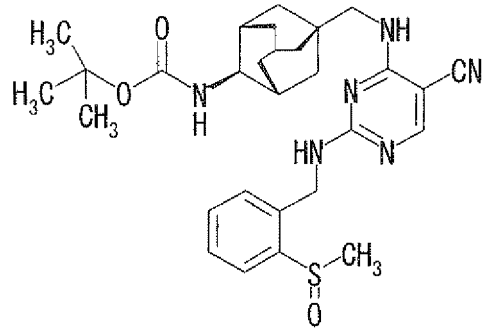 |
| 121 rel | 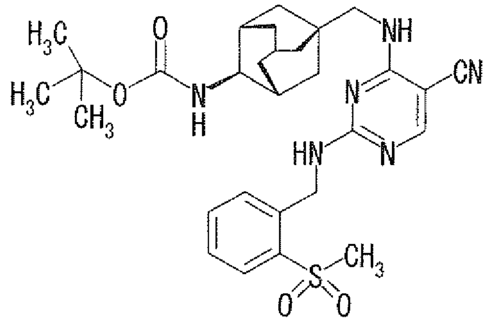 | 122 | 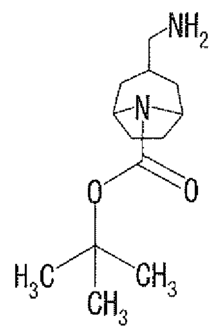 |
| 123 | 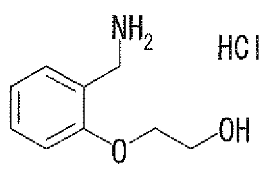 | 124 rel | 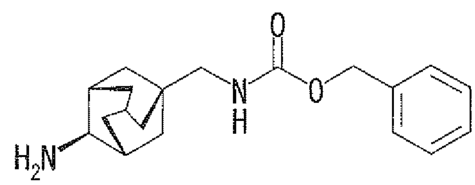 |
| 125 rel | 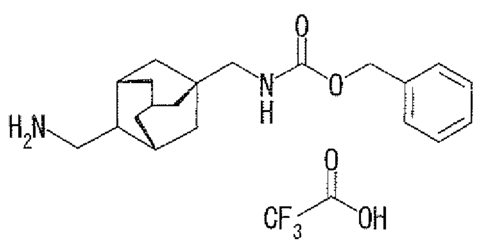 | 126 | 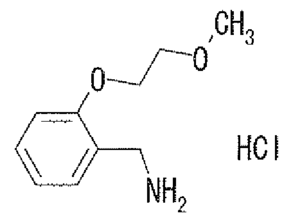 |
| 127 | 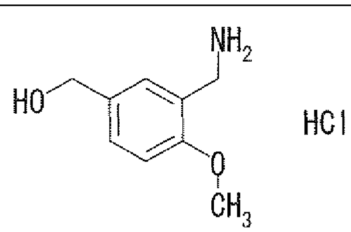 | 128 | 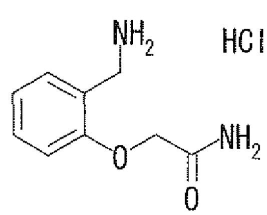 |

[Table 16]

| PEx | Str | PEx | Str |
|---|---|---|---|
| 129 | 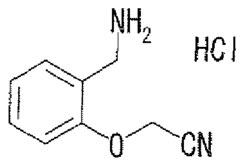 | 130 | 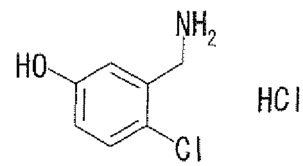 |
| 131 | 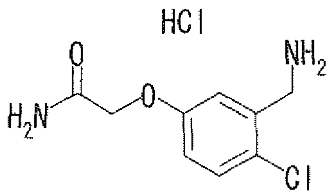 | 132 | 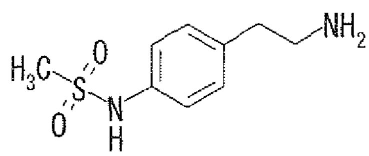 |
| 133 rel | 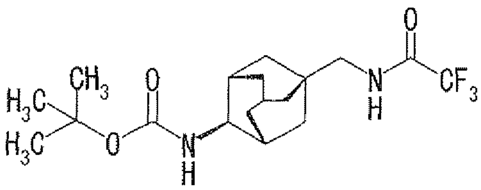 | 134 rel | 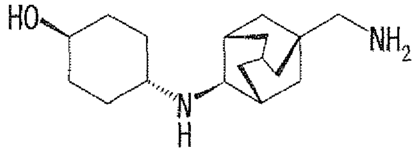 |
| 135 rel | 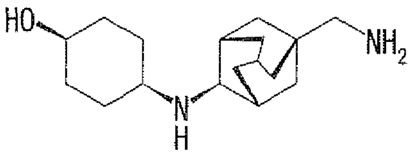 | 136 rel | 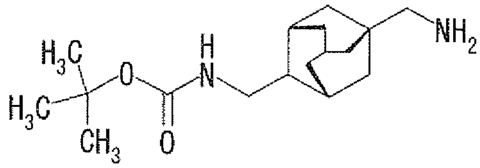 |
| 137 | 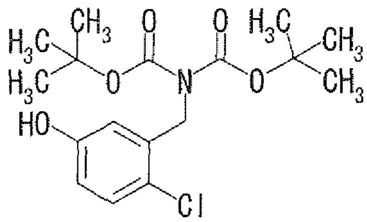 | 138 rel | 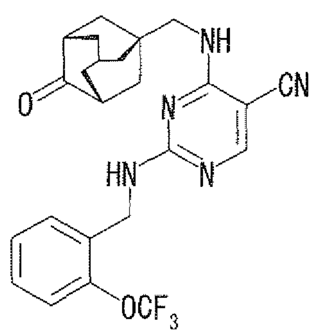 |
| 139 rel | 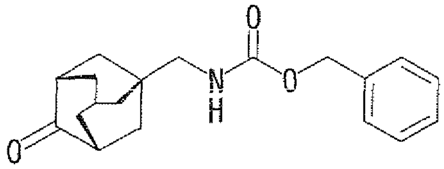 | 140 rel | 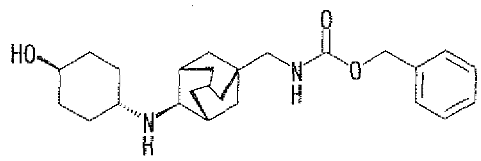 |
| 141 rel | 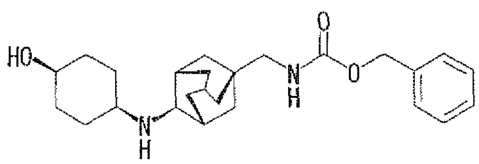 | 142 | 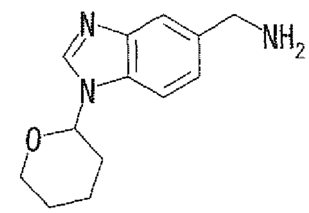 |

[Table 17]

| PEx | Str | PEx | Str |
|---|---|---|---|
| 143 rel | 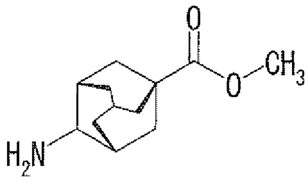 | 144 | 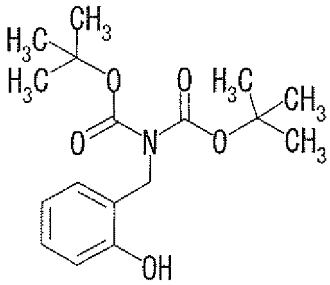 |
| 145 | 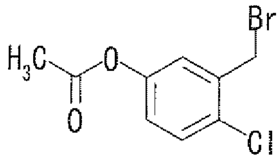 | 146 | 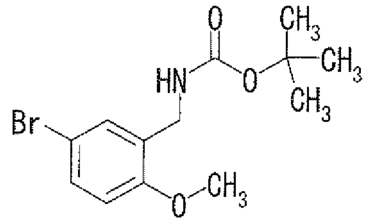 |
| 147 | 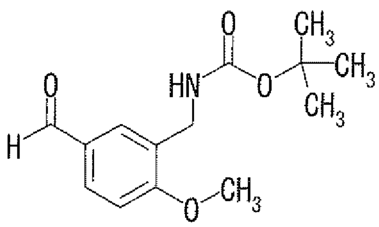 | 148 | 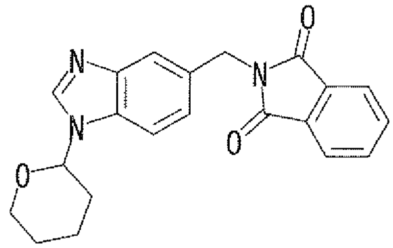 |
| 149 | 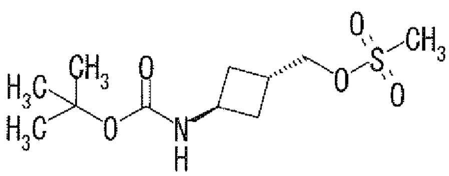 | 150 | 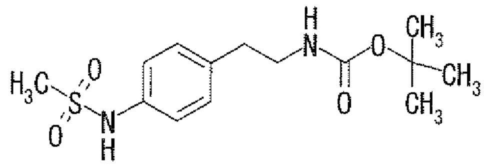 |
| 151 | 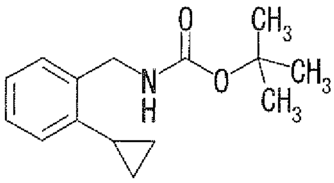 | 152 rel | 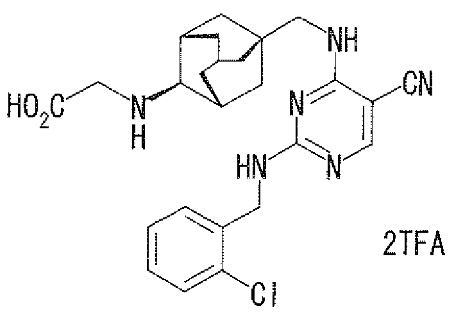 2TFA |
| 153 | 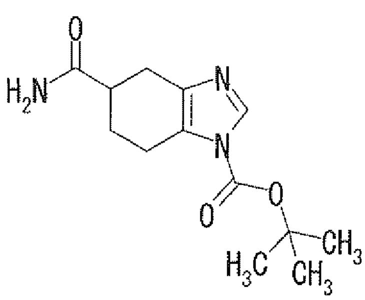 | 154 | 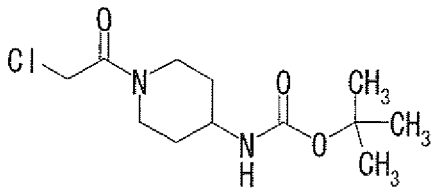 |

[Table 18]

| PEx | Str | PEx | Str |
|---|---|---|---|
| 155 | 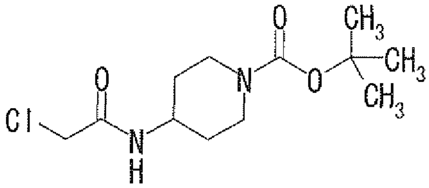 | 156 | 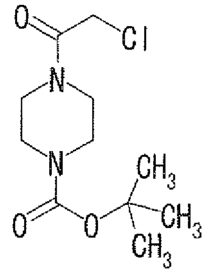 |
| 157 | 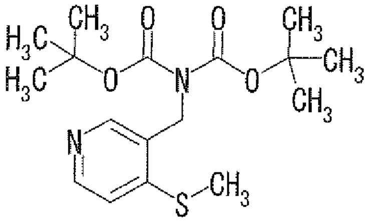 | 158 | 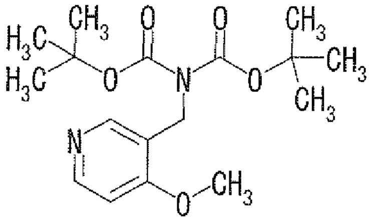 |
| 159 | 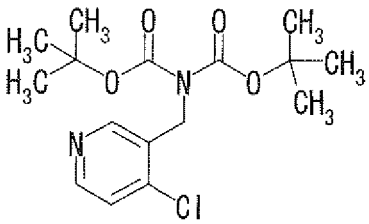 | 160 | 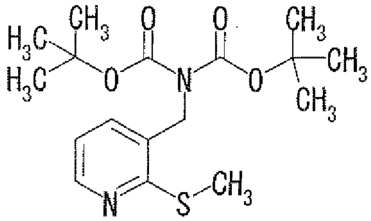 |
| 161 rel | 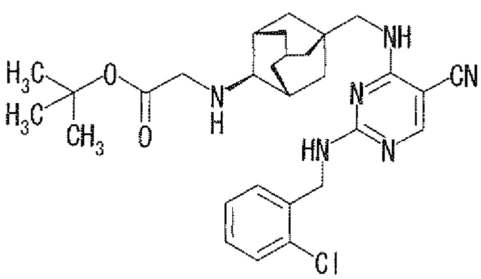 | 162 rel | 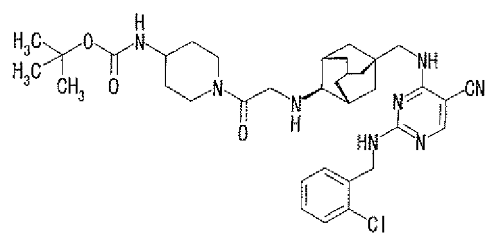 |
| 163 rel | 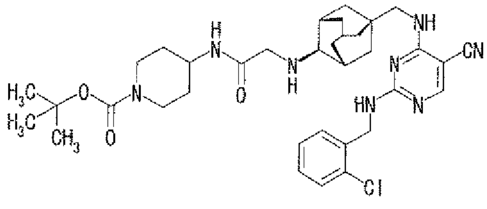 | 164 rel | 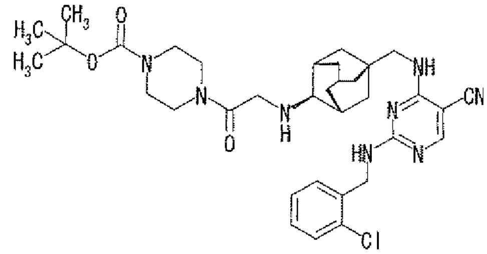 |
| 165 | 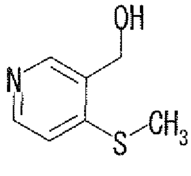 | 166 rel | 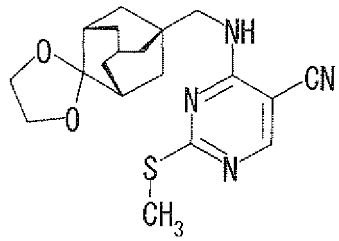 |

[Table 19]

| PEx | Str | PEx | Str |
|---|---|---|---|
| 167 | 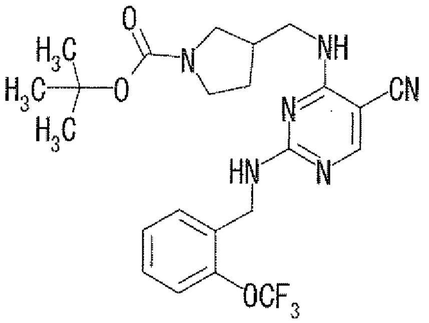 | 168 rel | 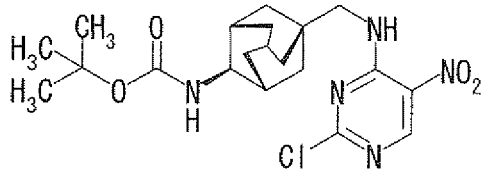 |
| 169 rel | 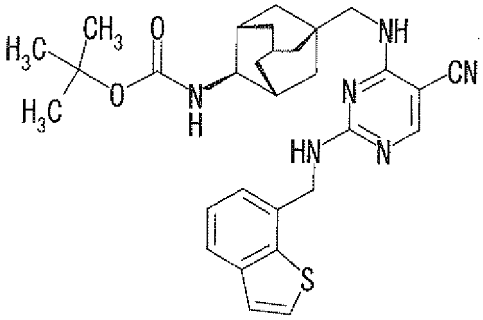 | 170 rel | 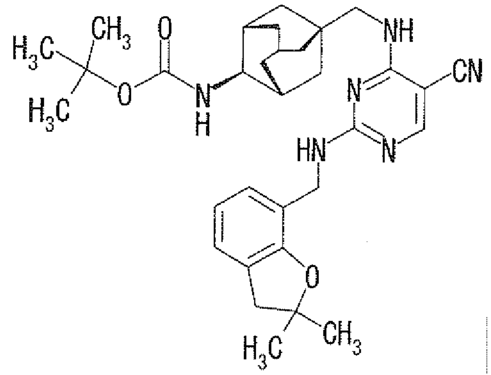 |
| 171 rel | 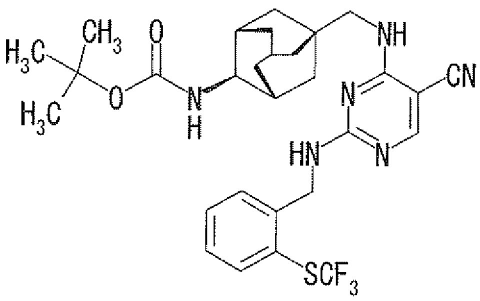 | 172 rel | 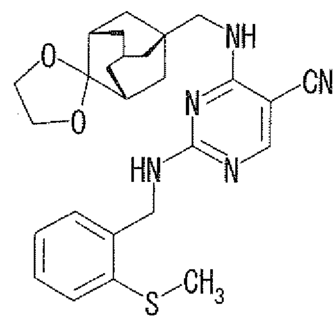 |
| 173 rel | 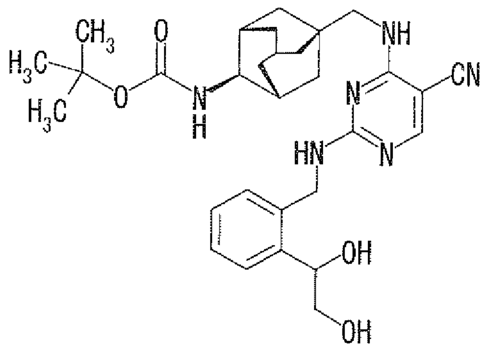 | 174 rel | 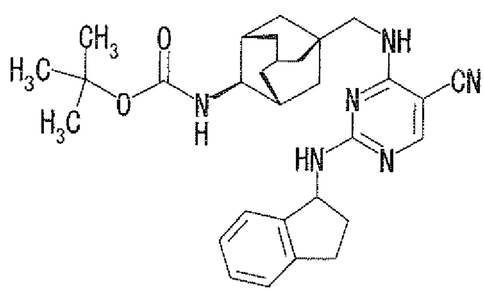 |

[Table 20]

| PEx | Str | PEx | Str |
|---|---|---|---|
| 175 rel | 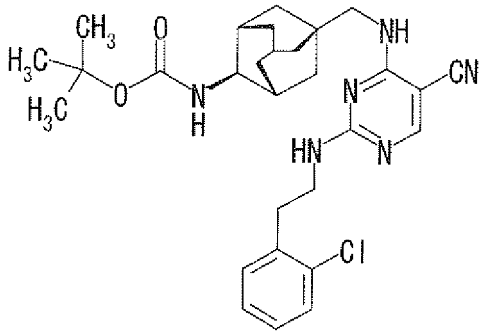 | 176 rel | 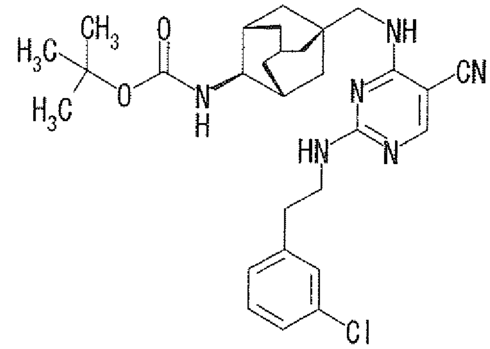 |
| 177 rel | 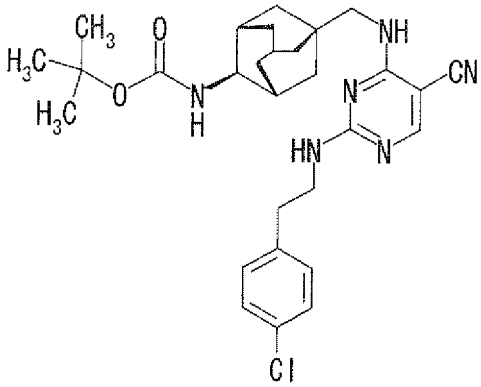 | 178 rel | 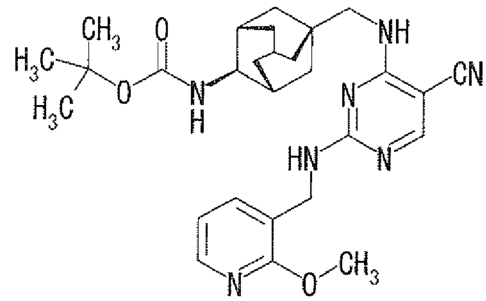 |
| 179 rel | 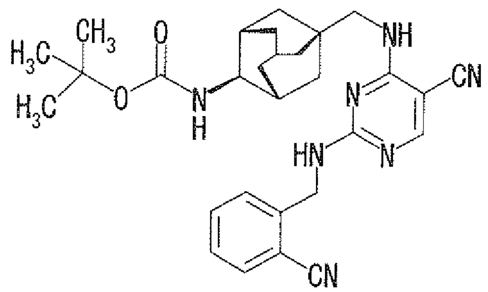 | 180 rel | 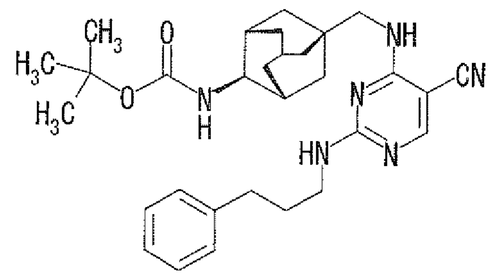 |
| 181 rel | 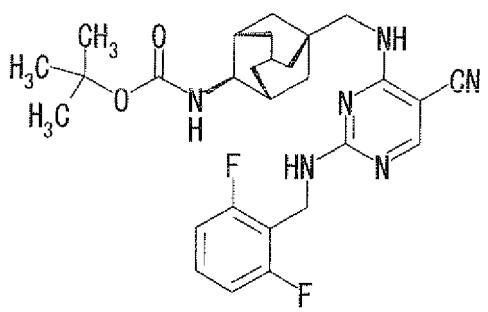 | 182 rel | 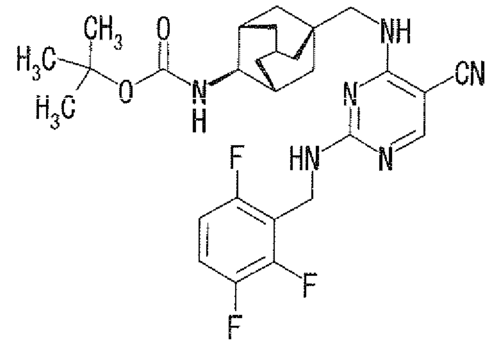 |

[Table 21]

| PEx | Str | PEx | Str |
|---|---|---|---|
| 183 rel | 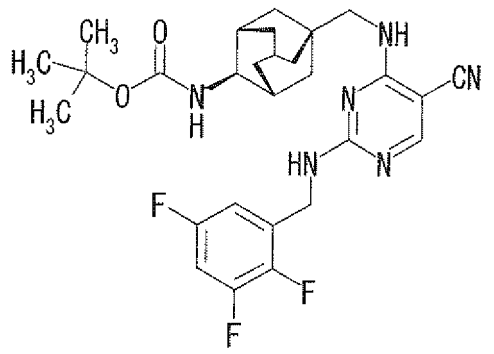 | 184 rel | 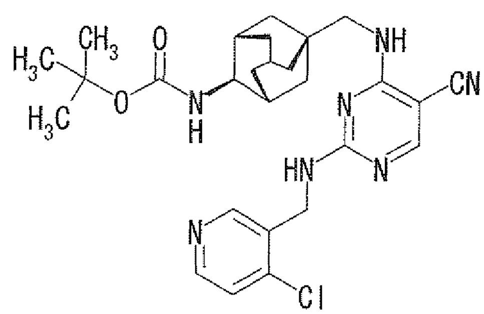 |
| 185 rel | 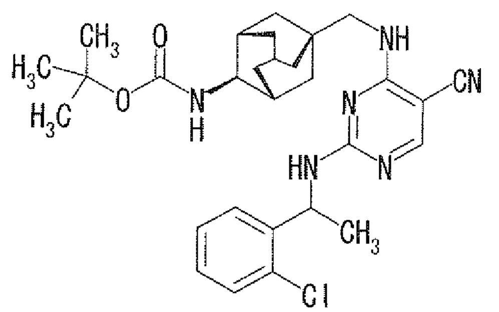 | 186 rel | 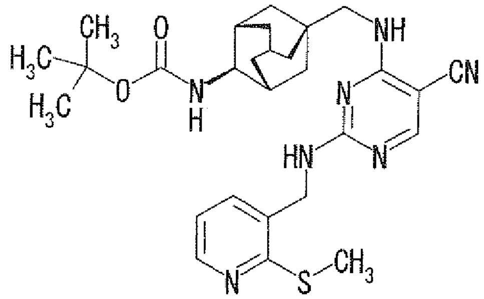 |
| 187 rel | 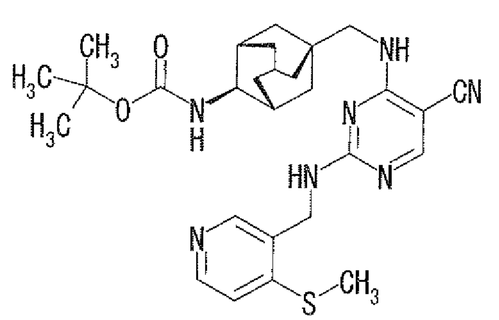 | 188 rel | 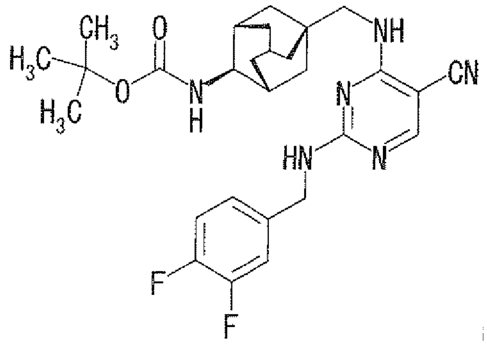 |
| 189 rel | 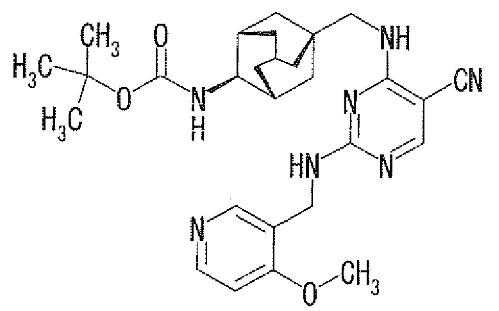 | 190 rel | 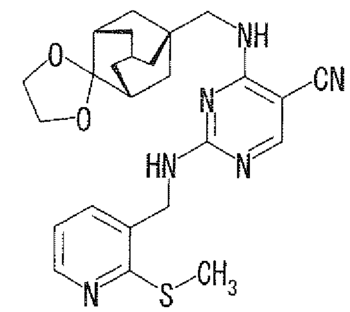 |

[Table 22]

| PEx | Str | PEx | Str |
|---|---|---|---|
| 191 rel | 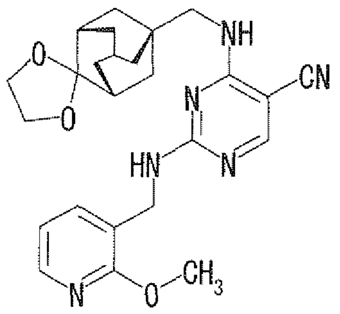 | 192 rel | 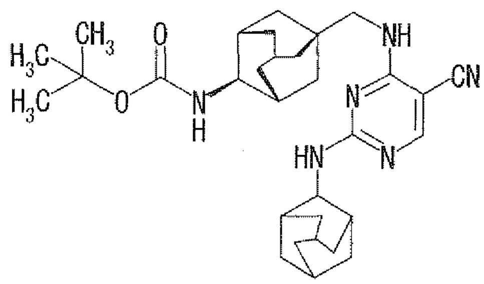 |
| 193 rel | 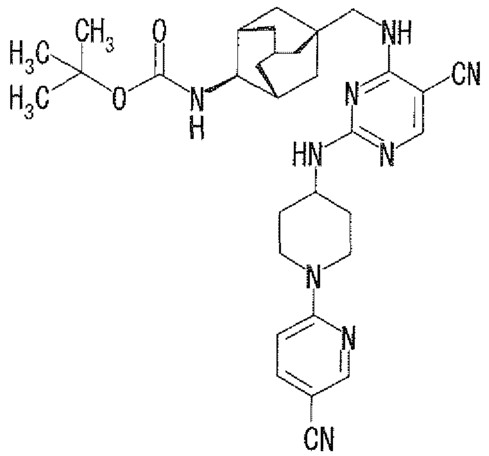 | 194 rel | 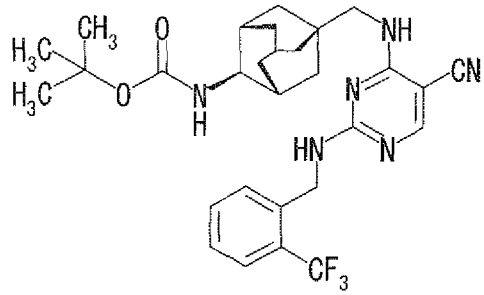 |
| 195 rel | 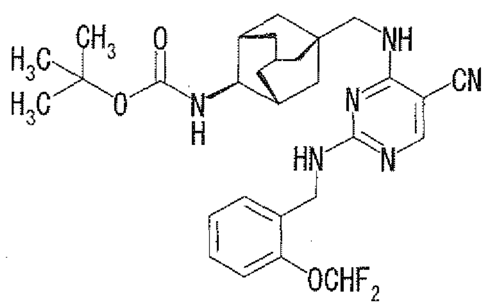 | 196 rel | 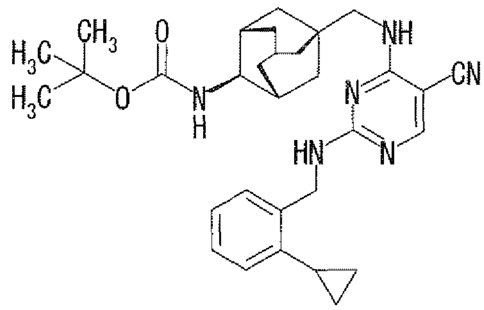 |
| 197 rel | 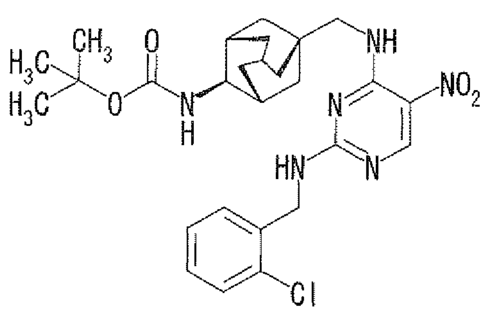 | 198 rel | 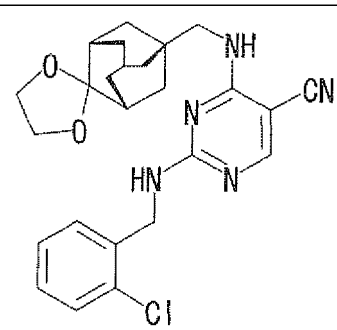 |

[Table 23]

| PEx | Str | PEx | Str |
|---|---|---|---|
| 199 rel | 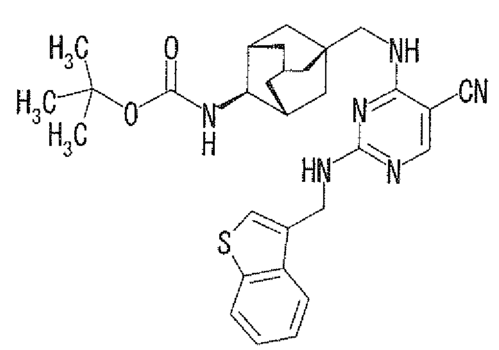 | 200 rel | 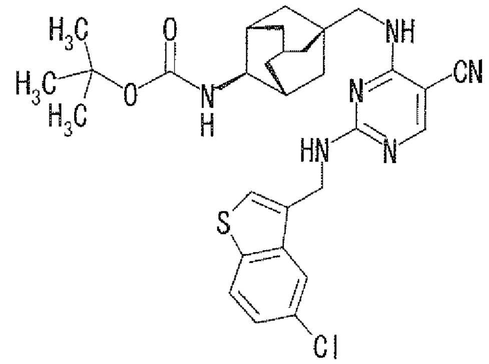 |
| 201 rel | 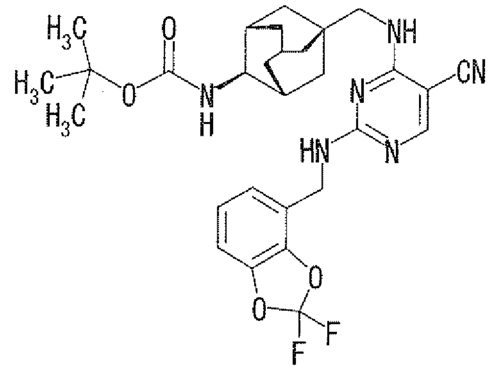 | 202 | 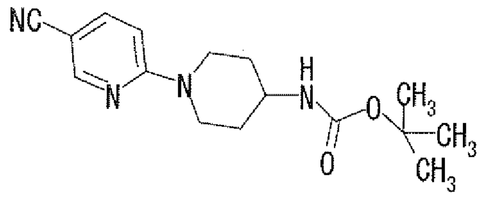 |
| 203 rel | 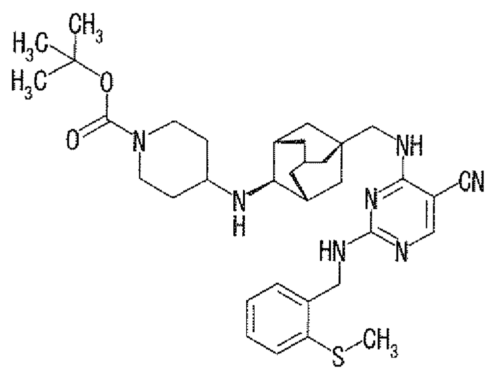 | 204 rel | 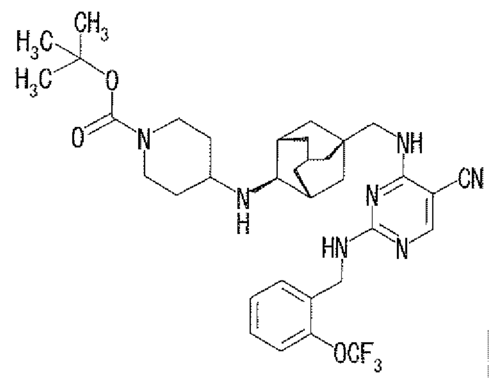 |
| 205 rel | 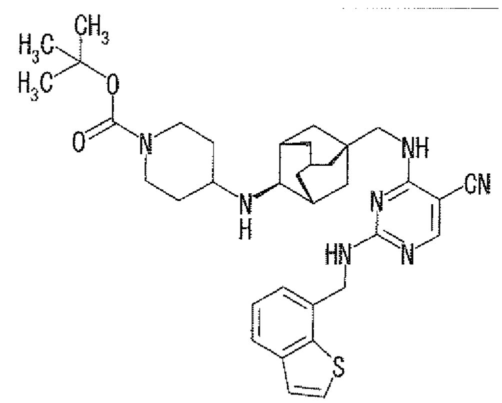 | 206 rel | 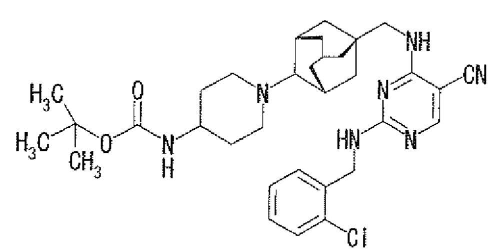 |

[Table 24]

| PEx | Str | PEx | Str |
|---|---|---|---|
| 207 rel | 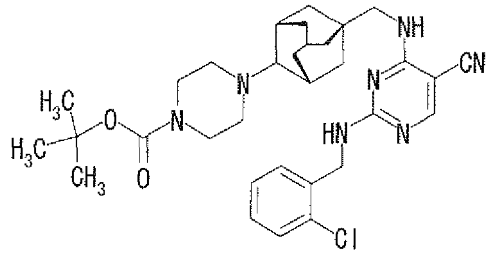 | 208 rel | 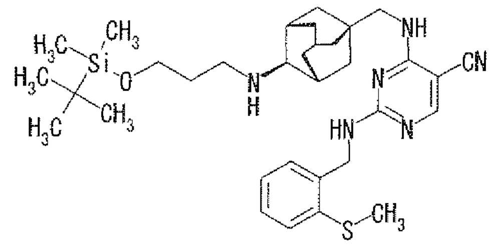 |
| 209 rel | 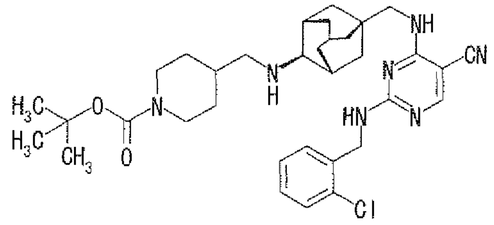 | 210 rel | 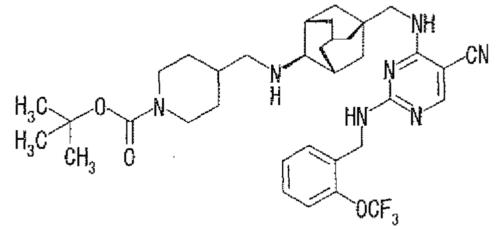 |
| 211 rel | 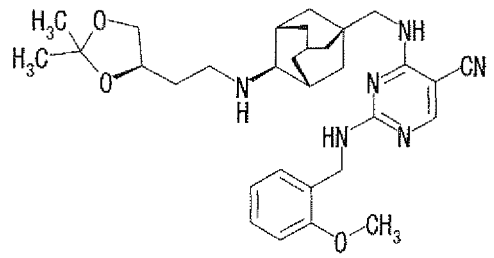 | 212 rel | 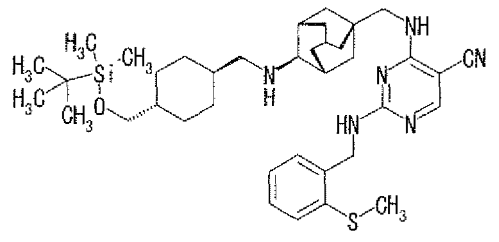 |
| 213 rel | 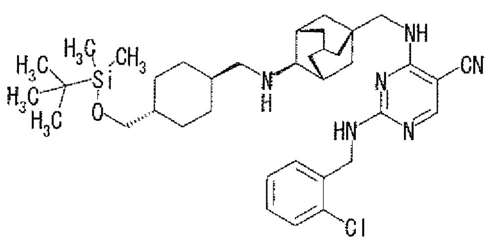 | 214 rel | 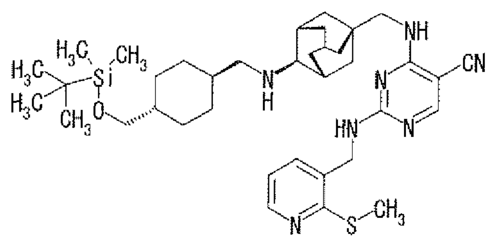 |
| 215 rel | 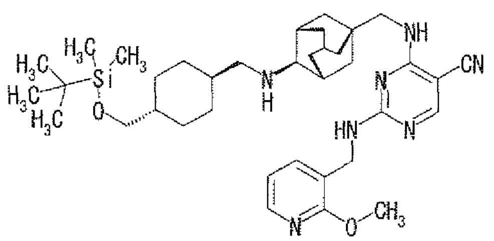 | 216 rel | 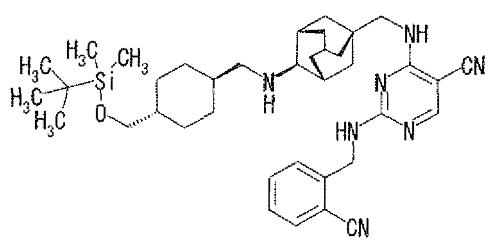 |
| 217 rel | 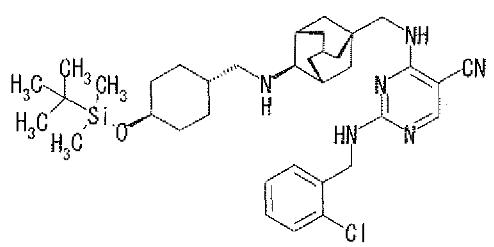 | 218 rel | 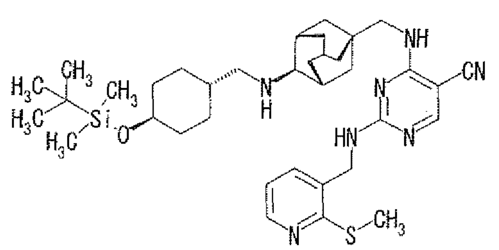 |

[Table 25]

| PEx | Str | PEx | Str |
|---|---|---|---|
| 219 rel | 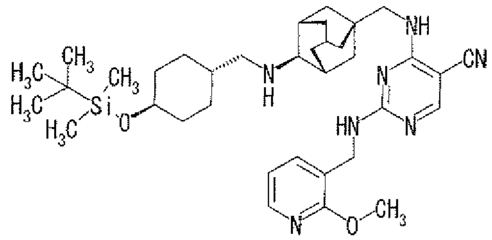 | 220 rel | 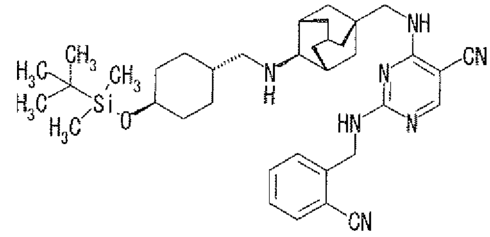 |
| 221 rel | 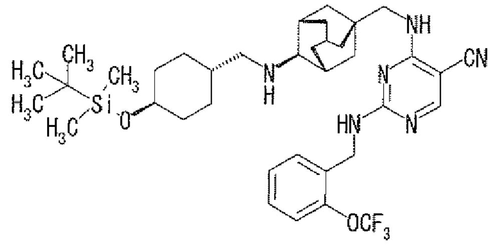 | 222 rel | 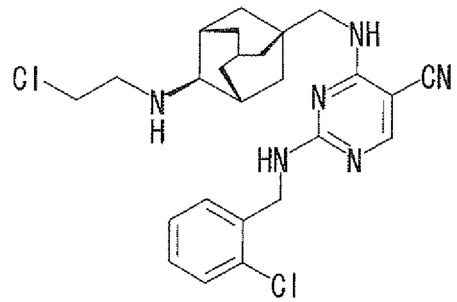 |
| 223 rel | 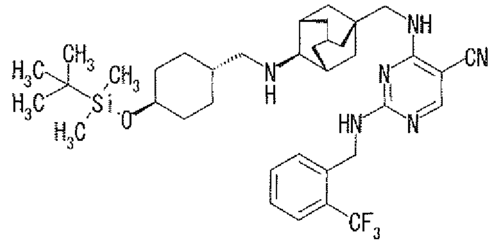 | 224 rel | 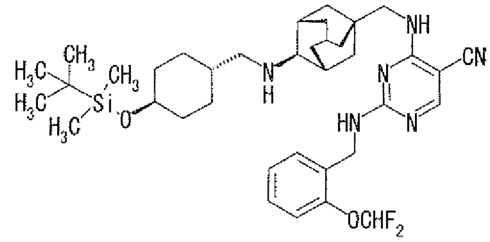 |
| 225 rel | 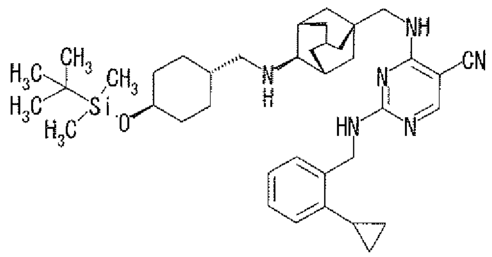 | 226 rel | 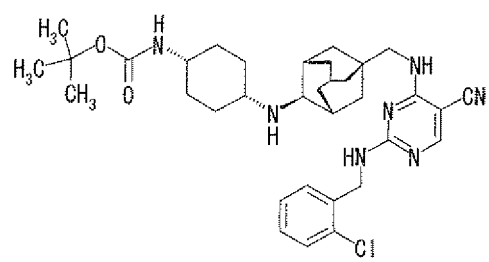 |
| 227 rel | 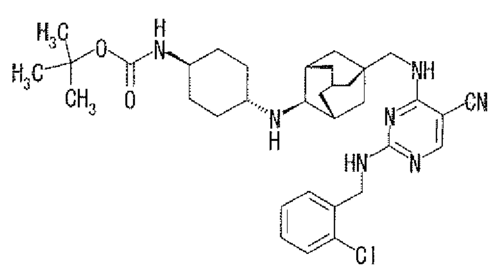 | 228 rel | 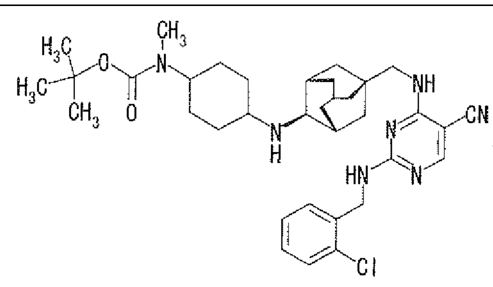 isomer A |

[Table 26]

| PEx | Str | PEx | Str |
|---|---|---|---|
| 229 rel | 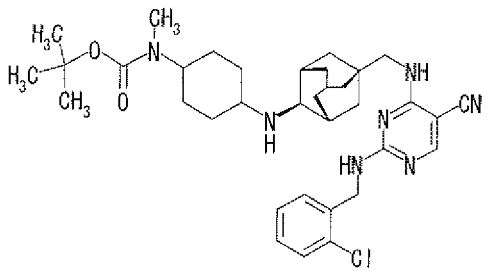 isomer B | 230 rel | 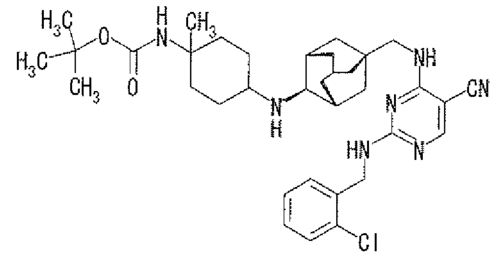 |
| 231 rel | 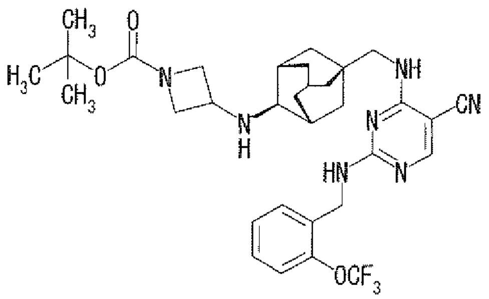 | 232 rel | 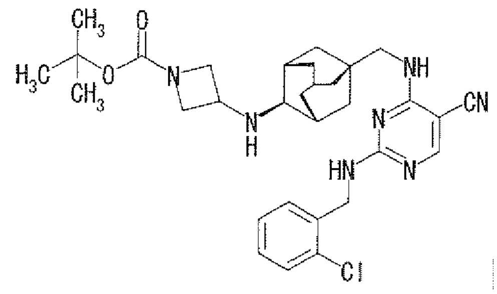 |
| 233 rel | 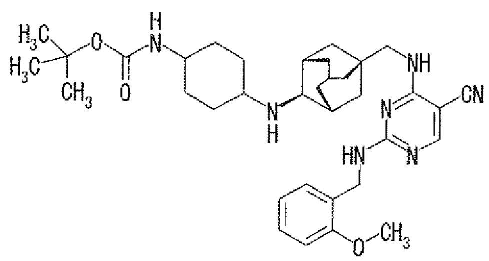 isomer A | 234 rel | 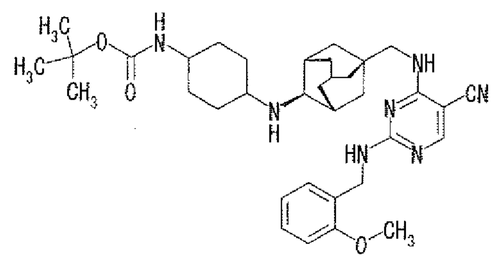 isomer B |
| 235 rel | 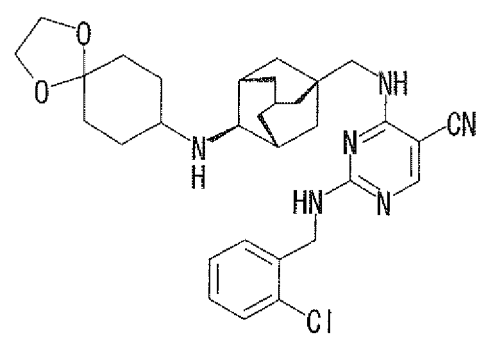 | 236 rel | 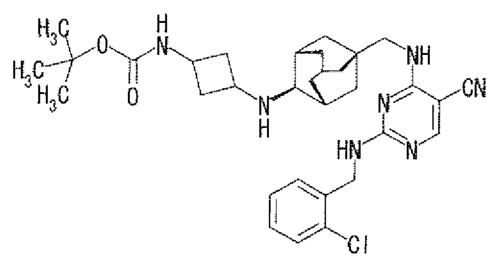 |
| 237 rel | 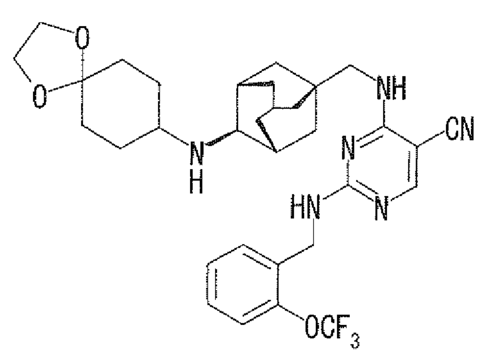 | 238 rel | 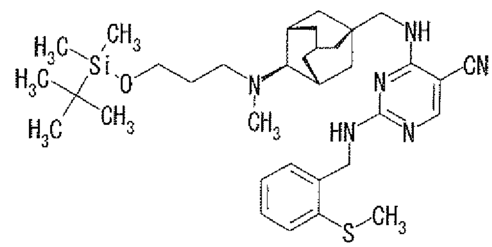 |

[Table 27]

| PEx | Str | PEx | Str |
|---|---|---|---|
| 239 rel | 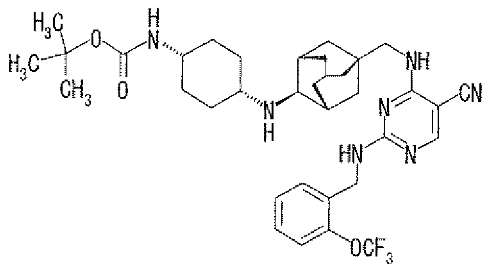 | 240 rel | 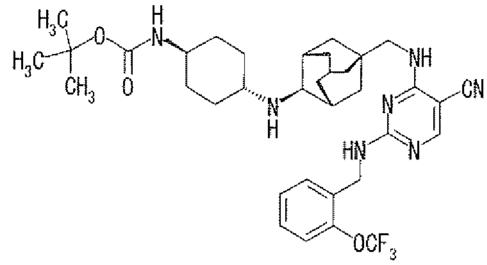 |
| 241 | 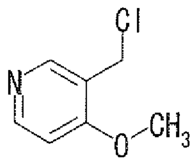 | 242 | 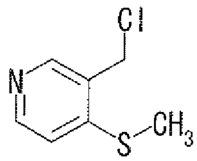 |
| 243 rel | 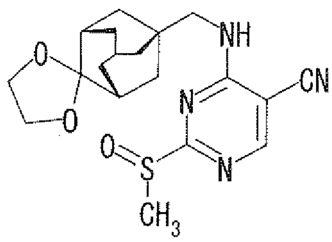 | 244 rel | 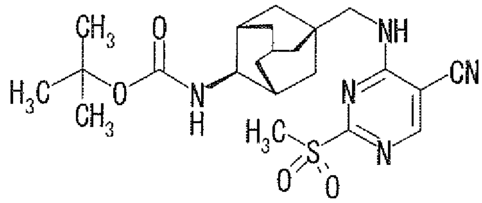 |
| 245 | 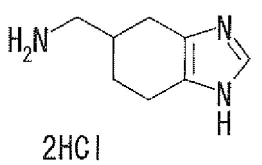 | 246 | 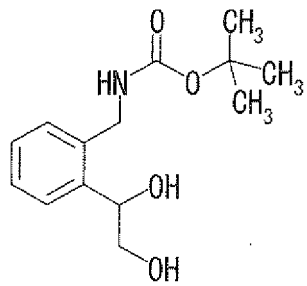 |
| 247 | 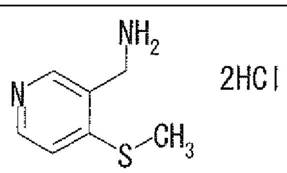 | 248 | 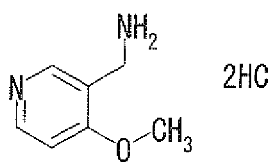 |
| 249 | 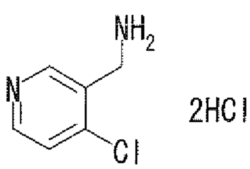 | 250 | 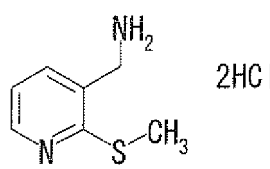 |
| 251 | 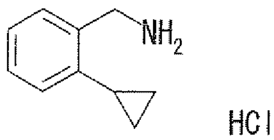 | 252 | 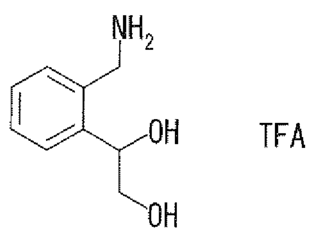 |

[Table 28]

| PEx | Str | PEx | Str |
|---|---|---|---|
| 253 | 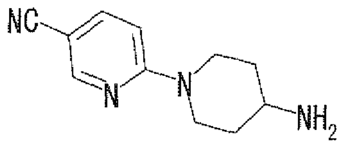 | 254 rel | 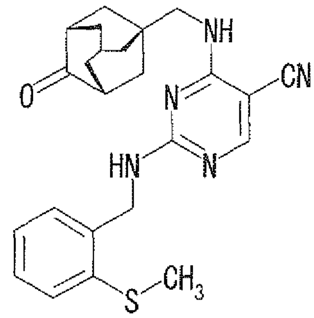 |
| 255 rel | 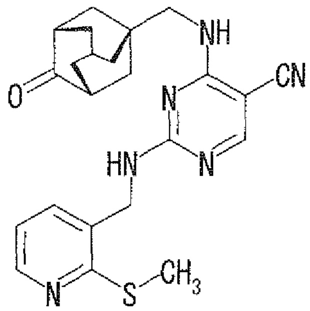 | 256 rel | 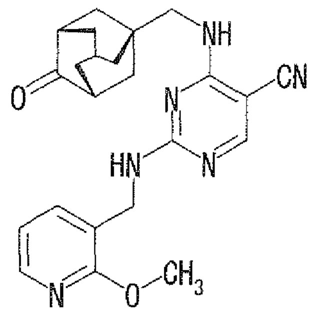 |
| 257 rel | 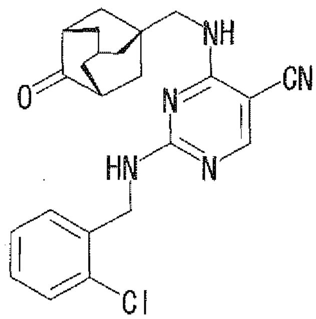 | 258 rel | 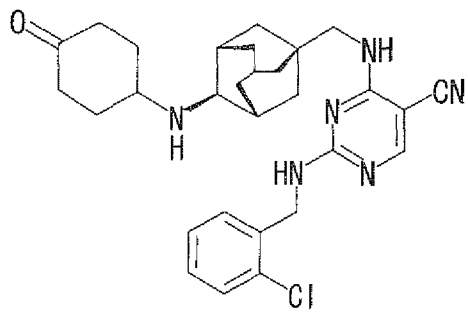 |
| 259 rel | 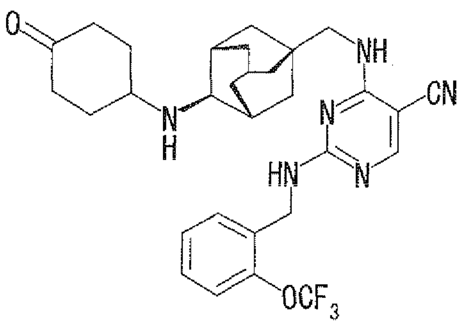 | 260 | 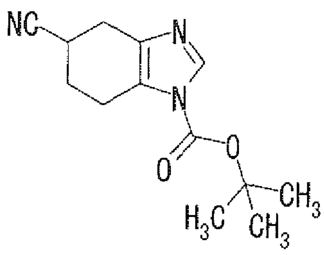 |
| 261 | 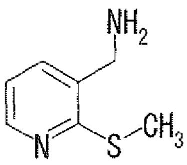 | 262 rel | 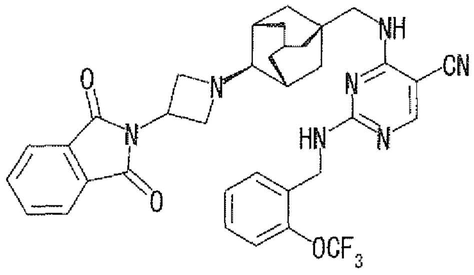 |

[Table 29]

| PEx | Str | PEx | Str |
|---|---|---|---|
| 263 rel | 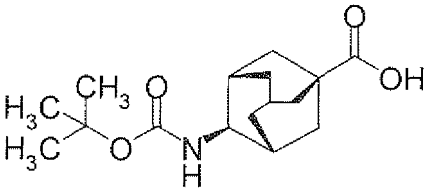 | 264 | 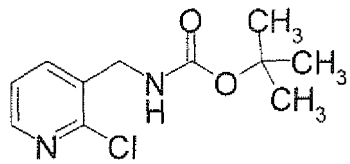 |
| 265 | 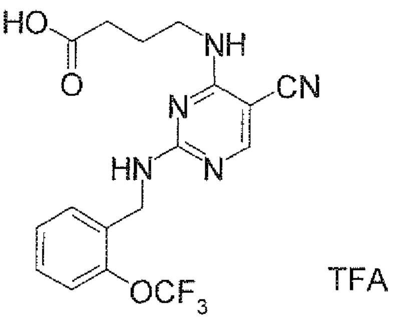 | 266 | 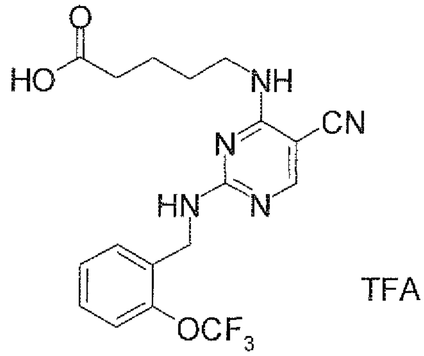 |
| 267 | 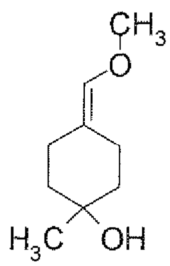 | 268 rel | 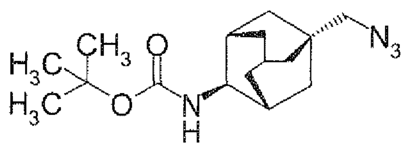 |
| 269 rel | 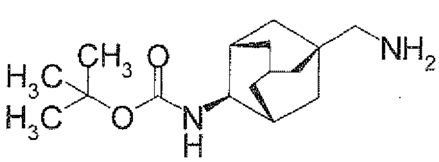 | 270 | 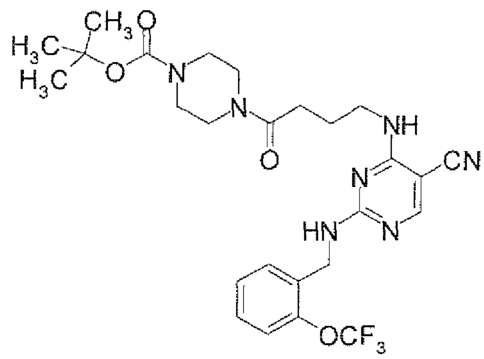 |
| 271 | 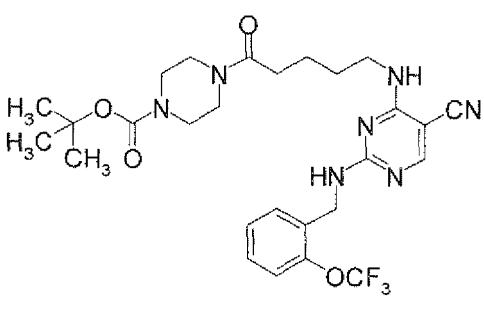 | 272 | 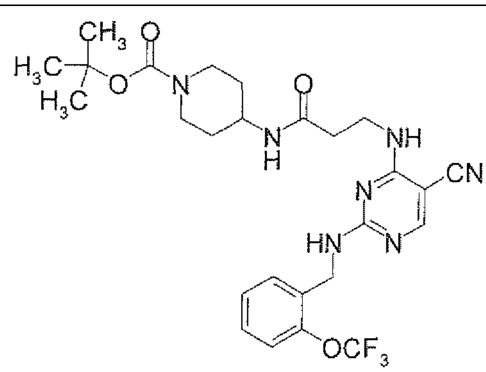 |

[Table 30]

| PEx | Str | PEx | Str |
|---|---|---|---|
| 273 | 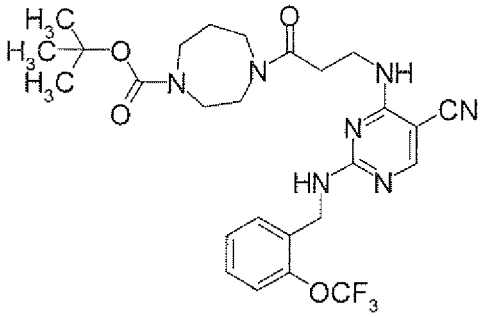 | 274 | 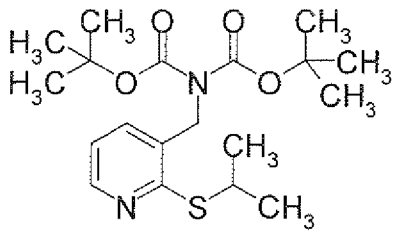 |
| 275 | 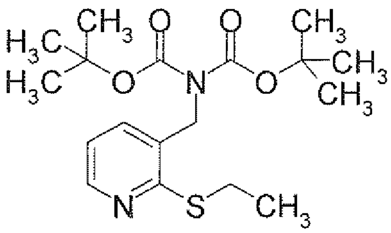 | 276 rel | 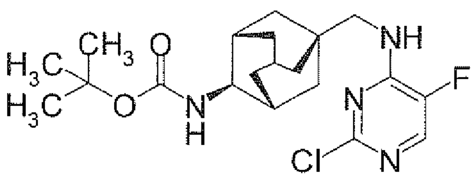 |
| 277 | 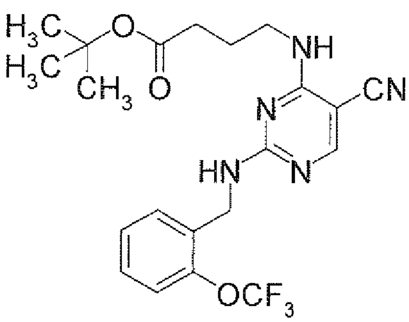 | 278 | 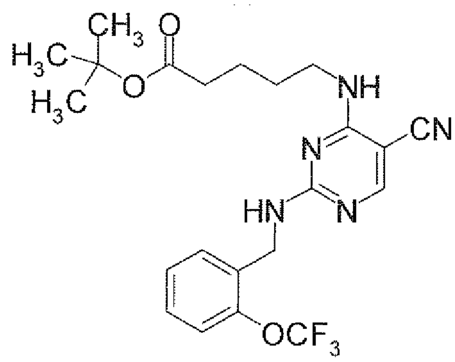 |
| 279 rel | 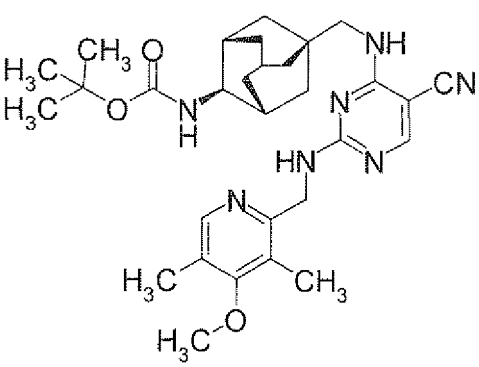 | 280 rel | 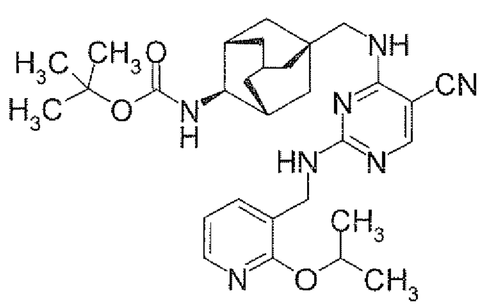 |

[Table 31]

| PEx | Str | PEx | Str |
|---|---|---|---|
| 281 rel | 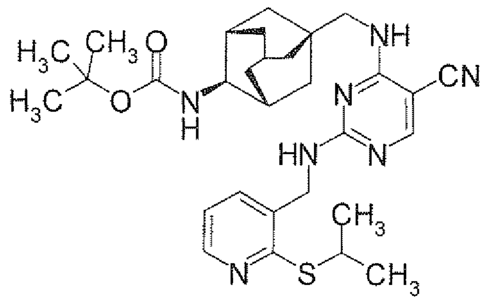 | 282 rel | 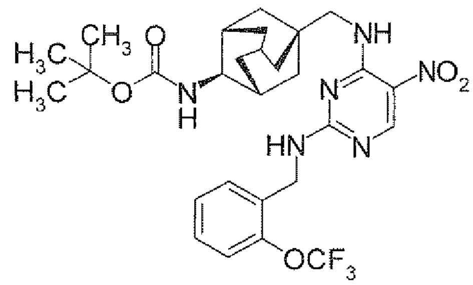 |
| 283 rel | 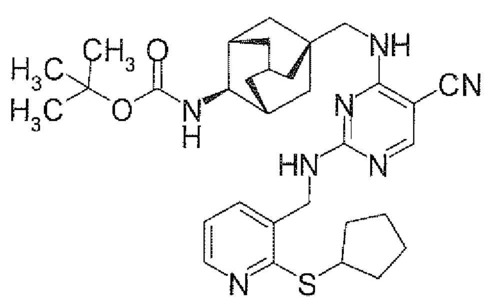 | 284 rel | 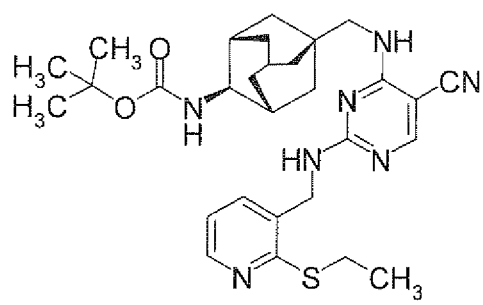 |
| 285 rel | 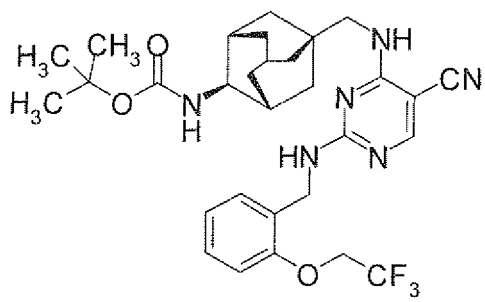 | 286 rel | 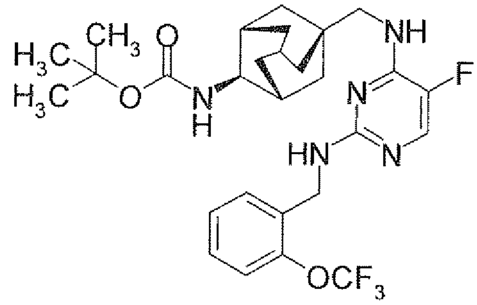 |
| 287 rel | 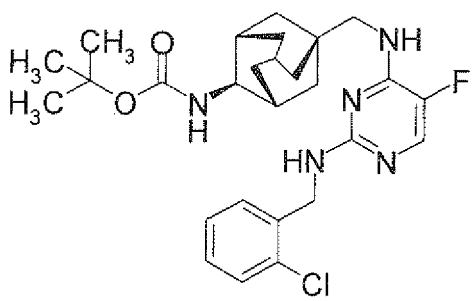 | 288 | 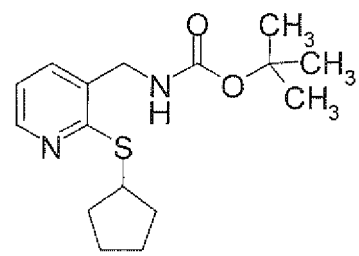 |
| 289 rel | 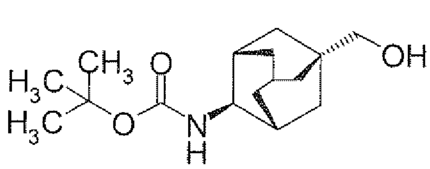 | 290 | 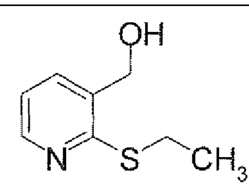 |

[Table 32]

| PEx | Str | PEx | Str |
|---|---|---|---|
| 291 | 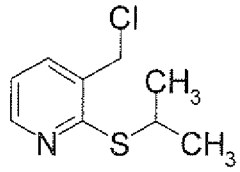 HCl | 292 | 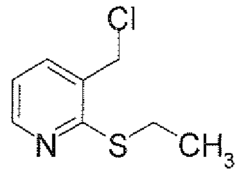 HCl |
| 293 rel | 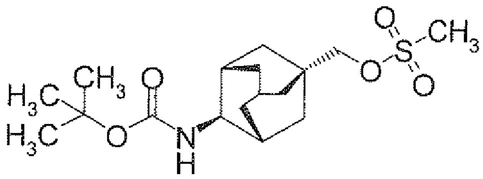 | 294 rel | 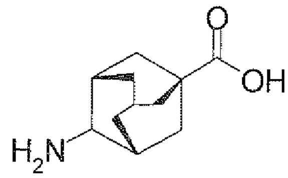 |
| 295 rel | 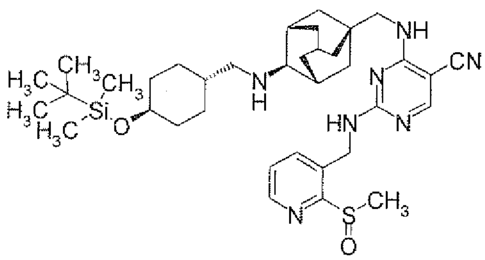 | 296 rel | 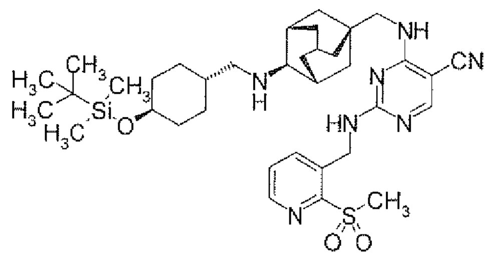 |
| 297 rel | 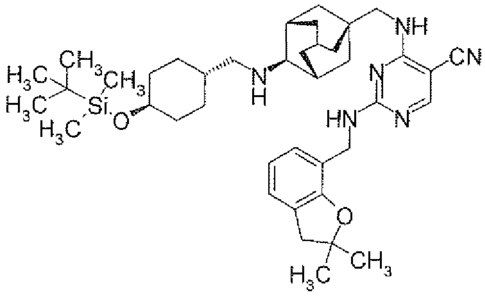 | 298 rel | 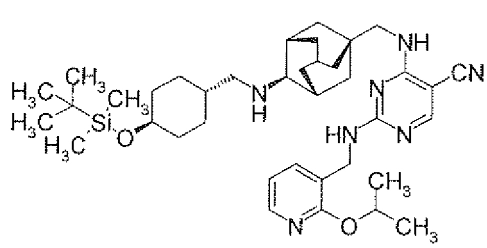 |
| 299 rel | 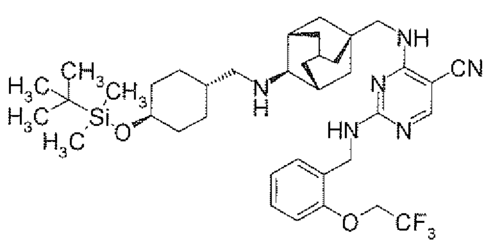 | 300 rel | 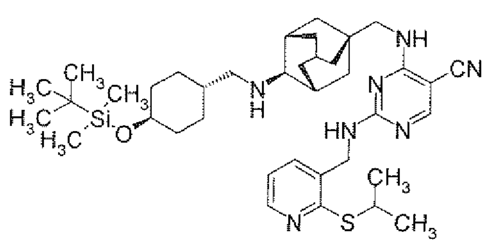 |

[Table 33]

| PEx | Str | PEx | Str |
|---|---|---|---|
| 301 rel | 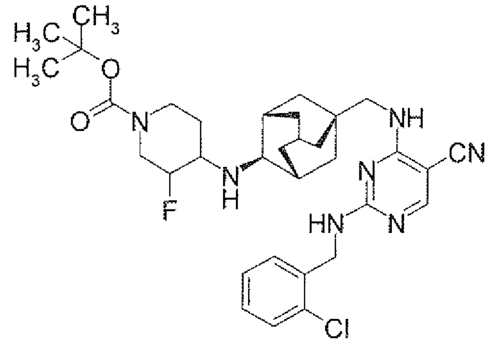 | 302 rel | 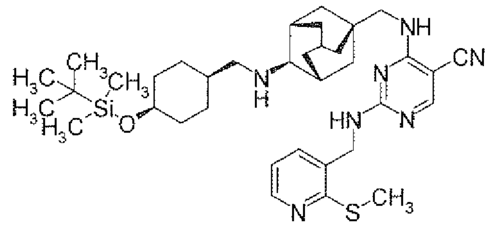 |
| 303 rel | 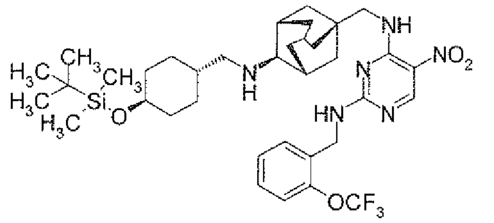 | 304 rel | 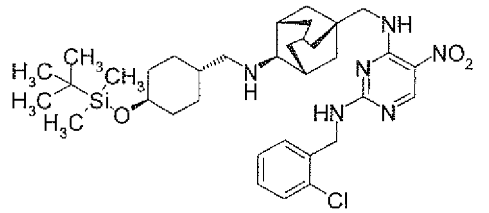 |
| 305 rel | 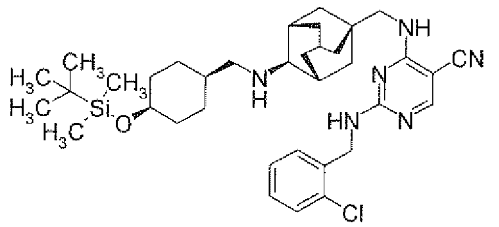 | 306 rel | 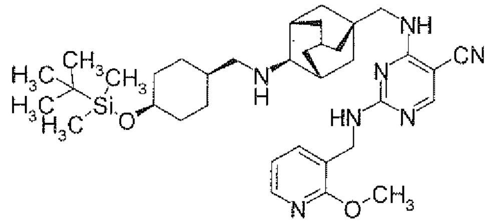 |
| 307 rel | 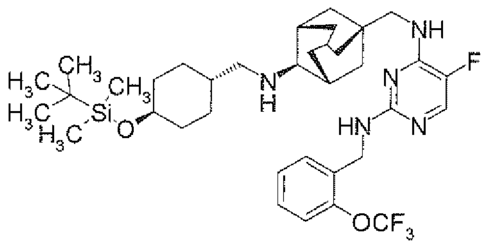 | 308 rel | 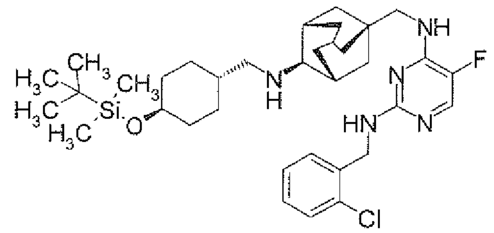 |
| 309 rel | 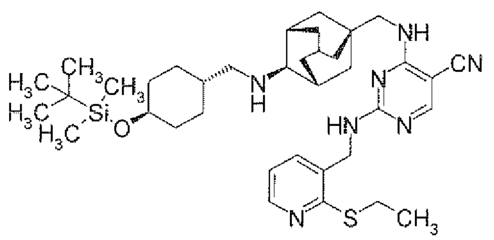 | 310 rel | 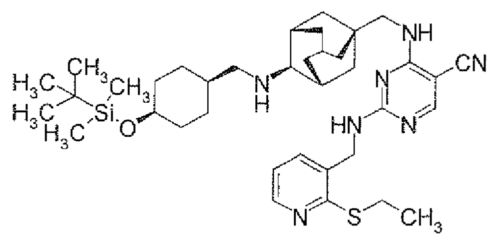 |
| 311 rel | 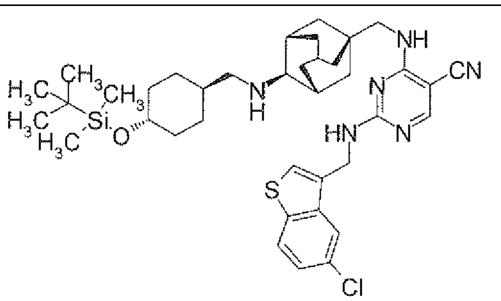 | 312 rel | 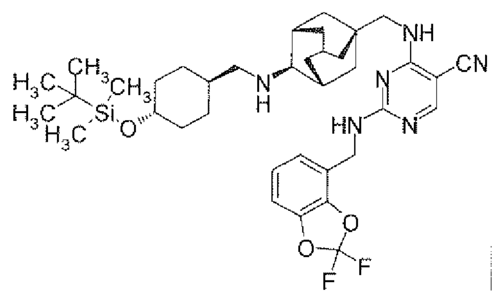 |

[Table 34]

| PEx | Str | PEx | Str |
|---|---|---|---|
| 313 rel | 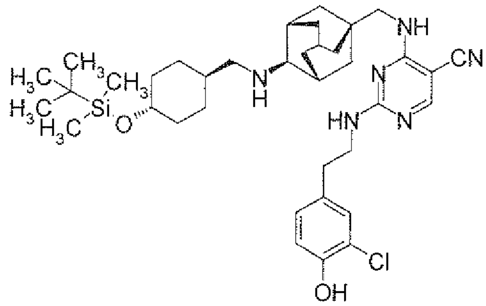 | 314 rel | 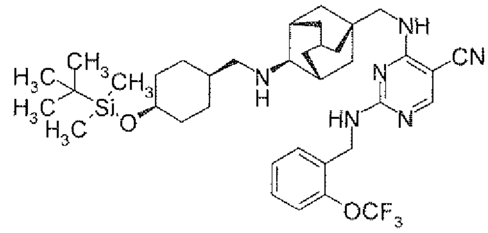 |
| 315 rel | 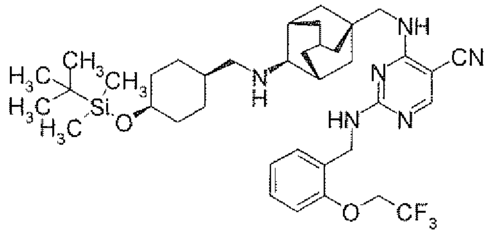 | 316 rel | 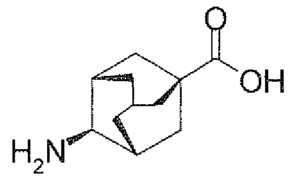 |
| 317 rel | 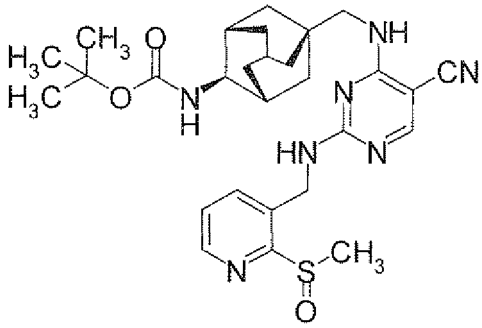 | 318 rel | 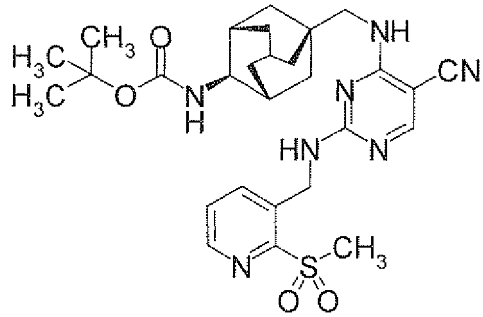 |
| 319 | 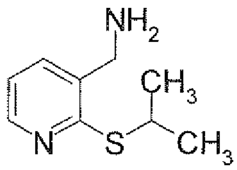 2HCl | 320 | 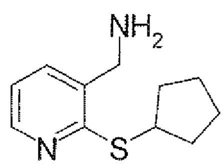 2HCl |
| 321 | 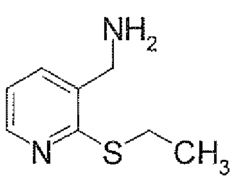 2HCl | 322 | 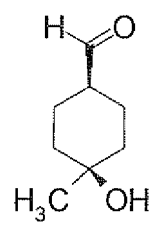 |
| 323 | 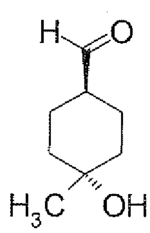 | | |

[Table 351

| PEx | Syn | Dat | PEx | Syn | Dat |
|---|---|---|---|---|---|
| 1 | P1 | ESI+:451 | 2 | P2 | ESI+: 384 |
| 3 | P3 | ESI+: 594 | 4 | P4 | ESI+: 368 |
| 5 | P4 | ESI+: 382 | 6 | P6 | ESI+:283 |
| 7 | P7 | ESI+: 437 | 8 | P7 | ESI+: 344 |
| 9 | P9 | East+: 358 | 10 | P10 | ESI+: 249 |
| 11 | P11 | ESI+: 223 | 12 | P12 | ESI+: 510 |
| 13 | P12 | ESI+: 536 | 14 | P12 | ESI+: 536 |
| 15 | P12 | ESI+: 572 | 16 | P12 | ESI+: 572 |
| 17 | P12 | ESI+: 572 | 18 | P12 | ESI+: 558 |
| 19 | P19 | ESI+: 411 | 20 | P20 | ESI+: 351 |
| 21 | P21 | ESI+: 351 | 22 | P20 | ESI+: 238 |
| 23 | P23 | ESI+: 281 | 24 | P24 | ESI+: 315 |
| 25 | P24 | ESI+: 315 | 26 | P24 | ESI+: 224 |
| 27 | P27 | ESI+: 422, 424 | 28 | P28 | FAB+: 414 |
| 29 | P29 | ESI+: 559 | 30 | P29 | ESI+: 559 |
| 31 | P31 | ESI+: 403 | 32 | P31 | ESI+: 385 |
| 33 | P31 | ESI+: 404 | 34 | P31 | ESI+: 434 |
| 35 | P31 | ESI+: 437, 439 | 36 | P36 | ESI+: 290 |
| 37 | P37 | ESI+: 430 | 38 | P37 | ESI+: 529 |
| 39 | P37 | ESI+: 555 | 40 | P37 | ESI+: 607 |
| 41 | P37 | ESI+: 607 | 42 | P37 | ESI+: 424 |
| 43 | P37 | ESI+:516 | 44 | P37 | ESI+:507 |
| 45 | P37 | ESI+: 507 | 46 | P37 | ESI+: 464 |
| 47 | P37 | ESI+: 474, 476 | 48 | P37 | ESI+: 613, 615,617 |
| 49 | P37 | ESI+: 621 | 50 | P37 | ESI+: 587 |
| 51 | P37 | ESI+: 460 | 52 | P37 | ESI+: 524 |
| 53 | P37 | ESI+: 493 | 54 | P54 | ESI+: 313, 315 |
| 55 | P54 | ESI+: 531 | 56 | P54 | ESI+: 519 |
| 57 | P54 | ESI+: 597 | 58 | P54 | ESI+: 503 |
| 59 | P54 | ESI+: 519 | 60 | P54 | ESI+: 490 |
| 61 | P54 | ESI+: 490 | 62 | P54 | ESI+: 490 |
| 63 | P54 | ESI+: 523, 525 | 64 | P54 | ESI+: 523, 525 |
| 65 | P54 | ESI+: 523, 525 | 66 | P54 | ESI+: 567, 569 |
| 67 | P54 | ESI+: 529 | 68 | P54 | ESI+: 515 |
| 69 | P54 | ESI+: 505 | 70 | P54 | ESI+: 589,591 |
| 71 | P54 | ESI+: 549 | 72 | P54 | ESI+: 579, 581, 583 |
| 73 | P54 | ESI+: 579, 581,583 | 74 | P54 | ESI+: 535 |

[Table 36]

| PEx | Syn | Dat | PEx | Syn | Dat |
|---|---|---|---|---|---|
| 75 | P54 | no data | 76 | P54 | ESI+: 557, 559, 561 |
| 77 | P54 | ESI+: 519 | 78 | P54 | ESI+: 553, 555 |
| 79 | P54 | ESI+: 596 | 80 | P54 | ESI+: 549 |
| 81 | P54 | ESI+: 524, 526 | 82 | P54 | ESI+: 579 |
| 83 | P54 | ESI+: 351, 353 | 84 | P54 | no data |
| 85 | P54 | no data | 86 | P54 | ESI+: 571 |
| 87 | P54 | ESI+: 584 | 88 | P54 | ESI+: 571 |
| 89 | P54 | ESI+: 575, 577 | 90 | P54 | ESI+: 566 |
| 91 | P54 | ESI+: 563 | 92 | P54 | ESI+: 541 |
| 93 | P54 | ESI+: 541 | 94 | P54 | ESI+: 571 |
| 95 | P54 | ESI+: 555 | 96 | P54 | ESI+: 561, 563 |
| 97 | P54 | ESI+: 618, 620 | 98 | P54 | ESI+: 301, 303 |
| 99 | P54 | ESI+: 297, 299 | 100 | P100 | ESI+: 372, 374 |
| 101 | P101 | ESI+: 372, 374 | 102 | P100 | ESI+: 650 |
| 103 | P100 | ESI+: 650 | 104 | P104 | ESI+: 587,589 |
| 105 | P105 | ESI+:380 | 106 | P106 | ESI+: 352 |
| 107 | P107 | ESI+: 255 | 108 | P108 | ESI+: 408 |
| 109 | P109 | ESI+: 300 | 110 | P109 | ESI+: 661 |
| 111 | P109 | ESI+: 677 | 112 | P112 | ESI+: 527 |
| 113 | P113 | ESI+: 527 | 114 | P112 | ESI+: 649 |
| 115 | P112 | ESI+: 611 | 116 | P112 | ESI+: 606, 608 |
| 117 | P117 | ESI+:468 | 118 | P117 | ESI+: 480 |
| 119 | P117 | ESI+: 496 | 120 | P120 | ESI+: 551 |
| 121 | P121 | ESI+: 589 | 122 | P122 | no data |
| 123 | P123 | ESI+: 168 | 124 | P124 | ESI+: 315 |
| 125 | P124 | ESI+: 329 | 126 | P126 | ESI+: 182 |
| 127 | P126 | ESI+: 168 | 128 | P126 | ESI+: 181 |
| 129 | P126 | NMR1: 3.91-4.01(2 H, m), 5.23(2 H, s), 7.09-7.14(1H, m), 7.19-7.23(1H, m), 7.30-7.51 (2 H, m), 8.27-8.45(3 H, m) | 130 | P126 | ESI+: 158, 160 |
| 131 | P126 | ESI+: 215, 217 | 132 | P132 | ESI+: 215 |
| 133 | P133 | ESI-: 375 | 134 | P134 | ESI+: 279 |
| 135 | P134 | ESI+: 279 | 136 | P134 | ESI+: 295 |
| 137 | P137 | ESI+: 380, 382 | 138 | P138 | ESI+: 472 |
| 139 | P139 | ESI+: 314 | 140 | P140 | ESI+: 413 |

[Table 371

| PEx | Syn | Dat | PEx | Syn | Dat |
|---|---|---|---|---|---|
| 141 | P140 | ESI+: 413 | 142 | P142 | ESI+: 232 |
| 143 | P143 | EST+: 210 | 144 | P144 | FAB+: 324 |
| 145 | P145 | ESI+: 285, 287,289 | 146 | P146 | ESI+: 338, 340 |
| 147 | P147 | ESI+: 288 | 148 | P148 | ESI+: 362 |
| 149 | P149 | ESI+: 302 | 150 | P150 | ESI+: 337 |
| 151 | P3 | ESI+: 270 | 152 | P4 | ESI+: 481, 483 |
| 153 | P20, P21 | ESI+: 288 | 154 | P154 | ESI+: 299, 301 |
| 155 | P154 | ESI+: 299, 301 | 156 | P154 | ESI+: 285, 287 |
| 157 | P27 | ESI+: 355 | 158 | P27 | ESI+: 339 |
| 159 | P27 | ESI+: 365, 367 | 160 | P27 | ESI+: 377 |
| 161 | P29 | ESI+: 537, 539 | 162 | P29 | ESI+: 663, 665 |
| 163 | P29 | ESI+: 663, 665 | 164 | P29 | ESI+: 649, 651 |
| 165 | P37 | ESI+: 156 | 166 | P37 | ESI+: 373 |
| 167 | P37 | ESI+: 515 | 168 | P37 | ESI+: 460, 462 |
| 169 | P54 | ESI+: 545 | 170 | P54 | ESI+: 559 |
| 171 | P54 | ESI+: 611 | 172 | P54 | ESI+:478 |
| 173 | P54 | ESI+: 571 | 174 | P54 | ESI+: 537 |
| 175 | P54 | ESI+: 559, 561 | 176 | P54 | ESI+: 559,561 |
| 177 | P54 | ESI+: 559, 561 | 178 | P54 | ESI+: 542 |
| 179 | P54 | ESI+: 535 | 180 | P54 | ESI+:539 |
| 181 | P54 | ESI+: 547 | 182 | P54 | ESI+:565 |
| 183 | P54 | ESI+: 565 | 184 | P54 | ESI+: 546, 548 |
| 185 | P54 | ESI+: 559, 561 | 186 | P54 | ESI+: 558 |
| 187 | P54 | ESI+: 536 | 188 | P54 | ESI+: 547 |
| 189 | P54 | ESI+: 520 | 190 | P54 | ESI+: 501 |
| 191 | P54 | ESI+: 485 | 192 | P54 | ESI+: 533 |
| 193 | P54 | ESI+:584 | 194 | P54 | ESI+: 557 |
| 195 | P54 | ESI+: 555 | 196 | P54 | ESI+: 529 |
| 197 | P54 | ESI+: 565, 567 | 198 | P54 | ESI+: 466, 468 |
| 199 | P54 | ESI+: 567 | 200 | P54 | ESI+: 601, 603 |
| 201 | P54 | ESI+: 569 | 202 | P202 | ESI+: 325 |
| 203 | P109 | ESI+: 618 | 204 | P109 | ESI+: 656 |
| 205 | P109 | ESI+: 628 | 206 | P109 | ESI+: 606, 608 |
| 207 | P109 | ESI+: 592, 594 | 208 | P112, P113 | ESI+: 607 |
| 209 | P112, P113 | ESI+: 620, 622 | 210 | P112, P113 | ESI+: 670 |
| 211 | P112, P113 | ESI+: 547 | 212 | P112, P113 | ESI+: 675 |
| 213 | P112, P113 | ESI+: 663, 665 | 214 | P112, P113 | ESI+: 676 |

[Table38]

| PEx | Syn | Dat | PEx | Syn | Dat |
|---|---|---|---|---|---|
| 215 | P112, P113 | ESI+: 660 | 216 | P112, P113 | ESI+: 654 |
| 217 | P112, P113 | ESI+: 649, 651 | 218 | P112, P113 | ESI+: 662 |
| 219 | P112, P113 | ESI+: 646 | 220 | P112, P113 | ESI+: 640 |
| 221 | P112, P113 | ESI+: 699 | 222 | P112, P113 | ESI+: 485, 487,489 |
| 223 | P112, P113 | ESI+: 683 | 224 | P112, P113 | ESI+: 681 |
| 225 | P112 P113 | ESI+: 655 | 226 | P112, P113 | ESI+: 620,622; TLC2: Rf=0.5 |
| 227 | P112, P113 | ESI+: 620,622; TLC2: Rf=0.45 | 228 | P112, P113 | ESI+: 634,636; TLC1: Rf=0.8 |
| 229 | P112, P113 | ESI+: 634,636; TLC1: Rf=0.7 | 230 | P112, P113 | ESI+:634,636 |
| 231 | P112, P113 | ESI+: 650 | 232 | P112, P113 | ESI+: 578, 580 |
| 233 | P112, P113 | ESI+: 616; TLC2: Rf=0.5 | 234 | P112, P113 | ESI+: 616; TLC2: Rf=0.45 |
| 235 | P112, P113 | no data | 236 | P112, P113 | ESI+: 592, 594 |
| 237 | P112, P113 | no data | 238 | P238 | ESI+:621 |
| 239 | 239, P240 | ESI+: 670; TLC2: Rf=0.5 | 240 | P239, P240 | ESI+: 670; TLC2: Rf=0.45 |
| 241 | P241 | ESI+: 158, 160 | 242 | P241 | ESI+: 174, 176 |
| 243 | P117 | ESI+: 389 | 244 | P117 | ESI+: 484 |
| 245 | P245 | ESI+: 152 | 246 | P246 | ESI+:290 |
| 247 | P126 | ESI+: 155 | 248 | P126 | ESI+: 139 |
| 249 | P126 | ESI+: 143, 145 | 250 | P126 | ESI+: 155 |
| 251 | P126 | ESI+: 148 | 252 | P132 | ESI+:168 |
| 253 | P132 | ESI+: 203 | 254 | P138 | ESI+:434 |
| 255 | P138 | ESI+: 457 | 256 | P138 | ESI+:441 |
| 257 | P138 | ESI+: 422, 424 | 258 | P138 | no data |
| 259 | P138 | no data | 260 | P260 | ESI+: 270 |
| 261 | P261 | ESI+: 155 | 262 | P262 | ESI+: 658 |
| 263 | P263 | ESI+: 318 | 264 | P263 | NMR1: 1.40(9 H, s), 4.11-4.28(2H, m), 7.38-7.76(2 H, m), 7.64-7.76 (1H, m), 8.25-8.33(1 H, m) |
| 265 | P4 | ESI+: 395 | 266 | P4 | ESI-: 408 |

[Table 39]

| PEx | Syn | Dat | PEx | Syn | Dat |
|---|---|---|---|---|---|
| 267 | P267 | NMR2: 1.23(3 H, s), 1.43-1.50 (2H, m), 1.56-1.64(2H, m), 1.88-1.94(1H, m), 2.14-2.23(2 H, m), 2.33-2.39(1 H, m), 3.54 (3 H, s), 5.78(1 H, s) | 268 | P10 | ESI+: 329 |
| 269 | P269 | ESI+: 281 | 270 | P12 | ESI-: 562 |

(continued)

| PEx | Syn | Dat | PEx | Syn | Dat |
|---|---|---|---|---|---|
| 271 | P12 | ESI-: 576 | 272 | P12 | ESI+: 563 |
| 273 | P12 | ESI+: 563 | 274 | P27 | ESI+: 383 |
| 275 | P27 | ESI+: 369 | 276 | P37 | ESI+: 411, 413 |
| 277 | P37 | ESI-: 450 | 278 | P37 | ESI+: 466 |
| 279 | P54 | ESI+: 548 | 280 | P54 | ESI+: 548 |
| 281 | P54 | ESI+: 564 | 282 | P54 | ESI+: 593 |
| 283 | P54 | ESI+: 591 | 284 | P54 | ESI+: 550 |
| 285 | P54 | ESI+: 587 | 286 | P286 | ESI+: 566 |
| 287 | P286 | ESI+: 516, 518 | 288 | P288 | ESI+: 309 |
| 289 | P289 | ESI+: 304 | 290 | P289 | ESI+: 170 |
| 291 | P241 | ESI+: 202, 204 | 292 | P241 | ESI+: 188, 190 |
| 293 | P149 | ESI+:382 | 294 | P294 | ESI+: 196 |
| 295 | P112, P113 | ESI+: 678 | 296 | P112, P113 | ESI+: 694 |
| 297 | P112, P113 | ESI+: 689 | | 298 P112, P113 | ESI+: 675 |
| 299 | P112, P113 | ESI+: 713 | 300 | P112, P113 | ESI+: 691 |
| 301 | P112, P113 | ESI+: 624, 626 | 302 | P112, P113 | ESI+: 662 |
| 303 | P112, P113 | ESI+: 719 | 304 | P112, P113 | ESI+: 669, 671 |
| 305 | P112, P113 | ESI+: 649, 651 | 306 | P112, P113 | ESI+: 646 |
| 307 | P112, P113 | ESI+: 692 | 308 | P112, P113 | ESI+: 642, 644 |
| 309 | P112, P113 | ESI+: 677 | 310 | P112, P113 | ESI+: 676 |
| 311 | P112, P113 | ESI+: 705, 707 | 312 | P112, P113 | ESI+: 695 |
| 313 | P112, P113 | ESI+: 679, 681 | 314 | P112, P113 | ESI+: 699 |
| 315 | P112, P113 | ESI+: 713 | 316 | P316 | NMR3: 1.66-1.75(2H, m), 1.82-1.95(6 H, m), 1.95-2.08(3 H, m), 2.12-2.20(2 H, m), 3.50-3.55(1 H, m) |

[Table 40]

| PEx | Syn | Dat | PEx | Syn | Dat |
|---|---|---|---|---|---|
| 317 | P120 | ESI+: 574 | 318 | P120 | ESI+: 568 |
| 319 | P126 | ESI+: 183 | 320 | P126 | ESI+: 209 |
| 321 | P126 | ESI+: 169 | 322 | P322, P323 | NMR2: 1.26(3 H, s), 1.40-1.48 (2H, m), 1.68-1.86(6 H, m), 2.13-2.19(1 H, m), 9.64(1 H, s) |
| 323 | P322, P323 | NMR2: 1.22(3 H, s), 1.46-1.73(6 H, m), 1.92-1.99(2 H, m), 2.30-2.36(1 H, m), 9.69(1 H, s) | | | |

Example 1

To a solution of benzyl rel-1-[(1R,3S,5S)-5-{[(5-cyano-2-{[2-(trifluoromethoxy)benzyl]amino}pyrimidin-4-yl)amino]methyl}adamantan-2-yl]-D-prolinate (100 mg) in EtOH (5 ml) was added 10% Pd/C (wetted with 50% water, 20 mg), followed by stirring at room temperature at a normal pressure under a hydrogen atmosphere. After completion of the reaction, the catalyst was separated by filtration through Celite, and the filtrate was concentrated under reduced pressure. The obtained residue was purified by preparative alumina thin layer chromatography (chloroform-MeOH) to obtain 74.4 mg of rel-1-[(1R,3S,5S)-5-{[(5-cyano-2-{[2-(trifluoromethoxy)benzyl]amino}pyrimidin-4-yl)amino]methyl}adamantan-2-yl]-D-proline.

Example 3

To a solution of rel-4-({[(1S,3R,4S,5S)-4-aminoadamantan-1-yl]methyl}amino)-2-{[2-(trifluoromethoxy)benzyl]amino}pyrimidine-5-carbonitrile (50 mg) in DMF (0.2 ml) were added iodobenzene (14.2 μl), cesium acetate (50.8 mg) and copper (I) iodide (20.2 mg), followed by stirring at 90˚C for 24 hours under irradiation with microwaves. The mixed reaction liquid was diluted with EtOAc, and then the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 5.1 mg of rel-4-({[(1S,3R,4S,5S)-4-anilinoadamantan-1-yl]methyl}amino)-2-{[2-(trifluoromethoxy)benzyl]amino}pyrimidine-5-carbonitrile.

Example 4

Under ice-cooling, to a solution of rel-4-({[(1S,3R,4S,5S)-4-aminoadamantan-1-yl]methyl}amino)-2-{[2-(trifluoromethoxy)benzyl]amino}pyrimidine-5-carbonitrile (50 mg) in DMF (1.0 ml) were sequentially added DIPEA (27.7 μl) and acetyl chloride (10.3 μl), followed by stirring at the same temperature for 1 hour. The reaction mixture was diluted with EtOAc, and then the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 46 mg of rel-N-[(1R,2S,3S,5S)-5-{[(5-cyano-2-{[2-{trifluoromethoxy}benzyl]amino}pyrimidin-4-yl)amino]methyl}adamantan-2-yl]acetamide.

Example 5

To a solution of rel-(4R)-1-[(1R,3S,5S)-5-{[(5-cyano-2-{[2-(trifluoromethoxy)benzyl]amino}pyrimidzn-4-yl)amino]methyl} adamantan-2-yl]-4-hydroxy-D-proline (23 mg) in DMF (0.5 ml) were sequentially added ammonium chloride (6.3 mg), HOBt (15.9 mg), and WSC (18.3 mg), followed by stirring at room temperature overnight. The reaction mixture was diluted with EtOAc, then sequentially washed with water and saturated brine, and dried over anhydrous sodium sulfate. After the desiccant was removed, the solvent was evaporated under reduced pressure. The obtained residue was purified by preparative silica gel thin layer chromatography (THF), and then purified by amino silica gel flash column chromatography (chloroform-MeOH) to obtain 10 mg of rel-(4R)-1-[(1R,3S,5S)-5-{[(5-cyano-2-{[2-(trifluoromethoxy)benzyl]amino}pyrimidin-4-yl)amino]methyl}adamantan-2-yl]-4-hydroxy-D-prolinamide.

Example 6

To a solution of rel-4-({[(1S,3R,4S,5S)-4-aminoadamantan-1-yl]methyl}amino)-2-{[2-(trifluoromethoxy)benzyl]amino}pyrimidlne-5-carbonitrile (50 mg) in DMF (1.0 ml) were sequentially added nicotinic acid (14.3 mg), HOBt (15.7 mg), and WSC (18.1 mg), followed by stirring at room temperature overnight. The reaction mixture was diluted with EtOAc, and then the organic layer was sequentially washed with water and saturated brine, and dried over anhydrous sodium sulfate. After the desiccant was removed, the solvent was evaporated under reduced pressure. The obtained residue was purified by amino silica gel flash column chromatography (hexane-EtOAc) to obtain 58 mg of rel-N-[(1R,2S,3S,5S)-5-{[(5-cyano-2-{[2-(trifluoromethoxy)benzyl]amino}pyrimidin-4-yl)amino]methyl}adamantan-2-yl]nicotinamide.

Example 7

To a solution of rel-4-({[(1S,3R,4S,5S)-4-aminoadamantan-1-yl]methyl}amino)-2-{[2-(trifluoromethoxy)benzyl]amino}pyrimidine-5-carbonitrile (50 mg) in N,N-dimethylacetamide (2.5 ml) were added sodium carbonate (44.8 mg)

and 1,3-dibromopropane (43.2 μl), followed by stirring at 100°C for 30 minutes under irradiation with microwaves. The reaction mixture was diluted with EtOAc, and then the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 45 mg of rel-4-({[(1S,3R,4S,5S)-4-azetidin-1-yladamantan-1-yl]methyl}amino)-2-{[2-(trifluoromethoxy) benzyl]amino}pyrimidine-5-carbonitrile.

Example 16

To a solution of rel-4-({[(1S,3R,45,5S)-4-aminoadamantan-1-yl]methyl}amino)-2-{[2-(trifluoromethoxy)benzyl] amino}pyrimidine-5-carbonitrile (30 mg) in DMF (0.6 ml) were added DIPEA (22.1 μl) and 2-bromoethanol (4.5 μl), followed by heating and stirring at 60°C. The reaction mixture was diluted with EtOAc, and then the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH), and then by preparative alumina thin layer chromatography (chloroform-MeOH) to obtain 25 mg of rel-4-[({(1S,3R,4S,5S)-4-[(2-hydroxyethyl)amino]adamantan-1-yl}methyl)amino]-2-{ [2-(trifluoromethoxy)benzyl]amino}pyrimidine-5-carbonitrile.

Example 32

To a solution of 4-({[(1S,3R,4S,5S)-4-aminoadarnantan-1-yl]methyl}amino)-2-{[2-(trifluoromethoxy)benzyl]ammo}pyrimidine-5-carbonitrile (50 mg) in DMF (1 ml) were added (2,2-dimethyl-1,3-dioxan-5-yl)methyl 4-methylbenzenesulfonate (47.7 mg) and potassium carbonate (29.2 mg), followed by stirring at 70°C for 12 hours. To the reaction mixture was added water, followed by extraction with EtOAc. The organic layer was dried over magnesium sulfate, the desiccant was removed, and then the solvent was evaporated under reduced pressure. The obtained residue was purified by silica gel column chromatography (hexane-EtOAc) to obtain 21 mg of 4-({[(1S,3R,4S,5S)-4-{[(2,2-dimethy)-1,3-dioxan-5-yl)methyl]amino}adamantan-1-yl]mehyl}amino)-2-{[2-(trifluoromethoxy)benzyl]amino}pyrimidine-5-carbonitrile.

Example 36

To a mixed solution of rel-4-({[(1S,3R,4S,SS)-4-aminoadamantan-1-yl]methyl}amino)-2-{[2-(trifluoromethoxy) benzyl]amino}pyrimidine-5-carbonitril(50 mg) in EtOH (1.0 ml) and THF (0.5 ml) was added oxiran-2-ylmethanol (8.2 μl), followed by stirring at room temperature for 24 hours. Oxylan-2-yl methanol (82.1 μl) was added thereto, followed by stirring at room temperature for additional 48 hours. The mixed reaction liquid was purified by amino silica gel flash column chromatography (chloroform-MeOH) to obtain 25 mg of rel-4-[({(1S,3R,4S,5S)-4-[(2,3-dihydroxypropyl)amino] adamantan-1-yl}methyl)amino]-2-{[2-(trifluoromethoxy)benzyl]amino}pyrimidine-5-carbonitrile.

Example 39

To a solution of rel-4-({[(1S,3R,4S,5S)-4-aminoadamantan-1-yl]methyl}amino)-2-{[2-(trifluoromethoxy)benzyl] amino}pyrimidine-5-carbonitrile (50 mg) in 1,3-dimethylimidazolidin-2-one (1.0 ml) were added DIPEA (36.9 μl) and 2,2,2-trifluoroethyl trifluoromethanesulfonate (18.3 μl), followed by stirring at room temperature. After completion of the reaction, the mixed reaction liquid itself was purified by silica gel flash column chromatography (chloroform-MeOH) to obtain 55 mg of rel-4-[({(1S,3R,4S,5S)-4-[(2,2,2-trifluoroethyl)amino]adamantan-1-yl}methyl)amino]-2-{[2-(trifluoromethoxy)benzyl]amino}pyrimidine-5-carbonitrile,

Example 40

To a solution of 4-({[(1S,3R,4S,5S)-4-aminoadamantan-1-yl]methyl)amino)-2-{[2-(trifluoromethoxy)benzy]amino}pyrimidine-5-carbonitrile (50 mg) in 1,3-dimethylimidazolidin-2-one (0.5 ml) were added 2,2-bis(bromomethyl)propane-1,3-diol (221.8 mg), and potassium carbonate (146.3 mg), followed by stirring at room temperature for 4 days. The reaction mixture was purified by silica gel column chromatography (chloroform-MeOH) as it was to obtain 5 mg of [({(1S,3R,4S,5S)-4-[3,3-bis(hydroxymethyl)azetidin-1-yladamantan-1-yl}methyl)amino]-2-{[2-(trifluoromethoxy)benzyl] amino}pyrimidine-5-carbonitrile.

Examples 41 and 42

A mixture of steric isomers (39 mg) of rel-4-[({(1R,3S,5R)-4-[(trans-4-hydroxycyclohexyl)amino]adamantan-1-yl}methyl)amino]-2-{[2-(trifluoromethoxy)benzyl]amino}pynmidine-5-carbonitrile was subjected to separation and purification by reverse phase liquid chromatography (eluent: a mixed liquid of 0.2% formic acid/MeOH and water) to obtain a formate of rel-4-[({(1R,35,4R,5R)-4-[(trans-4-hydroxycyclohexyl)amino]adamantan-1-yl}methyl)amino]-2-{[2-(trifluoromethoxy)benzyl]amino}pyrimidine-5-carbonitrile and a formate of rel-4-[({(1R,3S,4S,5R)-4-[(trans-4-hydroxycydohexyl)amino]adamantan-1-yl}methyl)amino]-2-{[2-(triftuoromethoxy)benzyl]amino}pyrimidine-5-carbonitrile as single isomers, respectively. Then, their formates were each purified by amino silica gel flash column chromatography (hexane-EtOAc) to obtain 19.1 mg of rel-4-[({(1R,3S,4R,5R)-4-[(trans-4-hydroxycyclohexyl)amino]adamantan-1-yl}methyl)amino]-2-{[2-(tnfluoromethoxy)benzyl]amino}pyrimidine-5-carbonitrile and 10.4 mg of rel-4-[({(1R,3S,4S,5R)-4-[(trans-4-hydroxycyclohexyl)amino]adamantan-1-yl}methyl)amino]-2-{[2-(trifluoromethoxy)benzyl]amino}pyrimidine-5-carbonitrile.

Example 43

To a solution of rel-trans-4-{[(1R,2S,3S,5S)-5-(aminomethyl)adamantan-2-yl]amino}cyclohexanol (80 mg) in 1,3-dimethylimidazolidin-2-one (1.0 ml) were added DIPEA (0.20 ml) and 4-chloro-2-{[2-(methylsulfanyl)benzyl]amino}pyrimidine-5-carbonitrile (108.6 mg), followed by stirring at room temperature overnight. The mixed reaction liquid was purified by amino silica gel flash column chromatography (chloroform-MeOH) to obtain 73.4 mg of rel-4-[({(1R,3S,4R,5R)-4-[(trans-4-hydroxycyclohexyl)amino]adamantan-1-yl}methyl)amino]-2-{[2-(methylsulfanyl)benzyl]amino}pynmidine-5-carbonitrile.

Example 63

To a solution of ethyl rel-N-[(1R,2S,3S,5S)-5-{[(5-cyano-2-{[2-(trifluoromethoxy)benzyl]amino}pyrimidin-4-yl)amino]methyl}adamantan-2-yl]glycinate (24 mg) in THF (2.4 ml) was added a 4 M aqueous lithium hydroxide solution (0.1 ml), followed by stirring at room temperature. After completion of the reaction, to the mixed reaction liquid were added 1 M hydrochloric acid (0.4 ml), followed by stirring, and then the mixed reaction liquid was concentrated under reduced pressure. To the obtained residue was added water, and the solid was collected by filtration and then dried under reduced pressure. The obtained solid was suspended by the addition of EtOAc and diisopropyl ether, and then the solid was collected by filtration and dried under reduced pressure to obtain 11.0 mg of rel-N-[(1R,2S,3S,5S)-5-{[(5-cyano-2-{[2-(trifluoromethoxy)benzyl]amino}pyrimidin-4-yl)amino]methyl}adamantan-2-yl]glycine.

Example 64

To a solution of rel-4-({[(1S,3R,5S)-4-oxoadamantan-1-yl]methyl}amino)-2-([2-(trifluoromethoxy)benzyl]amino}pyrimidine-5-carbonitrile (40 mg) in dichloromethane (0.8 ml) were added trans-4-aminocyclohexanol (14.7 mg) and sodium triacetoxyborohydride (53.9 mg), followed by stirring at room temperature overnight. To the mixed reaction liquid was added saturated aqueous sodium bicarbonate, followed by extraction with EtOAc, and then the organic layer was washed with saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 18 mg of rel-4-[({1R,3S,5R)-4-[(trans-4-hydroxycyclohexyl)amino]adamantan-1-yl}methyl)amino]-2-{[2-(trifluoromethoxy)benzyl]amino}pyrimidine-5-carbonitrile.

Example 68

To a solution of rel-4-({[(1S,3R,4S,5S)-4-aminoadamantan-1-yl]methyl}amino)-2-{[2-(methylsulfanyl)benzyl]amino}pyrimidine-5-carbonitrile (50 mg) in dichloromethane (3.5 ml) were added 4-hydroxycyclohexanone (39.4 mg) and sodium triacetoxyborohydride (146.3 mg), followed by stirring at room temperature for 2 hours. To the mixed reaction liquid was added saturated aqueous sodium bicarbonate, followed by extraction with EtOAc, then washing with saturated brine, and drying over anhydrous sodium sulfate. After the desiccant was removed, the solvent was evaporated under reduced pressure. The obtained residue was purified by amino silica gel flash column chromatography (chloroform-MeOH) to obtain 60.6 mg of rel-4-[({1S,3R,4S,5S}-4-[(4-hydroxycyclohexyl)amino]adamantan-1-yl}methyl)amino]-2-{[2-(methylsulfanyl)benzyl]amino}pyrimidine-5-carbonitrile.

Example 105

To a solution of rel-4-[({(1S,3R,4S,5S)-4-[(4-hydroxycyclohexyl)amino]adamantan-1-yl}methyl)amino]-2-{

[2-(trifluoromethoxy)benzyl]amino}pyrimidine-5-carbonitrile (50 mg) in MeOH (2 ml) were added a 37% aqueous formalin solution (28.4 μl) and sodium cyanoborohydride (8.3 mg), followed by stirring at room temperature for 5 hours. To the mixed reaction liquid was added saturated aqueous sodium bicarbonate, followed by extraction with EtOAc, and then washed with saturated brine, and dried over anhydrous sodium sulfate. After the desiccant was removed, the solvent was evaporated under reduced pressure. The obtained residue was purified by amino silica gel flash column chromatography (chloroform-MeOH) to obtain 37.6 mg of rel-4-[({(1S,3R,4S,5S)-4-[(4-hydroxycyclohexyl)(methyl)amino]adamantan-1-yl}methyl)amino]-2-{[2-(trifluoromethoxy)benzyl]amino}pyrimidine-5-carbonitrile.

Example 117

To a solution of rel-4-({[(1S,3R,5S)-4-oxoadamantan-1-yl]methyl}amino)-2-{[2-(trifluoromethoxy)benzyl]amino}pyrimidine-5-carbonitrile (30 mg) in MeOH (3 ml) was added sodium borohydride (13.5 mg), followed by stirring at room temperature. After completion of the reaction, the reaction mixture was diluted with EtOAc, and then the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 25.6 mg of rel-4-({[(1S,3R,5S)-4-hydroxyadamantan-1-yl]methyl}amino)-2-{[2-(trifluoromethoxy)benzyl]amino}pyrimidine-5-carbonitrile.

Example 118 and 119

Under ice-cooling, to a solution of rel-2-[(2-methoxybenzyl)amino]-4-[({(1S,3R,4S,5S)-4-[(4-oxocyclohexyl)amino]adamantan-1-yl}methyl)amino]pyrimidine-5-carbonitrile (40 mg) in THF (2 ml) was added dropwise a 0.97 M solution (0.40 ml) of methylmagnesium bromide in THF under a nitrogen atmosphere, followed by stirring at the same temperature for 3 hours. To the mixed reaction liquid was added water, followed by extraction with EtOAc, and then the organic layer was washed with saturated brine, and dried over anhydrous sodium sulfate. After the desiccant was removed, the solvent was evaporated under reduced pressure. The obtained residue was purified by preparative silica gel thin layer chromatography (chloroform-MeOH-28% aqueous ammonia) to first obtain 4.3 mg of rel-4-[({(1S,3R,4S,5S)-4-[(4-hydroxy-4-methylcyclohexyl)amino]adamantan-1-yl}methyl)amino]-2-[(2-methoxybenzyl)amino]pyrimidine-5-carbonitrile (isomer A) and then obtain 5.3 mg of rel-4-[({(1S,3R,4S,5S)-4-[(4-hydroxy-4-methylcyclohexyl)aminoladamantan-1-yl}methyl)amino]-2-[(2-methoxybenzyl)amino]pyrimidine-5-carbonitrile (isomer B).

Example 120

To a solution of 4-[(1-azabicyclo[2.2.2]oct-3-ylmethyl)amino]-2-{[2-(trifluoromethoxy)benzyl]amino}pyrimidine-5-carbonitrile (40 mg) in dichloromethane (1 ml) was added 77% MCPBA (contains water, 21 mg) under ice-cooling, followed by stirring at room temperature for 3 hours. To the reaction mixture was added saturated aqueous sodium hydrogen carbonate solution, followed by extraction with EtOAc. The organic layer was dried over anhydrous sodium sulfate, the desiccant was removed, and then the solvent was evaporated under reduced pressure. The obtained residue was purified by amino silica gel flash column chromatography (chloroform-MeOH) to obtain 19 mg of 4-[(1-oxide-azabicyclo[2.2.2]oct-3-ylmethyl)amino]-2-{[2-(trifluoromethoxy)benzyl]amino}pyrimidine-5-carbonitrile.

Example 121

To a solution of rel-4-({[(1S,3R,4S,5S)-4-(tetrahydro-2H-thiopyran-4-ylamino)adamantan-1-yl]methyl}amino)-2-{[2-(trifluoromethoxy)benzyl]amino}pyrimidine-5-carbonitrile (88 mg) in dichloromethane (2 ml) was added 75% MCPBA (contains water, 106.1 mg), followed by stirring at room temperature for 6 hours. To the mixed reaction liquid was added saturated aqueous sodium bicarbonate, followed by extraction with EtOAc, and the organic layer was washed with saturated brine and then dried over anhydrous sodium sulfate. After the desiccant was removed, the solvent was evaporated under reduced pressure. The obtained residue was purified by silica gel flash column chromatography (chloroform-MeOH), and then by preparative silica gel thin layer chromatography (chloroform-MeOH) to obtain 8.8 mg of rel-4-[({(1S,3R,4,5S)-4-[(1,1-dioxidotetrahydro-2H-thiopyraran-4-yl)amino]adamantan-1-yl}methyl)amino]-2-{[2-(trifluoromethoxy)benzyl]amino}pyrimidine-5-carbonitrile.

Example 122

To a solution of 4-{[(3-endo)-8-benzyl-8-azabicyclo[3.2.1]oct-3-yl]amino}-2-{[2-(trifluoromethoxy)benzyl]amino}pyrimidine-5-carbonitrile (16 mg) in MeOH (1ml) were added ammonium formate (100 mg) and a catalytic amount of 10% Pd/C (wetted with 50% water), followed by heating and refluxing for 6 hours. The mixed reaction liquid was left

to be cooled to room temperature, then the catalyst was removed, and the filtrate was concentrated under reduced pressure. The obtained residue was dissolved in EtOAc, sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 8.0 mg of 4-[{3-endo}-8-azabicyclo[3.2.1]oct-3-ylamino]-2-{[2-(trifluoromethoxy)benzyl]amino}pyrimidine-5-carbonitrile.

Example 123

To a solution of tert-butyl rel-{(1R,2S,3S,5S)-S-[({2-[(2-chlorobenzyl)amino]-5-cyanopyrimidin-4-yl}amino)methyl]adamantan-2-yl}carbamate (70 mg) in dichloromethane (1 ml) was added trifluoroacetic acid (0.135 ml), followed by stirring at room temperature overnight. The mixed reaction liquid was concentrated under reduced pressure, and to the obtained residue was added an aqueous potassium carbonate solution, followed by stirring at room temperature for 3 hours. The precipitated solid was collected by filtration, washed with water, and then dried under reduced pressure to obtain 55 mg of rel-4-({[(1S,3R,4S,5S)-4-aminoadamantan-1-yl]methyl}amino)-2-[(2-chlorobenzyl)amino]pyrimidine-5-carbonitrile.

Example 170

To a solution of tert-butyl 3-{[(5-cyano-2-{[2-(trifluoromethoxy)benzyl]amino}pyrimidin-4-yl)amino]methyl}-8-azabicyclo[3.2.1]octane-8-carboxylate in dioxane (0.6 ml) was added a 4 M hydrogen chloride dioxane solution (0.28 ml), followed by stirring at room temperature for 10 hours. The reaction mixture was concentrated under reduced pressure to obtain 56 mg of 4-[(8-azabicyclo[3.2.1]oct-3-ylmethyl)amino]-2-{[2-(trifluoromethoxy)benzyl]amino}pyrimidine-5-carbonitrile dihydrochloride.

Example 175

To a solution of 4-({[1-(tetrahydro-2H-pyran-2-yl)-1H-benzimidazol-5-yl]methyl}amino)-2-{[2-(trifluoromethoxy)benzyl]amino}pyrimidine-5-carbonitrile (50 mg) in EtOH (3 ml) was added 1 M hydrochloric acid (1ml), followed by stirring at 60˚C for 24 hours. To the mixed reaction liquid were added 1 M hydrochloric acid (1 ml), followed by stirring at 60˚C for additional 24 hours. The reaction liquid was concentrated under reduced pressure, and to the obtained residue were added EtOAc and EtOH. The precipitated solid was collected by filtration, washed with EtOAc, and then dried under reduced pressure to obtain 53.1 mg of 4-[(1H-benzimidazol-5-ylmethyl)amino]-2-{[2-(trifluoromethoxy)benzyl]amino}pyrimidine-5-carbonitrile dihydrochloride.

Example 176

To a solution of benzyl rel-[(1R,2S,3S,5S)-5-{[(5-cyano-2-{[2-(trifluoromethoxy)benzyl]amino}pyrimidin-4-yl)amino]methyl}adamantan-2-yl]carbamate (55 mg) in MeOH (3 ml) was added 10% Pd/C (wetted with 50% water, 15 mg), followed by stirring at room temperature for 6 hours at a normal pressure under a hydrogen atmosphere. The catalyst was removed by filtration, and the filtrate was concentrated under reduced pressure. The obtained residue was purified by preparative alumina thin layer chromatography (chloroform-MeOH) to obtain 20.0 mg of rel-4-({[(1S,3R,4S,5S)-4-aminoadamantan-1-yl]methyl}amino)-2-{[2-(trifluoromethoxy)benzyl]amino}pyrimidine-5-carbonitrile.

Example 186

To a solution of benzyl rel-{(1R,2S,3S,5S)-5-[({5-cyano-2[(2,5-dichlorobenzyl)amino]pyrimidin-4-yl}amino)methyl]adamantan-2-yl}carbamate (45 mg) in acetic acid (1.5 ml) was added 48% hydrobromic acid (1.5 ml), followed by stirring at room temperature for 24 hours. The reaction liquid was concentrated under reduced pressure, then the residue was dissolved in EtOAc, sequentially washed with an aqueous potassium carbonate solution, water, and saturated brine, and dried over anhydrous sodium sulfate. After the desiccant was removed, the solvent was evaporated under reduced pressure. The obtained residue was purified by amino silica gel flash column chromatography (chloroform-MeOH) to obtain 18 mg of rel-4-({[(1S,3R,4S,5S)-4-aminoadamantan-1-yl]methyl}amino)-2-[(2,5-dichlorobenzyl)amino]pyrimidine-5-carbonitrile.

Example 188

To a solution of rel-4-({[(1S,3R,4S,5S)-4-{[(2-phenyl-1,3-dioxan-4-yl)methyl]amino }adamantan-1-yl]methyl} amino)-2-{[2-(trifluoromethoxy)benzyl]amino}-5-carbonitrile (54 mg) in THF (1.5 ml) was added 1 M hydrochloric acid

(1.5 ml), followed by stirring at room temperature for 4 hours. The reaction mixture was cooled under ice, and saturated aqueous sodium bicarbonate was added thereto. The precipitate was collected by filtration and dried under reduced pressure to obtain a solid, which was purified by amino silica gel column chromatography (chloroform-MeOH) to obtain 41.2 mg of rel-4-[({(1S,3R,4S,5S)-4-[(2,4-dihydroxybutyl)amino]adamantan-1-yl}methyl)amino]-2-{[2-(trifluoromethoxy) benzyl]amino}pyrimidine-5-carbonitrile.

Example 191

To a solution of rel-4-[({(1S,3R,4S,5S)-4-[(2,2-dimethyl-1,3-dioxan-5-yl)amino]adamantan-1-yl}methyl)amino]-2-{[2-(trifluoromethoxy)benzyl]amino}pyrimidine-5-carbonitrile (55 mg) in THF (1.0 ml) was added 1 M hydrochloric acid (1.0 ml), followed by stirring at room temperature. After completion of the reaction, the mixed reaction liquid was concentrated under reduced pressure, and then to the residue was added dichloromethane, followed by concentration under reduced pressure. To the obtained residue was added diethyl ether, and the precipitated solid was collected by filtration, washed with diethyl ether, and then dried under reduced pressure to obtain 50.5 mg of rel-4-({[(1S,3R,4S,5S)-4-{[2-hydroxy- 1-(hydroxymethyl) ethyl] amino} adamantan- 1- yl] methyl} amino)- 2-[2-(trifluoromethoxy) benzyl] amino} pyrimidine-5-carbonitrile dihydrochloride.

Example 203

Under ice-cooling, to a solution of rel-4-({[(1S,3R,4S,5S)-4-aminoadamantan-1-yl]methyl}amino)-2-{[2-(trifluoromethoxy)benzyl]amino}pyrimidine-5-carbonitrile (50 mg) in DMF (1.0 ml) were sequentially added DIPEA (27.7 μl) and methanesulfonyl chloride (8.6 μL), followed by stirring at the same temperature for 1 hour. The mixed reaction liquid was diluted with EtOAc, and the organic layer was sequentially washed with water and saturated brine, and dried over anhydrous sodium sulfate. After the desiccant was removed, the solvent was evaporated under reduced pressure. The obtained residue was purified by preparative silica gel thin layer chromatography (chloroform-MeOH) to obtain 31 mg of rel-N-[(1R,2S,3S,5S)-5-{[(5-cyano-2-{[2-(trifluoromethoxy)benzyl]amino}pyrimidin-4-yl)amino]methyl}adamantan-2-yl]methane sulfonamide.

Example 206

Under ice-cooling, to a solution of rel-2-[(2-chlorobenzyl)amino]-4-({[(1S,3R,5S)-4-piperazin-1-yladamantan-1-yl]methyl}amino)pyrimidine-5-carbonitrile (18 mg) in DMF (360 μl) were sequentially added triethylamine (3.6 μl) and acetic anhydride (7.6 μl), followed by stirring at room temperature. After completion of the reaction, the mixed reaction liquid was diluted with EtOAc, washed with water (three times) and saturated brine, and dried over anhydrous sodium sulfate. The desiccant was removed, then the solvent was evaporated under reduced pressure, and the residue was purified by amino silica gel flash column chromatography (hexane-EtOAc) to obtain 16.2 mg of rel-4-({[(1S,3R,5S)-4-(4-acetylpiperazin-1-yl)adamantan-1-yl]methyl}amino)-2-[(2-chlorobenzyl)amino]pyrimidine-5-carbonitrile.

Example 207

To a solution of rel-2-[(2-chlorobenzyl)amino]-4-[({(1S,3R,4S,5S)-4-[(2-chloroethyl)amino]adamantan-1-yl}methyl)amino]pyrimidine-5-carbonitrile (27.0 mg) and (2S)-pyrrolidin-2-ylmethanol (7.9 mg) in DMF (0.27 ml) were added potassium iodide (13.8 mg) and DIPEA (0.02 ml), followed by stirring at 75˚C for 2 hours. Then,(2S)-pyrrolidin-2-ylmethanol (1.1 mg) was added thereto, followed by stirring at the same temperature for additional 2 hours. The solvent was evaporated under reduced pressure and the obtained residue was purified by amino silica gel flash column chromatography (chloroform- MeOH) to obtain 25.4 mg of 2-[(2-chlorobenzyl)amino]-4-({[(1R,3R,4S,5S)-4-({2-[(2S)-2-(hydroxymethylpyrrolidin-1-yl]ethyl}amino)adamantan-1-yl]methyl}amino)pyrimidine-5-carbonitrile.

Example 220

To a solution of rel-4-[({(1R,3S,4R,5R)-4-[(cis-4-ammocyclohexyl)amino]adamantan-1-yl}methyl)amino]-2-[(2-chlorobenzyl)amino]pyrimidine-5-carbonitrile (30 mg) in DMI (1.0 ml) were added DIPEA (80.4 μl) and 2-bromoethanol (8.2 μl), followed by heating and stirring at 120˚C. After completion of the reaction, the reaction mixture was diluted with chloroform and purified by amino silica gel flash column chromatography (chloroform-MeOH) as it was to obtain 17.6 mg of rel-2-[{2-chlorobenzyl}amino]-4-({[(1R,3S,4R,SR)-4-({cis-4-[(2-hydroxyethyl)amino]cyclohexyl}amino)adamantan-1-yl]methyl}amino)pyrimidine-5-carbonitrile.

Example 235

To a solution of rel-2-[(2-chlorobenzyl)amino]-4-[({(1S,3R,4S,5S)-4-[(4-oxocyclohexyl)amino]adamantan-1-yl}methyl)amino]pyrimidine-5-carbonitrile (50 mg) in dichloromethane (3.0 ml) were sequentially added 4,4-difluoropiperidine hydrochloride (30.4 mg), triethylamine (26.7 μl), and sodium triacetoxyborohydride (61.2 mg), followed by stirring at room temperature. After completion of the reaction, to the mixed reaction liquid was added saturated aqueous sodium bicarbonate, followed by extraction with chloroform, and the solvent was evaporated under reduced pressure. The obtained residue was purified by amino silica gel flash column chromatography (chloroform-MeOH) to obtain 13.1 mg of rel-2-[(2-chlorobenzyl)amino]-4-({[(1S,3R,4S,5S)-4-{[4-(4,4-difluoropiperidin-1-yl)cyclohexyl]amino}adamantan-1-yl]methyl}amino)pyrimidine-5-carbonitrile.

Example 281

To a solution of rel-4-[({(1R,3S,4R,5R)-4-[(cis-4-aminocyclohexyl)amino]adamantan-1-yl}methyl)amino] -2-[(2-chlorobenzyl)amino]pyrimidine-5-carbonitrile (40 mg) in dichloromethane (2.0 ml) were sequentially added 1-acetylpiperidin-4-one (21.7 mg) and sodium triacetoxyborohydride (48.9 mg), followed by stirring at room temperature. After completion of the reaction, to the mixed reaction liquid was added saturated aqueous sodium bicarbonate, followed by extraction with chloroform, and the solvent was evaporated under reduced pressure. The obtained residue was purified by amino silica gel flash column chromatography (chloroform-MeOH) to obtain 54 mg of rel-4-({[(1R,35,4R,5R)-4-({cis-4-[(1-acetylpiperidin-4-yl)amino]cyclohexyl}amino)adamantan-1-yl]methyl}amino)-2-[(2-chlorobenzyl)amino]pyrimidine-5-carbonitrile.

Example 338

To a suspension of rel-4-({[(1S,3R,4S,5S)-4-([trans-4-([tert-butyl (dimethyl)sllyl]oxylmethyl)cyclohexyl]methyllamino)adamantan-1-yl]methyl}amino)-2-[(2-cyanobenzyl)amino]pyrimidine-5-carbonitrile (68 mg) in MEOH) (1.4 ml) was added 1 M hydrochloric acid (0.6 ml), followed by stirring at room temperature for 1 hour. The solvent was evaporated under reduced pressure, and then the residue was diluted with chloroform, and saturated aqueous sodium bicarbonate was added thereto under ice-cooling. The mixture was extracted with a mixed solvent of chloroform-MeOH (10:1), and the organic layer was washed with water and saturated brine, and dried over anhydrous magnesium sulfate. After the desiccant was removed, the solvent was evaporated under reduced pressure and the obtained residue was purified by amino silica gel flash column chromatography (chloroform-MeOH) to obtain 49.4 mg of rel-2-[(2-cyanobenzyl)amino]-4-({[(1S,3R,4S,5S)-4-({[trans-4-(hydroxymethyl)cyclohexyl]methyl}amino)adamantan-1-yl]methyl}amino)pyrimidine-5-carbonitrile.

Example 351

Under ice-cooling, to a solution of 4-[({(1S,3R,4S,5S)-4-[(3-{[tert-butyldimethyl)silyl]oxypropyl)(methyl)amino]adamantan-1-yl}methyl)amino]-2-{[2-(methylsulfanyl)benzyl]amino}pyrimidine-5-carbonitrile (55 mg) in THF (2 mL) were added a solution (0.20 mL) of 1 M tetrabutylammonium fluoride in THF, followed by stirring at room temperature for 1 hour. The solvent was evaporated under reduced pressure, and purified by amino silica gel flash column chromatography (chloroform-MeOH) as it was to obtain 25 mg of 4-[({(1S,3R,4S,5S)-4-[(3-hydroxypropyl)(methyl)amino]adamantan-1-yl}methyl)amino]-2-{[2-(methylsulfanyl)benzyl]amino}pyrimidine-5-carbonitrile.

Example 353

To a mixed solution of rel-4-[({(1S,3R,4S,5S)-4-[3-(1,3-dioxo-1,3-dihydro-2H-isoindol-2-yl)azetidin-1-yl]adamantan-1-yl}methyl)amino]-2-{[2-(trifluoromethoxy)benzyl]amino}pyrimidine-5-carbonitrile (64 mg) in EtOH (1.28 ml) and THF (1.28 ml) was added hydrazine monohydrate (18.9 μl), followed by heating and refluxing, and the insoluble materials were removed by filtration, and then the filtrate was concentrated under reduced pressure. To the obtained residue was added chloroform, followed by washing with water twice and drying over anhydrous sodium sulfate. After the desiccant was removed, the solvent was evaporated under reduced pressure and the residue was purified by amino silica gel flash column chromatography (chloroform-MeOH) to obtain 14 mg of rel-4-({[(1S,3R,4S,5S)-4-(3-aminoazetidin-1-yl)adamantan-1-yl]methyl}amino)-2-{[2-(trifluoromethoxy) benzyl]amino}pyrimidine-5-carbonitrile.

Example 356

To a solution of rel-2-[(2-chlorobenzyl)amino]-4-[({(1S,3R,4S,5S)-4-[(piperidin-4-ylmethyl)amino]adamantan-1-yl}methyl)amino]pyrimidine-5-carbonitrile (40 mg) in DMI (1 ml) were added 1-fluoro-3-iodopropane (18 mg) and DIPEA (0.017 ml), followed by irradiation with microwaves at 100˚C for 1 hour. The reaction mixture was diluted with chloroform, and purified by amino silica gel flash column chromatography (hexane-EtOAc) as it was to obtain 20 mg of rel-2-[(2-chlorobenzyl)amino]-4-({[(1S,3R,4S,5S)-4-({[1-(3-fluoropropyl)piperidin-4-ylmethyl]methyl}amino)adamantan-1-yl} methyl}amino)pyrimidine-5-carbonitrile.

Example 376

Under ice-cooling, to a solution of tert-butyl 4-{4-[(5-cyano-2-{[2-(trifluoromethoxy)benzyl]amino}pyrimidin-4-yl)amino]butanolyl}piperazine-1-carboxylate (205 mg) in dichloromethane (4.1 ml) was added TFA (0.75 ml), followed by stirring at room temperature for 18 hours. The mixed reaction liquid was concentrated, and an aqueous potassium carbonate solution was added thereto under ice-cooling, followed by stirring at room temperature. The precipitated solid was collected by filtration, dried, and then purified by amino silica gel flash column chromatography (chloroform-MeOH). A fraction including a desired compound was concentrated, and to the residue was added a 4 M hydrogen chloride ethyl acetate solution, followed by concentration and solidification, to obtain 65.6 mg of 4-{[4-oxo-4-(piperazin-1-yl)butyl] amino}-2-{[2-(trifluoromethoxy)benzyl]amino}pyrimidine-5-carbonitrile dihydrochloride.

Example 412

To a solution of rel-4-[({(1S,3R,4S,5S)-4-[(1,4-dioxaspiro[4.5]dec-8-ylmethyl)amino]adamantan-1-yl}methyl) amino]-2-({[2-(methylsulfanyl)pyridin-3-yl]methyl}amino)pyrimidine-5-carbonitrile (140 mg) in THF (3 ml) was added 1 M hydrochloric acid (2 ml), followed by stirring at room temperature for 2 hours. The reaction mixture was concentrated under reduced pressure, and then saturated aqueous sodium bicarbonate was added thereto, followed by extraction with EtOAc. The organic layer was washed with saturated brine and dried over anhydrous sodium sulfate. The desiccant was removed, then the solvent was evaporated under reduced pressure, and the residue was purified by amino silica gel flash column chromatography (chloroform-MeOH) to obtain 100 mg of rel-2-({[2-(methylsulfanyl)pyridin-3-yl]methyl} amino)-4-({[(1S,3R,4S,5S)-4-{[(4-oxocyclohexyl)methyl]amino}adamantan-1-yl]methyl}amino)pyrimidine-5-carbonitrile.

Each of the Example compounds was prepared in the same manner as the methods of Examples above, using each of the corresponding starting materials. The structures, the production processes, and the physicochemical data of the Example compounds are shown in Tables below.

[Table 41]

| Ex | Str | Ex | Str |
|---|---|---|---|
| 1 rel | 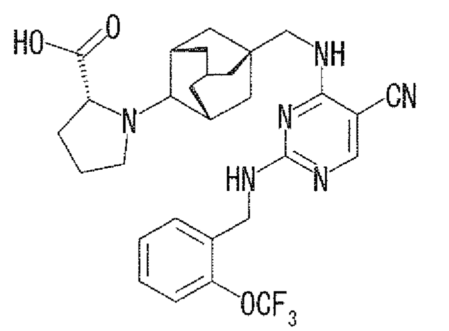 | 2 rel | 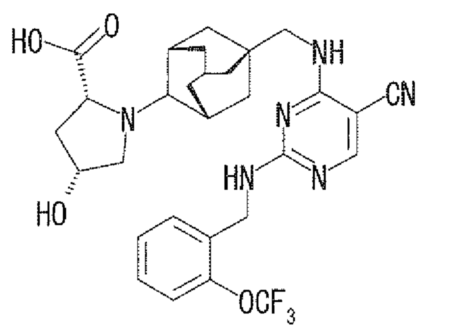 |
| 3 rel | 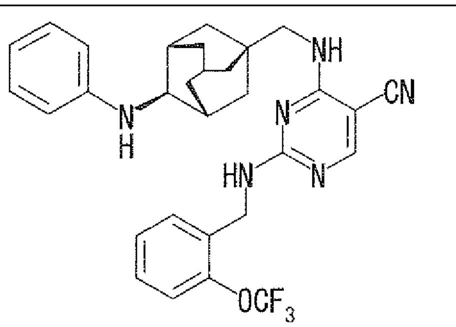 | 4 rel | 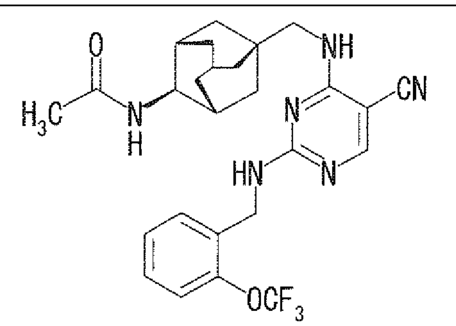 |

(continued)

| Ex | Str | Ex | Str |
|---|---|---|---|
| 5 rel | 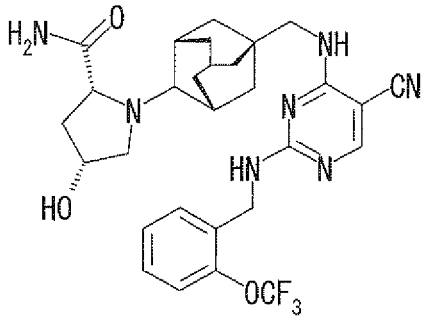 | 6 rel | 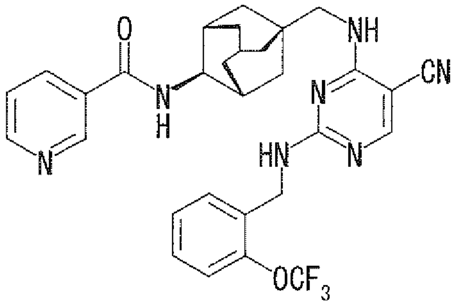 |
| 7 rel | 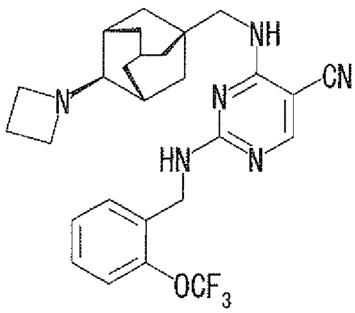 | 8 rel | 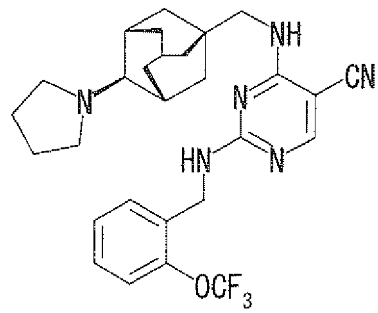 |

[Table 42]

| Ex | Str | Ex | Str |
|---|---|---|---|
| 9 rel | 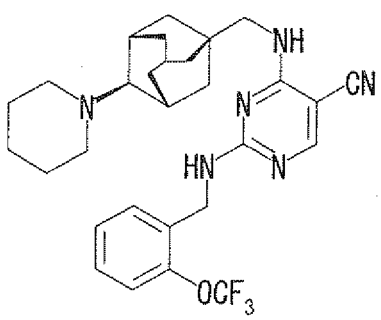 | 10 rel | 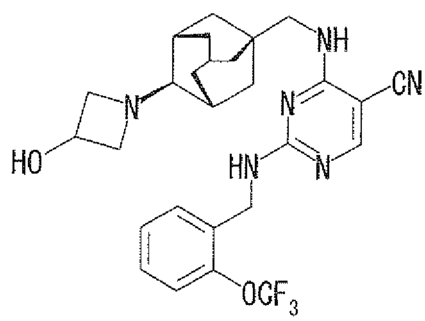 |
| 11 rel | 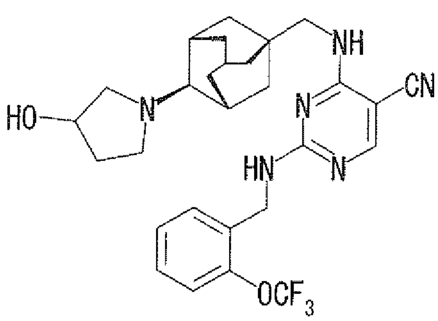 | 12 rel | 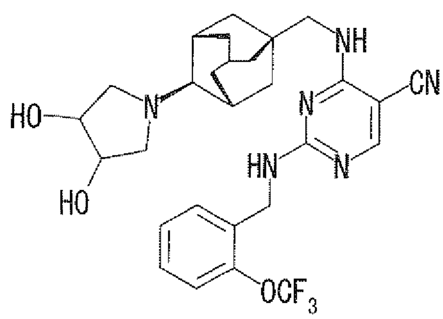 |

(continued)

| Ex | Str | Ex | Str |
|---|---|---|---|
| 13 rel | 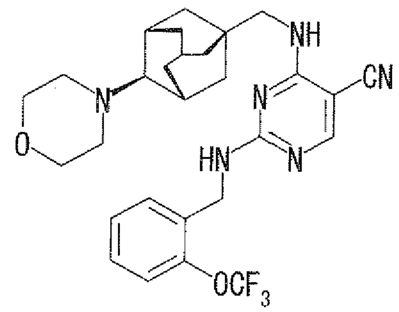 | 14 rel | 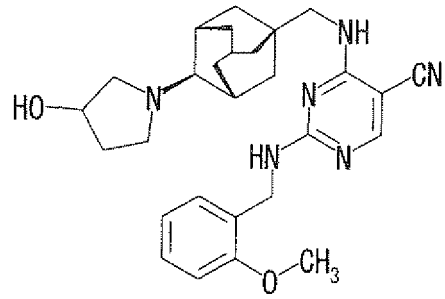 |
| 15 rel | 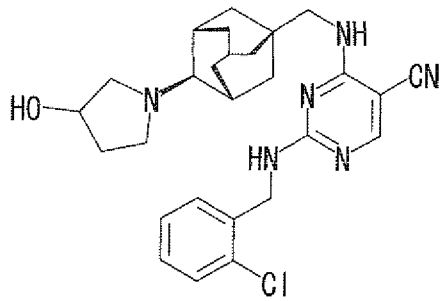 | 16 rel | 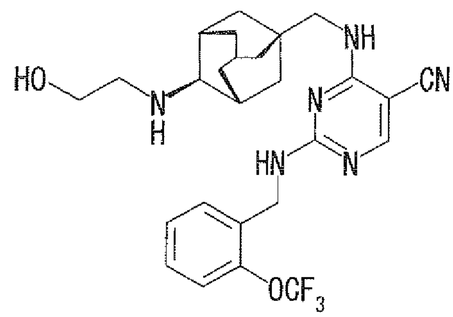 |

[Table 43]

| Ex | Str | Ex | Str |
|---|---|---|---|
| 17 rel | 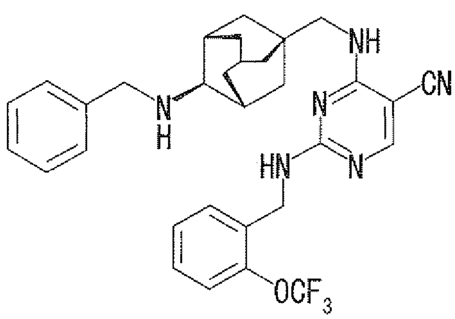 | 18 rel | 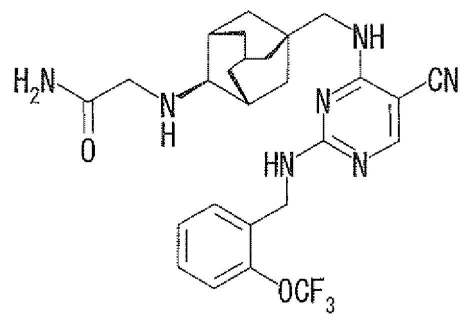 |
| 19 rel | 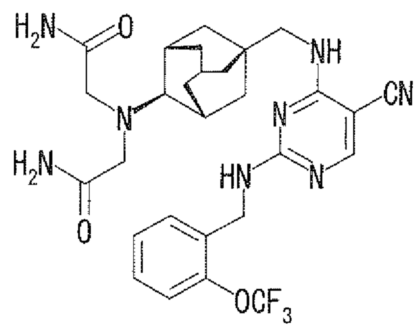 | 20 rel | 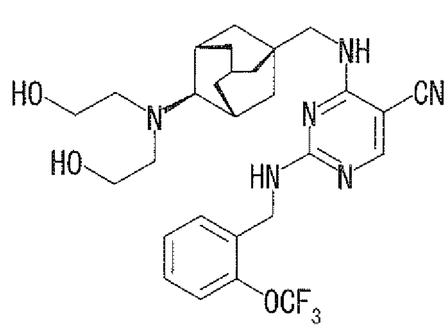 |

(continued)

| Ex | Str | Ex | Str |
|---|---|---|---|
| 21 rel | 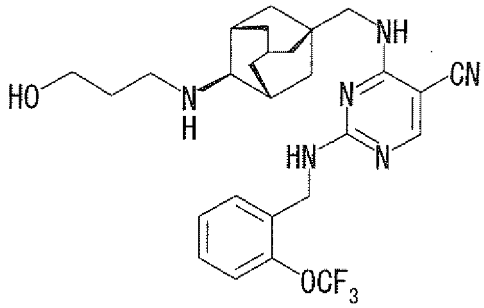 | 22 rel | 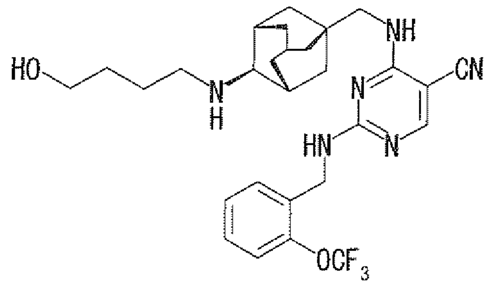 |
| 23 rel | 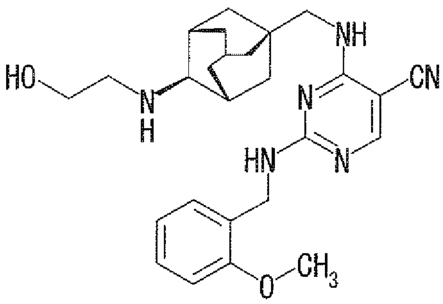 | 24 rel | 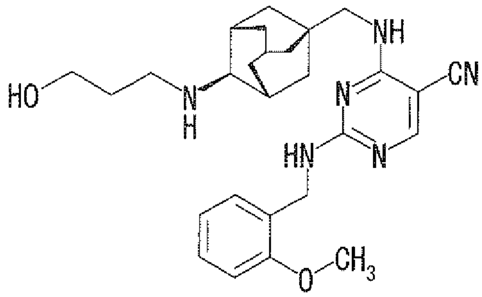 |

[Table 44]

| Ex | Str | Ex | Str |
|---|---|---|---|
| 25 rel | 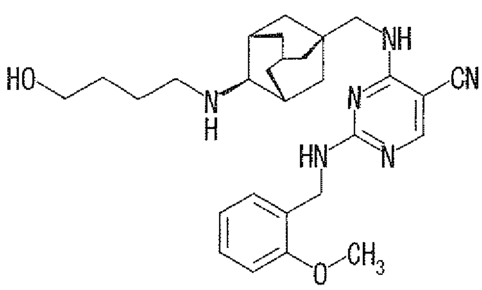 | 26 rel | 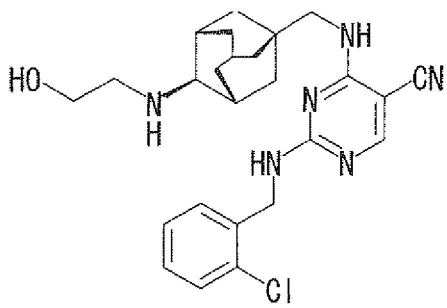 |
| 27 rel | 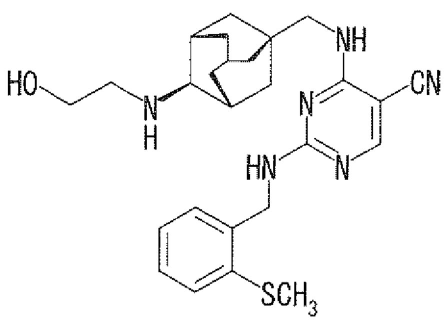 | 28 rel | 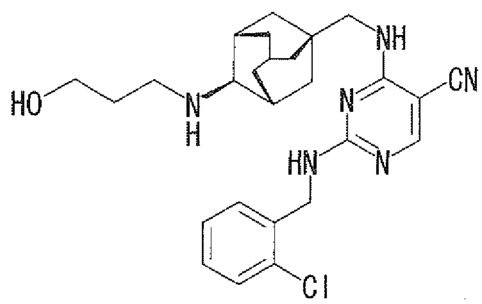 |

(continued)

| Ex | Str | Ex | Str |
|---|---|---|---|
| 29 rel | 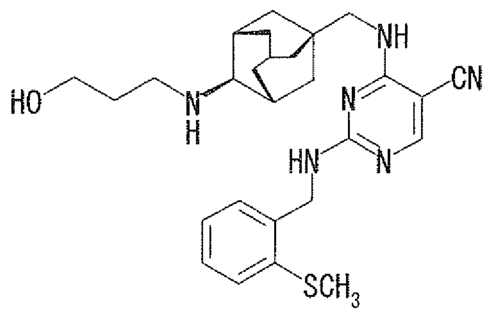 | 30 rel | 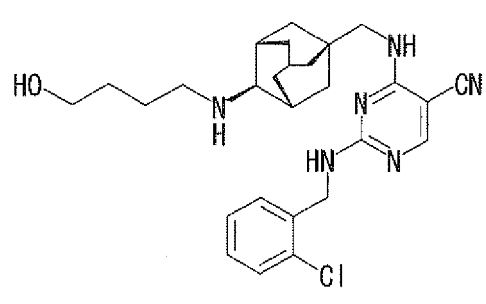 |
| 31 rel | 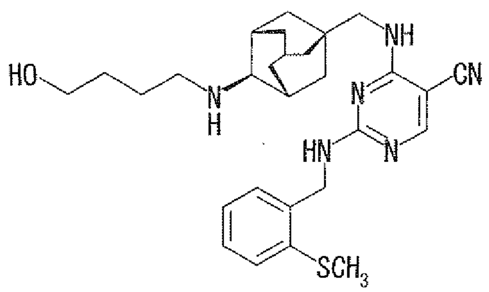 | 32 rel | 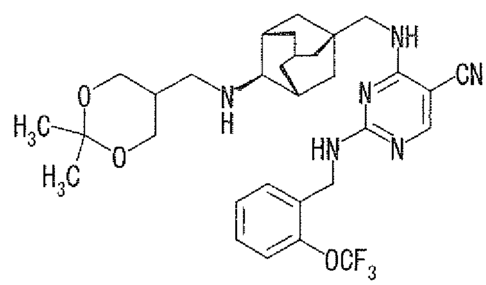 |

[Table 45]

| Ex | Str | Ex | Str |
|---|---|---|---|
| 33 rel | 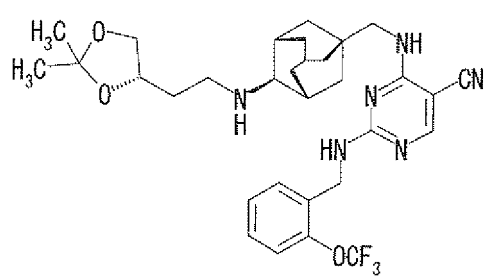 | 34 rel | 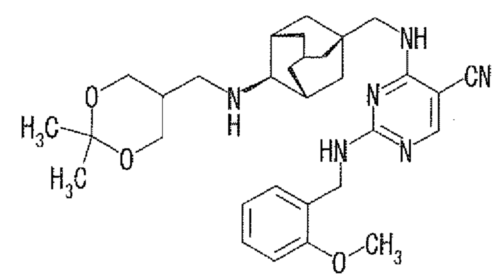 |
| 35 rel | 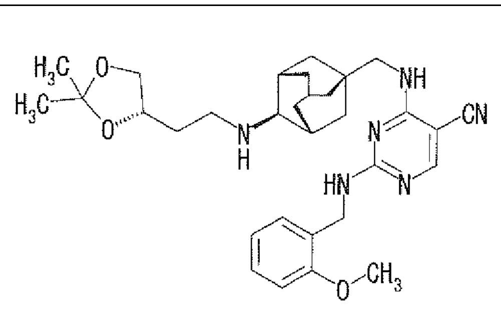 | 36 rel | 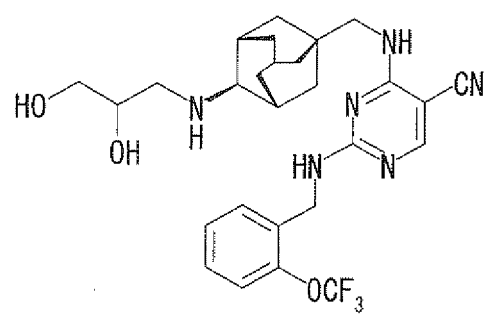 |

(continued)

| Ex | Str | Ex | Str |
|---|---|---|---|
| 37 rel | 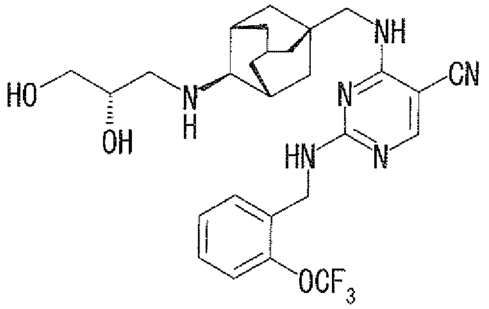 | 38 rel | 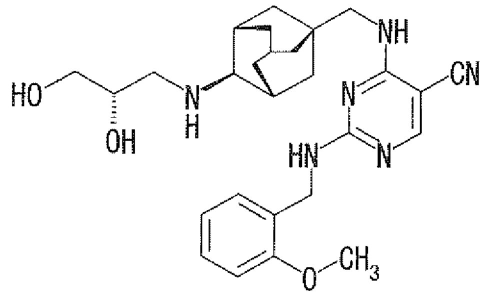 |
| 39 rel | 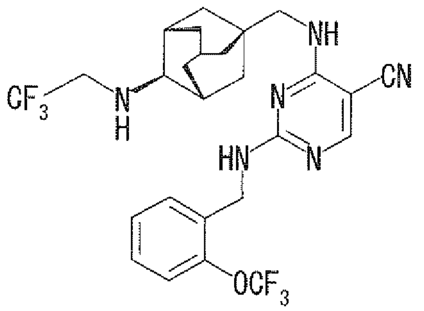 | 40 rel | 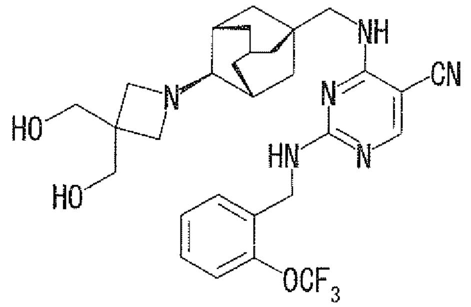 |

[Table 46]

| Ex | Str | Ex | Str |
|---|---|---|---|
| 41 rel | 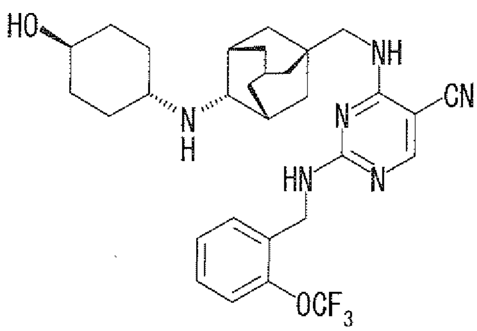 | 42 rel | 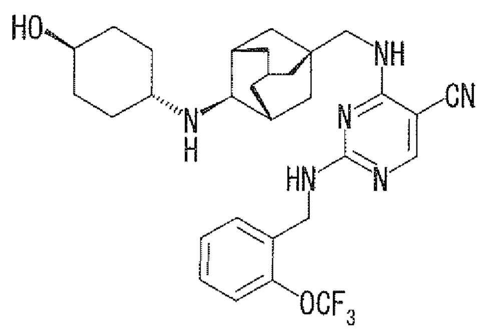 |
| 43 rel | 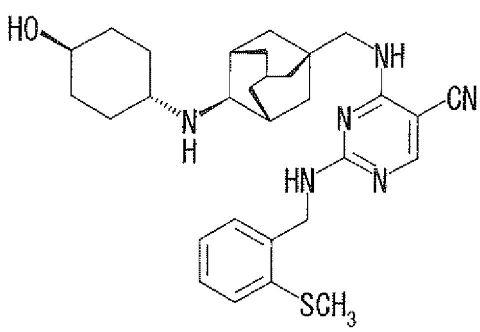 | 44 rel | 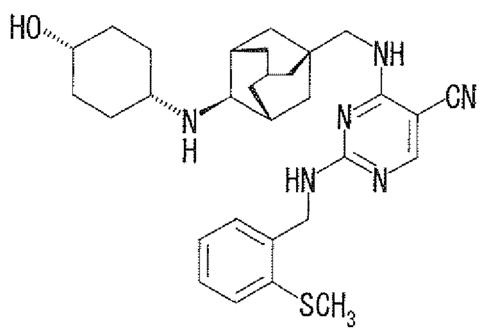 |

(continued)

| Ex | Str | Ex | Str |
|---|---|---|---|
| 45 rel | 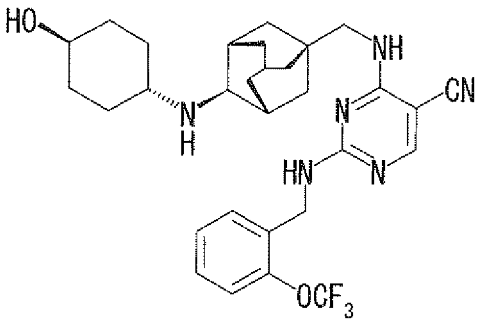 | 46 rel | 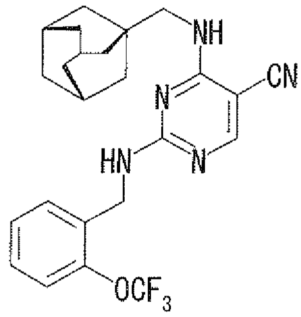 |
| 47 rel | 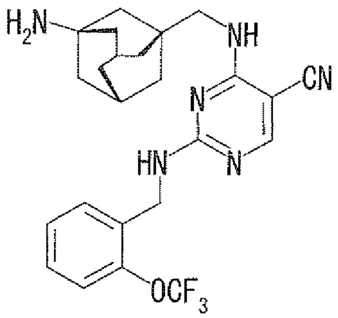 | 48 | 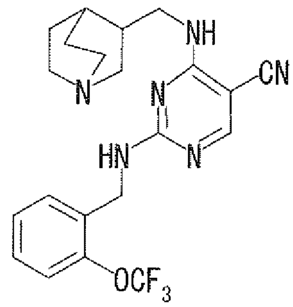 |

[Table 47]

| Ex | Str | Ex | Str |
|---|---|---|---|
| 49 | 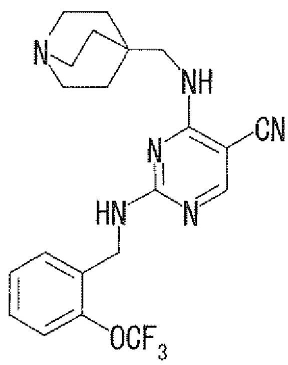 | 50 | 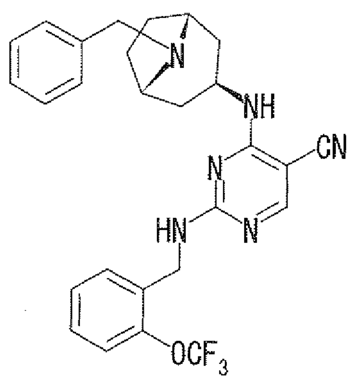 |
| 51 rel | 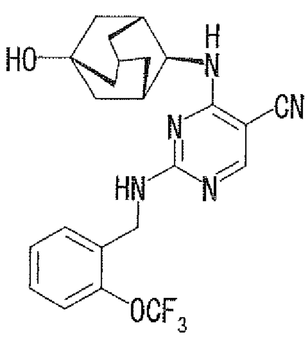 | 52 rel | 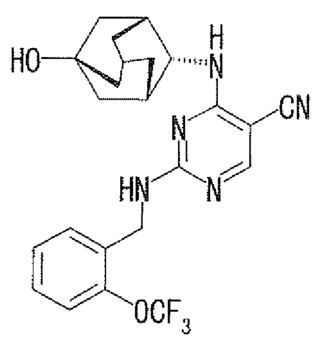 |

(continued)

| Ex | Str | Ex | Str |
|---|---|---|---|
| 53 rel | 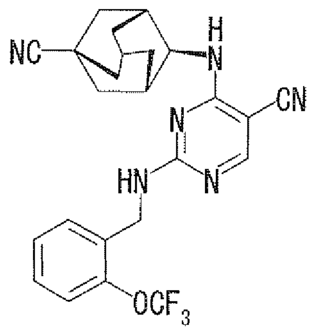 | 54 rel | 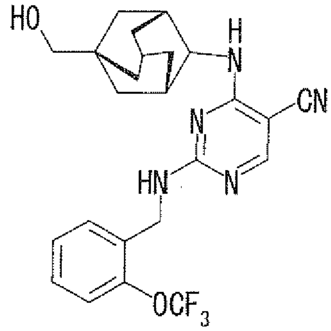 isomer A |
| 55 rel | 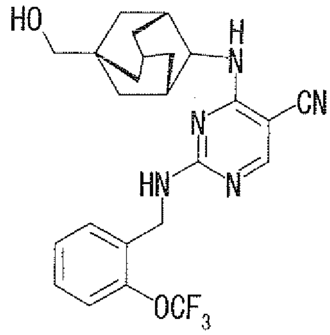 isomer B | 56 | 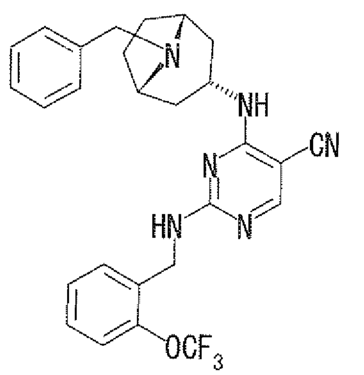 |

[Table 48]

| Ex | Str | Ex | Str |
|---|---|---|---|
| 57 rel | 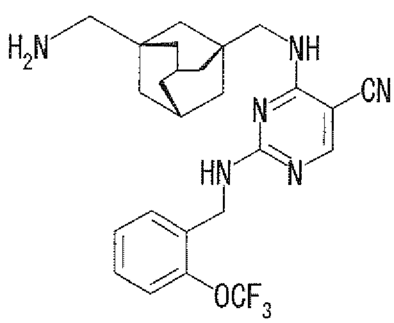 | 58 | 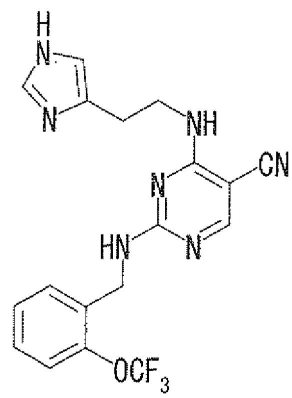 |

(continued)

| Ex | Str | Ex | Str |
|---|---|---|---|
| 59 rel | 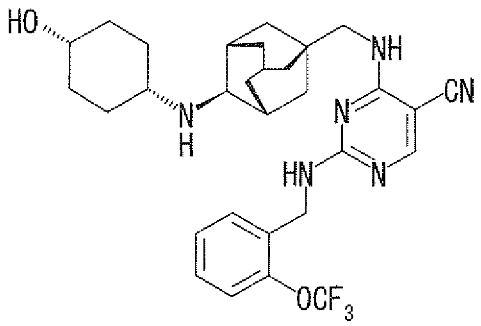 | 60 rel | 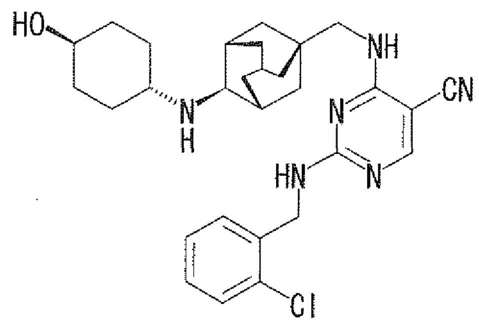 |
| 61 rel | 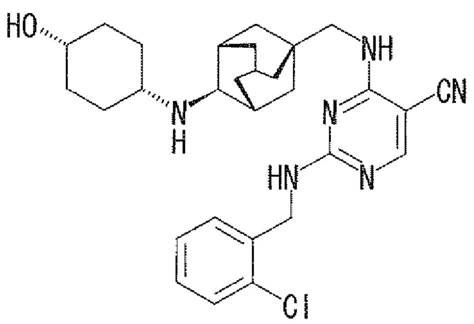 | 62 rel | 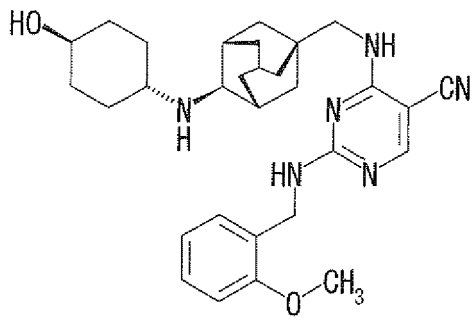 |
| 63 rel | 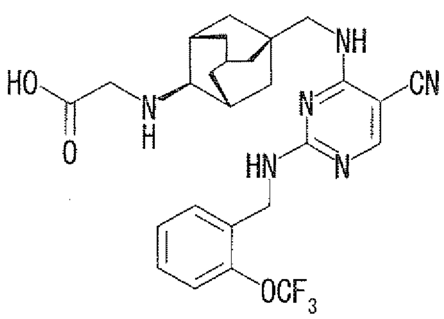 | 64 rel | 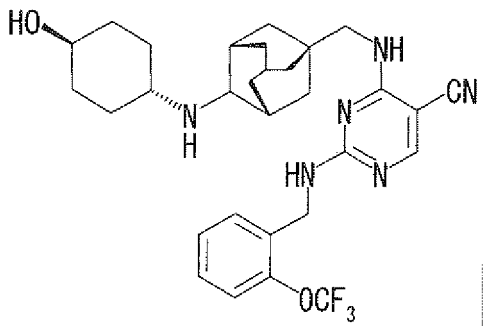 |

[Table 49]

| Ex | Str | Ex | Str |
|---|---|---|---|
| 65 rel | 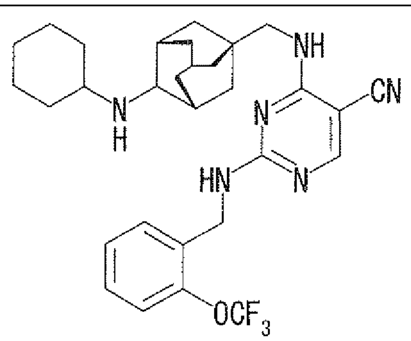 | 66 rel | 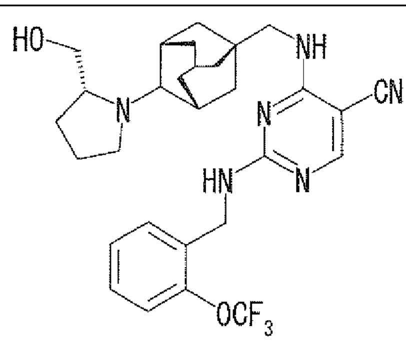 |

(continued)

| Ex | Str | Ex | Str |
|---|---|---|---|
| 67 rel | 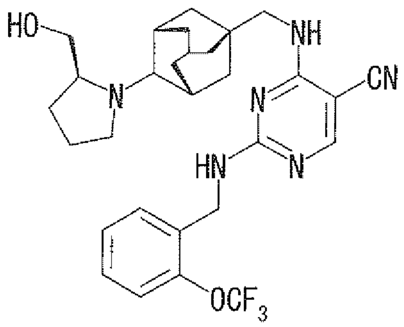 | 68 rel | 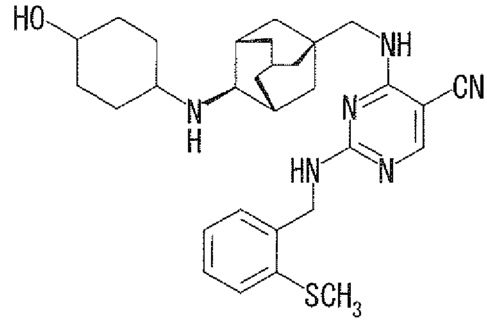 |
| 69 | 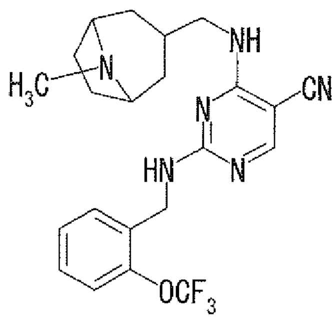 | 70 rel | 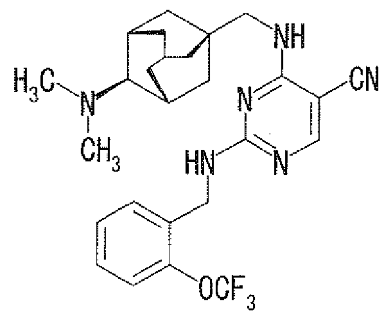 |
| 71 rel | 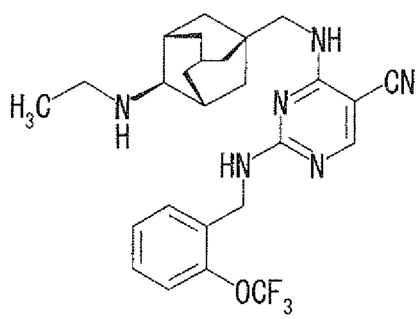 | 72 rel | 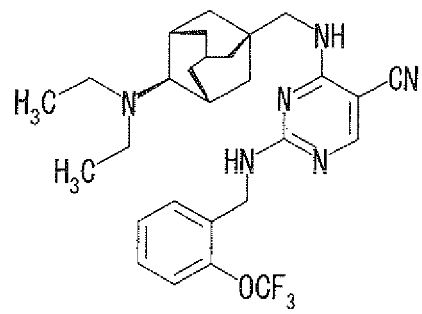 |

[Table 50]

| Ex | Str | Ex | Str |
|---|---|---|---|
| 73 rel | 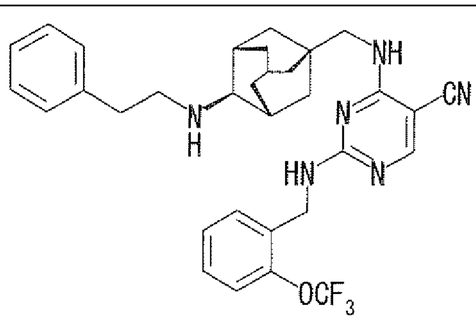 | 74 rel | 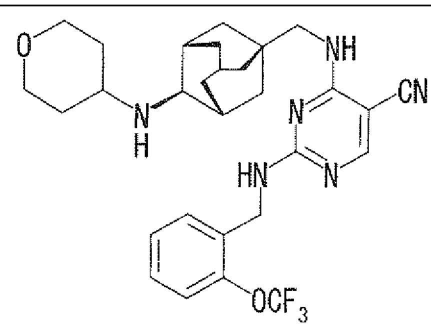 |

(continued)

| Ex | Str | Ex | Str |
|---|---|---|---|
| 75 rel | 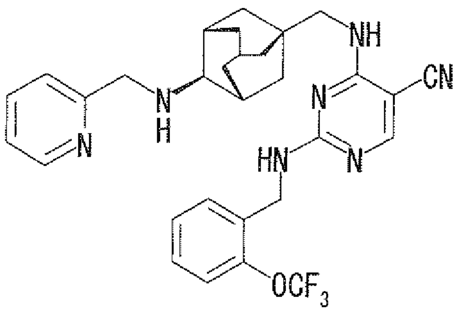 | 76 rel | 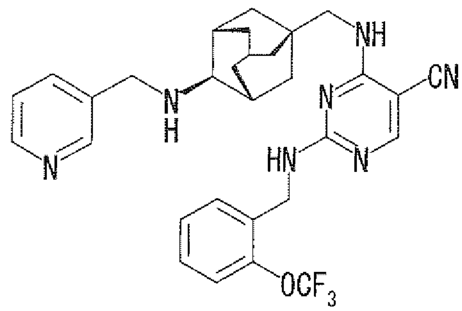 |
| 77 rel | 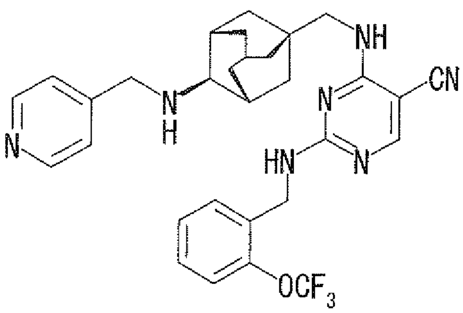 | 78 rel | 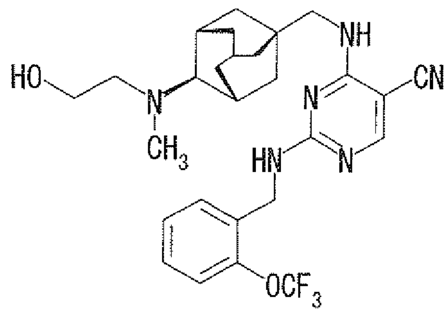 |
| 79 rel | 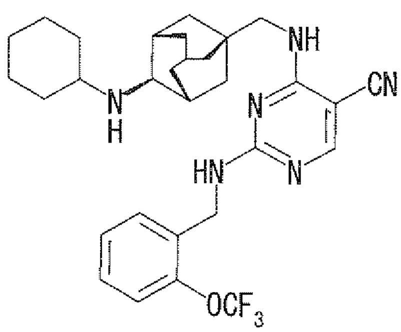 | 80 rel | 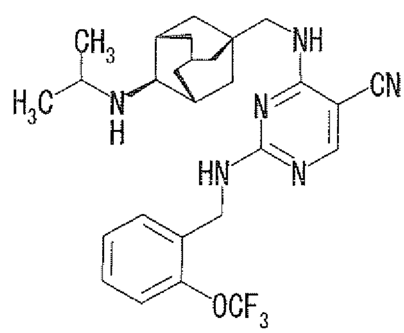 |

[Table 51]

| Ex | Str | Ex | Str |
|---|---|---|---|
| 81 rel | 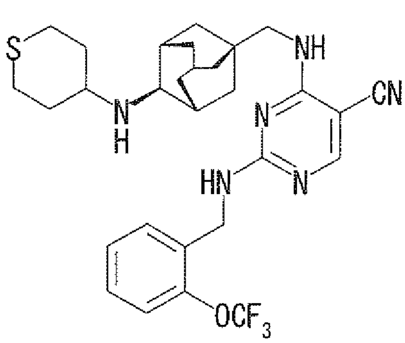 | 82 rel | 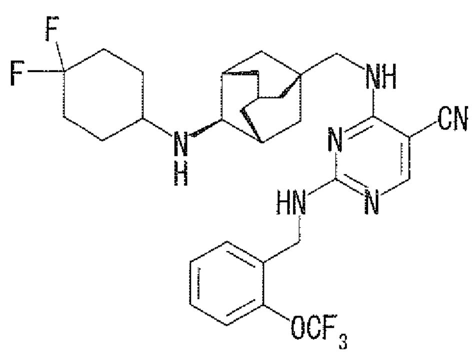 |

(continued)

| Ex | Str | Ex | Str |
|---|---|---|---|
| 83 rel | 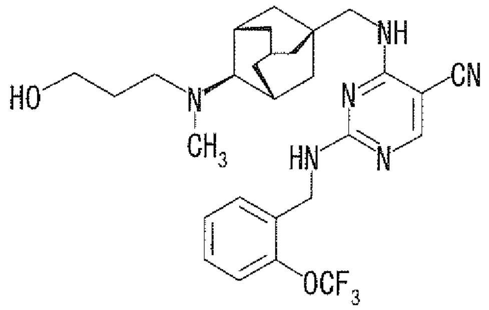 | 84 rel | 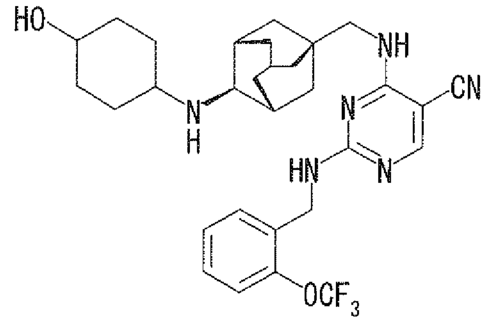 |
| 85 rel | 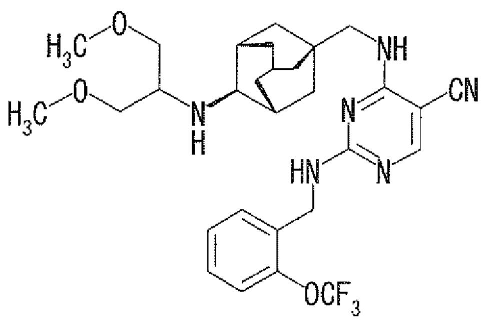 | 86 rel | 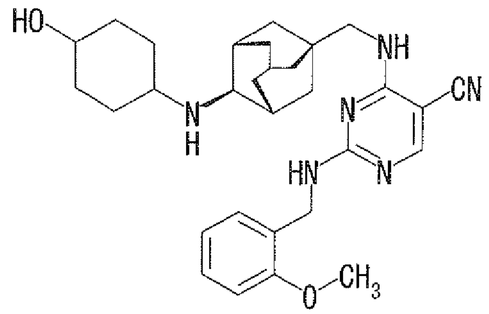 |
| 87 rel | 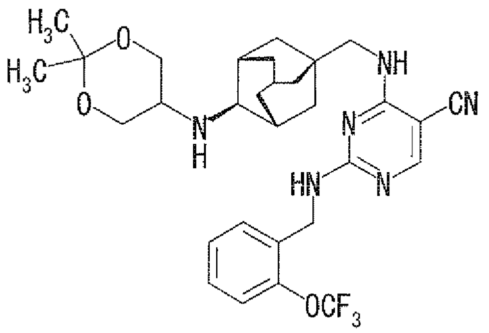 | 88 rel | 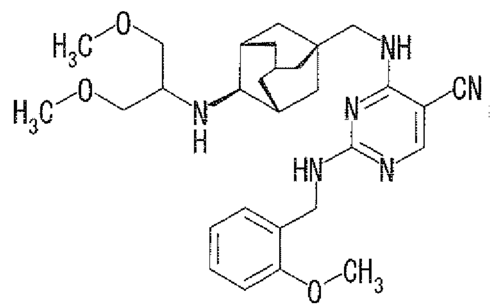 |

[Table 52]

| Ex | Str | Ex | Str |
|---|---|---|---|
| 89 rel | 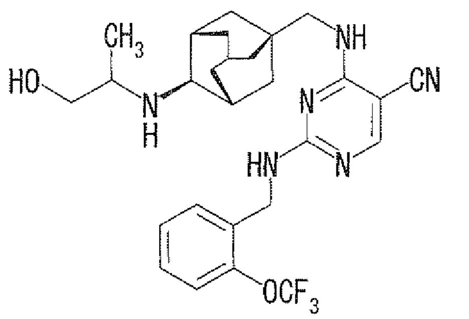 | 90 rel | 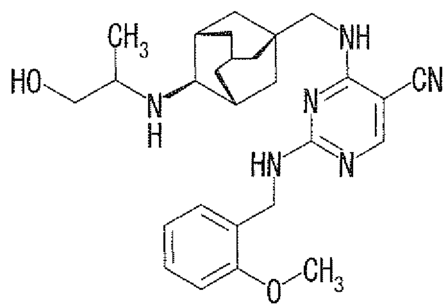 |

(continued)

| Ex | Str | Ex | Str |
|---|---|---|---|
| 91 rel | 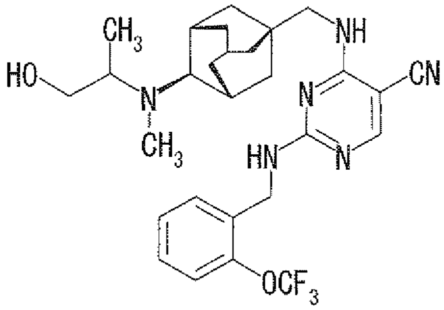 | 92 rel | 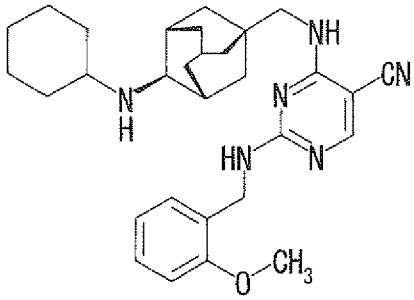 |
| 93 rel | 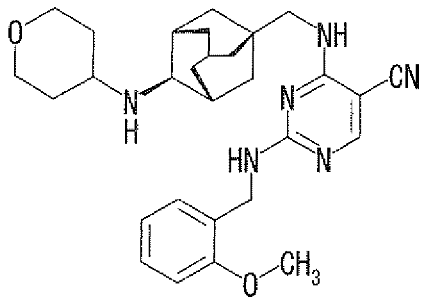 | 94 rel | 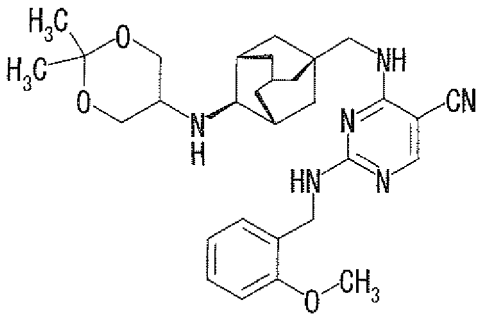 |
| 95 rel | 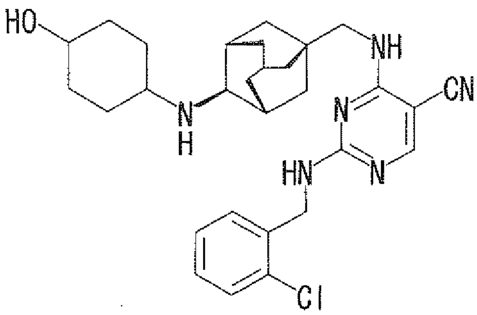 | 96 rel | 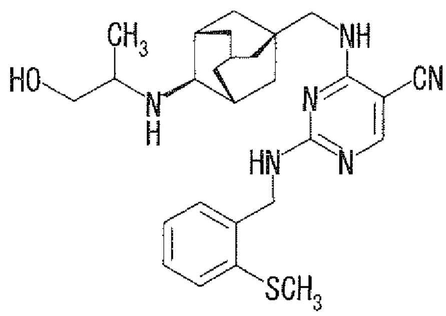 |

[Table 53]

| Ex | Str | Ex | Str |
|---|---|---|---|
| 97 rel | 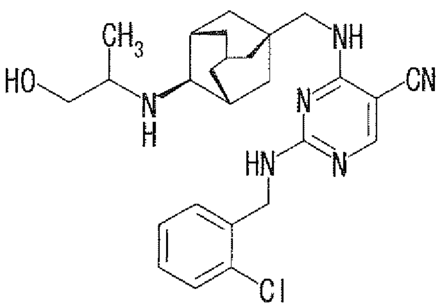 | 98 rel | 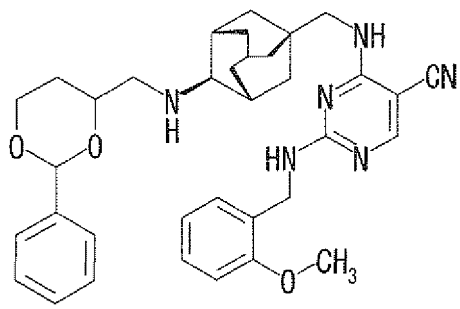 |

(continued)

| Ex | Str | Ex | Str |
|---|---|---|---|
| 99 rel | 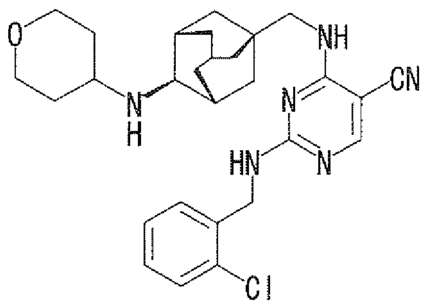 | 100 rel | 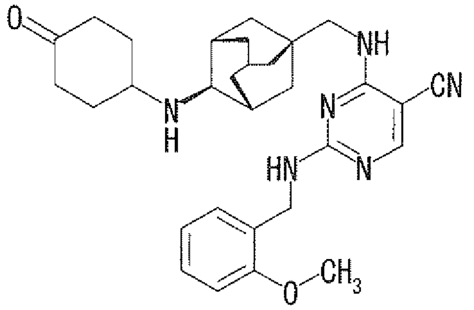 |
| 101 rel | 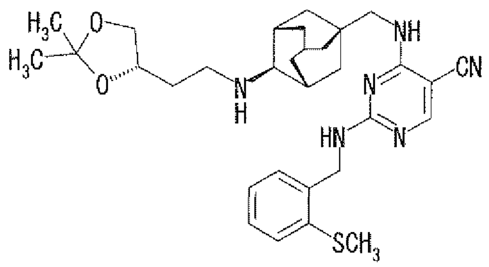 | 102 rel | 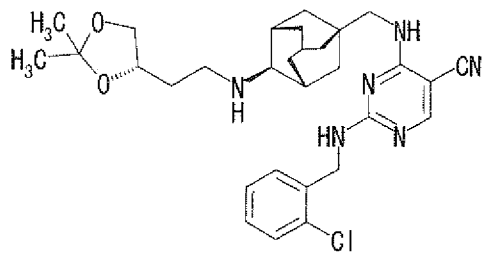 |
| 103 rel | 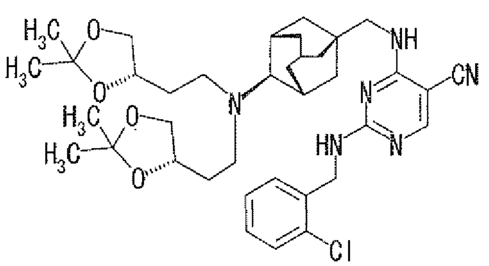 | 104 rel | 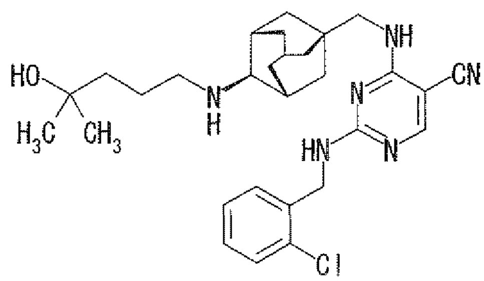 |

[Table 54]

| Ex | Str | Ex | Str |
|---|---|---|---|
| 105 rel | 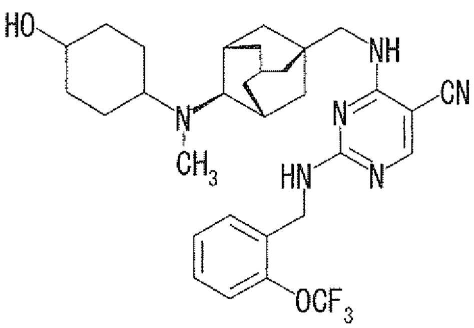 | 106 rel | 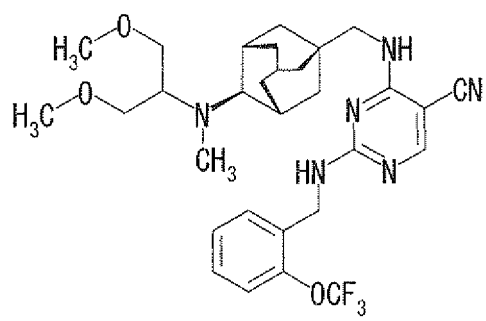 |

(continued)

| Ex | Str | Ex | Str |
|---|---|---|---|
| 107 rel | 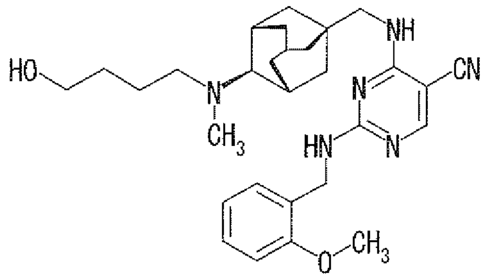 | 108 rel | 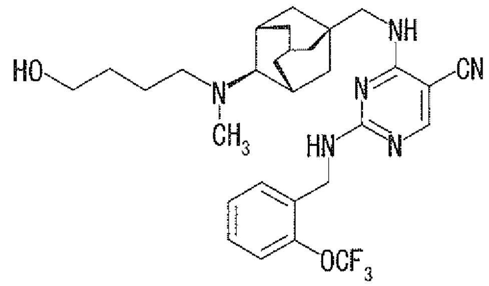 |
| 109 rel | 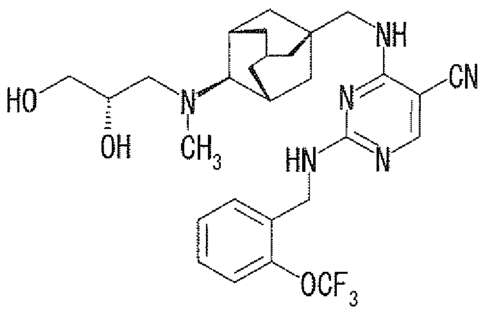 | 110 rel | 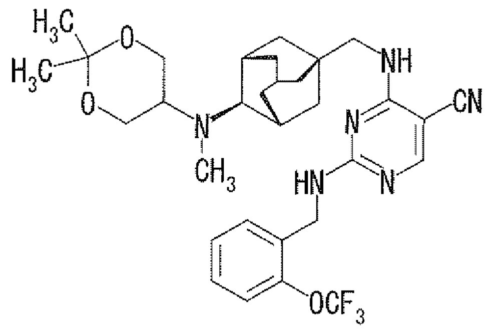 |
| 111 rel | 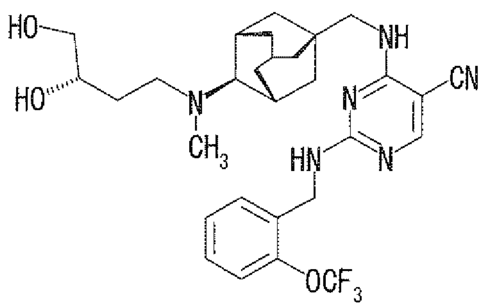 | 112 rel | 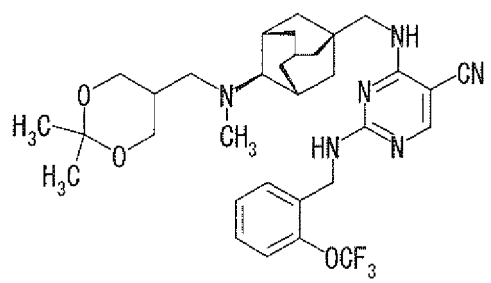 |

[Table 55]

| Ex | Str | Ex | Str |
|---|---|---|---|
| 113 rel | 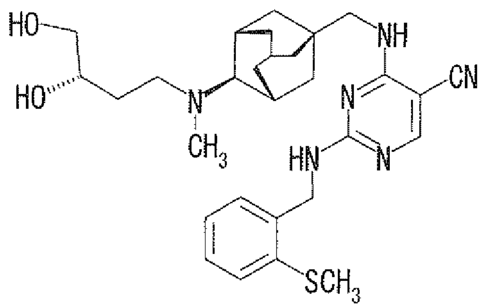 | 114 rel | 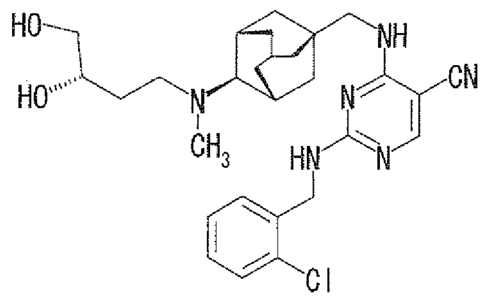 |

(continued)

| Ex | Str | Ex | Str |
|---|---|---|---|
| 115 rel | 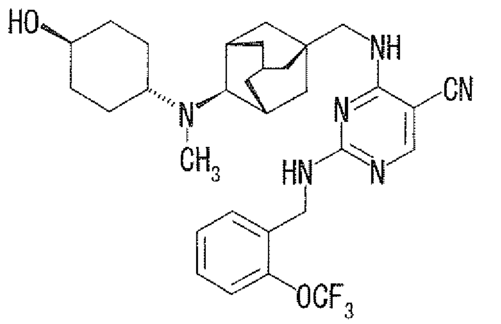 | 116 rel | 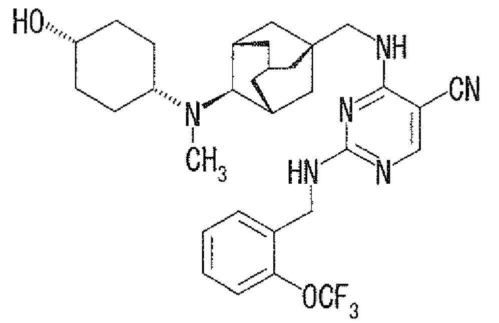 |
| 117 rel | 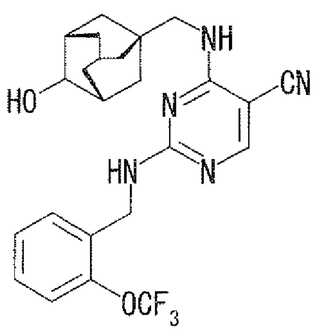 | 118 rel | 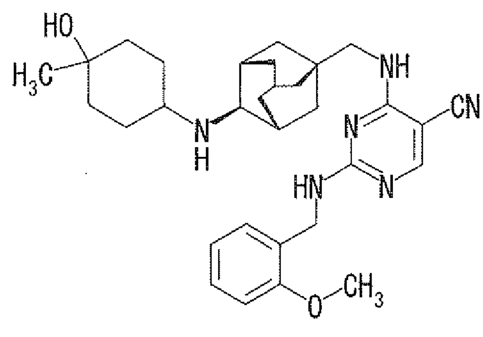 isomer A |
| 119 rel | 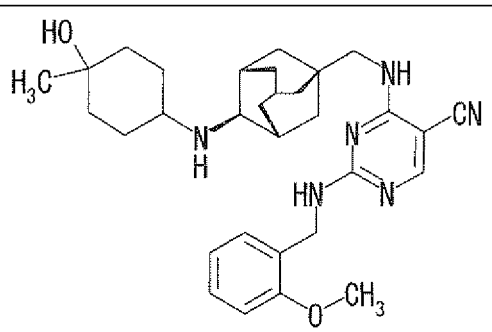 isomer B | 120 | 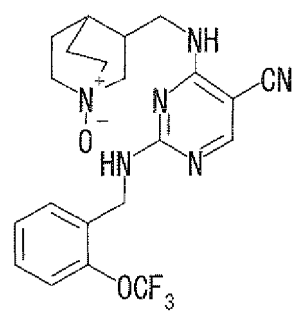 |

[Table 56]

| Ex | Str | Ex | Str |
|---|---|---|---|
| 121 rel | 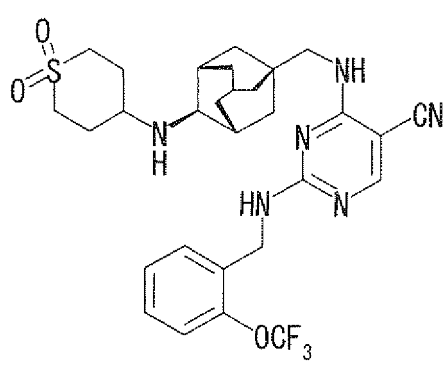 | 122 | 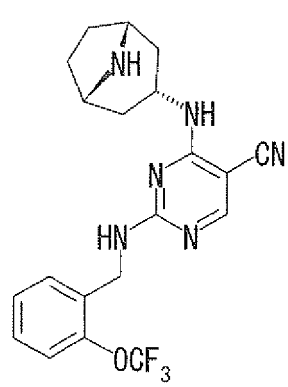 |

(continued)

| Ex | Str | Ex | Str |
|---|---|---|---|
| 123 rel | 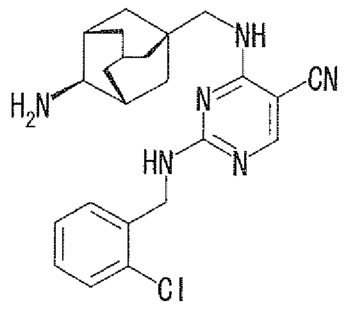 | 124 | 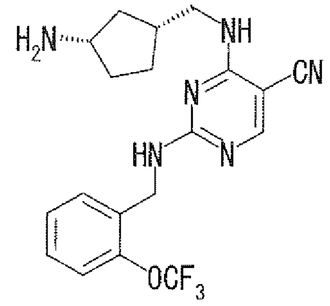 |
| 125 | 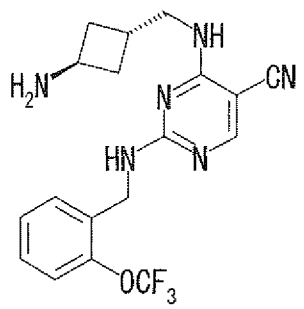 | 126 | 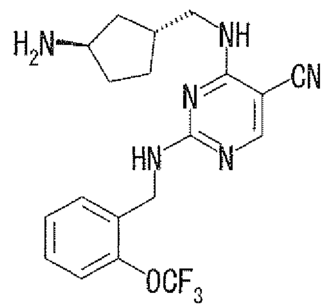 |
| 127 rel | 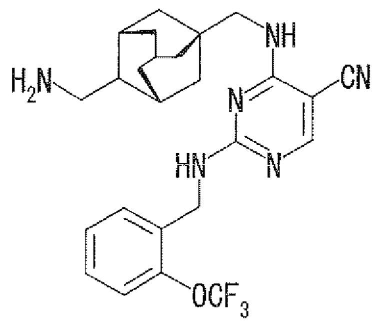 | 128 rel | 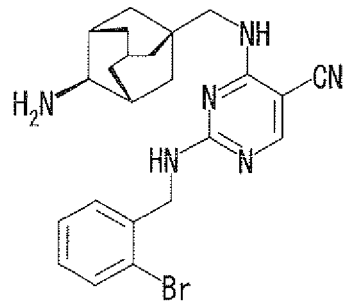 |

[Table 57]

| Ex | Str | Ex | Str |
|---|---|---|---|
| 129 rel | 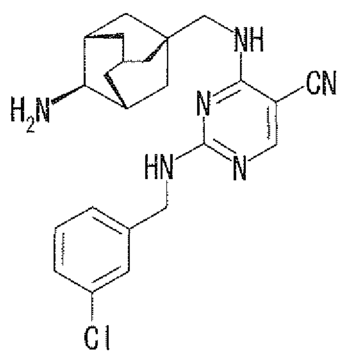 | 130 rel | 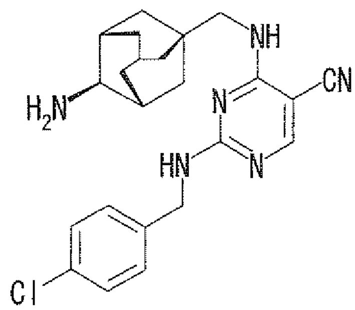 |

(continued)

| Ex | Str | Ex | Str |
|---|---|---|---|
| 131 rel | 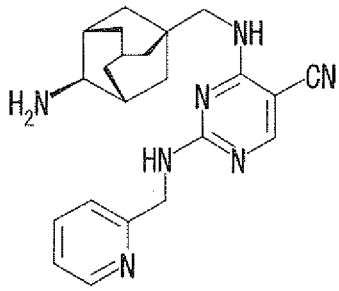 | 132 rel | 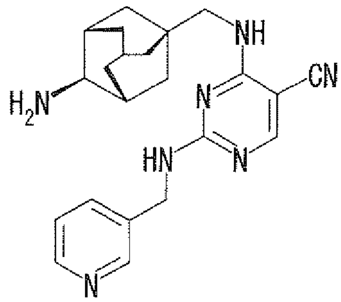 |
| 133 rel | 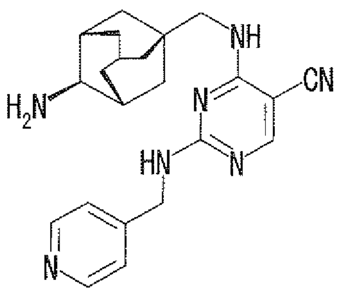 | 134 rel | 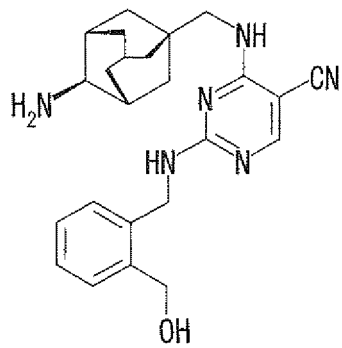 |
| 135 rel | 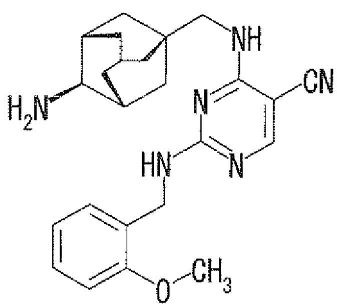 | 136 rel | 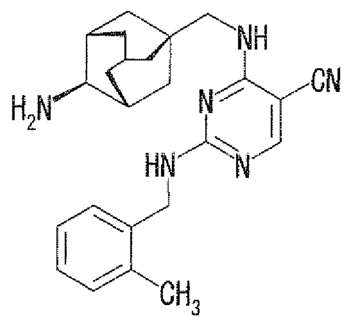 |

[Table 58]

| Ex | Str | Ex | Str |
|---|---|---|---|
| 137 rel | 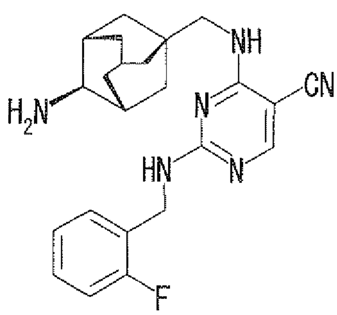 | 138 rel | 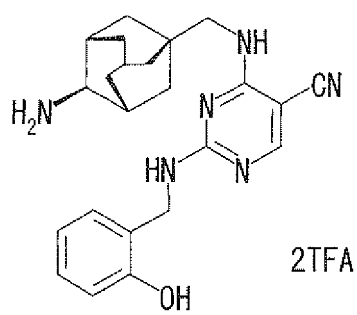 |

(continued)

| Ex | Str | Ex | Str |
|---|---|---|---|
| 139 rel | 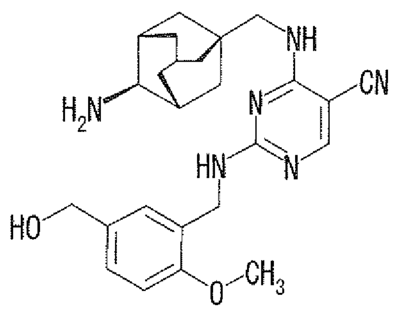 | 140 rel | 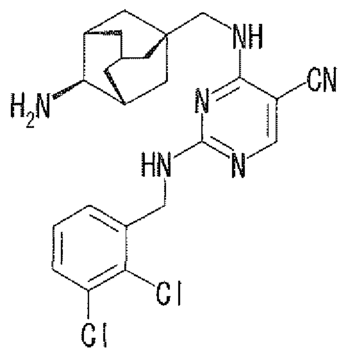 |
| 141 rel | 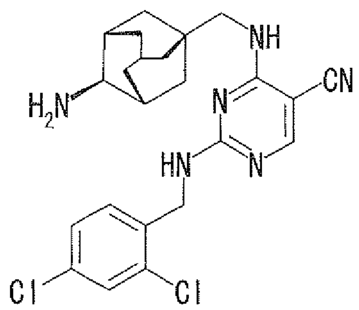 | 142 rel | 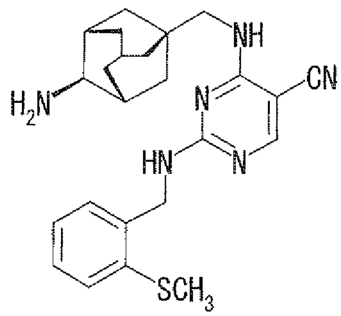 |
| 143 rel | 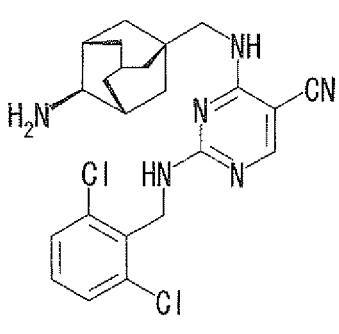 | 144 rel | 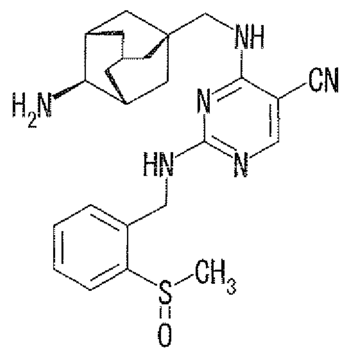 |

[Table 59]

| Ex | Str | Ex | Str |
|---|---|---|---|
| 145 rel | 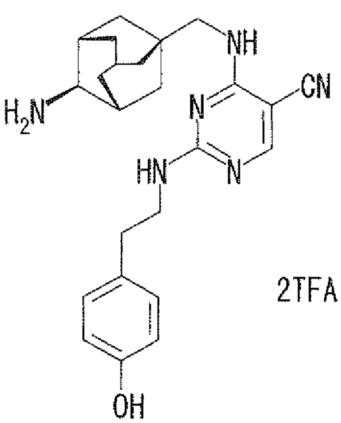 | 146 rel | 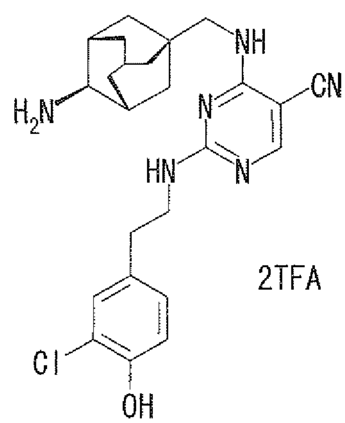 |

(continued)

| Ex | Str | Ex | Str |
|---|---|---|---|
| 147 rel | 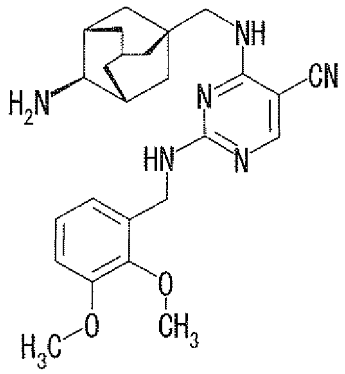 | 148 rel | 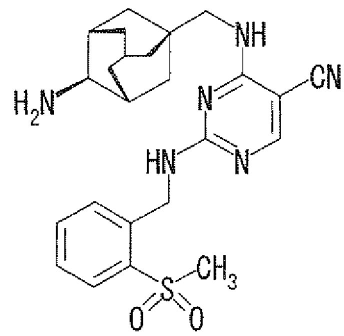 |
| 149 rel | 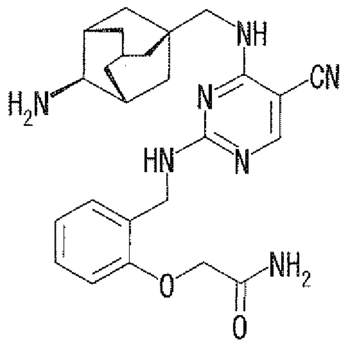 | 150 rel | 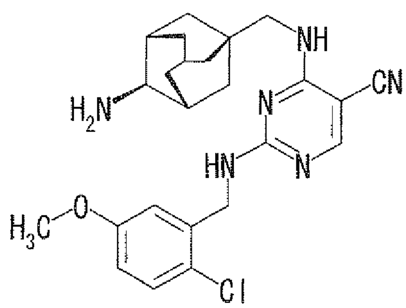 |
| 151 rel | 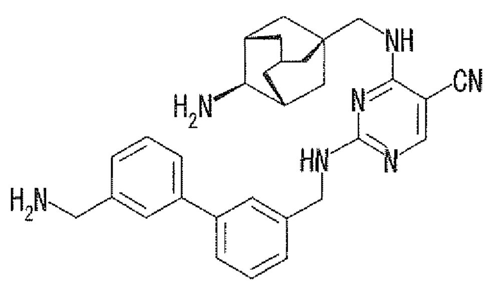 | 152 rel | 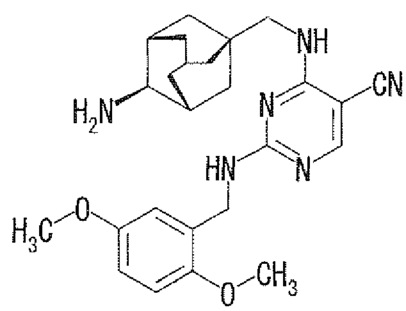 |

[Table 60]

| Ex | Str | Ex | Str |
|---|---|---|---|
| 153 rel | 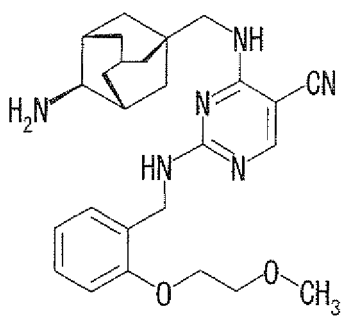 | 154 rel | 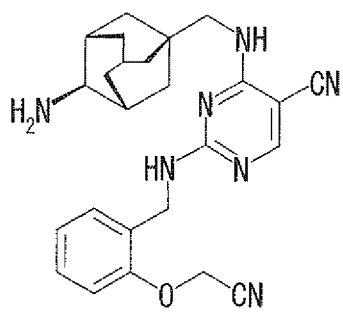 |

(continued)

| Ex | Str | Ex | Str |
|---|---|---|---|
| 155 rel | 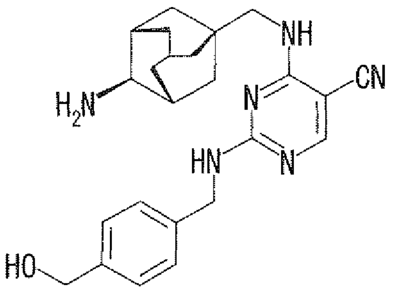 | 156 | 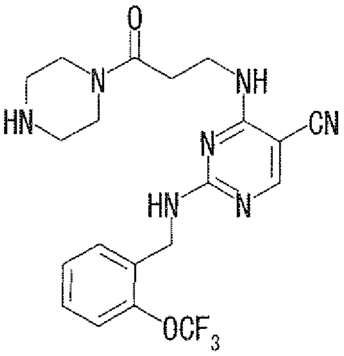 |
| 157 rel | 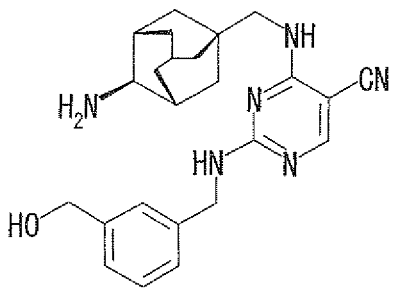 | 158 rel | 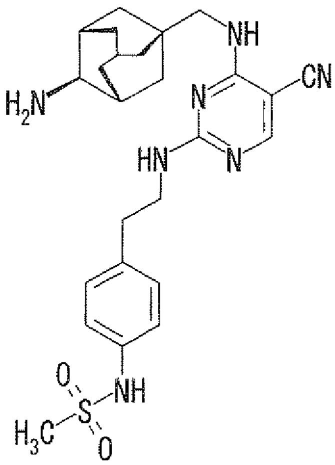 |
| 159 rel | 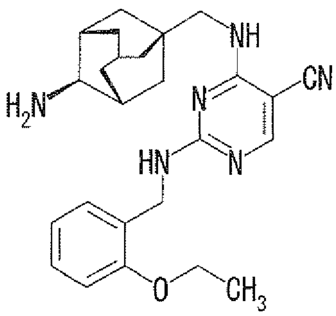 | 160 rel | 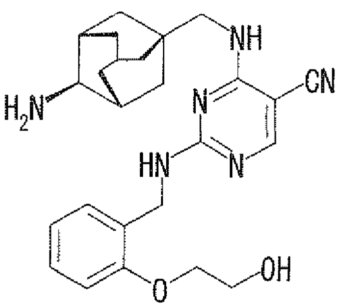 |

[Table 61]

| Ex | Str | Ex | Str |
|---|---|---|---|
| 161 | 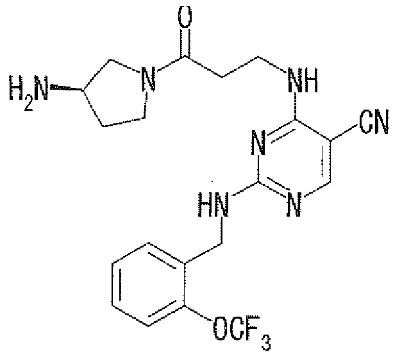 | 162 | 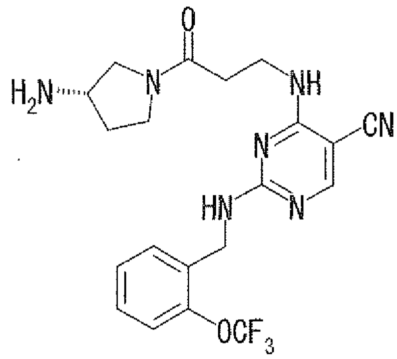 |
| 163 | 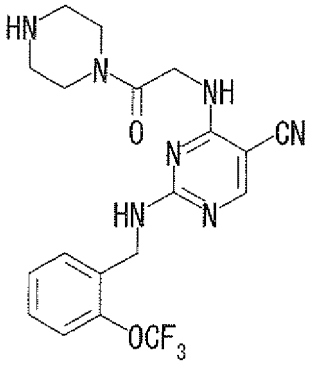 | 164 rel | 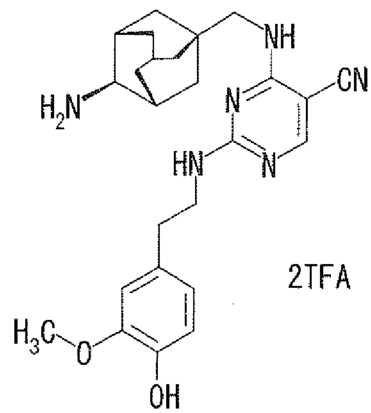 |
| 165 rel | 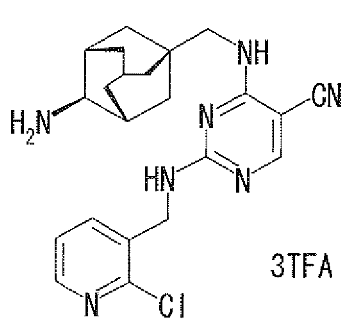 | 166 rel | 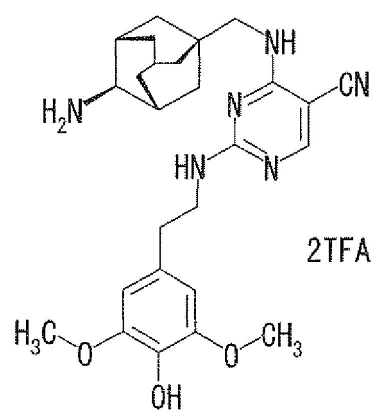 |
| 167 rel | 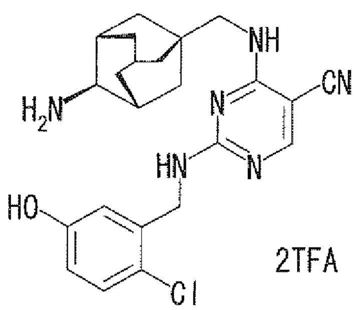 | 168 rel | 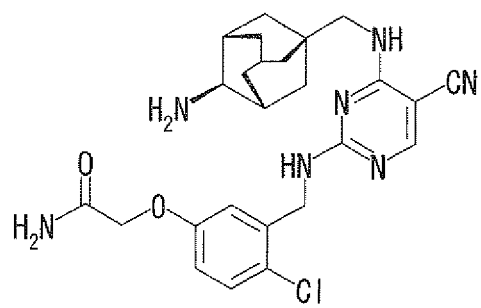 |

[Table 62]

| Ex | Str | Ex | Str |
|---|---|---|---|
| 169 rel | 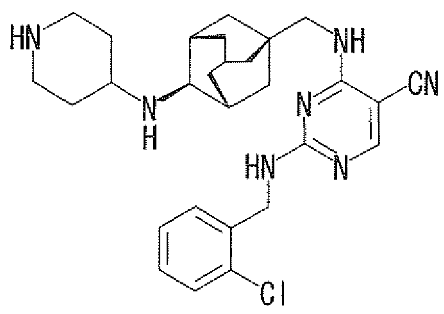 | 170 | 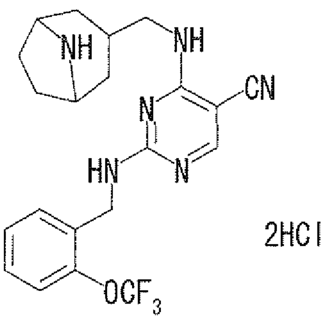 |
| 171 | 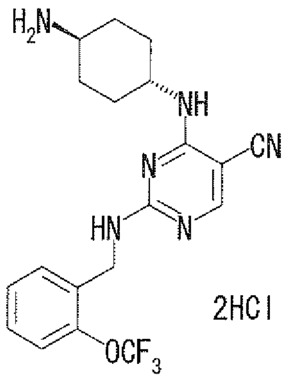 | 172 | 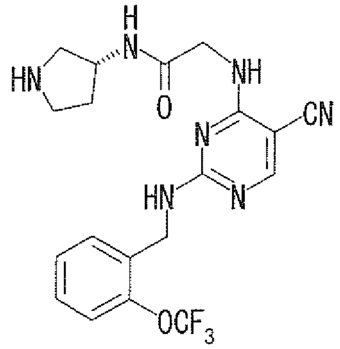 |
| 173 | 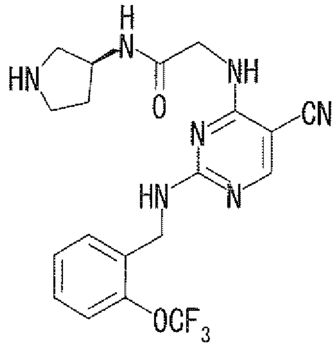 | 174 | 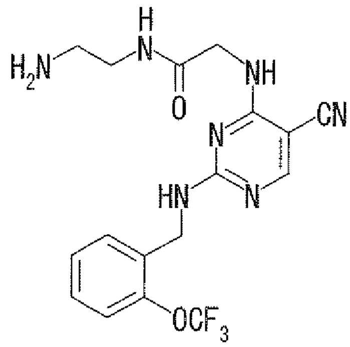 |
| 175 | 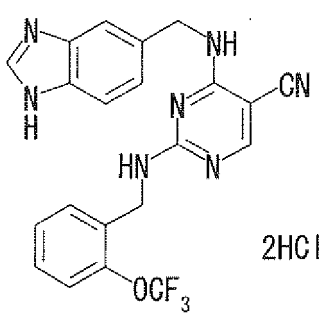 | 176 rel | 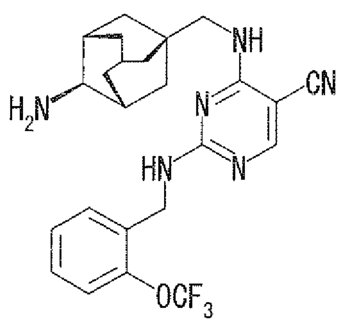 |

[Table 63]

| Ex | Str | Ex | Str |
|---|---|---|---|
| 177 rel | 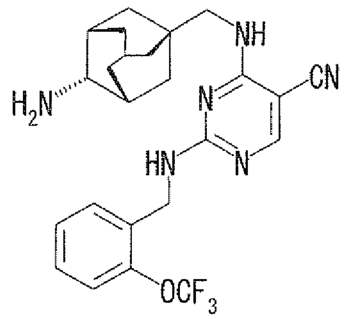 | 178 rel | 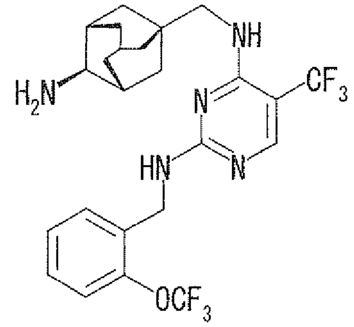 |
| 179 rel | 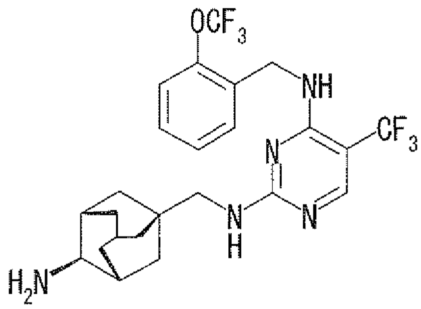 | 180 rel | 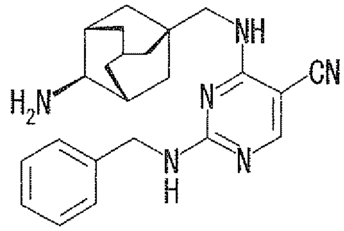 |
| 181 rel | 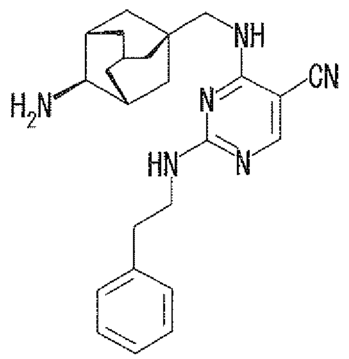 | 182 rel | 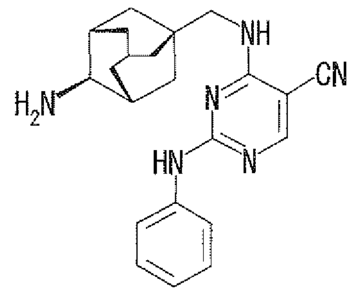 |
| 183 rel | 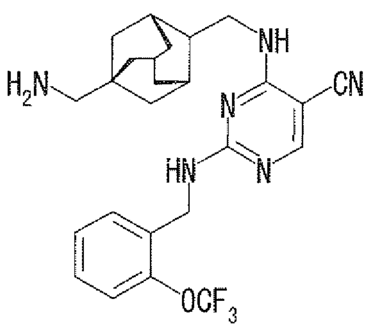 | 184 rel | 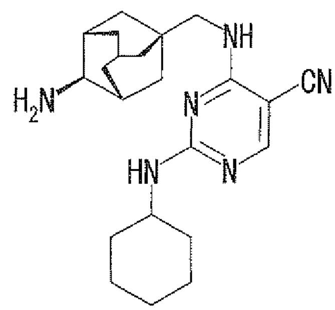 |

[Table 64]

| Ex | Str | Ex | Str |
|---|---|---|---|
| 185 rel | 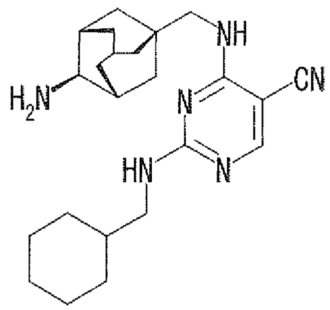 | 186 rel | 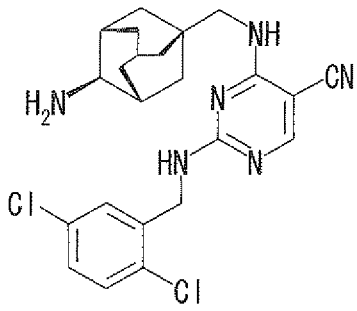 |
| 187 rel | 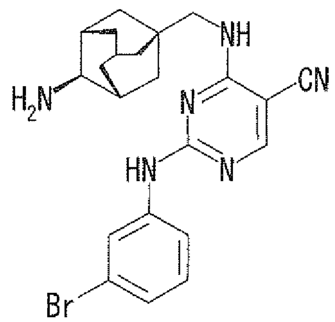 | 188 rel | 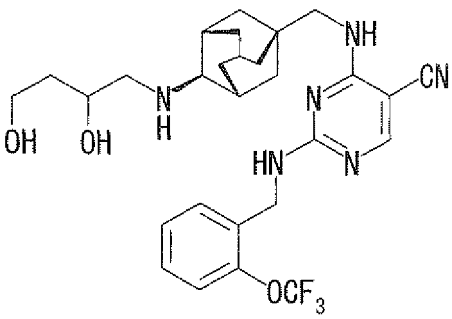 |
| 189 rel | 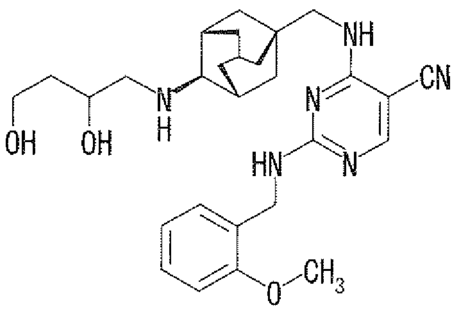 | 190 rel | 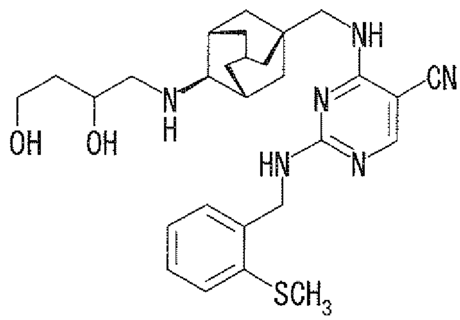 |
| 191 rel | 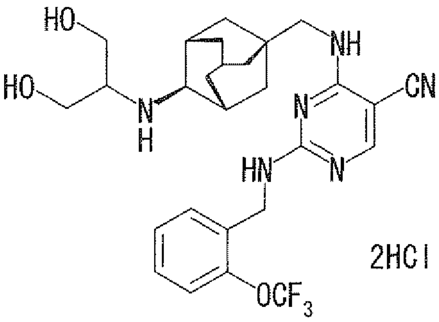 | 192 rel | 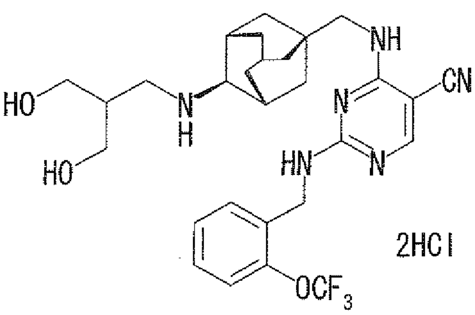 |

[Table 65]

| Ex | Str | Ex | Str |
|---|---|---|---|
| 193 rel | 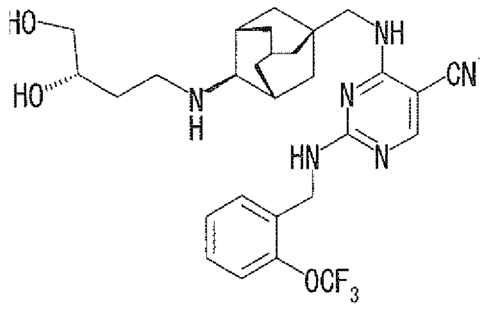 | 194 rel | 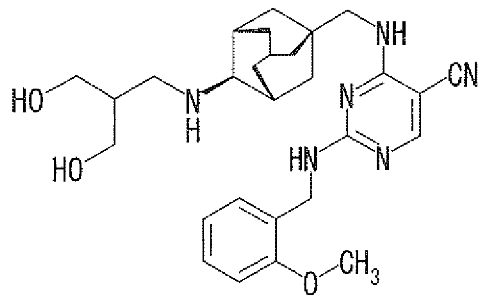 |
| 195 rel | 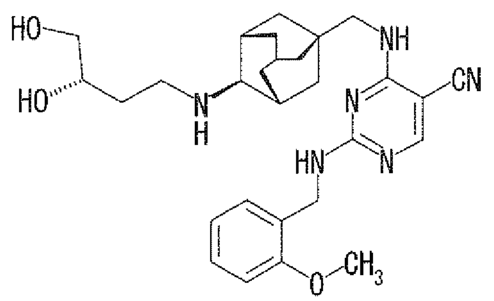 | 196 rel | 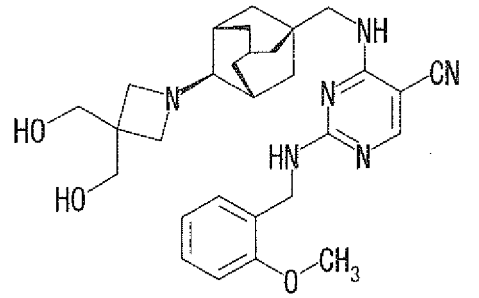 |
| 197 rel | 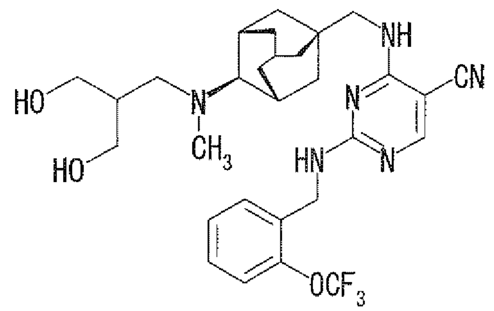 | 198 rel | 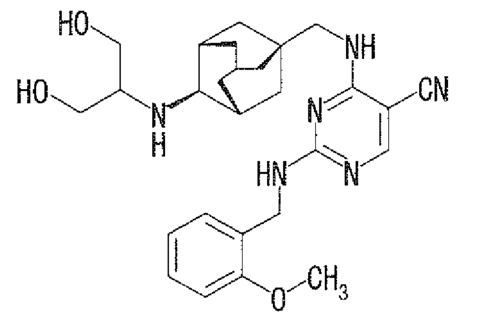 |
| 199 rel | 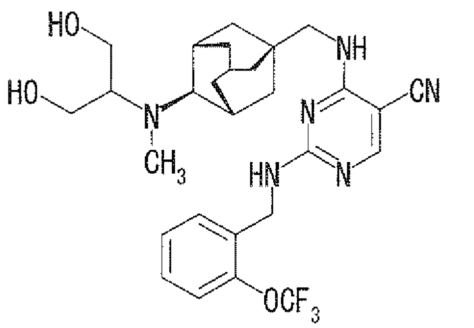 | 200 rel | 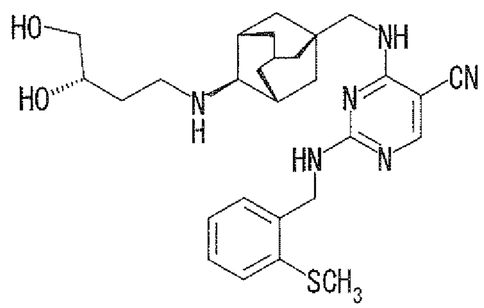 |

[Table 66]

| Ex | Str | Ex | Str |
|---|---|---|---|
| 201 rel | 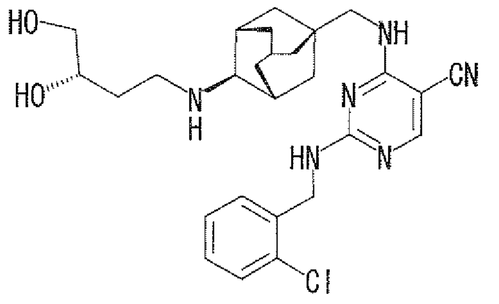 | 202 rel | 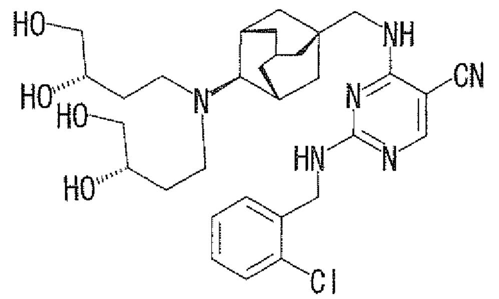 |
| 203 rel | 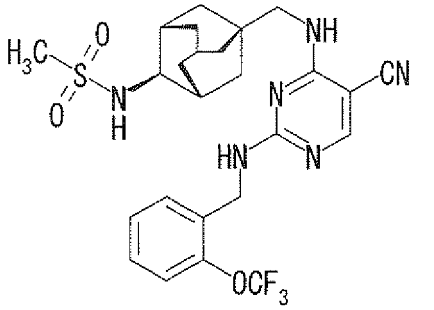 | 204 rel | 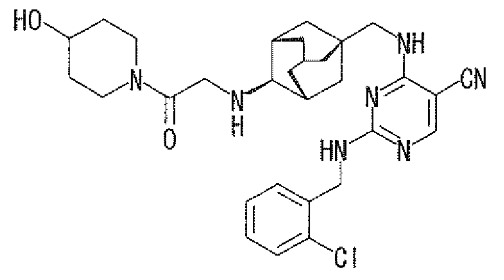 |
| 205 rel | 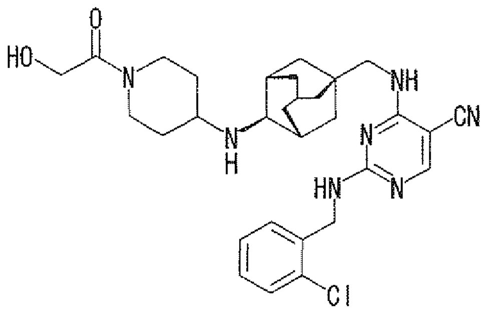 | 206 rel | 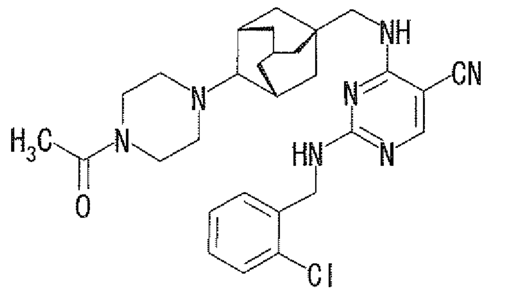 |
| 207 rel | 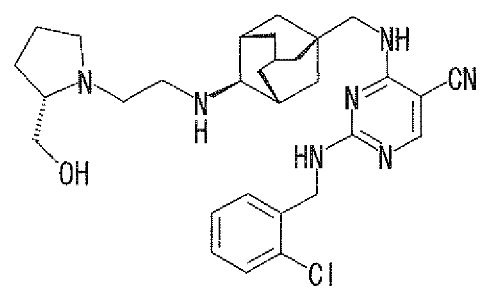 | 208 rel | 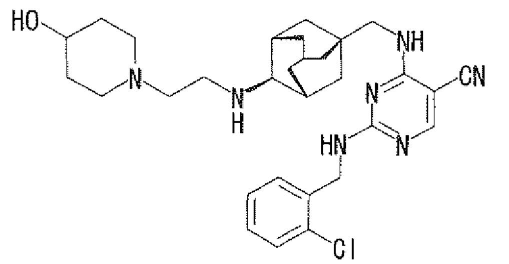 |
| 209 rel | 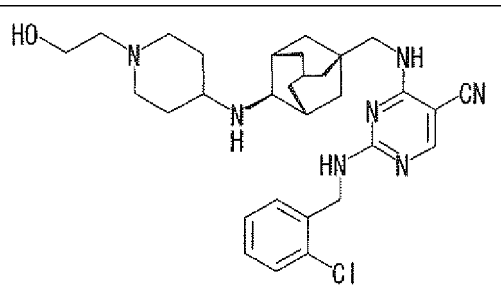 | 210 rel | 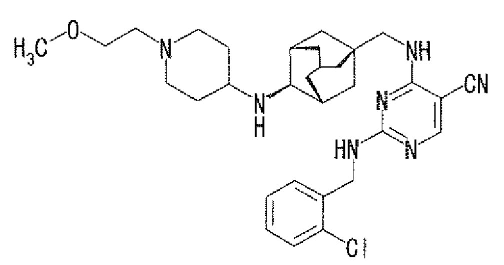 |

[Table 67]

| Ex | Str | Ex | Str |
|---|---|---|---|
| 211 rel | 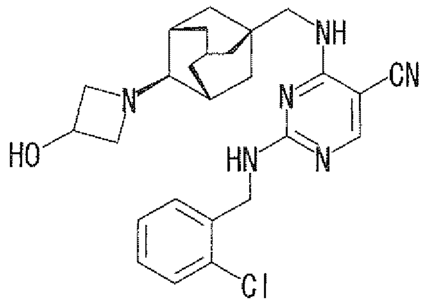 | 212 rel | 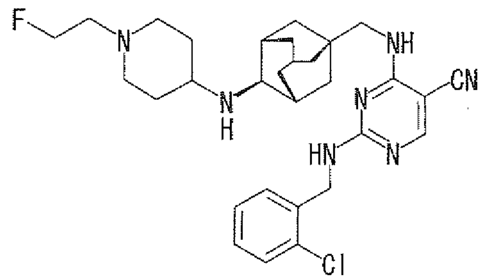 |
| 213 rel | 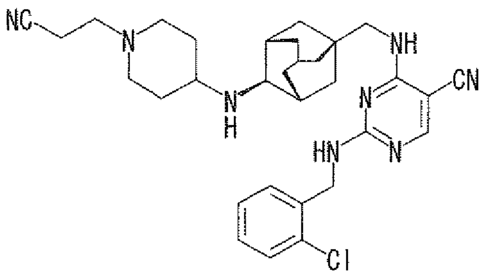 | 214 rel | 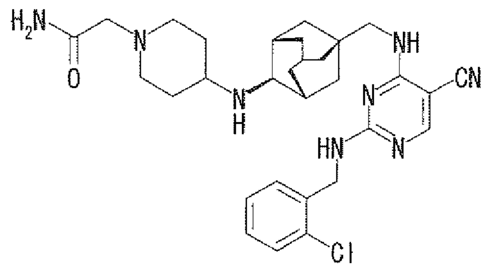 |
| 215 rel | 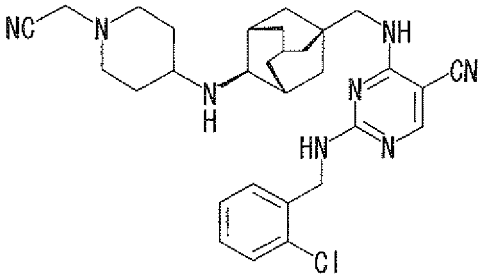 | 216 rel | 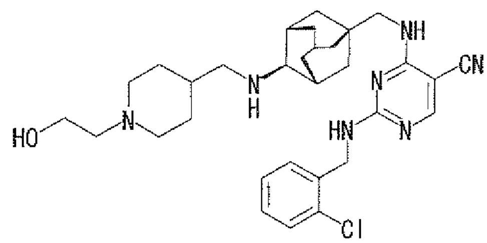 |
| 217 rel | 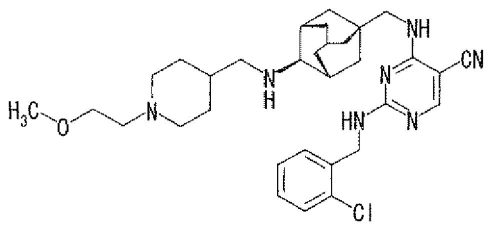 | 218 rel | 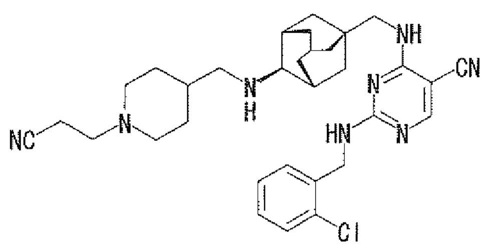 |
| 219 rel | 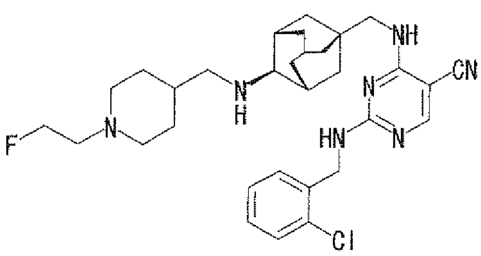 | 220 rel | 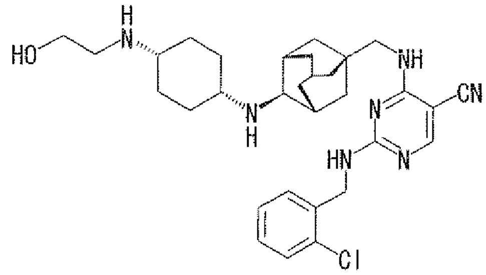 |

[Table 68]

| Ex | Str | Ex | Str |
|---|---|---|---|
| 221 rel | 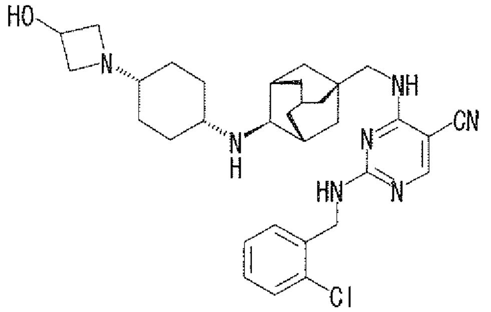 | 222 rel | 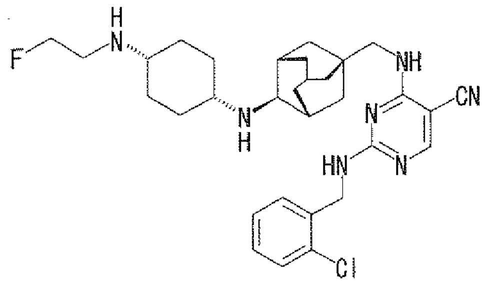 |
| 223 rel | 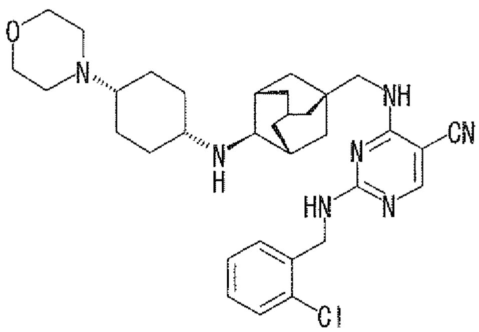 | 224 | 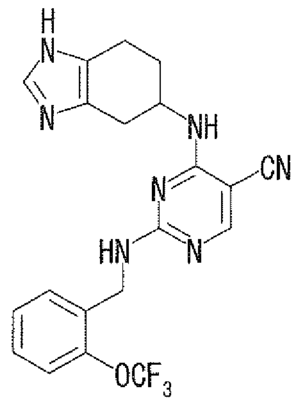 |
| 225 | 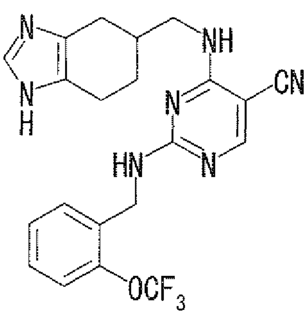 | 226 rel | 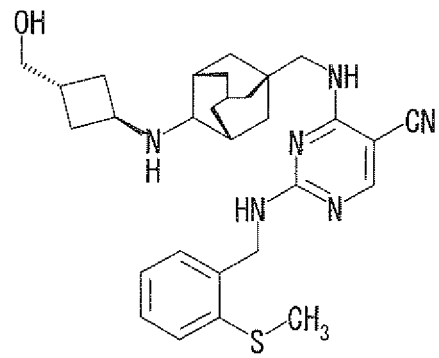 |
| 227 rel | 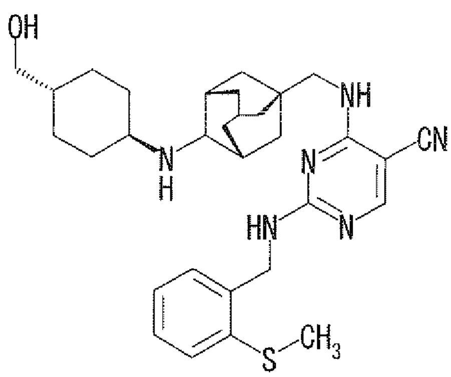 | 228 rel | 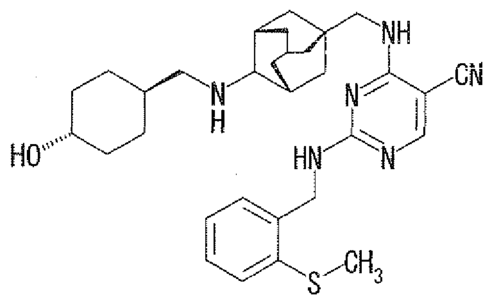 |

[Table 69]

| Ex | Str | Ex | Str |
|---|---|---|---|
| 229 rel | 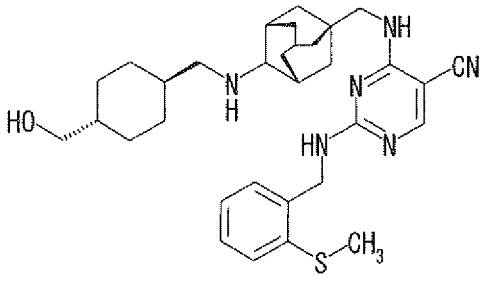 | 230 rel | 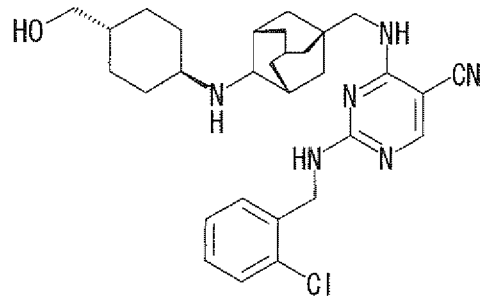 |
| 231 rel | 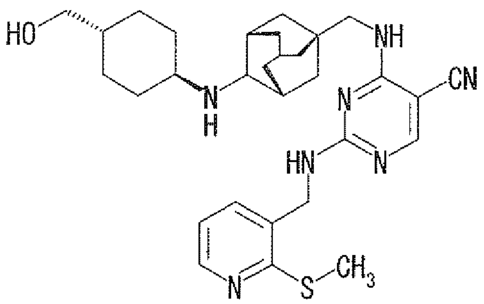 | 232 rel | 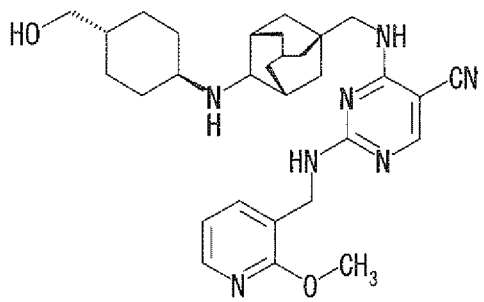 |
| 233 rel | 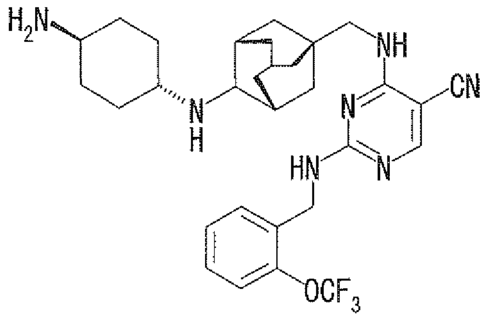 | 234 rel | 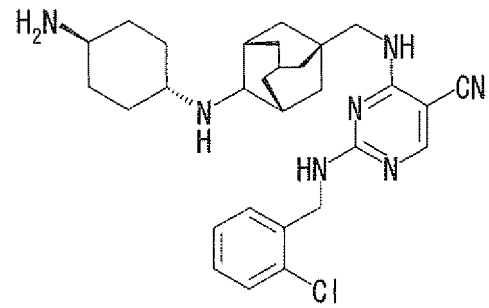 |
| 235 rel | 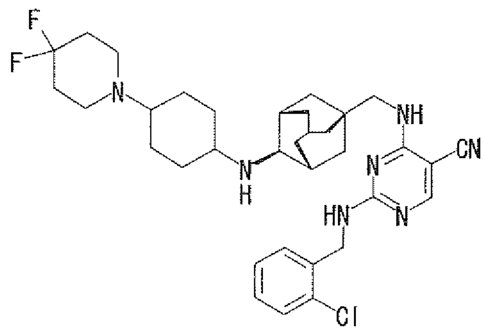 isomer A | 236 rel | 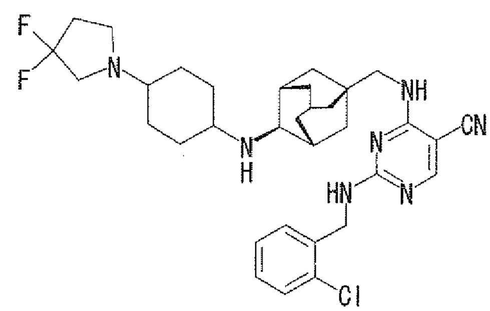 isomer A |

[Table 70]

| Ex | Str | Ex | Str |
|---|---|---|---|
| 237 rel | 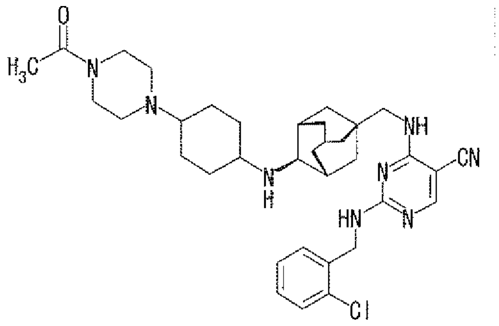 isomer A | 238 rel | 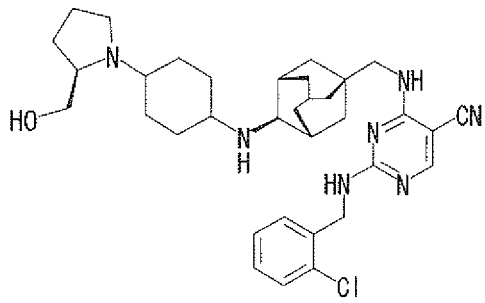 isomer A |
| 239 rel | 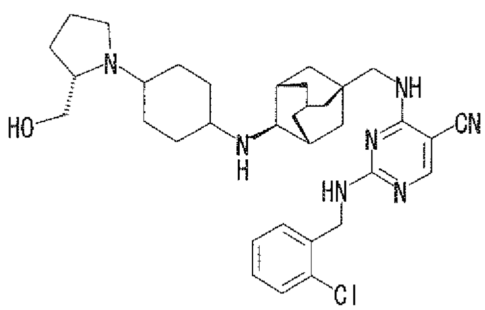 isomer A | 240 rel | 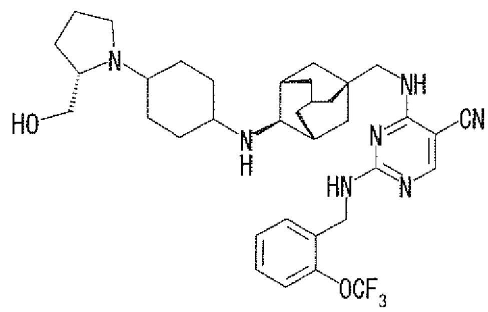 isomer A |
| 241 rel | 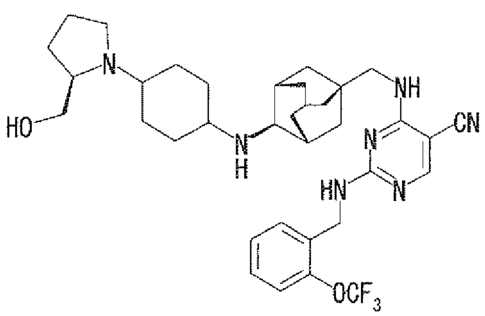 isomer A | 242 rel | 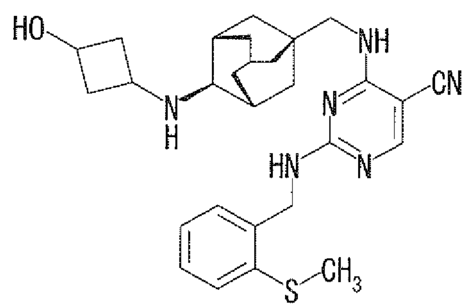 |
| 243 rel | 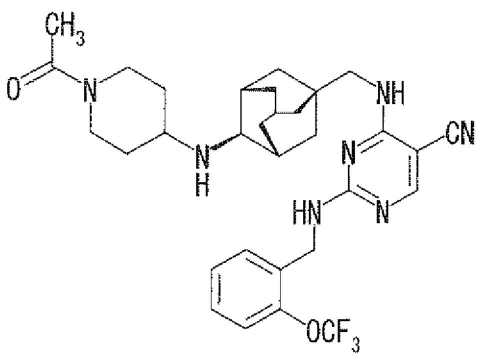 | 244 rel | 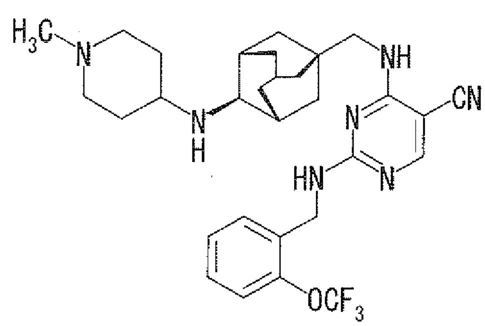 |

[Table 71]

| Ex | Str | Ex | Str |
|---|---|---|---|
| 245 rel | 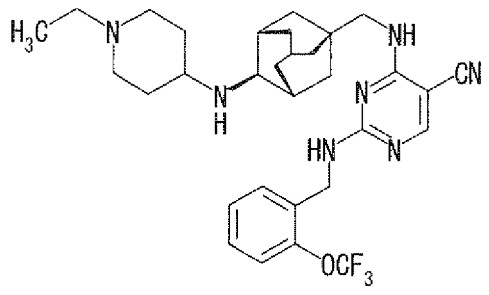 | 246 rel | 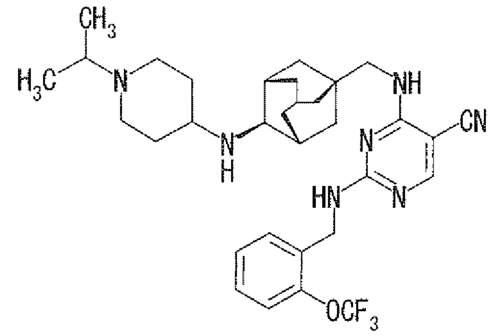 |
| 247 rel | 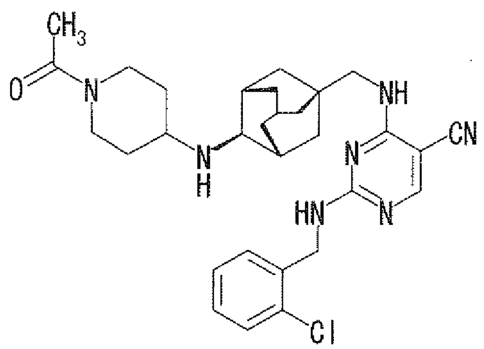 | 248 rel | 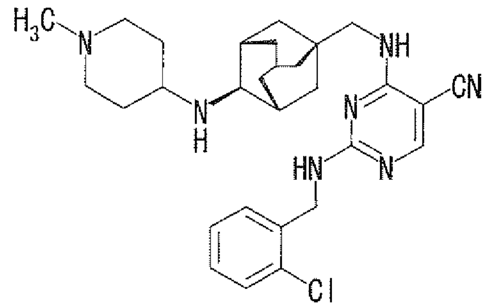 |
| 249 rel | 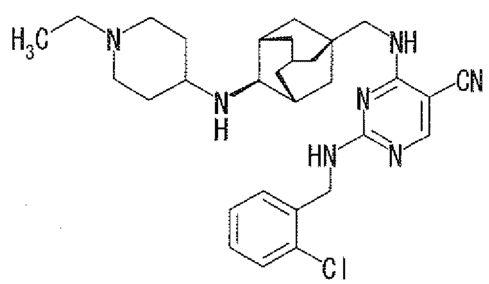 | 250 rel | 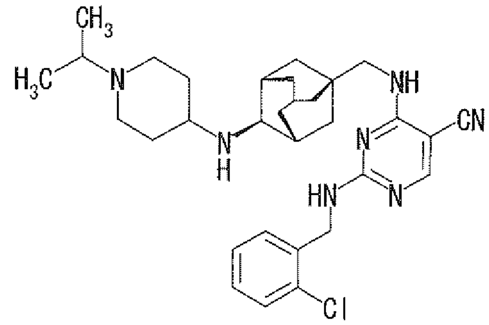 |
| 251 rel | 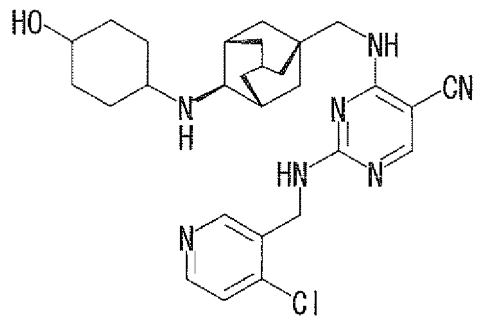 | 252 rel | 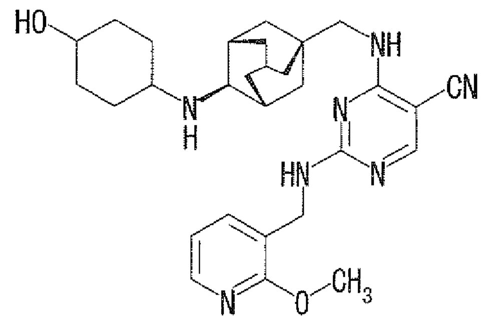 |

[Table 72]

| Ex | Str | Ex | Str |
|---|---|---|---|
| 253 rel | 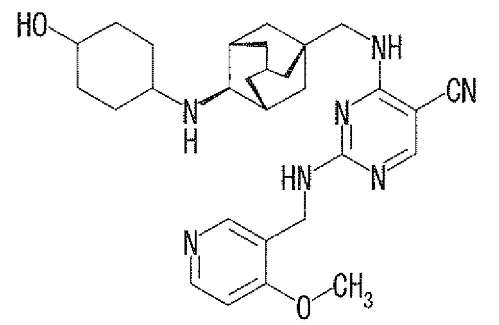 | 254 rel | 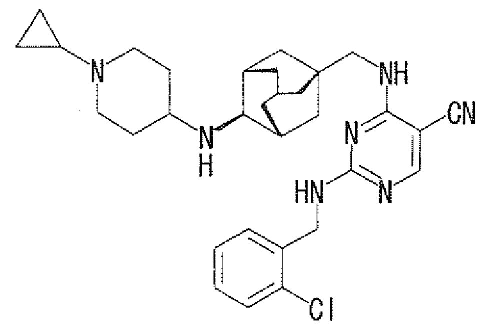 |
| 255 rel | 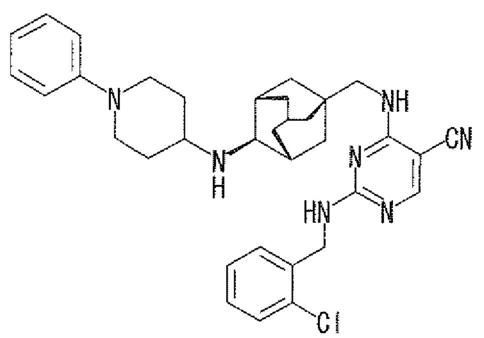 | 256 rel | 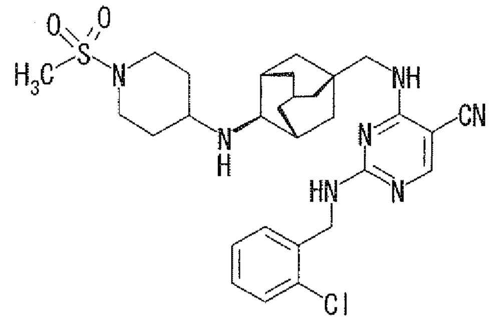 |
| 257 rel | 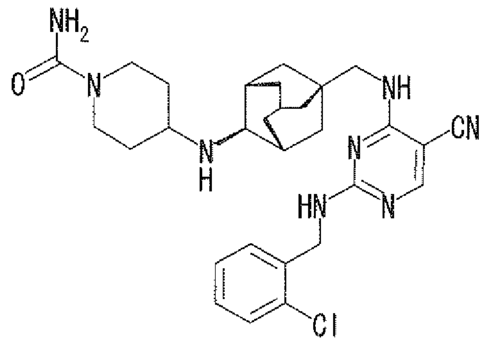 | 258 rel | 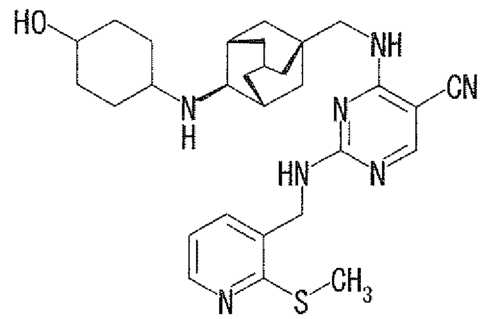 |
| 259 rel | 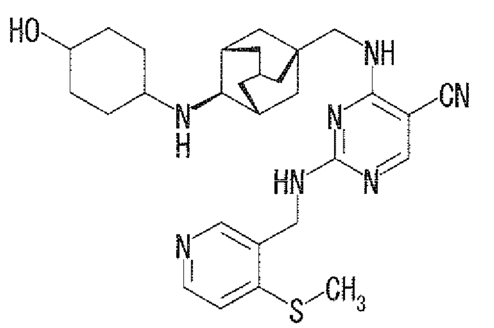 | 260 rel | 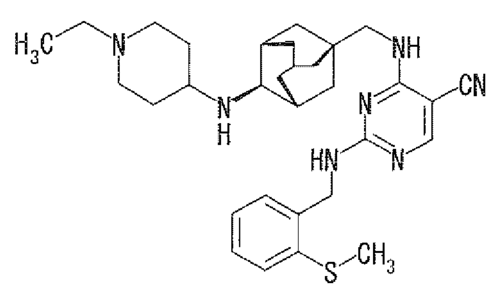 |

[Table 73]

| Ex | Str | Ex | Str |
|---|---|---|---|
| 261 rel | 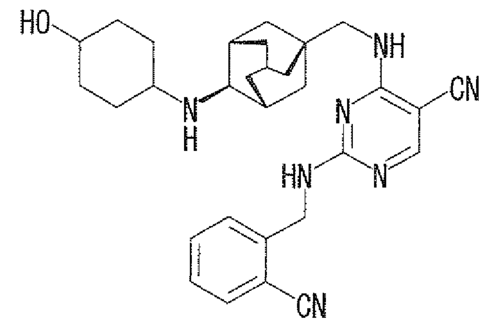 | 262 rel | 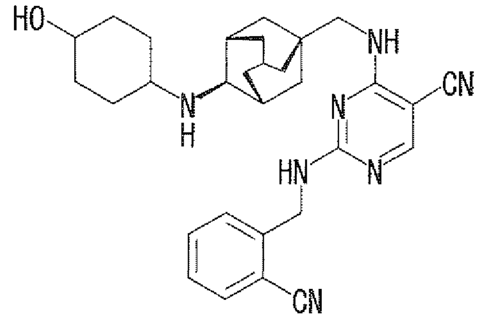 |
| 263 rel | 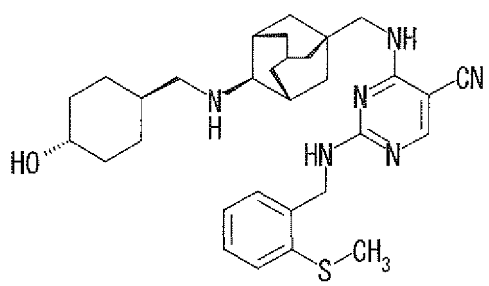 | 264 rel | 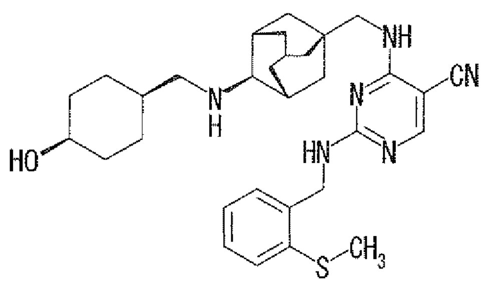 |
| 265 rel | 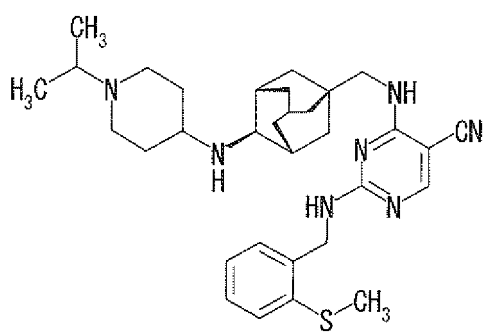 | 266 rel | 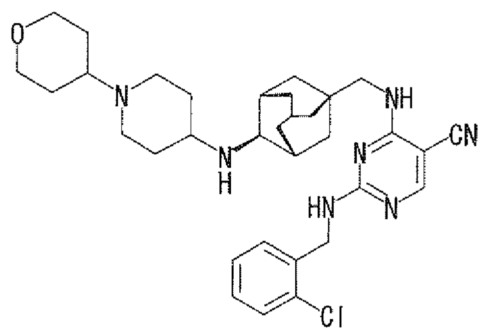 |
| 267 rel | 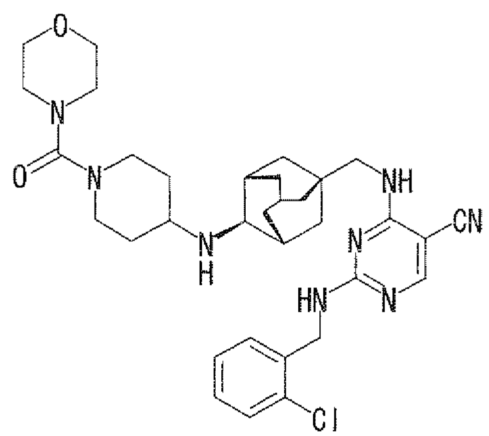 | 268 rel | 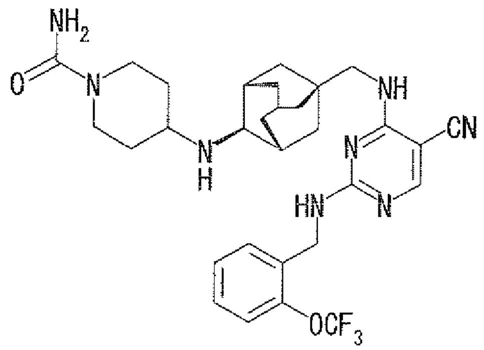 |

[Table 74]

| Ex | Str | Ex | Str |
|---|---|---|---|
| 269 rel | 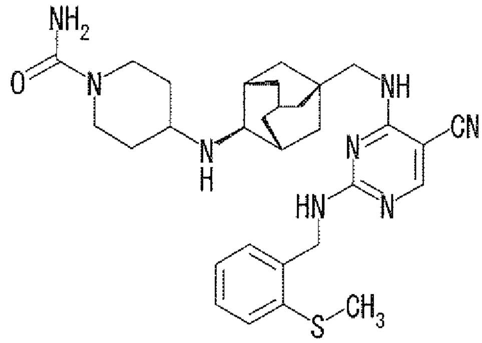 | 270 rel | 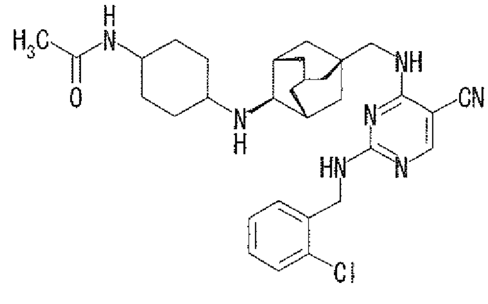 isomer A |
| 271 rel | 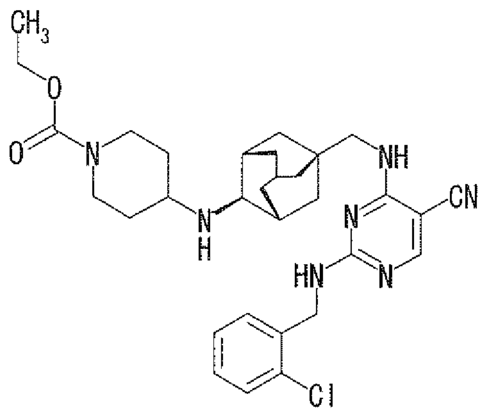 | 272 rel | 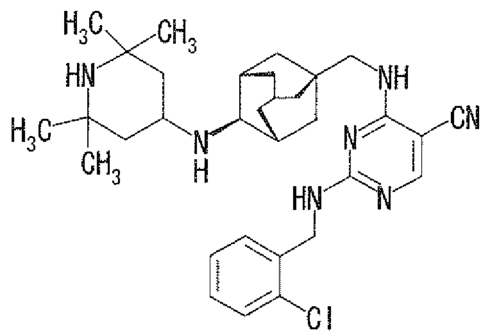 |
| 273 rel | 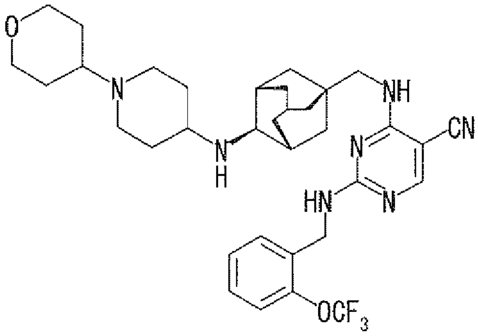 | 274 rel | 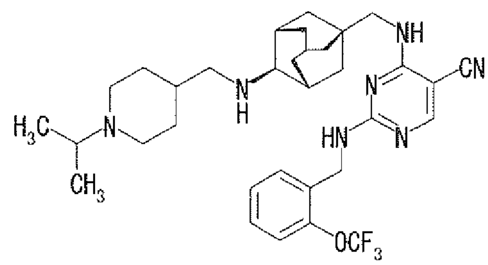 |
| 275 rel | 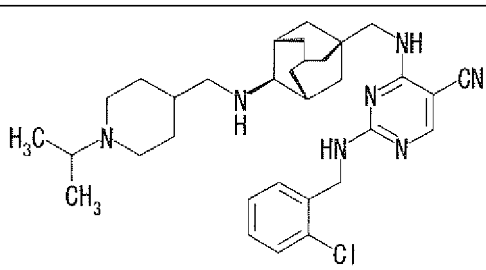 | 276 rel | 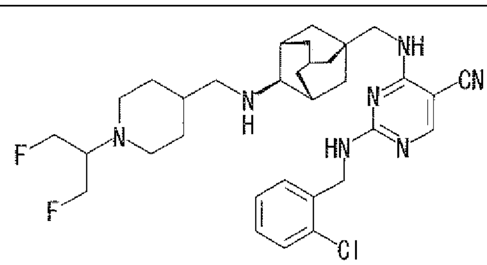 |

[Table 75]

| Ex | Str | Ex | Str |
|---|---|---|---|
| 277 rel | 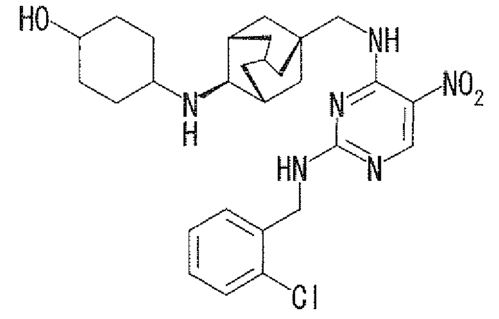 | 278 rel | 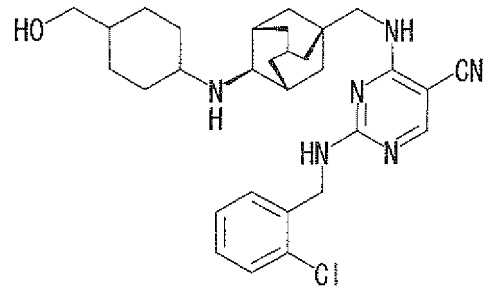 |
| 279 rel | 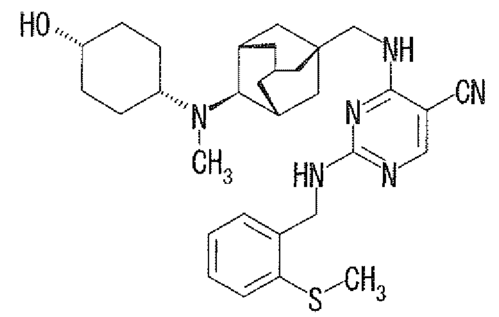 | 280 rel | 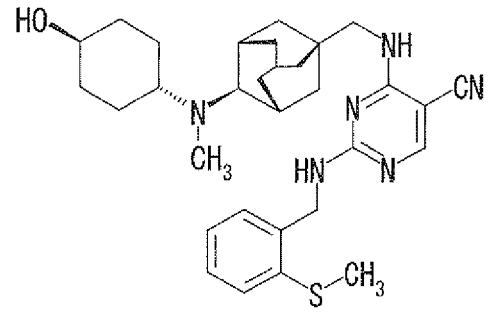 |
| 281 rel | 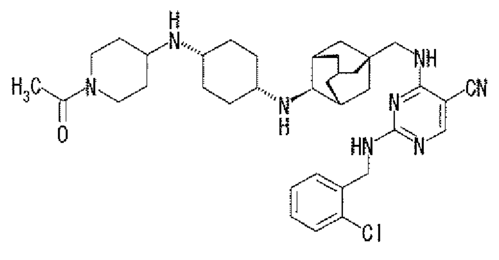 | 282 rel | 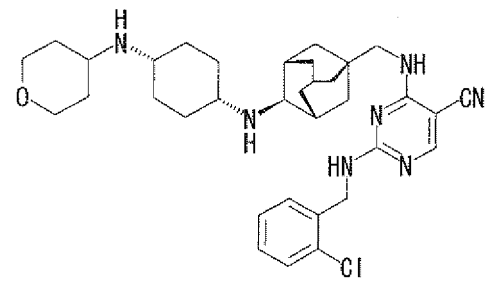 |
| 283 rel | 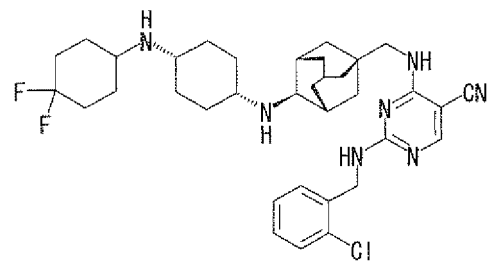 | 284 rel | 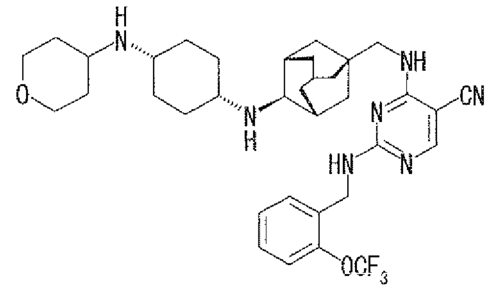 |
| 285 rel | 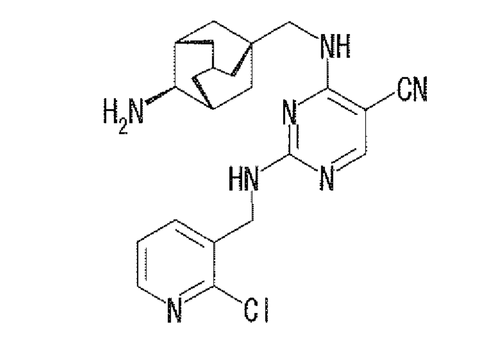 | 286 rel | 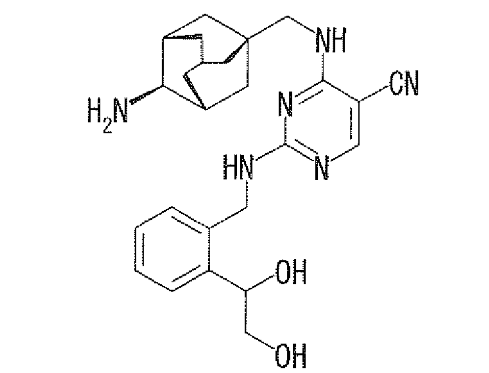 |

[Table 76]

| Ex | Str | Ex | Str |
|---|---|---|---|
| 287 rel | 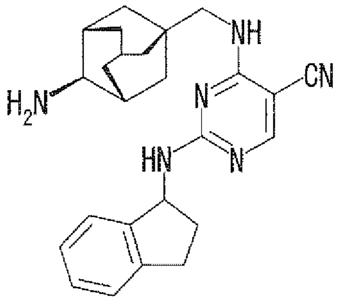 | 288 rel | 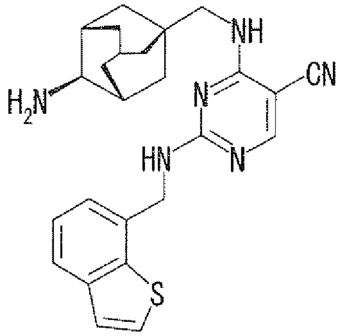 |
| 289 rel | 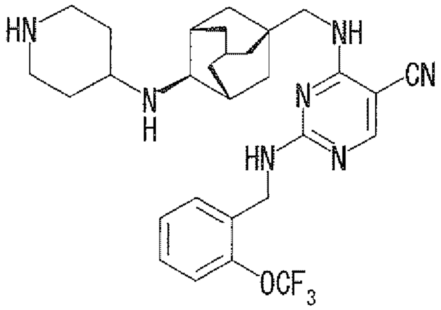 | 290 rel | 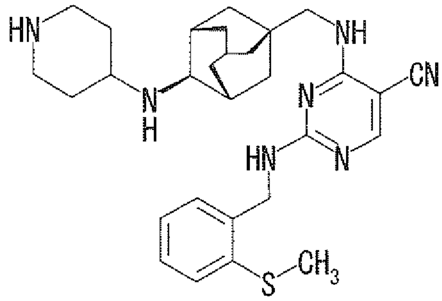 |
| 291 rel | 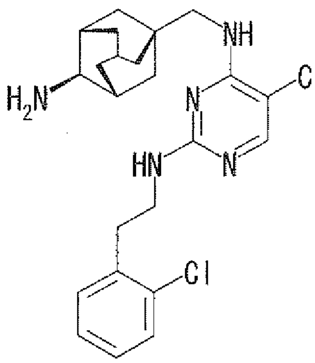 | 292 rel | 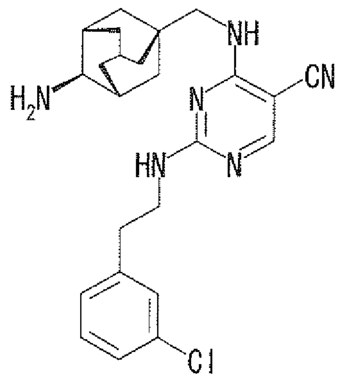 |
| 293 rel | 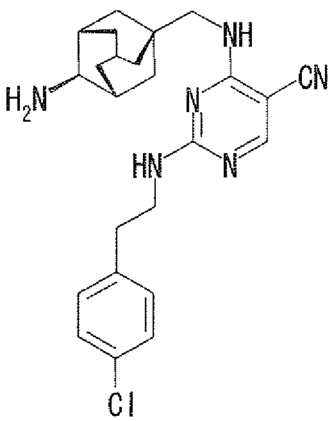 | 294 rel | 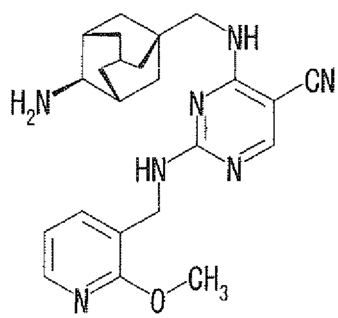 |

[Table 77]

| Ex | Str | Ex | Str |
|---|---|---|---|
| 295 rel | 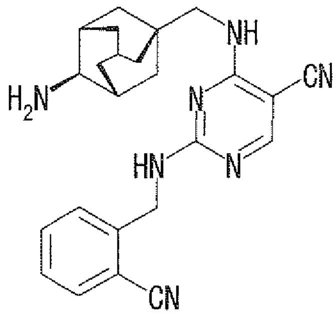 | 296 rel | 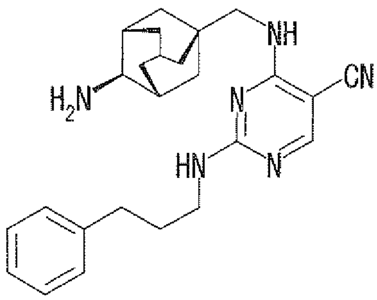 |
| 297 rel | 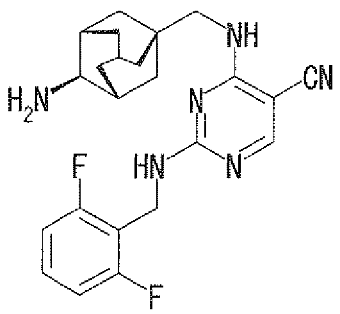 | 298 rel | 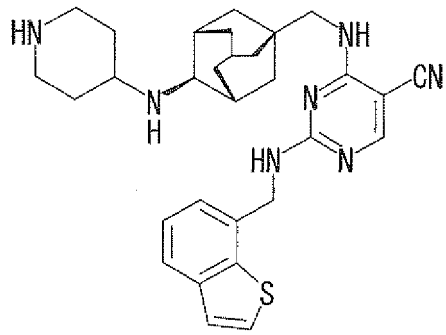 |
| 299 rel | 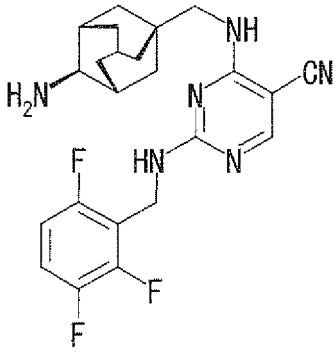 | 300 rel | 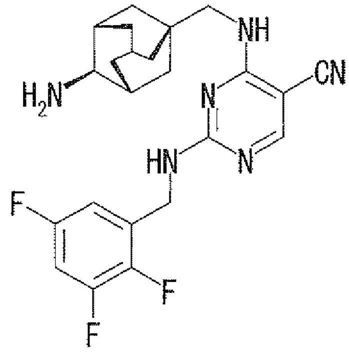 |
| 301 rel | 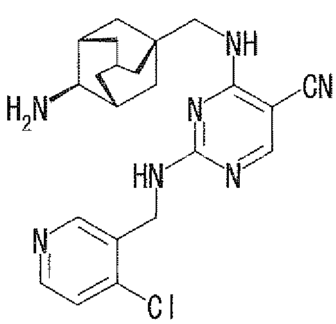 | 302 rel | 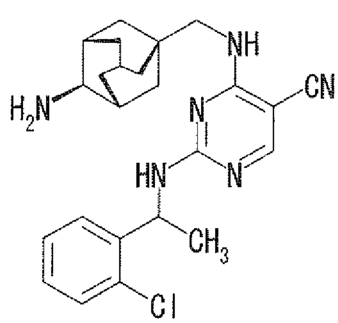 |

[Table 78]

| Ex | Str | Ex | Str |
|---|---|---|---|
| 303 rel | 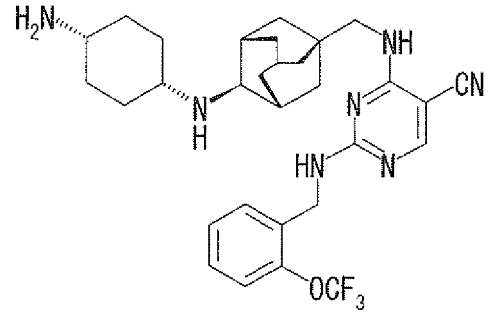 | 304 rel | 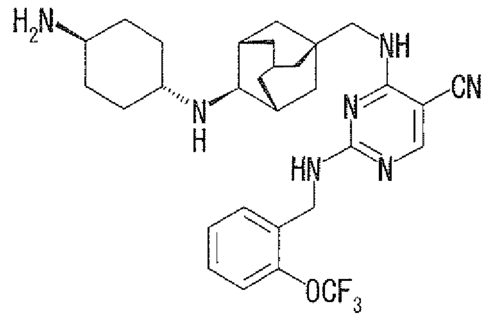 |
| 305 rel | 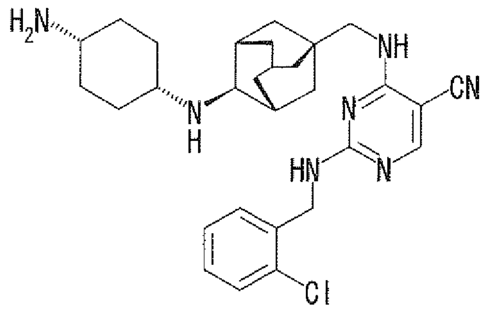 | 306 rel | 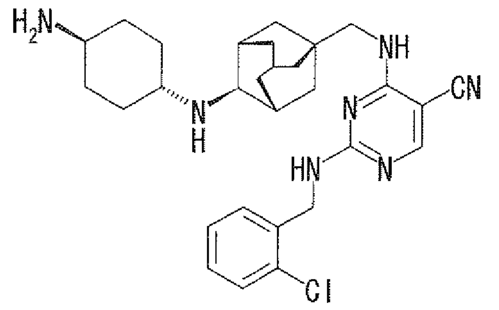 |
| 307 rel | 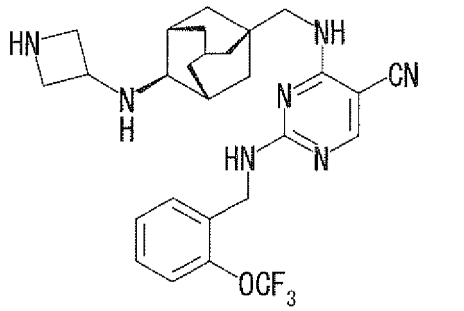 | 308 rel | 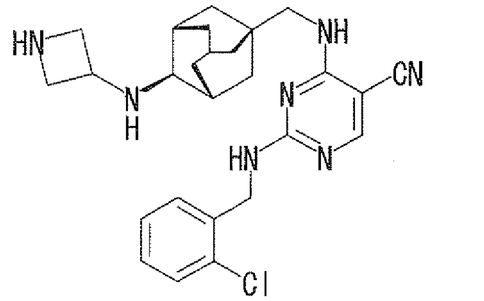 |
| 309 rel | 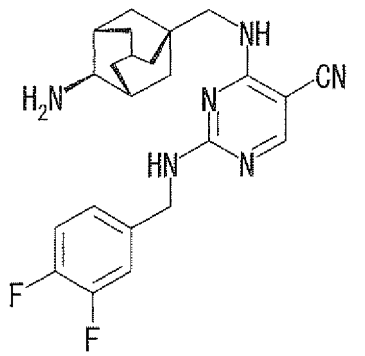 | 310 rel | 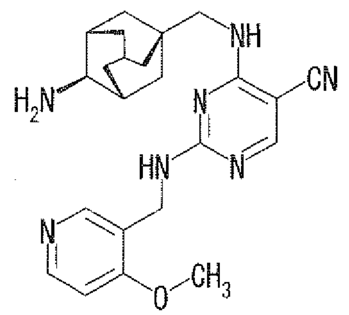 |

[Table 79]

| Ex | Str | Ex | Str |
|---|---|---|---|
| 311 rel | 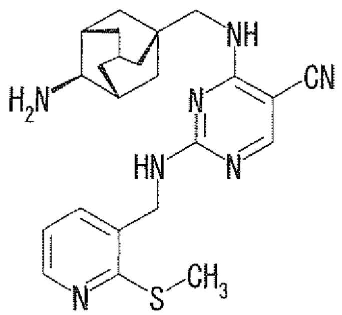 | 312 rel | 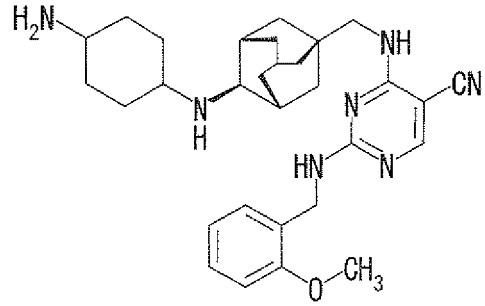 isomer A |
| 313 rel | 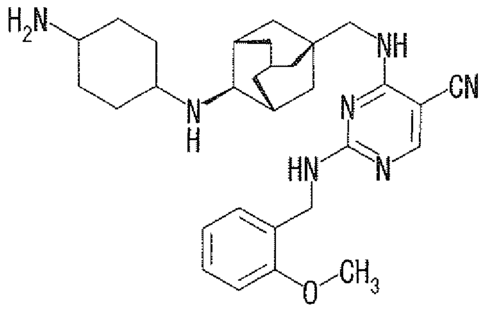 isomer B | 314 rel | 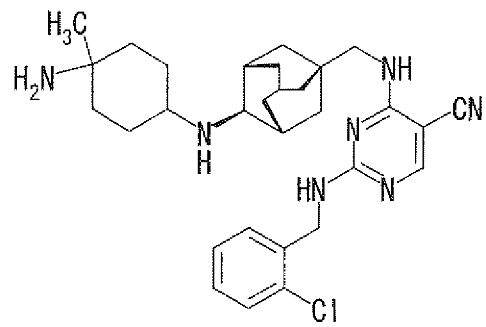 |
| 315 rel | 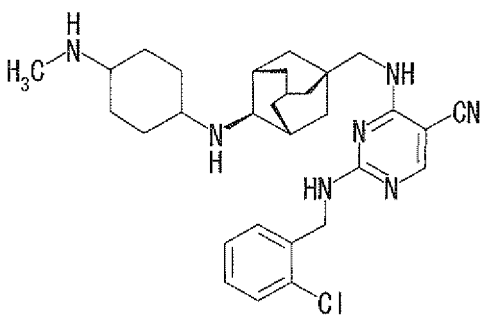 isomer A | 316 rel | 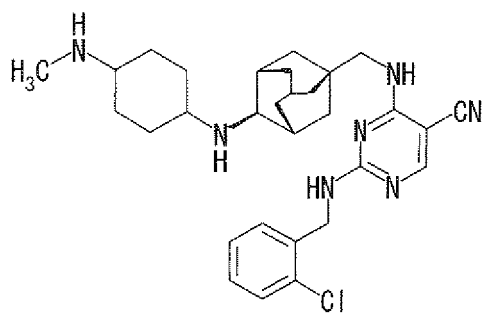 isomer B |
| 317 rel | 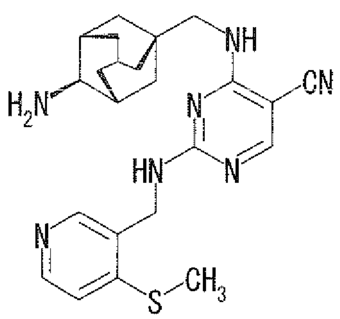 | 318 rel | 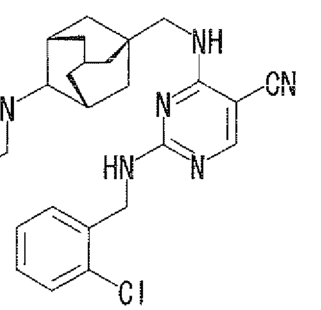 |

[Table 80]

| Ex | Str | Ex | Str |
|---|---|---|---|
| 319 rel | 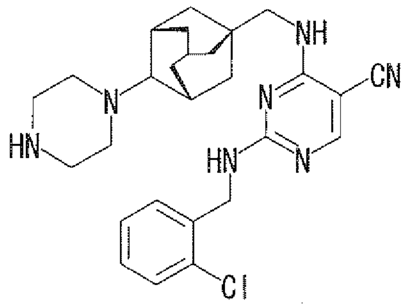 | 320 | 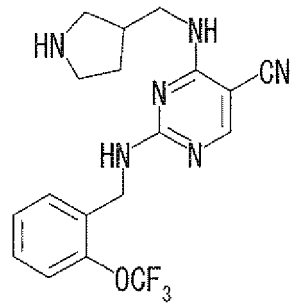 |
| 321 rel | 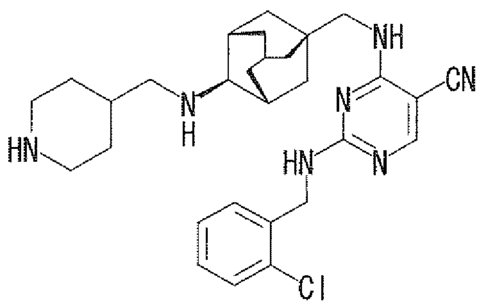 | 322 rel | 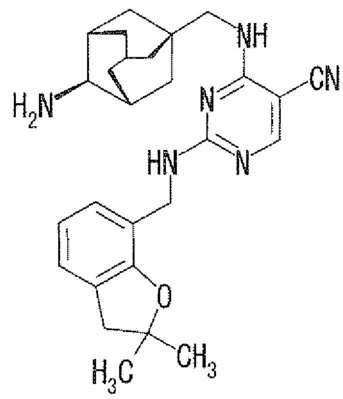 |
| 323 rel | 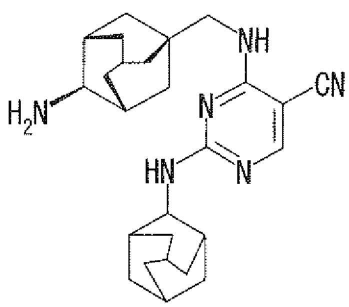 | 324 rel | 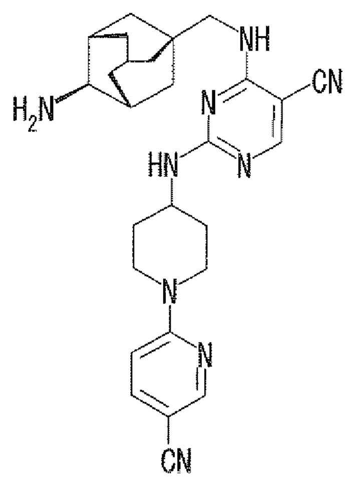 |
| 325 rel | 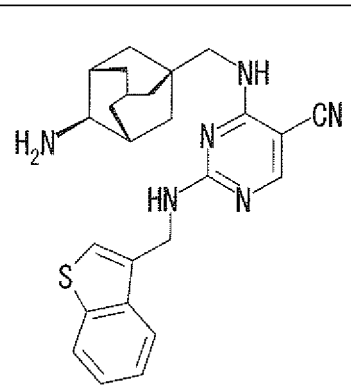 | 326 rel | 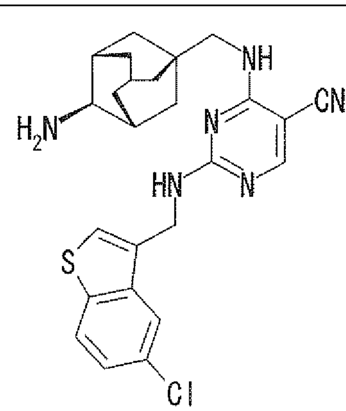 |

[Table 81]

| Ex | Str | Ex | Str |
|---|---|---|---|
| 327 rel | 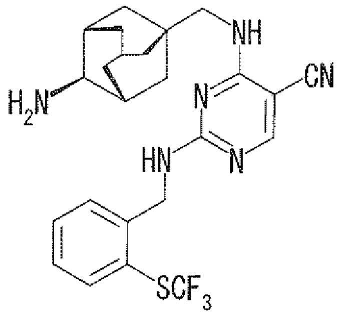 | 328 rel | 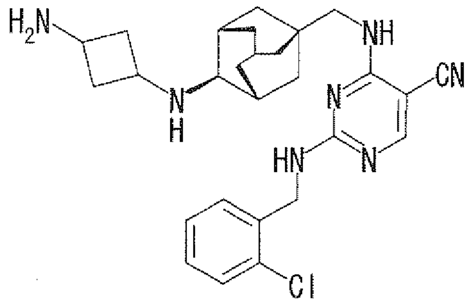 |
| 329 rel | 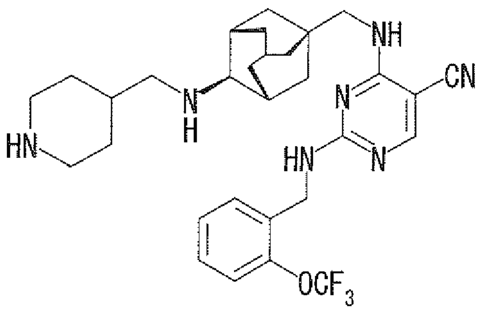 | 330 rel | 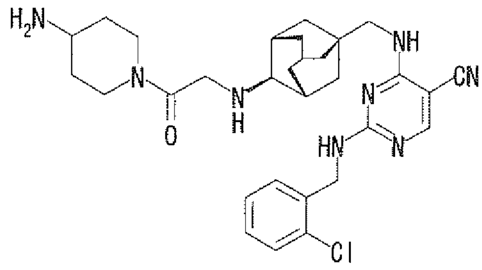 |
| 331 rel | 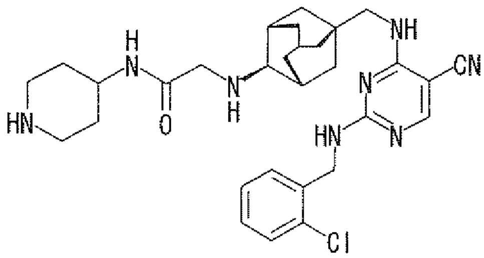 | 332 rel | 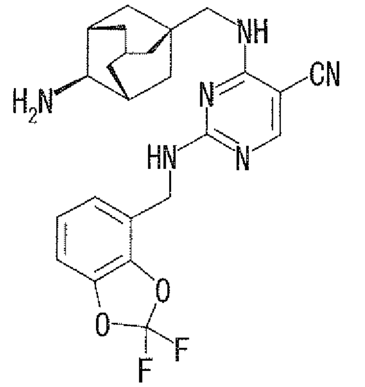 |
| 333 rel | 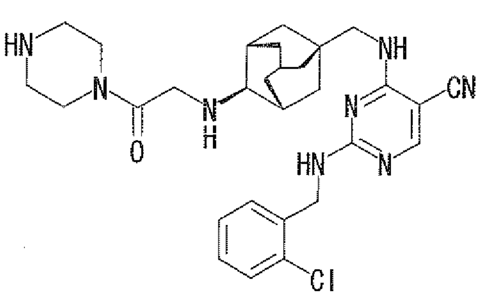 | 334 rel | 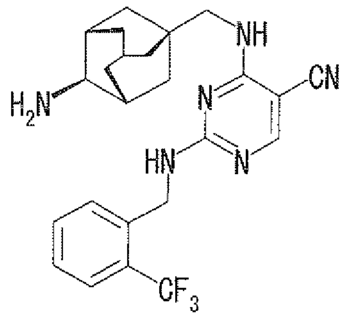 |

[Table 82]

| Ex | Str | Ex | Str |
|---|---|---|---|
| 335 rel | 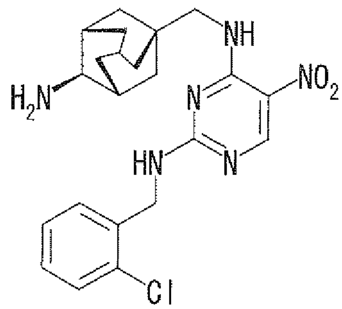 | 336 rel | 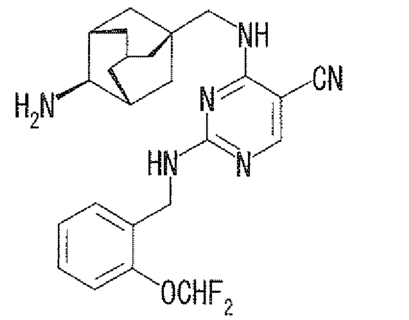 |
| 337 rel | 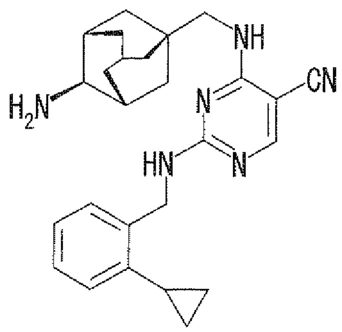 | 338 rel | 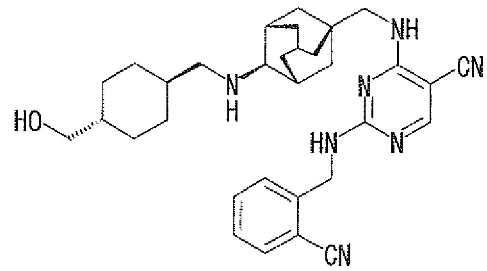 |
| 339 rel | 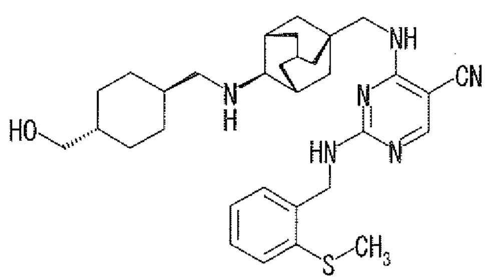 | 340 rel | 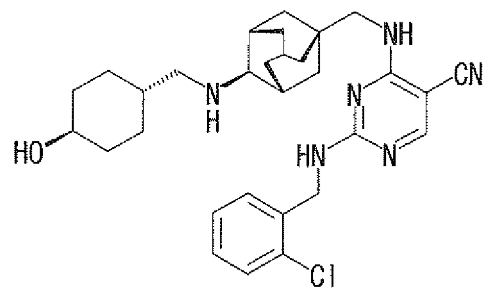 |
| 341 rel | 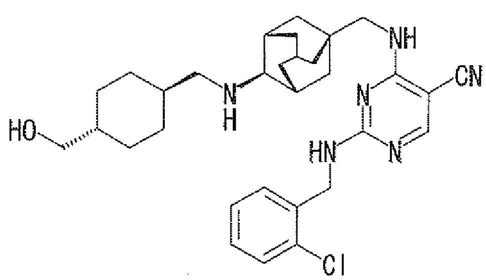 | 342 rel | 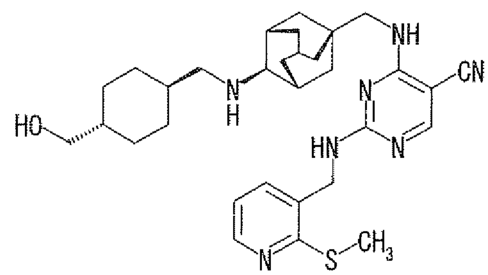 |
| 343 rel | 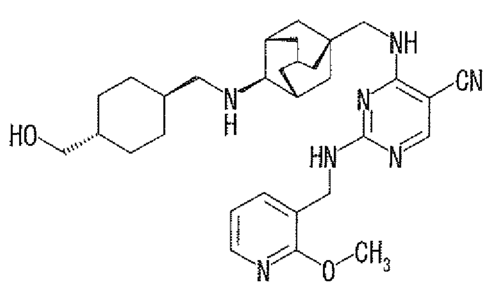 | 344 rel | 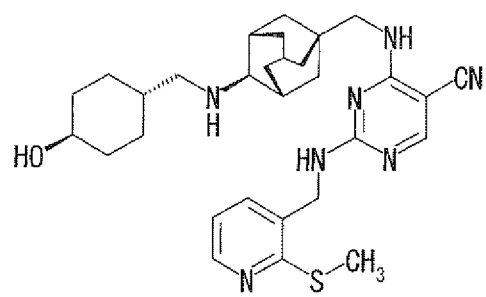 |

[Table 83]

| Ex | Str | Ex | Str |
|---|---|---|---|
| 345 rel | 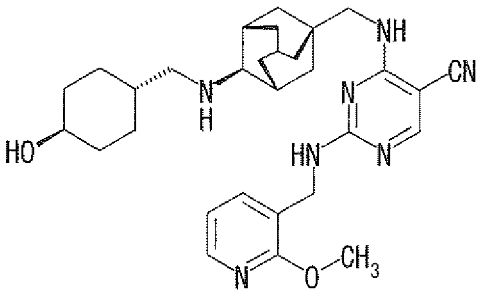 | 346 rel | 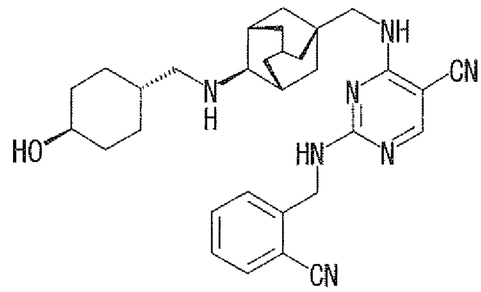 |
| 347 rel | 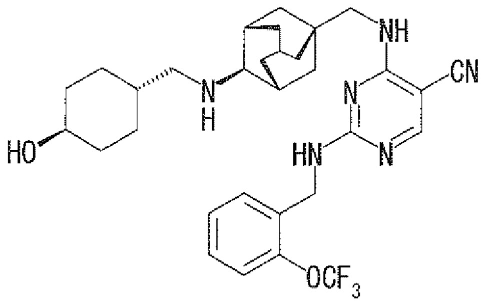 | 348 rel | 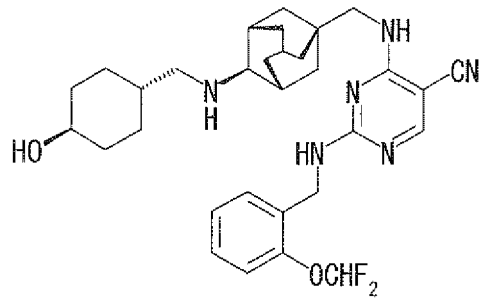 |
| 349 rel | 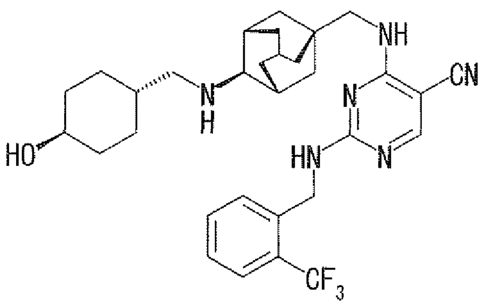 | 350 rel | 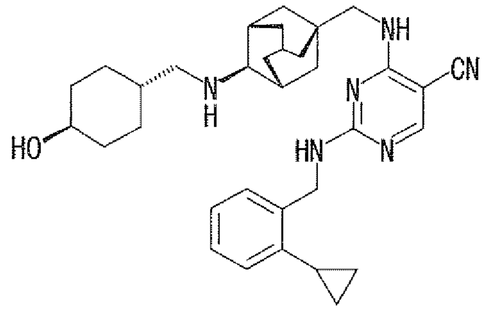 |
| 351 rel | 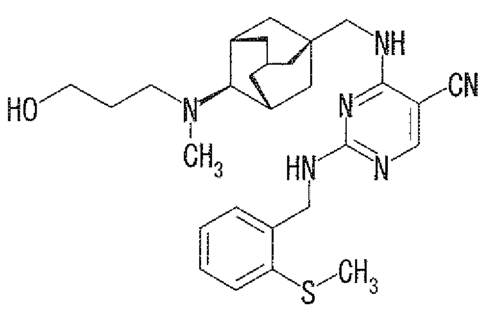 | 352 rel | 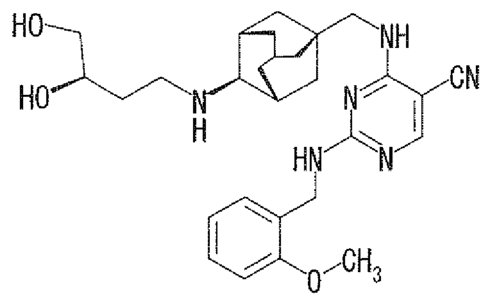 |
| 353 rel | 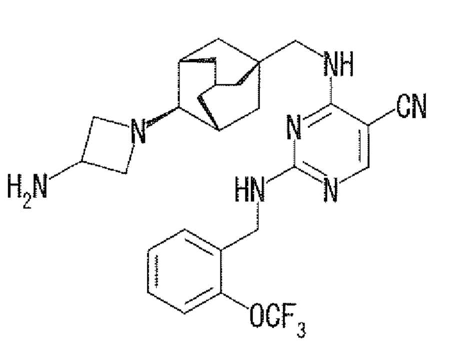 | 354 | 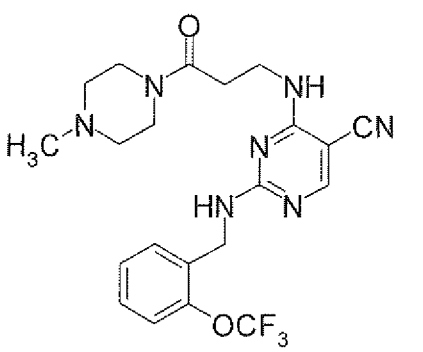 |

[Table 84]

| Ex | Str | Ex | Str |
|---|---|---|---|
| 355 | 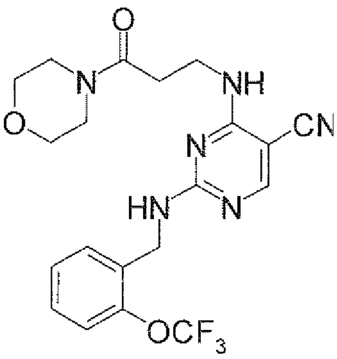 | 356 rel | 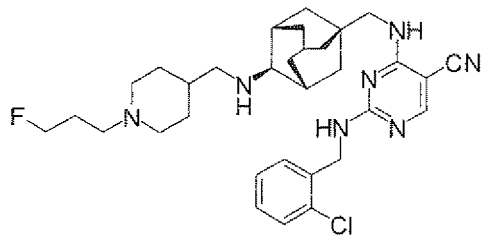 |
| 357 rel | 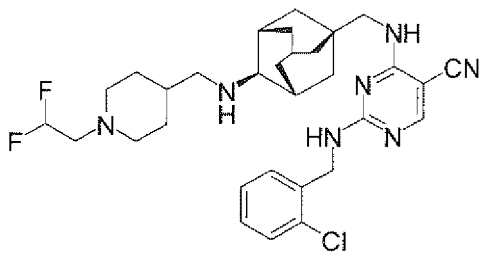 | 358 rel | 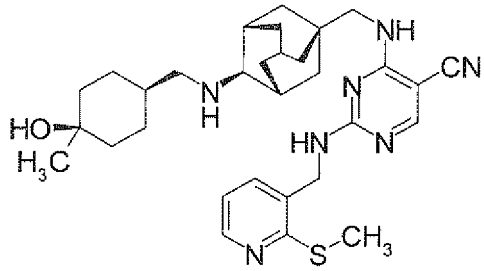 |
| 359 rel | 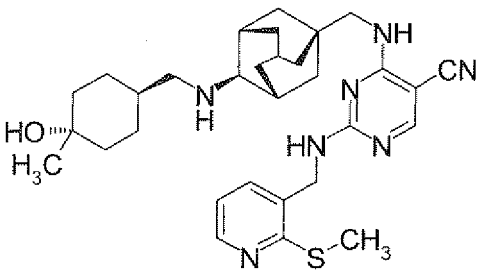 | 360 rel | 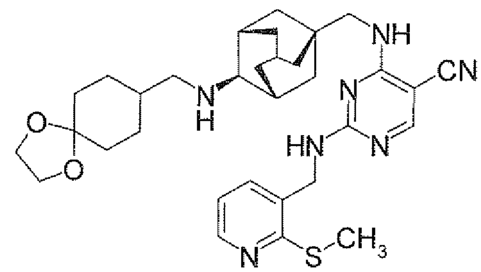 |
| 361 rel | 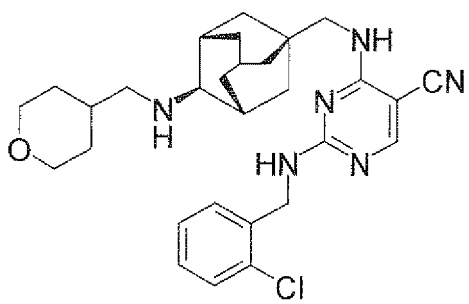 | 362 rel | 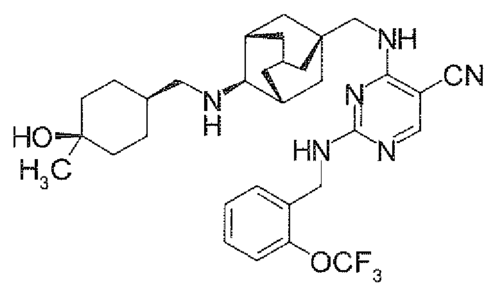 |
| 363 rel | 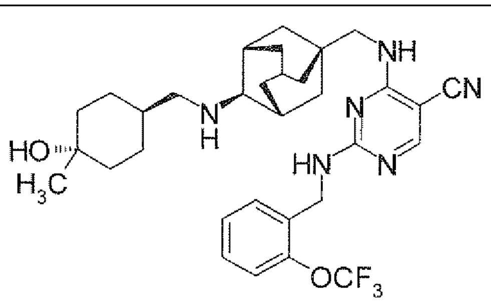 | 364 rel | 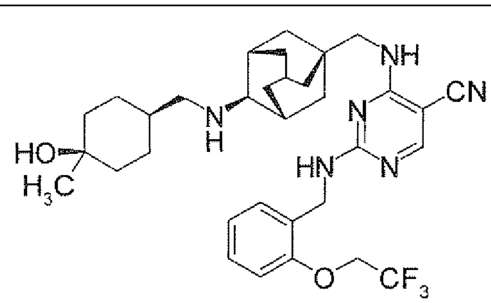 |

[Table 85]

| Ex | Str | Ex | Str |
|---|---|---|---|
| 365 rel | 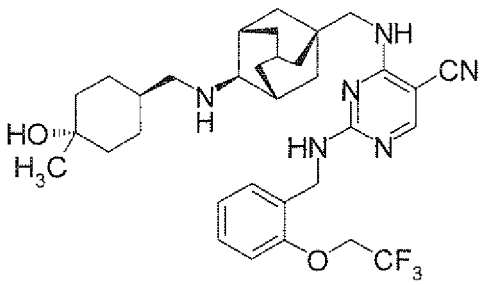 | 366 rel | 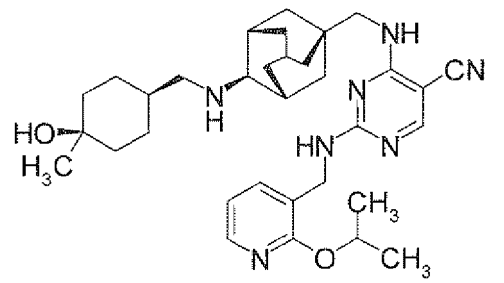 |
| 367 rel | 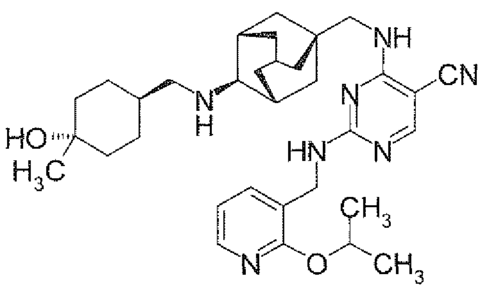 | 368 rel | 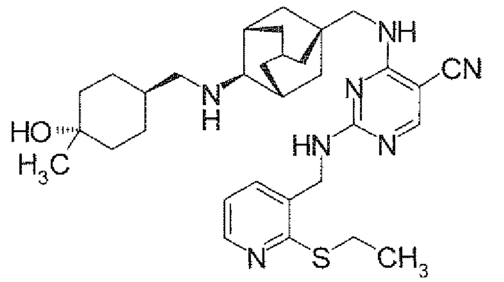 |
| 369 rel | 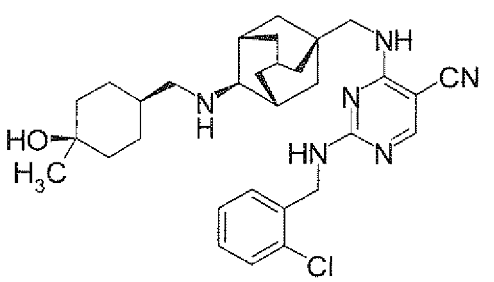 | 370 rel | 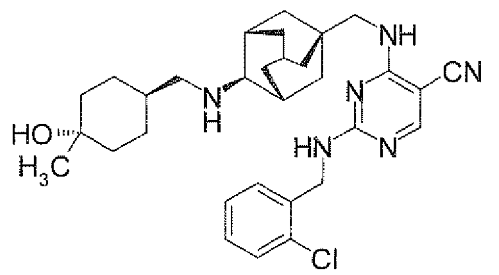 |
| 371 rel | 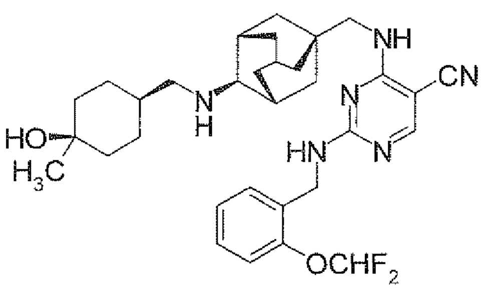 | 372 rel | 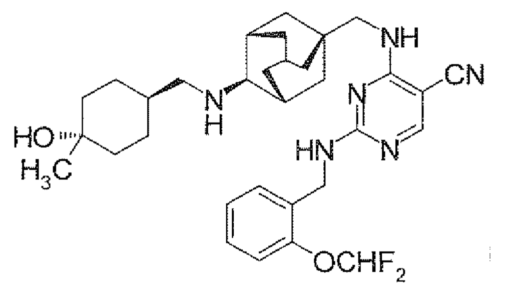 |
| 373 rel | 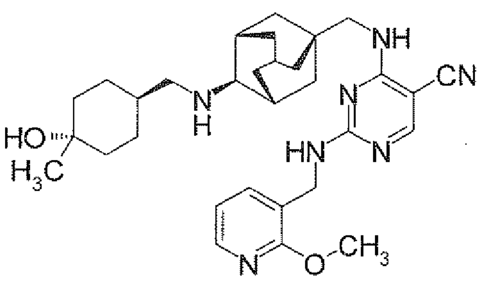 | 374 rel | 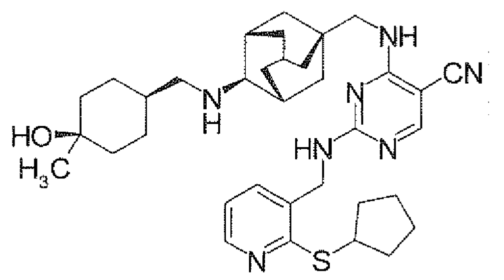 |

[Table 86]

| Ex | Str | Ex | Str |
|---|---|---|---|
| 375 rel | 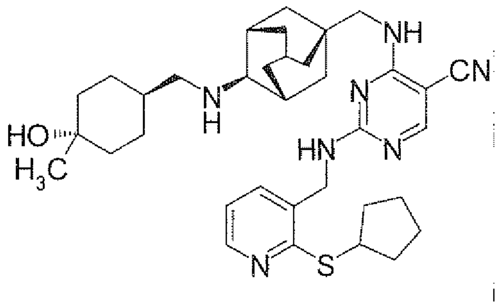 | 376 | 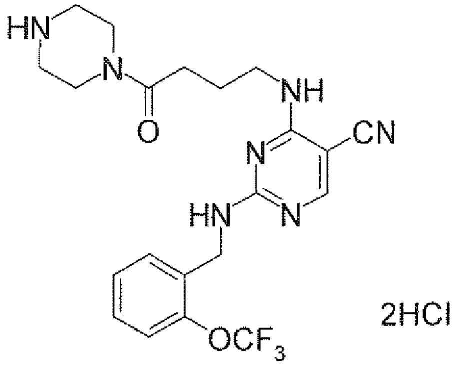 |
| 377 rel | 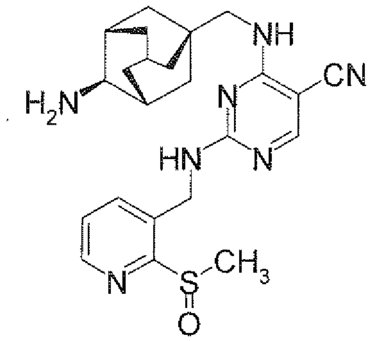 | 378 rel | 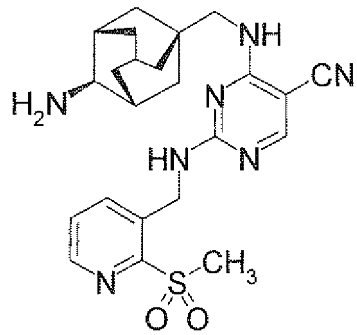 |
| 379 rel | 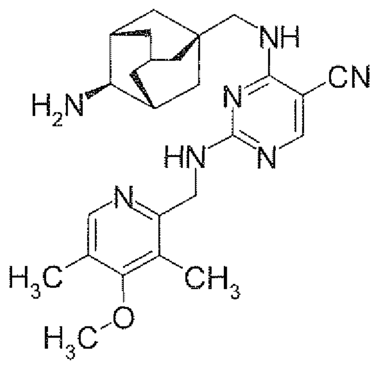 | 380 rel | 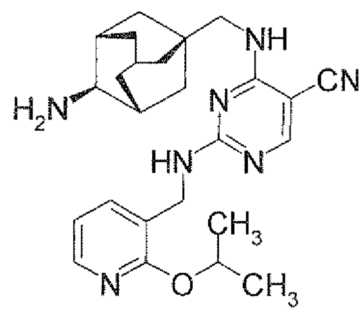 |
| 381 rel | 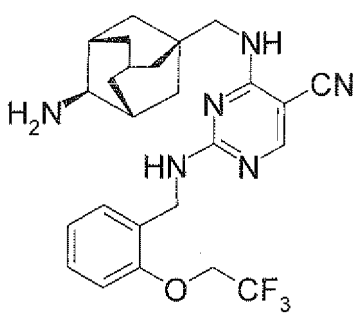 | 382 rel | 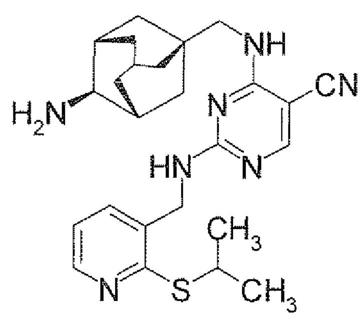 |

[Table 87]

| Ex | Str | Ex | Str |
|---|---|---|---|
| 383 rel | 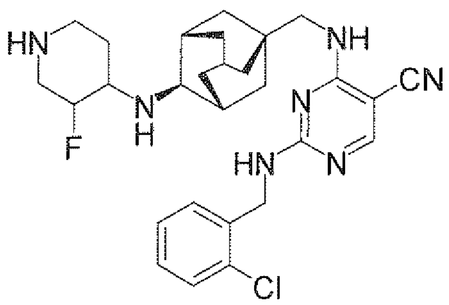 | 384 | 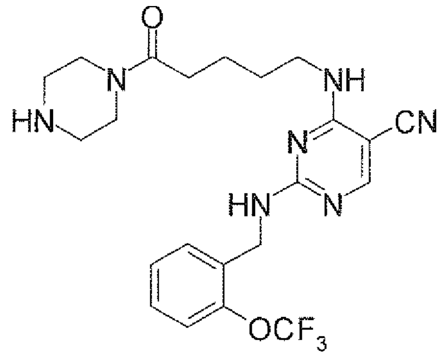 |
| 385 rel | 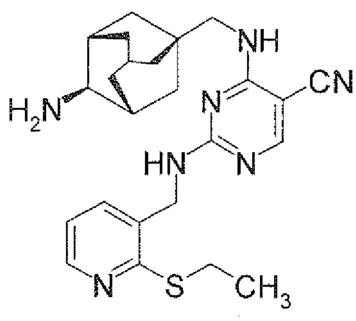 | 386 | 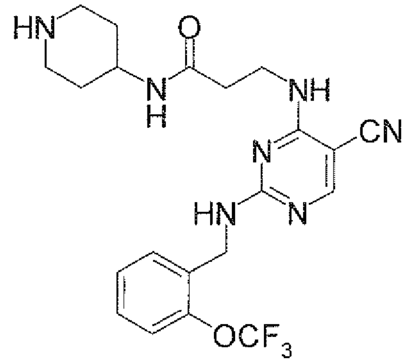 |
| 387 | 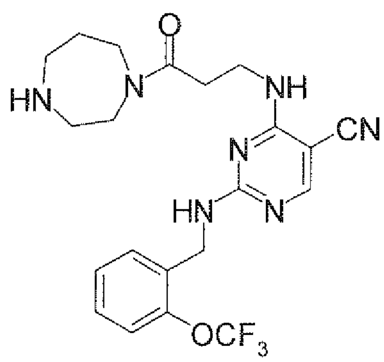 | 388 rel | 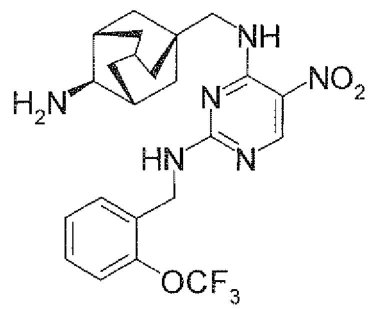 |
| 389 rel | 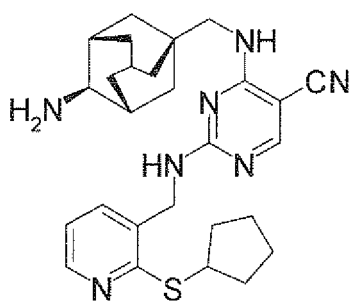 | 390 rel | 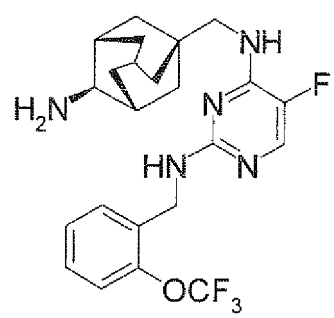 |

[Table 88]

| Ex | Str | Ex | Str |
|---|---|---|---|
| 391 rel | 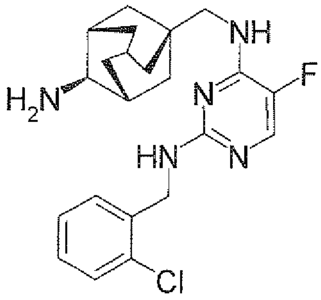 | 392 rel | 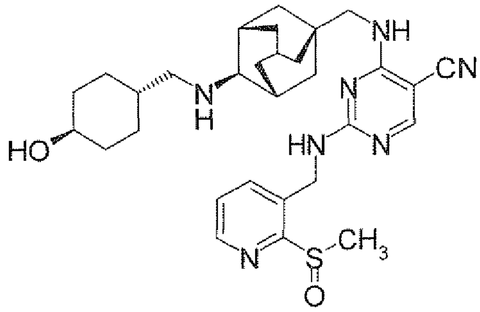 |
| 393 rel | 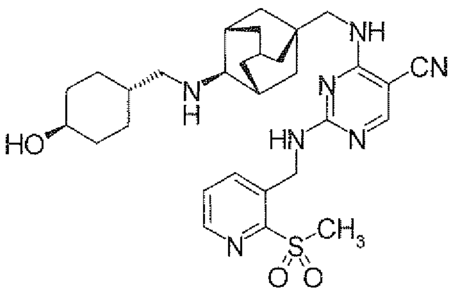 | 394 rel | 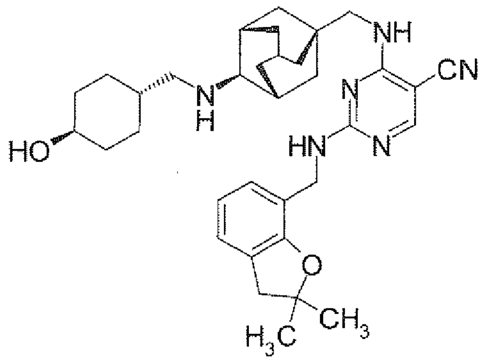 |
| 395 rel | 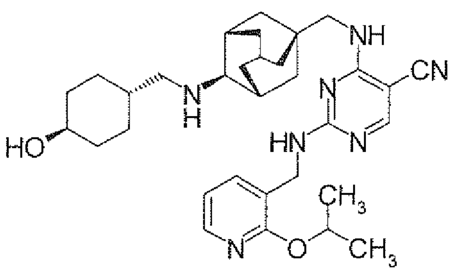 | 396 rel | 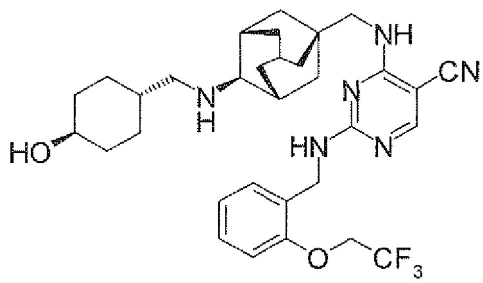 |
| 397 rel | 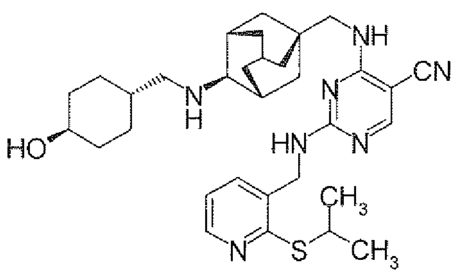 | 398 rel | 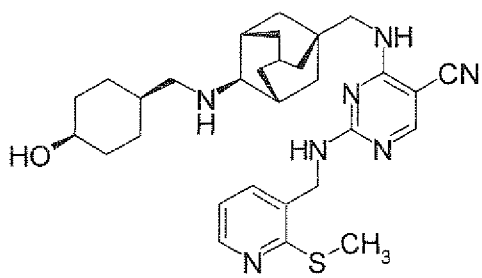 |

[Table 89]

| Ex | Str | Ex | Str |
|---|---|---|---|
| 399 rel | 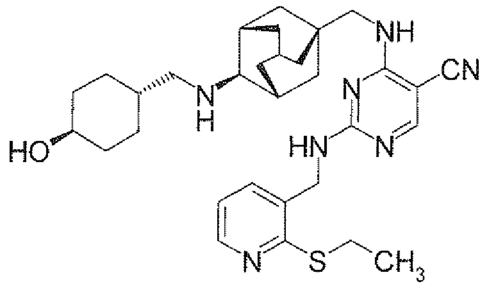 | 400 rel | 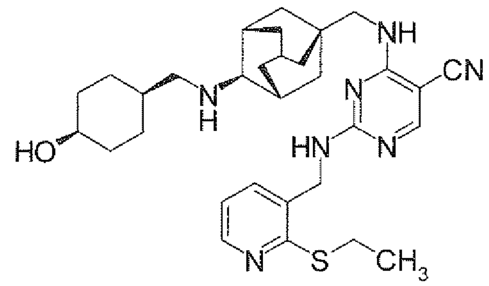 |
| 401 rel | 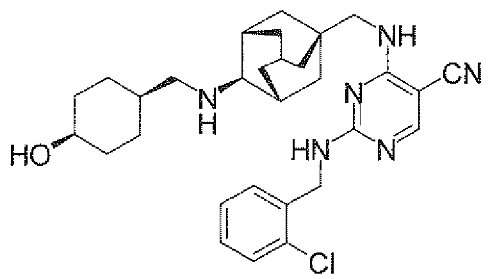 | 402 rel | 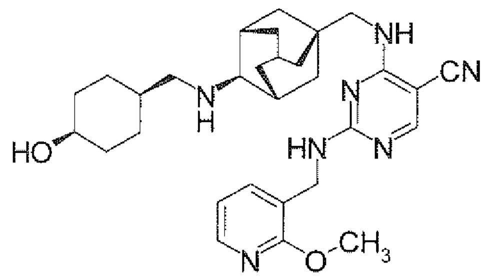 |
| 403 rel | 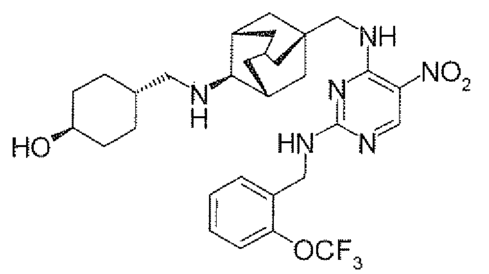 | 404 rel | 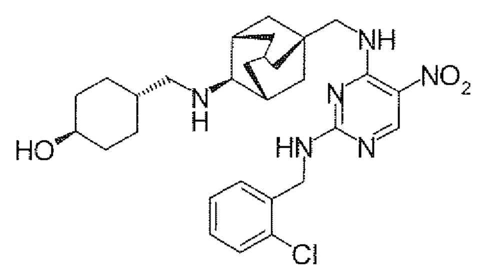 |
| 405 rel | 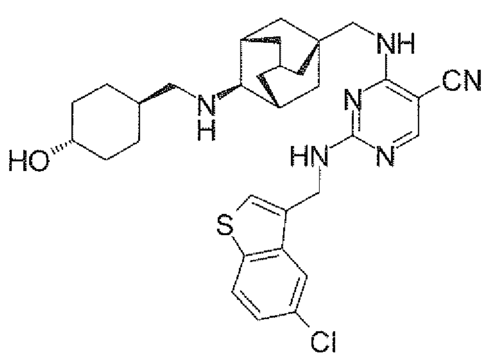 | 406 rel | 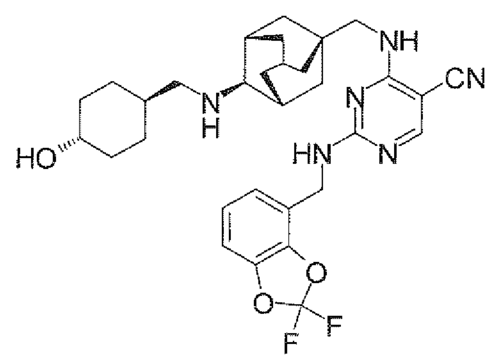 |
| 407 rel | 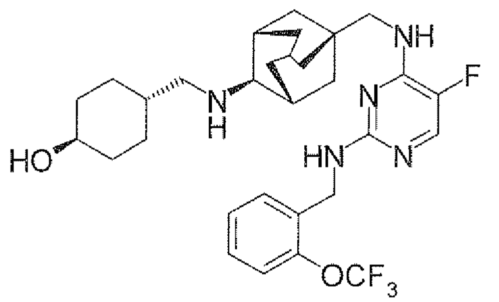 | 408 rel | 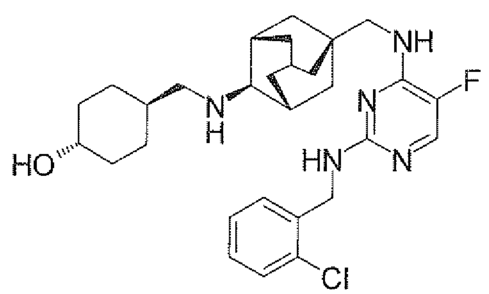 |

[Table 90]

| Ex | Str | Ex | Str |
|---|---|---|---|
| 409 rel | 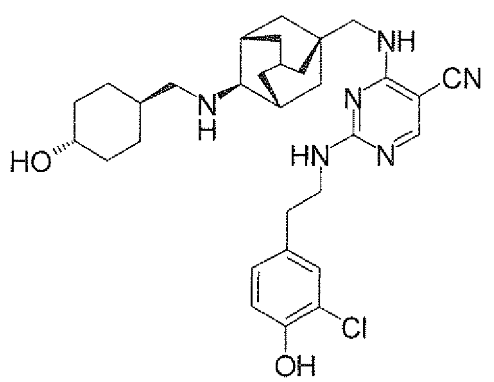 | 410 rel | 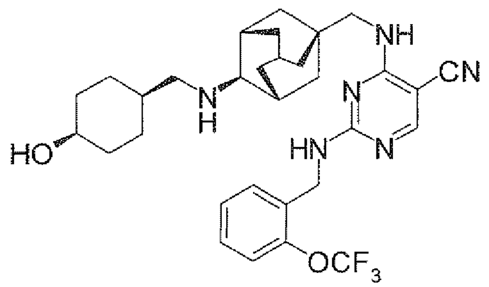 |
| 411 rel | 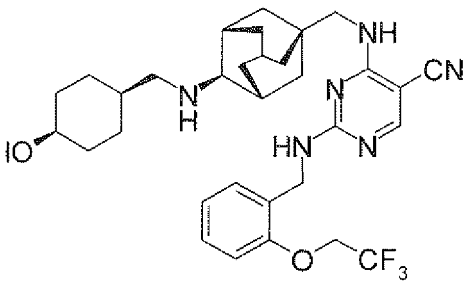 | 412 rel | 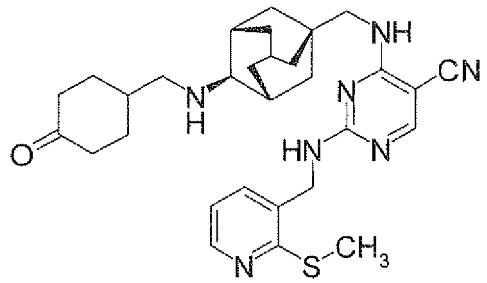 |

[Table 91]

| Ex | Syn | Dat |
|---|---|---|
| 1 | 1 | NMR1: 1.00-2.03 (18H, m), 2.67-2.98 (4H, m), 3.21-3.68 (2H, m), 4.52-4.56 (2H, m), 7.21-7.35 (5H, m), 8.03-8.18 (2H, m); ESI+: 571 |
| 2 | 1 | NMR1: 0.94-2.23 (16H, m), 2.61-3.15 (5H, m), 3.44-3.50 (2H, m), 4.20 (1H, brs), 4.53 (2H, d, J-6.0Hz), 7.13-7.35 (5H, m), 8.03-8.18 (2H, m); ESI+:587 |
| 3 | 3 | NMR1: 1.47-2.02 (13H, m), 2.99 (2H, d, J=6.2Hz), 3.17 (1H, brs), 4.56 (2H, d, J=6.2Hz), 5.36 and 5.45 (total 1H, each d, J =7.2Hz), 6.47 (1H, t, J= 7.8Hz), 6.55 and 6.61 (total 2H, each d, J=7.8Hz), 7.03 (2H, t, J=7.8Hz), 7.22-7.37 (5H, m), 7.92 and 8.16 (total 1H, each t, J=6.2Hz), 8.20 (1H, s); ESI+: 549 |
| 4 | 4 | NMR1: 1.18-1.95 (13H, m), 1.83 (3H, s), 2.95 and 3.15 (total 2H, each d, J =6.3Hz), 3.60 and 3.74 (total 1H, each d, J = 7.3Hz), 4.52 4.56 (total 2H, each d, J=6.0Hz), 7.23-7.35 (5H, m), 7.62 and 7.72 (total 1H, each d, J=7.7Hz), 7.91 and 8.16-8.13 (total 1H, each), 8.18 (1H, s); ESI+:515 |
| 5 | 5 | NMR1: 0.82-2.67 (17H, m), 2.84-3.14 (4H, m), 4.12 (1H, brs), 4.53 (2H, d, J =6.0Hz), 5.09-5.12 (1H, m), 6.91-7.02 (1H, m), 7.17-7.34 (6H, m), 7.89-8.18 (2H,m); ESI+: 586 |
| 6 | 6 | NMR1: 1.22-2.10 (13H, m), 2.99 and 3.19 (total 2H, each d, J=6.3Hz), 3.83 and 3.97 (total 1H, each m), 4.54 (2H, d, J=6.0Hz), 7.25-7.38 (5H, m), 7.48 (1H, dd, J=4.7,7.9Hz), 7.93-8.18 (3H, m), 8.19 (1H, s), 8.68 (1H, dd, J=1.6,4.8Hz), 8.95 (1H, d, J=2.2Hz); ESI+: 578 |
| 7 | 7 | NMR1: 1.04-2.33 (16H, m), 2.95 (4H, brs), 4.04 (2H, brs), 4.53 (2H, d, J= 6.0Hz), 7.28-7.37 (5H, m), 7.92-8.19 (2H, m); ESI+: 513 |
| 8 | 7 | NMR1: 1.20-2.33 (18H,m), 2.96 and 3.15 (total 2H, each d, J=6.3Hz), 3.00 (2H, brs), 3.54 (2H, brs), 4.53 (2H, d, J=6.0Hz), 7.28-7.42 (5H, m), 7.92-8.20 (2H, m); ESI+: 527 |
| 9 | 7 | NMR1: 1.19-2.79 (24H, m), 2.96 and 3.16 (total 2H, each d, J=6.3Hz), 4.53 (2H, d, J=6.0Hz), 7.28-7.36 (5H, m), 7.91 and 8.17 (total 1H, each m), 8.19 (1H, s); ESI+: 541 |

(continued)

| Ex | Syn | Dat |
|---|---|---|
| 10 | 7 | NMR1: 1.06-1.99 (16H, m), 2.94 and 3.14 (total 2H, each d, J=6.3Hz), 3.41 (2H, brs), 4.14 (1H, brs), 4.53 (2H, d, J=6.0Hz), 5.19 (1H, brs), 7.27-7.36 (5H, m), 7.91 and 8.17 (total 1H, each m), 8.19 (1H, s); ESI+: 529 |

[Table 92]

| Ex | Syn | Dat |
|---|---|---|
| 11 | 7 | NMR1: 1.19-1.99 (19H, m), 2.50 (1H, brs), 2.95 and 3.16 (total 2H, each d, J=6.3Hz), 3.31-3.32 (1H, brs), 4.21 (1H, brs), 4.53 (2H, d, J=6.0Hz), 7.28-7.35 (5H, m), 7.91 and 8.17 (total 1H, each m), 8.19 (1H, s); ESI+: 543 |
| 12 | 7 | NMR1: 1.05-2.20 (18H, m), 2.79 (1H, brs), 2.94 and 3.15 (total 2H, each d, J=6.3Hz), 3.91 (2H, brs), 4.48 (1H, brs), 4.53 (2H, d, J=6.0Hz), 7.19-7.34 (5H, m), 7.91 and 8.15 (total 1H, each t, J-6.3Hz), 8.18 (1H, s); ESI+: 559 |
| 13 | 7 | NMR1: 1.08-2.33 (18H, m), 2.95 and 3.15 (total 2H, each d, J=6.3Hz), 3.58 (4H,m), 4.54 (2H, d, J=6.1Hz), 7.21-7.34 (5H, m), 7.90 and 8.15 (total 1H, each t, J=6.3Hz), 8.19 (1H, s); ESI+: 543 |
| 14 | 7 | NMR1: 1.23-2.67 (21H, m), 2.99 and 3.17 (total 2H, each d, J=6.3Hz), 3.80-3.83 (3H, m), 4,21 (1H, brs), 4.43 (2H, d, J=6.0Hz), 6.84-6.87 (1H, m), 6.95-6.99 (1H, m), 7.05-7.08 (1H, m), 7.19-7.29 (2H, m), 7.71 and 7.98 (total 1H, each brs), 8.15 and 8.17 (total 1H, each s); ESI+: 489 |
| 15 | 7 | NMR1: 1.09-2.65 (20H, m), 2.95 and 3.17 (total 2H, each d, J=6.3Hz), 4.17 (1H, brs), 4.52 (2H, d, J=6.2Hz), 4.6 (1H, d, J=4.6Hz), 7.20-7.34 (4H, m), 7.41-7.43 (1H, m), 7.92-8.21 (2H, m); ESI+: 493, 495 |
| 16 | 16 | NMR1: 1.08-2.34 (15H, m), 2.53-2.61 (2H, m), 2.94 and 3.14 (total 2H, each d, J=6.3Hz), 3.44 (2H, m), 4.44 (1H, brs), 4.54 (2H, d, J=6.0Hz), 7.18-7.34 (5H, m), 7.91 and 8.1 (total 1H, each t, J=6.3Hz), 8.18 (1H, s); ESI+: 517 |
| 17 | 16 | NMR1: 1.02-2.08 (14H, m), 2.37 and 2.56 (total 1H, each brs), 2.92 and 3.14 (total 2H, each d, J=6.3Hz), 3.65 and 3.69 (total 2H, each s), 4.51 and 4.55 (total 2H, each d, J=6.2Hz), 7.19-7.35 (10H, m), 7.91 and 8.14-8.16 (total 1H, each m), 8.18 (1H, s); ESI+: 563 |
| 18 | 16 | NMR1: 1.05-2.00 (14H, m), 2.27 (1H, brs), 2.93 and 3.14 (total 2H, each d, J=6.3Hz), 2.99-3.04 (total 2H, each s), 4.52 (2H, d, J=6.0Hz), 7.05-7.36 (7H, m), 7.91 and 8.16 (total 1H, each t, J=6.3Hz), 8.18 (1H, s); ESI+: 530 |
| 19 | 16 | NMR1: 1.02-2.03 (13H, m), 2.53 (1H, brs), 2.94 (2H, d, J=6.3Hz), 3.13 and 3.19 (total 4H, each s), 4.52 (2H, d, J=6.0Hz), 7.00 (2H, brs), 7.22-7.35 (5H, m), 7.65 (2H, brs), 7.92 and 8.16 (total 1H, each t, J=6.3Hz), 8.18 (1H.s); ESI+: 587 |

[Table 93]

| Ex | Syn | Dat |
|---|---|---|
| 20 | 16 | NMR1: 1.04-2.67 (18H, m), 2.94 and 3.16 (total 2H, each d, J=6.3Hz), 3.38-3.42 (4H, m), 4.34 (2H, t, J=5.2Hz), 4.54 (2H, d, J=6.0Hz), 7.18-7.34 (5H, m), 7.90 and 8.14 (total 1H, each t, J=6.3Hz), 8.18 (1H, s); ESI+: 561 |
| 21 | 16 | NMR1: 1.00-2.01 (15H, m), 2.33 (1H, brs), 2.43-2.69 (2H, m), 2.94 (2H, d, J=6.4Hz), 3.09-3.33 (1H, m), 3.47 (2H, t, J=6.0Hz), 4.46-4.62 (1H, brs), 4.54 (2H, d, J=6.4Hz), 7.21-7.39 (5H, m), 8.14 (1H, t, J=6.4Hz), 8.18 (1H, s); ESI+: 531 |
| 22 | 16 | NMR1: 0.78-2.03 (17H, m), 2.28-2.63 (3H, m), 2.94 (2H, d, J=6.4Hz), 3.09-3.35 (1H, m), 3.38 (2H, t, J=6.0Hz), 4.54 (2H, d, J=6.0Hz), 4.69 (1H, brs), 7.23-7.41 (5H, m), 8.14 (1H, t, J=6.4Hz), 8.18 (1H, s); ESI+: 545 |

(continued)

| Ex | Syn | Dat |
|---|---|---|
| 23 | 16 | NMR1: 1.23-2.56 (14H, m), 2.86-3.07 (3H, m), 2.99 and 3.17 (total 2H, each d, J=6.3Hz), 3.43 and 3.63 (total 2H, each m), 3.80-3.83 (3H, m), 4.42-4.43 (2H, m), 5.00 (1H, brs), 6.84-6.87 (1H, m), 6.95-7.00 (1H, m), 7.07 (1H, d, J=7.4Hz), 7.17-7.31 (2H, m), 7.71 and 7.99 (total 1H, each t, J=6.3Hz), 8.16 and 8.17 (total 1H, each s); ESI+: 463 |
| 24 | 16 | NMR1: 1.00-2.00 (15H, m), 2.36 (1H, brs), 2.44-2.60 (2H, m), 2.97 (2H, d, J =6.4Hz), 3.11-3.44 (1H, m), 3.45 (2H, t, J=6.0Hz), 3.83 (3H, s), 4.43 (2H, d, J=6.0Hz), 4.57 (1H, brs), 6.71-7.29 (5H, m), 7.66-8.17 (2H, m); ESI+: 477 |
| 25 | 16 | NMR1: 1.03-2.05 (17H, m), 2.28-2.56 (3H, m), 2.91-3.34 (3H, m), 3.35-3.44 (2H, m), 3.83 (3H, s), 4.43 (2H, d, J=5.6Hz), 4.69 (1H, br), 6.80-7.27 (5H, m), 7.67-8.18 (2H, m); ESI+: 491 |
| 26 | 16 | NMR1: 1.08-2.57 (17H, m), 2.95 and 3.17 (total 2H, each d, J=6.3Hz), 3.45 (2H, m), 4.47 (1H, brs), 4.52 (2H, d, J=6.2Hz), 7.20-7.34 (4H, m), 7.42-7.44 (1H, m), 7.92-8.23 (2H, m); ESI+: 467, 469 |
| 27 | 16 | NMR1: 1.07-2.54 (20H, m), 2.96 and 3.17 (2H, d, J=6.3Hz), 3.44 (2H, q, J= 5.6Hz), 4.43-4.45 (3H, m), 7.06-7.30 (5H, m), 7.83 and 8.11 (total 1H, each t, J=6.3Hz), 8.16 (1H, s); ESI+: 479 |
| 28 | 16 | NMR1: 0.77-2.06 (15H, m), 2.34 (1H, brs), 2.39-2.72 (2H, m), 2.95 (2H, d, J=6.0Hz), 3.11-3.43 (1H, m), 3.47 (2H, t, J-6.0Hz), 4.44-4.63 (3H, m), 7.16-7.47 (5H, m), 8.14-8.23 (2H, m); ESI+: 481, 483 |
| 29 | 16 | NMR1: 1.00-2.05 (15H, m), 2.33 (1H, brs), 2.39-2.68 (5H, m), 2.95 (2H, d, J=6.0Hz), 3.09-3.42 (1H, m), 3.48 (2H, t, J=6.0Hz), 4.45 (2H, d, J=6.0Hz), 4.58 (1H, br), 7.04-7.32 (5H, m), 8.12 (1H, t, J-6.0Hz), 8.17 (1H, s); ESI+:493 |

[Table 94]

| Ex | Syn | Dat |
|---|---|---|
| 30 | 16 | NMR1: 0.81-2.00 (17H, m), 2.31-2.36 (1H, m), 2.38-2.70 (2H, m), 2.95 (2H, d, J=6.4Hz), 3.11-3.36 (1H, m), 3.39 (2H, d, J=6.4Hz), 4.52 (2H, d, J=6.4Hz), 4.70 (1H, br), 7.14-7.49 (5H, m), 8.13-8.25 (2H, m); ESI+: 495, 497 |
| 31 | 16 | NMR1: 1.01-2.05 (17H, m), 1.59 (3H, s), 2.29-2.36 (1H, m), 2.37-2.69 (2H, m), 2.95 (2H, d, J=6.0Hz), 3.13-3.35 (1H, m), 3.39 (2H, d, J=6.0Hz), 4.44 (2H, d, J=6.0Hz), 4.70 (1H, brs), 7.05-7.35 (5H, m), 8.11 (1H, t, J=6.0Hz), 8.16 (1H, s); ESI+: 507 |
| 32 | 32 | NMR1: 1.00-2.01 (14H, m), 1.29 (3H, s), 1.31 (3H, s), 2.25-2.70 (3H, m), 2.94 (2H, d, J=6.0Hz), 3.35-3.43 (1H, m), 3.52-3.66 (2H, m), 3.76-3.89 (2H, m), 4.54 (2H, d, J=6.0Hz), 7.24-7.40 (5H, m), 8.14 (1H, t, J=6.0Hz), 8.18 (1H, s); EST+: 601 |
| 33 | 32 | NMR1: 1.01-2.00 (15H, m), 1.25 (3H, s), 1.30 (3H, s), 2.29-2.69 (3H, m), 2.94 (2H, d, J=6.0Hz), 3.09-3.34 (1H, m), 3.46 (1H, t, J=7.6Hz), 3.99 (1H, dd, J=7.6 and 6.0Hz), 4.04-4.13 (1H, m), 4.53 (2H, d, J-6.0Hz), 7.24- 7.43 (5H, m), 8.14 (1H, t, J=6.0Hz), 8.18 (1H, s); ESI+: 601 |
| 34 | 32 | NMR1: 0.97-2.01 (14H, m), 1.29 (3H, s), 1.31 (3H, s), 2.18-2.69 (3H, m), 2.97 (2H, d, J=6.0Hz), 3.08-3.38 (1H, m), 3.52-3.63 (2H, m), 3.75-3.89 (2H, m), 3.82 (3H, s), 4.43 (2H, d, J=6.0Hz), 6.77-7.29 (5H, m), 7.66-8.00 (1H, m), 8.15 (1H, s); ESI+: 547 |
| 35 | 32 | NMR1: 0.99-2.05 (15H, m), 1.25 (3H, s), 1.30 (3H, s), 2.30-2.70 (3H, m), 2.97 (2H, d, J=6.0Hz), 3.12-3.33 (1H, m), 3.46 (1H, t, J=6.0Hz), 3.82 (3H, s), 3.94-4.13 (2H, m), 4.23 (2H, d, J=6.0Hz), 6.79-7.28 (5H, m), 7.66- 7.99 (1H, m), 8.15 (1H, s); ESI+: 547 |
| 36 | 36 | NMR1: 1.08-2.59 (18H, m), 2.94 and 3.15 (total 2H, each d, J=6.3Hz), 3.29-3.38 (2H, m), 3.50-3.51 (1H, m), 4.53-4.55 (3H, m), 7.16-7.34 (5H, m), 7.90 and 8.14 (total 1H, each t, J=6.3Hz), 8.18 (1H, s); ESI+: 547 |
| 37 | 36 | NMR1: 1.07-2.67 (21H, m), 2.94 and 3.15 (total 2H, each d, J=6.3Hz), 3.36 and 3.50 (total 1H, each m), 4.53-4.57 (2H, m), 7.18-7.34 (5H, m), 7.91 and 8.15 (total 1H, each t, J=6.3Hz), 8.18 (1H, s); ESI+: 547 |

(continued)

| Ex | Syn | Dat |
|---|---|---|
| 38 | 36 | NMR1: 1.08-2.67 (19H, m), 2.97 and 3.16 (total 2H, each d, J=6.3Hz), 3.35 and 3.51 (total 1H, each brs), 3.80-3.83 (3H, m), 4.43 (2H, d, J= 6.0Hz), 4.53-4.58 (2H, m), 6.82-6.86 (1H, m), 6.96-6.98 (1H, m), 7.05-7.26 (3H, m), 7.71 and 7.96 (total 1H, each t, J=6.3Hz), 8.15 and 8.16 (total 1H, each s); ESI+: 493 |

[Table 95]

| Ex | Syn | Dat |
|---|---|---|
| 39 | 39 | NMR1: 1.07-2.00 (11H, m), 2.20-2.23 (1H, m), 2.43 (1H, brs), 2.94 (2H, d, J =6.3Hz), 3.11-3.21 (4H, m), 4.53 (2H, d, J=6.0Hz), 7.19-7.34 (5H, m), 7.90 and 8.15 (total 1H, each t, J=6.3Hz), 8.18 (1H, s); ESI+: 555 |
| 40 | 40 | NMR1: 0.73-2.07 (13H, m), 2.25-2.76 (4H, m), 2.94 (2H, d, J=6.0Hz), 3.13-3.64 (1H, m), 4.27 (4H, s), 4.54 (2H, d, J=6.0Hz), 4.87 (2H, brs), 7.21- 7.39 (5H, m), 8.10-8.20 (2H, m); ESI+: 573 |
| 41 | 41 | NMR1: 0.79-2.71 (24H, m), 2.86-3.14 (2H, m), 3.27-3.52 (1H, m), 4.41-4.59 (3H, m), 7.02-7.41 (5H, m), 7.92 and 8.12 (total 1H, each t, J=6.3Hz), 8.17 and 8.18 (total 1H, each s); ESI+: 571; HPLC: rt=16.3 min |
| 42 | 42 | NMR1: 0.76-2.69 (24H, m), 2.88-3.19 (2H, m), 3.25-3.45 (1H, m), 4.41-4.59 (3H, m), 7.13-7.41 (5H, m), 7.89 and 8.14 (total 1H, each t, J=6.3Hz), 8.18 (1H, s); ESI+: 571; HPLC: rt=15.1 min |
| 43 | 43 | NMR1: 0.72-2.71 (25H, m), 2.49 (3H, s), 2.90-3.18 (2H, m), 3.27-3.41 (1H, m), 4.41-4.48 (3H, m), 7.05-7.37 (5H, m), 7.82 and 8.11 (total 1H, each t, J=6.2Hz), 8.17 (1H, s); ESI+: 533 |
| 44 | 43 | NMR1: 0.72-2.72 (24H, m), 2.49 (3H, s), 2.90-3.19 (2H, m), 3.55-3.65 (1H, m), 4.23-4.48 (3H, m), 7.05-7.37 (5H, m), 7.82 and 8.11 (total 1H, each t, J=6.2Hz), 8.17 (1H, s); ESI+: 533 |
| 45 | 43 | NMR1: 0.69-2.69 (24H, m), 2.88-3.17 (2H, m), 3.22-3.41 (1H, m), 4.41-4.59 (3H, m), 7.13-7.43 (5H, m), 7.90 and 8.14 (total 1H, each t, J=6.3Hz), 8.18 (1H, s); ESI+: 571;HPLC: rt=15.1 min |
| 46 | 43 | NMR1: 1.18-1.94 (15H, m), 2.93 and 3.14 (total 2H, each d, J=6.3Hz), 4.53 (2H, d, J=6.0Hz), 7.16-7.34 (5H, m), 7.90 and 8.15 (total 1H, each t, J=6.3Hz), 8.18 (1H, s); ESI+: 458 |
| 47 | 43 | NMR1: 1.03-1.47 (14H, m), 1.84-2.03 (2H, m), 2.97 and 3.18 (total 2H, each d, J=6.4Hz), 4.53 (2H, d, J=6.4Hz), 7.18-7.36 (5H, m), 7.90 and 8.15 (total 1H, each t, J=6.4Hz), 8.18 (1H, s); ESI+: 473 |
| 48 | 43 | NMR1: 1.13-2.10 (5H, m), 2.51-2.67 (6H, m), 3.13 (2H, t, J=6.0Hz), 4.15 (1H, d, J=6.0Hz), 4.55 (2H, d, J=6.0Hz), 7.33-7.36 (4H, m), 7.56-7.57 (1H, m), 8.14-8.18 (2H, d); ESI+: 433 |
| 49 | 43 | NMR1: 0.97-1.53 (6H, m), 2.67-2.78 (6H, m), 2.99 (2H, d, J=6.4Hz), 4.50 (2H, d, J=6.0Hz), 7.31-7.38 (5H, m), 8.17-8.20 (2H, m); ESI+: 433 |

[Table 96]

| Ex | Syn | Dat |
|---|---|---|
| 50 | 43 | NMR1: 1.26-1.97 (8H, m), 2.99 and 3.15 (total 2H, each brs), 3.55 and 3.64 (total 2H, each s), 4.21 and 4.43 (total 1H, each brs), 4.55-4.58 (2H, m), 7.19-7.36 (10H, m), 7.88 and 8.11 (total 1H, each t, J=6.3Hz), 8.19 (1H, s); ESI+: 509 |
| 51 | 43 | NMR1: 1.25-2.20 (13H, m), 3.77 (1H, brs), 4.38 (1H, brs), 4.51-4.58 (2H, m), 6.27 (1H, d, J=5.2Hz), 7.29-7.38 (4H, m), 8.20-8.27 (2H, m); ESI+: 458 |
| 52 | 43 | NMR1: 1.23-2.51 (13H, m), 3.68-3.70 (1H, m), 4.42 (1H, brs), 4.51 (2H, d, J=6.0Hz), 6.26 (1H, d, J=5.6Hz), 7.29-7.39 (4H, m), 8.21-8.24 (2H, m); ESI+: 482 |

(continued)

| Ex | Syn | Dat |
|---|---|---|
| 53 | 43 | NMR1: 1.39-2.23 (13H, m), 3.84 (1H, brs), 4.51 (2H, d, J=6.0Hz), 6.63 (1H, d, J=5.2Hz), 7.27-7.41 (4H, m), 8.23-8.26 (2H, m); ESI-: 467 |
| 54 | 43 | NMR1: 1.08-2.09 (13H, m), 2.93 (2H, d, J=5.2Hz), 3.82 (1H, brs), 4.32 (1H, t, J=5.2Hz), 4.50 (2H, d, J-6.0Hz), 6.28 (1H, d, J=6.0Hz), 7.31-7.38 (4H, m), 8.19-8.24 (2H, m); ESI+: 474; TLC1: Rf=0.5 |
| 55 | 43 | NMR1: 1.14-2.14 (13H, m), 2.92 (2H, d, J=5.2Hz), 3.81-4.07 (1H, m), 4.31 (1H, t, J=5.2Hz), 4.51 (2H, d, J=6.0Hz), 6.18 (1H, d, J=5.6Hz), 7.31- 7.36 (4H, m), 8.20-8.23 (2H, m); ESI+: 496; TLC1: Rf=0.4 |
| 56 | 43 | NMR1: 1.59-2.05 (8H, m), 2.98 and 3.12 (total 2H, each brs), 3.44 and 3.47 (total 2H, each s), 3.98 and 4.21 (total 1H, each brs), 4.53 and 4.56 (total 2H, each d, J=6.0Hz), 6.52 and 6.60 (total 1H, each d, J= 5.3Hz), 7.21-7.37 (9H, m), 8.03 and 8.20-8.22 (total 1H, each m), 8.21 (1H, s); ESI+: 509 |
| 57 | 43 | NMR1: 0.97-1.54 (14H, m), 1.81 and 2.00 (total 2H, each brs), 2.10 and 2.20 (total 2H, each s), 3.96 and 3.25 (total 2H, each d, J=6.3Hz), 4.54 (2H, d, J=6.0Hz), 7.14-7.34 (5H, m), 7.90 and 8.14 (total 1H, each t, J=6.3Hz), 8.18 and 8.24 (total 1H, each s); ESI+: 487 |
| 58 | 43 | NMR1: 2.57-2.83 (2H, m), 3.44 and 3.59 (total 2H, each brs), 4.55-4.56 (2H, m), 6.45-6.89 (1H, m), 7.32-7.58 (6H, m), 8.01 and 8.16-8.21 (total 1H, each), 8.18 (1H, s), 11.75 (1H, brs); ESI+: 404 |
| 59 | 43 | NMR1: 0.71-2.70 (24H, m), 2.90-3.19 (2H, m), 3.56-3.64 (1H, m), 4.23-4.59 (3H, m), 7.14-7.41 (5H, m), 7.90 and 8.14 (total 1H, each t, J=6.3Hz), 8.18 (1H,s); ESI+: 571 |
| 60 | 43 | NMR1: 0.92-2.69 (24H, m), 2.91-3.18 (2H, m), 3.27-3.43 (1H, m), 4.42-4.58 (3H, m), 7.15-7.44 (5H, m), 7.86-8.23 (2H, m); ESI+: 521, 523 |

table 971

| Ex | Syn | Dat |
|---|---|---|
| 61 | 43 | NMR1: 0.90-2.61 (24H, m), 2.90-3.19 (2H, m), 3.58-3.66 (1H, m), 4.23-4.30 (1H, m), 4.49-4.56 (2H, m), 7.14-7.45 (5H, m), 7.85-8.22 (2H, m); ESI+: 521, 523 |
| 62 | 43 | NMR1: 0.81-2.62 (24H, m), 2.94-3.18 (2H, m), 3.29-3.39 (1H, m), 3.80 and 3.82 (total 3H, each s), 4.41-4.57 (3H, m), 6.81-7.28 (5H, m), 7.66-7.99 (1H, m), 8.15 and 8.16 (total 1H, each s); ESI+: 517 |
| 63 | 63 | NMR1: 1.11-2.09 (14H, m), 2.82 (1H, brs), 2.94 (2H, d, J=6.3Hz), 3.13-3.18 (2H, m), 3.60 (1H, brs), 4.52 (2H, d, J=6.0Hz), 7.26-7.38 (5H, m), 7.93 and 8.18 (total 1H, each t, J=6.2Hz), 8.18 (1H, s); ESI+: 531 |
| 64 | 64 | NMR1: 0.81-2.71 (24H, m), 2.87-3.19 (2H, m), 3.19-4.61 (4H, m), 7.01-7.42 (5H, m), 7.85-7.96 and 8.07-8.17 (total 1H, each m), 8.17 and 8.18 (total 1H, each s); ESI+: 571; HPLC: rt=15.2 min, 16.3 min |
| 65 | 64 | NMR1: 0.87-1.99 (25H, m), 2.93-3.1 3 (3H, m), 4.54 (2H, d, J-6.1Hz), 7.24-7.35 (5H, m), 7.90 and 8.15 (total 1H, each t, J=6.0Hz), 8.18 and 8.19 (total 1H, each s); ESI+: 555 |
| 66 | 64 | NMR1: 0.85-2.74 (21H, m), 2.94-3.27 (4H, m), 4.34 (1H, brs), 4.47-4.60 (2H, m), 7.21-7.34 (5H, m), 7.91 and 8.14 (total 1H, each m), 8.18 and 8.19 (total 1H, each s); ESI+: 557 |
| 67 | 64 | NMR1: 0.84-2.67 (21H, m), 2.94-3.28 (4H, m), 4.32 (1H, brs), 4.52-4.56 (2H, m), 7.06-7.35 (5H, m), 7.91 and 8.13 (total 1H, each m), 8.18 and 8.19 (total 1H, each s); ESI+: 557 |
| 68 | 68 | NMR1: 0.68-2.71 (27H, m), 2.89-3.19 (2H, m), 3.28-3.65 (1H, m), 4.23-4.51 (3H, m), 7.03-7.34 (5H, m), 7.79-7.85 and 8.07-8.14 (total 1H, each m), 8.17 and 8.32 (total 1H, each s); ESI+: 533 |
| 69 | 68 | NMR1: 0.75-2.35 (9H, m), 2.09 (3H, s), 2.82-3.37 (4H, m), 4.55 (2H, d, J= 6.0Hz), 7.14-7.62 (5H, m), 7.89-8.24 (2H, m); ESI+: 447 |
| 70 | 68 | NMR1: 1.06-1.95 (14H, m), 2.07 and 2.13 (total 6H, each s), 2.94 and 3.16 (total 2H, each d, J=6.3Hz), 4.54 (2H, d, J=6.0Hz), 7.21-7.34 (5H, m), 7.90 and 8.1 (total 1H, each t, J=6.3Hz), 8.19 (1H, s); ESI+: 501 |

[Table 98]

| Ex | Syn | Dat |
|---|---|---|
| 71 | 68 | NMR1: 1.00 (3H, t, J=7.1Hz), 1.05-1.88 (15H, m), 2.46 (2H, q, J=7.1Hz), 2.94 and 3.15 (total 2H, each d, J=6.3Hz), 4.54 (2H, d, J=6.0Hz), 7.18-7.33 (5H, m), 7.91 and 8.15 (total 1H, each t, J=6.3Hz), 8.18 (1H, s); ESI+: 501 |
| 72 | 68 | NMR1: 0.86 (6H, t, J=7.0Hz), 1.06-2.15 (14H, m), 2.34-2.59 (4H, m), 2.95 and 3.16 (total 2H, each d, J=6.3Hz), 4.54 (2H, d, J= 6.0Hz), 7.20-7.34 (5H, m), 7.90 and 8.15 (total 1H, each t, J=6.3Hz), 8.18 (1H, s); ESI+: 529 |
| 73 | 68 | NMR1: 1.06-2.60 (15H, m), 2.70-2.72 (4H, m), 2.93 and 3.15 (total 2H, each d, J=6.3Hz), 4.53 (2H, d, J=6.0Hz), 7.16-7.33 (10H, m), 7.91 and 8.15 (total 1H, each t, J=6.3Hz), 8.18 (1H, s); ESI+: 577 |
| 74 | 68 | NMR1: 1.02-1.89 (18H, m), 2.54-2.74 (2H, m), 2.94 and 3.17 (total 2H, each d, J=6.3Hz), 3.27 (2H, t, J=11.6Hz), 3.81 (2H, d, J=11.6Hz), 4.54 (2H, d, J=6.0Hz), 7.16-7.34 (5H, m), 7.89 and 8.14 (total 1H, each t, J=6.3 Hz), 8.15 (1H, s); ESI+: 557 |
| 75 | 68 | NMR1: 1.05-2.12 (14H, m), 2.38 and 2.58 (total 1H, each brs), 2.93 and 3.14 (total 2H, each d, J=6.3Hz), 3.75 and 3.79 (total 2H, each s), 4.51 and 4.55 (total 2H, each d, J=6.0Hz), 7.16-7.33 (6H, m), 7.45 (1H, d, J=7.8Hz), 7.72-7.76 (1H, m), 7.89 and 8.13 (total 1H, each t, J=6.3Hz), 8.17 (1H, s), 8.49 (1H, d, J=4.1Hz); ESI+: 564 |
| 76 | 68 | NMR1: 1.04-2.04 (14H, m), 2.35 and 2.55 (total 1H, each brs), 2.93 and 3.14 (total 2H, each d, J=6.3Hz), 3.67 and 3.71 (total 2H, each s), 4.52 and 4.55 (total 2H, each d, J=6.0Hz), 7.15-7.35 (6H, m), 7.75 (1H, d, J=7.8Hz), 7.89 and 8.13 (total 1H, each t, J=6.3Hz), 8.17 (1H, s), 8.43 (1H, dd, J=1.6,4.7Hz), 8.53 (1H, d, J=1.6Hz); ESI+: 564 |
| 77 | 68 | NMR1: 1.04-2.13 (14H, m), 2.34 (1H, s), 2.93 and 3.14 (total 2H, each d, J=6.3Hz), 3.68 and 3.72 (total 2H, each s), 4.51 and 4.55 (total 2H, each d, J=6.0Hz), 7.15-7.34 (5H, m), 7.36 (2H, d, J=5.8Hz), 7.88 and 8.13 (total 1H, each t, J=6.3Hz), 8.17 (1H, s), 8.48 (2H, dd, J=1.4,4.4Hz); ESI+: 564 |
| 78 | 68 | NMR1: 1.05-2.33 (13H, m), 2.42 (2H, t, J=6.7Hz), 2.95 (2H, d, J=6.3Hz), 3.17 (3H, d, J=5.4Hz), 3.42-3.47 (2H, m), 4.09 (1H, q, J=5.4Hz), 4.27 (1H, t, J=5.4Hz), 4.54 (2H, d, J=6.2Hz), 7.18-7.34 (5H, m), 7.89 and 8.14 (total 1H, each t, J=6.2Hz), 8.18 (1H, s); ESI+: 531 |

[Table 99]

| Ex | Syn | Dat |
|---|---|---|
| 79 | 68 | NMR1: 0.91-1.22 (14H, m), 1.45-1.99 (11H, m), 2.32 (1H, m), 2.93 and 3.15 (total 2H, each d, J=6.3Hz), 4.54 (2H, d, J=6.0Hz), 7.16-7.35 (5H, m), 7.89 and 8.14 (total 1H, each t, J=6.3Hz), 8.18 (1H, s); ESI+: 555 |
| 80 | 68 | NMR1: 0.94 (6H, d, J=6.2Hz), 0.97-1.97 (14H, m), 2.46 and 2.65 (total 1H, each brs), 2.70-2.75 (1H, m), 2.94 and 3.15 (total 2H, each d, J= 6.3Hz), 4.54 (2H, d, J=6.0Hz), 7.16-7.35 (5H, m), 7.90 and 8.14 (total 1H, each t, J=6.3Hz), 8.18 (1H, s); ESI+: 515 |
| 81 | 68 | NMR1: 1.07-2.71 (24H, m), 2.93 and 3.14 (total 2H, each d, J=6.3Hz), 4.54 (2H, d, J=6.0Hz), 7.17-7.34 (5H, m), 7.90 and 8.14 (total 1H, each t, J=6.3Hz), 8.18 (1H, s); ESI+: 573 |
| 82 | 68 | NMR1: 1.08-1.99 (22H, m), 2.46 (1H, brs), 2.59-2.66 (1H, m), 2.94 and 3.15 (total 2H, each d, J=6.3Hz), 4.54 (2H, d, J=6.0Hz), 7.17-7.34 (5H, m), 7.90 and 8.14 (total 1H, each d, J=6.3Hz), 8.18 (1H, s); ESI+: 591 |
| 83 | 68 | NMR1: 0.96-1.96 (15H, m), 2.08 (3H, s), 2.36-2.44 (2H, m), 2.65-2.68 (1H, m), 2.94 (2H, d, J=6.4Hz), 3.36-3.44 (2H, m), 4.40 (1H, brs), 4.54 (2H, d, J=6.4Hz), 7.12-7.44 (5H, m), 8.14 (1H, t, J=6.4Hz), 8.18 (1H, s); ESI+: 545 |
| 84 | 68 | NMR1: 0.79-2.70 (24H, m), 2.88-3.20 (2H, m), 3.28-3.65 (1H, m), 4.23-4.60 (3H, m), 7.12-7.41 (5H, m), 7.86-7.93 and 8.10-8.18 (total 1H, each m), 8.18 (1H, s); ESI+: 571 ;HPLC: rt=15.1 min, 15.8 min |

(continued)

| Ex | Syn | Dat |
|---|---|---|
| 85 | 68 | NMR1: 1.05-1.96 (14H, m), 2.45-2.53 (1H, m), 2.73-2.86 (1H, m), 2.94 and 3.15 (total 2H, each d, J=6.2Hz), 3.21-3.27 (10H, m), 4.50-4.58 (2H, m), 7.14-7.40 (5H, m), 7.90 and 8.15 (total 1H, each t, J=6.2Hz), 8.18 and 8.32 (total 1H, each s); ESI+: 575 |
| 86 | 68 | NMR1: 0.90-2.71 (24H, m), 2.91-3.20 (2H, m), 3.26-3.65 (1H, m), 3.75-3.89 (3H, m), 4.22-4.50 (3H, m), 6.79-7.27 (5H, m), 7.66-7.73 and 7.91-7.99 (total 1H, each m), 8.12-8.18 (1H, m); ESI+: 517 |
| 87 | 68 | NMR1: 1.05-2.68 (22H, m), 2.93 and 3.15 (total 2H, each d, J=6.3Hz), 3.48 (2H, dd, J=8.2, 11.3Hz), 3.76 (2H, dd, J=4.3, 11.3Hz), 4.54 (2H, d, J=6.0Hz), 7.17-7.34 (5H, m), 7.90 and 8.14 (total 1H, each t, J=6.3Hz), 8.18 (1H, s); ESI+: 587 |

[Table 100]

| Ex | Syn | Dat |
|---|---|---|
| 88 | 68 | NMR1: 1.08-1.95 (14H, m), 2.75-2.87 (1H, m), 2.97 and 3.15 (total 2H, each d, J=6.6Hz), 3.21-3.33 (11H, m), 3.80 and 3.83 (total 3H, each s), 4.40-4.46 (2H, m), 6.80-6.89 (1H, m), 6.94-6.99 (1H, m), 7.04-7.27 (3H, m), 7.70 and 7.96 (total 1H, each t, J=6.6Hz), 8.15 and 8.16 (total 1H, each s); ESI+: 521 |
| 89 | 68 | NMR1: 0.86 (3H, d, J=6.4Hz), 1.02-2.00 (13H, m), 2.42-2.47 (1H, m), 2.53-2.70 (2H, m), 2.94 (2H, d, J=6.0Hz), 3.10-3.28 (2H, m), 4.46 (1H, t, J=5.2Hz), 4.54 (2H, d, J=6.0Hz), 7.23-7.40 (5H, m), 8.14 (1H, t, J=6.0Hz), 8.18 (1H, s); ESI+: 531 |
| 90 | 68 | NMR1: 0.88 (3H, d, J=6.0Hz), 1.05-2.00 (13H, m), 2.56-2.68 (2H, m), 2.97 (2H, d, J=6.0Hz), 3.11-3.28 (3H, m), 3.83 (3H, s), 4.33-4.50 (3H, m), 6.78-7.28 (5H, m), 7.96 (1H, t, J=6.0Hz), 8.15 (1H, s); ESI+: 477 |
| 91 | 68 | NMR1: 0.78 (3H, s), 1.00-1.89 (13H, m), 1.97 (3H, s), 2.42-2.62 (1H, m), 2.64-2.69 (1H, m), 2.95 (2H, d, J=6.0Hz), 3.10-3.44 (2H, m), 4.04-4.11 (1H, m), 4.55 (2H, d, J=6.0Hz), 7.22-7.40 (5H, m), 8.14 (1H, t, J=6.0Hz), 8.18 (1H, s); ESI+: 545 |
| 92 | 68 | NMR1: 0.95-2.71 (27H, m), 2.97 and 3.15 (total 2H, each d, J=6.3Hz), 3.80-3.82 (3H, m), 4.43 (2H, d, J=6.0Hz), 6.82-6.86 (1H, m), 6.95 (1H, d, J=8.0Hz), 7.07-7.23 (2H, m), 7.70 and 7.95 (total 1H, each t, J=6.3Hz), 8.14 and 8.16 (total 1H, each s); ESI+: 501 |
| 93 | 68 | NMR1: 1.10-2.74 (20H, m), 2.97 and 3.15 (total 2H, each d, J=6.3Hz), 3.24-3.30 (2H, m), 3.80-3.82 (5H, m), 4.43 (2H, d, J=6.0Hz), 6.82-6.86 (1H, m), 6.95 (1H, d, J=7.5Hz), 7.06-7.25 (3H, m), 7.70 and 7.95 (total 1H, each t, J=6.3Hz), 8.14 and 8.16 (total 1H, each s); ESI+: 503 |
| 94 | 68 | NMR1: 1.07-2.55 (22H, m), 2.97 and 3.15 (total 2H, each d, J=6.3Hz), 3.46-3.51 (2H, m), 3.75-3.82 (5H, m), 4.43 (2H, d, J=6.0Hz), 6.83-6.86 (1H, m), 6.95-6.97 (1H, m), 7.05-7.24 (3H, m), 7.70 and 7.96 (total 1H, each t, J=6.3Hz), 8.15 and 8.16 (total 1H, each s); ESI+: 533 |
| 95 | 68 | NMR1: 0.78-2.71 (24H, m), 2.89-3.21 (2H, m), 3.28-3.65 (1H, m), 4.23-4.59 (3H, m), 7.13-7.37 (4H, m), 7.37-7.45 (1H, m), 7.88-7.95 and 8.14-8.34 (total 2H, each m); ESI+: 521, 523 |
| 96 | 68 | NMR1: 0.87 (3H, d, J=6.0Hz), 1.05-1.98 (13H, m), 2.42-2.68 (3H, m), 2.49 (3H, s), 2.95 (2H, d, J=6.4Hz), 3.10-3.27 (2H, m), 4.34-4.52 (3H, m), 7.04-7.52 (5H, m), 8.11 (1H, t, J=6.4Hz), 8.17 (1H, s); ESI+: 493 |

[Table 101]

| Ex | Syn | Dat |
|---|---|---|
| 97 | 68 | NMR1: 0.87 (3H, s, J=6.0Hz), 1.02-2.00 (13H, m), 2.42-2.69 (3H, m), 2.95 (2H, d, J=6, 4Hz), 3.10-3.29 (2H, m), 4.40-4.57 (3H, m), 7.05-7.45 (5H, m), 8.07-8.22 (2H, m); ESI+: 481 |

(continued)

| Ex | Syn | Dat |
|---|---|---|
| 98 | 68 | NMR1: 1.07-1.29 (7H, m), 1.37-1.73 (6H, m), 1.78-2.02 (2H, m), 2.31-2.49 (1H, m), 2.56-2.69 (3H, m), 2.97 and 3.16 (total 2H, each d, J=each 6.4Hz), 3.80 and 3.81 (total 3H, each s), 3.88-3.98 (2H, m), 4.12-4.19 (1H, m), 4.40-4.46 (2H, m), 5 .52 and 5.53 (total 1H, each s), 6.80-6.89 (1H, m), 6.93-6.97 (1H, m), 7.03-7.28 (3H, m), 7.30-7.44 (5H, m), 7.71 and 7.94 (total 1H, each t, J=6.4Hz), 8.15 and 8.16 (total 1H, each s); ESI+: 595 |
| 99 | 68 | NMR1: 1.10-2.65 (19H, m), 2.95 and 3.15 (total 2H, each d, J=6.3Hz), 3.17 (1H, s), 3.23-3.32 (2H, m), 3.78-3.86 (2H, m), 4.49-4.55 (2H, m), 7.21-7.43 (5H, m), 7.91-8.21 (2H, m); ESI+: 507, 509 |
| 100 | 68 | NMR1: 1.12-2.71 (23H, m), 2.87-2.91 (1H, m), 2.99 and 3.16 (total 2H, each d, J=6.3Hz), 3.80-3.82 (3H, m), 4.44 (2H, d, J=6.1Hz), 6.83-6.88 (1H, m), 6.95-6.98 (1H, m), 7.06-7.27 (3H, m), 7.70 and 7.96 (total 1H, each t, J=6.3Hz), 8.15 and 8.16 (total 1H, each s); ESI+: 515 |
| 101 | 68 | NMR1: 1.02-2.03 (15H, m), 1.25 (3H, s), 1.30 (3H, s), 1.58 (3H, s), 2.28-2.68 (3H, m), 2.95 (2H, d, J=6.0Hz), 3.13-3.39 (1H, m), 3.46 (1H, t, J=7.6Hz), 3.95-4.14 (2H, m), 4.44 (2H, d, J=6.0Hz), 7.04-7.33 (5H, m), 8.11 (1H, t, J=6.0Hz), 8.17 (1H, s); ESI+: 563 |
| 102 | 68 | NMR1: 0.98-2.04 (15H, m), 1.25 (3H, s), 1.30 (3H, s), 2.26-2.77 (3H, m), 2.95 (2H, d, J=6.4Hz), 3.10-3.38 (1H, m), 3.46 (1H, t, J=7.6Hz), 3.93- 4.15 (2H, m), 4.51 (2H, d, J=6.4Hz), 7.14-7.46 (5H, m), 8.13-8.23 (2H, m); ESI+:551,553 |
| 103 | 68 | NMR1: 0.77-2.17 (17H, m), 1.25 (6H, s), 1.30 (6H, s), 2.28-2.69 (5H, m), 2.95 (2H, d, J=6.0Hz), 3.44 (2H, t, J=6.4Hz), 3.90-4.06 (4H, m), 4.52 (2H, d, J=6.0Hz), 7.16-7.45 (5H, m), 8.15-8.22 (2H, m); ESI+: 679, 681 |
| 104 | 68 | NMR1: 0.99-2.04 (17H, m), 1.06 (6H, s), 2.28-2.69 (3H, m), 2.85 (2H, d, J=6.4Hz), 3.12-3.37 (1H, m), 4.46-4.56 (3H, m), 7.14-7.45 (5H, m), 8.14-8.22 (2H, m); ESI+: 523, 525 |
| 105 | 105 | NMR1: 1.04-2.55 (26H, m), 2.95 and 3.16 (total 2H, each d, J=6.3Hz), 3.29 and 3.77 (total 1H, each brs), 4.20 and 4.45 (total 1H, each d, J= 3.1Hz), 4.55 (2H, d, J=6.0Hz), 7.28-7.33 (5H, m), 7.90 and 8.13 (total 1H, each t, J=6.3Hz), 8.18 (1H, s); ESI+: 585 |

[Table 102]

| Ex | Syn | Dat |
|---|---|---|
| 106 | 105 | NMR1: 1.00-1.97 (13H, m), 2.10 and 2.15 (total 3H, each s), 2.30-2.36 (1H, m), 2.84 and 2.95 (total 2H, each d, J= 6.2Hz), 3.05-3.25 (7H, m), 3.28-3.46 (4H, m), 4.49-4.57 (2H, m), 7.13-7.42 (5H, m), 7.91 and 8.15 (total 1H, each t, J=6.2Hz), 8.18 and 8.32 (total 1H, each s); ESI+: 589 |
| 107 | 105 | NMR1: 1.00-2.00 (17H, m), 2.07 (3H, s), 2.26-2.71 (2H, m), 2.93-3.50 (5H, m), 3.82 (3H, s), 4.32-4.54 (3H, m), 6.80-7.32 (5H, m), 7.66-8.19 (2H, m); ESI+: 505 |
| 108 | 105 | NMR1: 0.78-1.96 (17H, m), 2.07 (3H, s), 2.24-2.60 (2H, m), 2.95 (2H, d, J=6.0Hz), 3.10-3.30 (1H, m), 3.35-3.42 (2H, m), 4.40 (1H, t, J=5.2Hz), 4.54 (2H, d, J=6.0Hz), 7.15-7.40 (5H, m), 8.14 (1H, t, J=6.0Hz), 8.18 (1H, s); ESI+: 559 |
| 109 | 105 | NMR1: 1.05-2.41 (19H, m), 2.95 and 3.16 (total 2H, each d, J=6.3Hz), 3.29 (1H, m), 3.36 (1H, m), 3.59 (1H, m), 4.29 (1H, d, J=4.4Hz), 4.50 (1H, t, J=5.5Hz), 4.54 (2H, d, J=6.0Hz), 7.19-7.34 (5H, m), 7.90 and 8.14 (total 1H, each t, J=6.3Hz), 8.18 (1H, s); ESI+: 561 |
| 110 | 105 | NMR1: 1.05-2.55 (24H, m), 2.95 and 3.16 (total 2H, d, J=6.3Hz), 3.62-3.66 (2H, m), 3.79-3.84 (2H, m), 4.54 (2H, d, J=6.1Hz), 7.21-7.35 (5H, m), 7.89 and 8.14 (total 1H, each t, J=6.3Hz), 8.18 (1H, s); ESI+: 601 |
| 111 | 105 | NMR1: 1.00-1.98 (15H, m), 2.08 (3H, s), 2.30-2.71 (2H, m), 2.95 (2H, d, J=6.4Hz), 3.13-3.51 (4H, m), 4.48 (1H, brs), 4.54 (2H, d, J=6.4Hz), 4.60 (1H, brs), 7.20-7.40 (5H, m), 8.14 (1H, t, J=6.4Hz), 8.18 (1H, s); ESI+: 575 |

(continued)

| Ex | Syn | Dat |
|---|---|---|
| 112 | 105 | NMR1: 0.78-1.96 (14H, m), 1.30 (6H, s), 2.04 (3H, s), 2.15-2.70 (3H, m), 2.94 (2H, d, J=6.4Hz), 3.48-3.87 (4H, m), 4.54 (2H, d, J=6.4Hz), 7.22-7.40 (5H, m), 8.14 (1H, t, J=6.4Hz), 8.18 (1H, s); ESI+; 615 |
| 113 | 105 | NMR1: 1.00-2.17 (15H, m), 1.82 (3H, s), 2.08 (3H, s), 2.28-2.69 (3H, m), 2.96 (2H, d, J=6.0Hz), 3.11-3.45 (3H, m), 4.39-4.65 (4H, m), 7.05-7.34 (5H, m), 8.12 (1H, t, J=6.0Hz), 8.17 (1H, s); ESI+: 537 |
| 114 | 105 | NMR1: 1.00-1.88 (15H, m), 2.08 (3H, s), 2.30-2.72 (3H, m), 2.96 (2H, d, J=6.0Hz), 3.16-3.45 (3H, m), 4.45-4.62 (4H, m), 7.17-7.45 (5H, m), 8.15-8.22 (2H, m); ESI+: 525, 527 |
| 115 | 105 | NMR1: 1.05-2.55 (26H, m), 2.95 and 3.16 (total 2H, each d, J=6.2Hz), 3.27-3.29 (1H, m), 4.45 (1H, d, J=4.4Hz), 4.54 (2H, d, J=6.2Hz), 7.18-7.33 (5H, m), 7.90 and 8.14 (total 1H, each t, J=6.3Hz), 8.16 (1H, s); ESI+: 585 |

[Table 103]

| Ex | Syn | Dat |
|---|---|---|
| 116 | 105 | NMR1: 1.05-2.55 (26H, m), 2.95 (2H, d, J=6.4Hz), 3.77 (1H, brs), 4.20 (1H, d, J=3.1Hz), 4.54 (2H, d, J-6.2Hz), 7.18-7.33 (5H, m), 7.90 and 8.14 (total 1H, each t, J=6.2Hz), 8.18 (1H, s); ESI+: 585 |
| 117 | 117 | NMR1: 0.86-1.93 (13H, m), 2.90-3.14 (2H, m), 3.43 and 3.49 (total 1H, each brs), 4.42 and 4.45 (total 1H, each d, J=3.1Hz), 4.53 (2H, d, J=6.0Hz), 7.11-7.34 (5H, m), 7.91 and 8.15 (total 1H, each m), 8.17 and 8.18 (total 1H, each s); ESI+: 474 |
| 118 | 118 | NMR1: 1.08-2.67 (27H, m), 2.97 and 3.15 (total 2H, each d, J=6.2Hz), 3.80-4.11 (4H, m), 4.43 (2H, d, J=6.0Hz), 6.67-7.24 (5H, m), 7.71 and 7.97 (total 1H, each t, J=6.3Hz), 8.15 and 8.16 (total 1H, each s); ESI+: 531;HPLC: rt=13.0 min |
| 119 | 118 | NMR1: 1.07-2.67 (27H, m), 2.97 and 3.15 (total 2H, each d, J=6.2Hz), 3.80-3.90 (4H, m), 4.43 (2H, d, J=5.9Hz), 6.82-7.25 (5H, m), 7.70 and 7.96 (total 1H, each t, J=6.3Hz), 8.15 and 8.16 (total 1H, each s); ESI+: 531;HPLC: rt=14.3 min |
| 120 | 120 | NMR1: 1.20-2.20 (6H, m), 2.62-3.23 (8H, m), 4.50-4.61 (2H, m), 7.26- 7.45 (4H, m), 7.58-7.75 (1H, m), 8.05-8.32 (1H, m), 8.21 (1H, s); ESI+: 449 |
| 121 | 121 | NMR1: 1.05-2.39 (19H, m), 2.64-2.71 (2H, m), 2.86 (1H, brs), 2.95 and 3.16 (total 2H, each d, J=6.3Hz), 3.26-3.28 (2H, m), 4.54 (2H, d, J=6.0Hz), 7.18-7.34 (5H, m), 7.90 and 8.15 (total 1H, t, J=6.3Hz), 8.18 (1H, s); ESI+: 605 |
| 122 | 122 | NMR1: 1.16-1.99 (10H, m), 3.23 (1H, brs), 3.93 and 4.20 (total 1H, each brs), 4.51 and 4.56 (total 2H, each d, J=6.1Hz), 6.47-6.51 (1H, m), 7.32-7.39 (4H, m), 8.04 and 8.19-8.23 (total 1H, each m), 8.20 (1H, s); ESI+: 419 |
| 123 | 123 | NMR1: 1.07-2.03 (15H, m), 2.62 and 2.80 (total 1H, each brs), 2.94 and 3.16 (total 2H, each d, J=6.3Hz), 4.51-4.54 (2H, m), 7.15-7.31 (4H, m), 7.42 (1H, d, J=7.4Hz), 7.92 and 8.19 (total 1H, each t, J=6.3Hz), 8.18 (1H, s); ESI+: 423, 425 |
| 124 | 123 | NMR1: 0.79-2.03 (9H, m), 2.99-3.39 (2H, m), 3.44-3.75 (1H, m), 4.41-4.61 (2H, m), 7.29-7.60 (5H, m), 7.87-8.25 (2H, m); ESI+: 407 |
| 125 | 123 | NMR1: 1.46-2.44 (7H, m), 3.17-3.47 (3H, m), 4.52 and 4.56 (total 2H, each d, J=6.2Hz), 7.30-7.42 (4H, m), 7.42-7.57 (1H, m), 7.93-8.01 and 8.12-8.22 (total 2H, each m); ESI+: 393 |

[Table 104]

| Ex | Syn | Dat |
|---|---|---|
| 126 | 123 | NMR1: 0.92-1.84 (8H, m), 2.08-2.33 (1H, m), 3.00-3.17 (2H, m), 3.20-3.40 (1H, m), 4.47-4.60 (2H, m), 7.29-7.55 (5H, m), 7.90-8.20 (2H, m); ESI+: 407 |
| 127 | 123 | NMR1: 0.95-1.86 (15H, m), 2.43-2.58 (2H, m), 2.91-3.15 (3H, m), 4.54 (2H, d, J=6.0Hz), 6.58-7.35 (5H, m), 7.90 and 8.14 (total 1H, each brs), 8.18 (1H, s); ESI+: 487 |
| 128 | 123 | NMR1: 1.13-2.01 (15H, m), 2.74 and 2.91 (total 1H, each brs), 2.94 and 3.16 (total 2H, each d, J=6.3Hz), 4.46 (2H, d, J=6.0Hz), 7.14-7.20 (2H, m), 7.29-7.33 (2H, m), 7.60 (1H, d, J=7.8Hz), 7.91 and 8.21 (total 1H, each t, J=6.3Hz), 8.18 (1H, s); ESI+: 467, 469 |
| 129 | 123 | NMR1: 1.13-2.02 (15H, m), 2.68 and 2.79 (total 1H, each brs), 2.99 and 3.13 (total 2H, each d, J=6.3Hz), 4.44 and 4.47 (total 2H, each d, J= 6.0Hz), 7.13-7.35 (5H, m), 7.96 and 8.19 (total 1H, each t, 1=6.3Hz), 8.17 and 8.20 (total 1H, each s); ESI+: 423, 425 |
| 130 | 123 | NMR1: 1.12-2.02 (15H, m), 2.66 and 2.79 (total 1H, each brs), 2.98 and 3.12 (total 2H, each d, J=6.3Hz), 4.42 and 4.45 (total 2H, each d, J= 6.0Hz), 7.11-7.28 (1H, m), 7.25 (2H, d, J=8.4Hz), 7.35 (2H, d, J=8.4Hz), 7.95 and 8.17 (total 1H, each t, J=6.3Hz), 8.15 (1H, s); ESI+: 423, 425 |
| 131 | 123 | NMR1: 1.08-2.03 (15H, m), 2.63 and 2.80 (total 1H, each brs), 2.93 and 3.15 (total 2H, each d, J=6.3Hz), 4.52 and 4.56 (total 2H, each d, J= 6.0Hz), 7.12-7.25 (3H, m), 7.68-7.74 (1H, m), 7.91 and 8.15 (total 1H, each d, J=6.3Hz), 8.18 (1H, s), 8.49 (1H, d, J=4.2Hz); ESI+: 390 |
| 132 | 123 | NMR1: 1.14-2.02 (15H, m), 2.69 and 2.79 (total 1H, each brs), 3.01 and 3.12 (total 2H, each d, J=6.3Hz), 4.46-4.50 (2H, m), 7.13 and 7.28 (total 1H, each t, J=6.3Hz), 7.32 (1H, dd, J=4.8,7.7Hz), 7.63-7.65 (1H, m), 7.97-8.21 (2H, m), 8.41-8.42 (1H, m), 8.49-8.50 (1H, m); ESI+: 390 |
| 133 | 123 | NMR1: 1.09-2.03 (15H, m), 2.65 and 2.79 (total 1H, each brs), 2.93 and 3.14 (total 2H, each d, J=6.3Hz), 4.45 and 4.48 (total 2H, each d, J= 6.1Hz), 7.15 and 7.27 (total 1H, each t, J=6.3Hz), 7.23 (2H, d, J=5.8Hz), 7.99 and 8.19 (total 1H, each t, J=6.1Hz), 8.17 (1H, s), 8.46 (2H, dd, J=1.6,4.5Hz); ESI+: 390 |

[Table 105]

| Ex | Syn | Dat |
|---|---|---|
| 134 | 123 | NMR1: 1.11-2.02 (15H, m), 2.65 and 2.79 (total 1H, each brs), 2.99 and 3.13 (total 2H, each d, J=6.3Hz), 4.80-4.52 (2H, m), 4.58 and 4.62 (total 2H, each d, 3=4.1Hz), 5.17 (1H, brs), 7.09-7.24 (4H, m), 7.36-7.38 (1H, m), 7.78 and 8.04 (total 1H, each t, J=6.3Hz), 8.14 and 8.18 (total 1H, each s); ESI+: 419 |
| 135 | 123 | NMR1: 1.09-2.03 (15H, m), 2.64 and 2.80 (total 1H, each brs), 2.97 and 3.15 (total 2H, each d, J=6.3Hz), 3.80 and 3.82 (total 3H, each s), 4.43 (2H, d, J=6.0Hz), 6.82-6.89 (1H, m), 6.95-6.98 (1H, m), 7.06-7.23 (3H, m), 7.71 and 7.95 (total 1H, each t, J=6.2Hz), 8.14 and 8.16 (total 1H, each s); ESI+: 419 |
| 136 | 123 | NMR1: 1.09-2.04 (15H, m), 2.27 and 2.31 (total 3H, each s), 2.64 and 2.79 (total 1H, each brs), 2.98 and 3.15 (total 2H, each d, J=6.3Hz), 4.42-4.46 (2H, m), 7.08-7.24 (5H, m), 7.82 and 8.09 (total 1H, each t, J =6.2Hz), 8.15 and 8.18 (total 1H, each s); ESI+: 403 |
| 137 | 123 | NMR1: 1.12-2.03 (15H, m), 2.67 and 2.79 (total 1H, each brs), 2.99 and 3.14 (total 2H, each d, J=6.3Hz), 4.49 and 4.52 (total 2H, each d, J= 6.0Hz), 7.10-7.28 (5H, m), 7.90-8.19 (2H, m); ESI+: 407 |
| 138 | 123 | NMR1: 1.27-2.00 (14H, m), 3.04 and 3.16 (total 2H, each d, J=6.2Hz), 3.11 and 3.26 (total 1H, each brs), 4.40 (2H, d, J=6.0Hz), 6.67-6.81 (2H, m), 6.99-7.05 (2H, m), 7.26 and 7.33 (total 1H, each t, J=6.3Hz), 7.74 and 7.98 (total 1H, each t, J=6.0Hz), 7.87-7.92 (3H, m), 8.16 and 8.22 (total 1H, each s), 9.50 (1H, brs); ESI+: 405 |
| 139 | 123 | NMR1: 1.11-2.32 (14H, m), 2.66 and 2.80 (total 1H, each brs), 2.98 and 3.15 (total 2H, each d, J=6.3Hz), 3.78 and 3.81 (total 3H, each s), 4.34 and 4.35 (total 2H, each s), 4.43 and 4.45 (total 2H, each d, J= 6.3Hz), 5.00 (1H, brs), 6.89-6.92 (2H, m), 7.08-7.23 (3H, m), 7.71 and 7.95 (total 1H, each t, J=6.3Hz), 8.14 and 8.16 (total 1H, each s); ESI+: 449 |

(continued)

| Ex | Syn | Dat |
|---|---|---|
| 140 | 123 | NMR1: 1.06-2.03 (15H, m), 2.63-2.67 (1H, m), 2.91 and 3.15 (total 2H, each d, J=6.3Hz), 4.52-4.56 (2H, m), 7.17 and 7.21 (total 1H, each d, J= 7.0Hz), 7.28-7.35 (2H, m), 7.49 and 7.53 (total 1H, each d, J=7.0Hz), 7.99 and 8.26 (total 1H, each t, J=6.2Hz), 8.19 (1H, s); ESI+: 457, 459,461 |

[Table 106]

| Ex | Syn | Dat |
|---|---|---|
| 141 | 123 | NMR1: 1.06-2.00 (15H, m), 2.65 (1H, brs), 2.93 and 3.15 (total 2H, each d, J=6.3Hz), 4.47 and 4.50 (total 2H, each d, J-6.1Hz), 7.19 and 7.24 (total 1H, each d, J=8.4Hz), 7.34 (1H, t, J= 6.4Hz), 7.3 6 (1H, dd, J=2.1, 8.4Hz), 7.60 (1H, d, J=2.1Hz), 7.95 and 8.23 (total 1H, each t, J=6.1Hz), 8.18 (1H, s); ESI+: 457, 459,461 |
| 142 | 123 | NMR1: 11.07-2.03 (15H, m), 2.49 (3H, s), 2.63 and 2.81 (total 1H, each brs), 2.95 and 3.15 (total 2H, each d, J=6.3Hz), 4.44 (2H, d, J=5.8Hz), 7.06-7.27 (5H, m), 7.84 and 8.11 (total 1H, each t, J=6.0Hz), 8.16 (1H, s); ESI+: 435 |
| 143 | 123 | NMR1: 1.22-1.25 (2H, m), 1.43-1.62 (10H, m), 1.77 (1H, brs), 1.98-2.01 (2H, m), 2.77 (1H, brs), 3.10 and 3.19 (total 2H, each d, J=6.3Hz), 4.71 (2H, d, J=6.0Hz), 7.08 and 7.28 (total 1H, each brs), 7.36 (1H, t, J=7.8Hz), 7.48 (2H, d, J=7.8Hz), 7.60 and 7.79 (total 1H, each brs), 8.13 and 8.27 (total 1H, each s); ESI+: 457, 459,961 |
| 144 | 123 | NMR1: 1.11-2.78 (16H, m), 2.70 (3H, s), 2.87-3.04 (2H, m), 3.14-3.17 (1H, m), 4.34-4.67 (2H, m), 7.18 and 7.25 (total 1H, each brs), 7.33-7.39 (1H, m), 7.45-7.52 (2H, m), 8.01 and 8.25 (total 1H, each brs), 8.17 (1H, s); ESI+: 451 |
| 145 | 123 | NMR1: 1.41-1.58 (8H, m), 1.89-1.92 (3H, m), 2.00 (2H, brs), 2.66-2.72 (2H, m), 3.13 and 3.23 (total 2H, each d, J=6.2Hz), 3.24 (1H, brs), 3.39-3.45 (2H, m), 6.66 and 6.68 (total 2H, each d, J=8.4Hz), 6.98 and 7.03 (total 2H, each d, J=8.4Hz), 7.28 and 7.70 (total 1H, each brs), 7.56 and 7.95 (total 1H, each brs), 7.92 (3 H, brs), 8.23 and 8.26 (total 1H, each s), 9.25 (1H, brs); ESI+: 419 |
| 146 | 123 | NMR1: 1.42-1.59 (9H, m), 1.90-1.93 (3H, m), 2.00 (2H, brs), 2.69-2.73 (2H, m), 3.13 and 3.22 (total 2H, each d, J=6.2Hz), 3.26 (1H, brs), 3.41-3.47 (2H, m), 6.86 and 6.89 (total 1H, each d, J=8.2Hz), 6.96 and 6.99 (total 1H, each dd, J=2.0,8.2Hz), 7.14 and 7.19 (total 1H, each d, J=2.0Hz), 7.3 and 7.73 (total 1H, each brs), 7.58 and 8.00 (total 1H, each brs), 7.94 (3H, brs), 8.24 and 8.25 (total 1H, each s), 10.01 (1H, brs); ESI+: 453, 455 |

[Table 107]

| Ex | Syn | Dat |
|---|---|---|
| 147 | 123 | NMR1: 1.07-1.31 (7H, m), 1.42-1.55 (3H, m), 1.60-1.71 (1H, m), 1.78-2.04 (2H, m), 2.65-2.70 and 2.82-2.85 (total 1H, each m), 3.00 and 3.14 (total 2H, each d, J=6.3Hz), 3.77 (3H, s), 3.78 (3H, s), 4.44-4.51 (2H, m), 6.66-6.71 and 6.74-6.78 (total 1H, each m), 6.85-7.00 (2H, m), 7.05-7.11 and 7.20-7.26 (1H, m), 7.72-7.78 and 7. 96-8.03 (total 1H, each m), 8.15 and 8.18 (total 1H, each s); ESI+: 449 |
| 148 | 123 | NMR1: 1.05-2.57 (15H, m), 2.94 and 3.15 (total 2H, each d, J=6.1Hz), 3.28 (3H, s), 3.34 (1H, m), 4.86 and 4.96 (total 2H, each d, J=5.9Hz), 7.20 and 7.28 (total 1H, each t, J=6.7Hz), 7.40-7.66 (3H, m), 7.90-7.96 (1H, m), 8.01 and 8.14 (total 1H, each t, J=6.6Hz), 8.20 (1H, s); ESI+: 467 |
| 149 | 123 | NMR1: 1.10-1.33 (6H, m), 1.41-1.55 (3H, m), 1.62-1.69 (1H, m), 1.77-2.04 (3H, m), 2.65-2.69 and 2.78-2.81 (1H, m), 3.02 and 3.13 (total 2H, each d, J=6.2Hz), 4.45-4.56 (4H, m), 6.85-6.94 (2H, m), 7.07-7.25 (3H, m), 7.42-7.61 (2H, m), 7.82 and 8.01 (total 1H, each t, J=6.2Hz), 8.14 and 8.17 (total 1H, each s); ESI+: 462 |

(continued)

| Ex | Syn | Dat |
|---|---|---|
| 150 | 123 | NMR1: 1.15-1.34 (6H, m), 1.41-1.99 (7H, m), 2.96-3.02 and 3.16-3.21 (total 2H, each m), 3.05-3.08 and 3.21-3.24 (total 1H, each), 3.67 and 3.69 (total 3H, each s), 4.43-4.53 (2H, m), 6.73-6.87 (2H, m), 7.27-7.74 (5H, m), 7.86-8.25 (2H, m); ESI+: 453 , 455 |
| 151 | 123 | NMR1: 1.10-2.01 (13H, m), 2.67 and 2.82 (total 1H, each hrs), 3.01 and 3.14 (total 214, each m), 3.25-3.51 (4H, m), 3.78 and 4.15 (total 2H, each brs), 4.51 and 4.55 (total 2H, each d, J=5.8Hz), 7.11-7.58 (9H, m), 7.99-8.24 (2H, m); ESI+: 494 |
| 152 | 123 | NMR1: 1.14-1.35 (7H, m), 1.42-2.02 (6H, m), 2.77-2.82 and 2.90-2.95 (total 1H, each m), 2.98 and 3.16 (total 2H, each d, J=6.3Hz), 3.61 and 3.63 (total 3H, each s), 3.74 and 3.77 (total 3H, each s), 4.35-4.44 (2H, m), 6.61-6.67 (1H, m), 6.70-6.77 (1H, m), 6.86-6.92 (1H, m), 7.12- 7.18 and 7.24-7.30 (total 1H, each m), 7.69-7.75 and 7.94- 8.01 (total 1H, each m), 8.16 and 8.17 (total 1H, each s); ESI+: 449 |
| 153 | 123 | NMR1; 1.11-1.33 (7H, m), 1.41-2.04 (6H, m), 2.72-2.76 and 2.87-2.90 (total 1H, each m), 3.00 and 3.15 (total 2H, each d, J=6.3Hz), 3.34 (3H, s), 3.66-3.73 (2H, m), 4.10-4.17 (2H, m), 4.37-4.49 (2H, m), 6.65- 6.89 (1H, m), 6.95-7.28 (4H, m), 7.56-7.61 and 7.84-7.92 (total 1H, each m), 8.15 and 8.17 (1H, each s); ESI+: 463 |

[Table 108]

| Ex | Syn | Dat |
|---|---|---|
| 154 | 123 | NMR1: 1.06-1.29 (7H, m), 1.43-1.53 (3H, m), 1.59-1.68 (1H, m), 1.77-2.04 (2H, m), 2.62-2.66 and 2.77-2.82 (total 1H, each m), 2.97 and 3.15 (total 2H, each d, J=6.2Hz), 4.43-4.49 (2H, m), 5.21 and 5.24 (total 2H, each s), 6.95-7.03 (1, m), 7.07-7.19 (2H, m), 7.21-7.30 (1H, m), 7.78 and 7.99 (total 1H, each t, J=6.2Hz), 8.16 and 8.17 (total 1H, each s); ESI+: 444 |
| 155 | 123 | NMR1: 1.16-1.38 (7H, m), 1.42-2.03 (6H, m), 2.71-2.75 and 2.88-2.92 (total 1H, each m), 3.00-3.16 (2H, m), 4.39-4.48 (4H, m), 5.05-5.15 (1H, br), 7.06-7.29 (5H, m), 7.87-8.21 (2H, m); ESI+: 419 |
| 156 | 123 | NMR1: 2.32-2.35 (2H, m), 2.50-2.65 (4H, m), 3.08-3.35 (5H, m), 3.43 and 3.56 (total 2H, each q, J=6.7Hz), 4.54 and 4.58 (total 2H, each d, J= 6.0Hz), 7.33-7.42 (5H, m), 8.03-8.21 (2H, m); ESI+: 450 |
| 157 | 123 | NMR1: 1.12-1.37 (6H, m), 1.42-1.57 (3H, m), 1.61-2.24 (4H, m), 2.66-2.72 and 2.77-2.81 (total 1H, each m), 3.00-3.06 and 3.11-3.15 (total 2H, each m), 4.40-4.53 (4H, m), 5.11-5.23 (1H, br), 7.06- 7.17 (2H, m), 7.19-7.27 (3H, m), 7.90-8.21 (2H, m); ESI+: 419 |
| 158 | 123 | NMR1: 1.21-2.00 (13H, m), 2.32-2.81 (4H, m), 2.91 (3H, s), 3.10 and 3.18 (total 2H, each d, J=6.2Hz), 3.41-3.48 (4H, m), 7.05 and 7.29 (total 1H, each t, J=6.3Hz), 7.08-7.19 (4H, m), 7.44 and 7.69 (total 1H, each t, J=6.0Hz), 8.11 and 8.20 (total 1H, each s); ESI+: 496 |
| 159 | 123 | NMR1: 1.10-1.39 (10H, m), 1.42-2.10 (6H, m), 2.69-2.75 and 2.85-2.89 (total 1H, each m), 2.98 and 3.15 (total 2H, each d, J=6.4Hz), 4.00- 4.1 (2H, m), 4.41-4.48 (2H, m), 6.79-6.88 (1H, m), 6.91-6.98 (1H, m), 7.03-7.31 (3H, m), 7.66 and 7.92 (total 1H, each t, J=6.4Hz), 8.15 and 8.17 (total 1H, each s); ESI+: 433 |
| 160 | 123 | NMR1: 1.11-1.32 (7H, m), 1.43-1.57 (3H, m), 1.62-1.71 (1H, m), 1.78-2.04 (2H, m), 2.69-2.73 and 2.82-2.86 (total 1H, each m), 3.02 and 3.14 (total 2H, each d, J=6.2Hz), 3.70-3.78 (2H, m), 3.98-4.05 (2H, m), 4.43-4.50 (2H, m), 4.84-5.00 (1H, br), 6.81-6.88 (1H, m), 6.93-6.98 (1H, m), 7.06-7.25 (3H, m), 7.68 and 7.90 (total 1H, each t, J=6.2Hz), 8.14 and 8.17 (total 1H, each s); ESI+: 449 |
| 161 | 123 | NMR1: 1.44-1.95 (4H, m), 2.16-3.08 (4H, m), 3.18-3.56 (5H, m), 4.53 and 4.57 (total 2H, each d, J=6.1Hz), 7.14-7.39 (5H, m), 8.06-8.21 (2H, m); ESI+: 450 |

[Table 109]

| Ex | Syn | Dat |
|---|---|---|
| 162 | 123 | NMR1: 1.51-1.87 (4H, m), 2.20-3.06 (4H, m), 3.18-3.55 (5H, m), 4.53 and 4.57 (total 2H, each d, J=6.1Hz), 7.32-7.40 (5H, m), 8.06-8.21 (2H, m); ESI+: 450 |
| 163 | 123 | NMR1: 2.31-2.70 (4H, m), 3.01-3.39 (5H, m), 4.01 and 4.18 (total 2H, each s), 4.51 and 4.57 (total 2H, each d, J=5.2Hz), 7.17-7.38 (5H, m), 8.01-8.26 (2H, m); ESI+; 436 |
| 164 | 123 | NMR1: 1.40-1.58 (8H, m), 1.89 (2H, brs), 1.92 (1H, brs), 1.99 (2H, brs), 2.67-2.74 (2H, m), 3.13-3.23 (3H, m), 3.45-3.47 (2H, m), 3.74 (3H, s), 6.57-6.77 (3H, m), 7.31-8.05 (5H, m), 8.26 (1H, s); ESI+: 449 |
| 165 | 123 | NMR1: 1.16-2.63 (15H, m), 2.95 and 3.18 (total 2H, each d, J=6.4Hz), 3.07 and 3.23 (total 1H, each brs), 4.50 (2H, d, J=6.0Hz), 7.37-7.40 (1H, m), 7.57 (1H, t, J=6.0Hz), 7.64 (1H, d, J=6.0Hz), 7.87 (3H, m), 8.21-8.30 (2H, m), 8.39 (1H, t, J=6.0Hz); ESI+: 424, 426 |
| 166 | 123 | NMR1: 1.39-1.57 (4H, m), 1.98-1.88 (3H, m), 2.71-2.76 (2H, m), 3.00-3.23 (2H, m), 3.46-3.47 (1H, m), 3.72-3.75 (8H, m), 6.44-6.50 (3H, m), 7.25-7.98 (5H, m), 8.23 (1H s); ESI+: 479 |
| 167 | 123 | NMR1: 1.16-1.35 (7H, m), 3.40-1.62 (1H, m), 1.68-2.03 (5H, m), 2.99 and 3.19 (total 2H, each d, J=6.2Hz), 3.07-3.14 and 3.24-3.31 (total 1H, each m), 4.39-4.46 (2H, m), 6.58-6.69 (2H, m), 7.15-7.23 (1H, m), 7.31 and 7.50 (total 1H, each t, J=6.2Hz), 7.75-7.93 (3H, m), 8.19-8.30 (2H, m), 9.46-9.64 (1H, br); ESI+: 439, 441 |
| 168 | 123 | NMR1; 1.13-1.34 (7H, m), 1.42-1.59 (2H, m), 1.62-1.67 (1H, m), 1.72-2.01 (3H, m), 2.74-2.78 and 2.90-2.93 (total 1H, each m), 2.95-3.00 and 3.15-3.19 (total 1H, each m), 4.32 and 4.36 (total 2H, each s), 4.44-4.52 (2H, m), 6.78-6.90 (2H, m), 7.16-7.41 (3H, m), 7.51-7.58 (1H, m), 7.86-8.21 (2H, m); ESI+: 496, 498 |
| 169 | 123 | NMR1: 0.95-2.92 (25H, m), 2.92-3.18 (2H, m), 4.49-4.56 (2H, m), 7.14-7.44 (5H, m), 7.87-7.94 and 8.15-8.22 (total 2H, each m); ESI+: 506, 508 |
| 170 | 170 | NMR1: 1.32-2.08 (9H, m), 3.08-3.29 (2H, m), 3.74-4.08 (3H, m), 4.66 (2H, d, J=6.0Hz), 7.30-7.50 (5H, m), 8.50-8.75 (2H, m), 8.85-9.18 (2H, m); ESI+:433 |
| 171 | 170 | NMR1: 1.16-1.56 (4H, m), 1.60-2.04 (4H, m), 2.78-2.98 (1H, m), 3.63-3.77 and 3.93-4.05 (total 1H, each m), 4.58 (2H, d, J=5.7Hz), 7.31- 7.47 (4H, m), 7.91-8.78 (6H, m); ESI+: 407 |

[Table 110]

| Ex | Syn | Dat |
|---|---|---|
| 172 | 170 | NMR1: 1.22-1.89 (211, m), 2.32-3.01 (3H, m), 3.23-3.57 (2H, m), 3.80-4.08 (3H, m), 4.52 and 4.56 (total 2H, each d, J=6.0Hz), 7.31-7.55 (5H, m), 7.84-7.97 (1H, m), 8.06-8.12 (1H, m), 8.22 and 8.25 (total 1H, each s); ESI+: 436 |
| 173 | 170 | NMR1: 1.23-2.4-0 (2H, m), 2.61-2.88 (2H, m), 3.21-3.51 (3H, m), 3.81-4.09 (3H, m), 4.52 and 4.57 (total 2H, each d, J=6.0Hz), 7.32-7.54 (5H, m), 7.87 and 7.97 (total 1H, each d, J=7.2Hz), 8.06-8.11 (1H, m), 8.22 and 8.24 (total 1H, each s); ESI+: 436 |
| 174 | 170 | NMR1: 1.79 (2H, brs), 2.47 and 2.57 (total 2H, each t, J=6.3Hz), 2.95 and 3.05 (total 2H, each q, J=6.0Hz), 3.82 and 3.92 (total 2H, each d, J =4.1Hz), 4.46-4.61 (2H, m), 7.33-7.40 (4H, m), 7.48 and 7.57 (total 1H, each brs), 7.77 and 7.87 (total 1H, each brs), 8.02 and 8.13 (total 1H, each t, J=6.2Hz), 8.22 and 8.25 (total 1H, each s); ESI+: 410 |
| 175 | 175 | NMR1: 4.55 and 4.58 (total 2H, each d, J=5.9Hz), 4.66 and 4.75 (total 2H, each d, J=5.9Hz), 7.15-7.82 (10H, m), 8.27 and 8.83 (total 1H, each brs), 8.32 and 8.52 (total 1H, each brs), 8.37 and 9.11 (total 1H, each brs), 9.57 and 9.60 (total 1H, each s); ESI+: 440 |
| 176 | 176 | NMR1: 1.01-2.02 (15H, m), 2.63 and 2.79 (total 1H, each s), 2.94 and 3.14 (total 2H, each d, J=6.4Hz), 4.54 (2H, d, J=6.4Hz), 7.14-7.34 (5H, m), 8.90 and 8.14 (total 1H, each t, J=6.4Hz), 8.18 (1H, s); ESI+: 473 |

(continued)

| Ex | Syn | Dat |
|---|---|---|
| 177 | 176 | NMR1: 0.88-1.85 (15H, m), 2.72 and 2.82 (total 1H, each brs), 2.91 and 3.11 (total 2H, each d, J=6.3Hz), 4.54 (2H, d, J=6.0Hz), 7.10-7.35 (5H, m), 7.91 and 8.13 (total 1H, each t, J=6.3Hz), 8.17 (1H, s); ESI+: 473 |
| 178 | 176 | NMR1: 1.04-2.03 (15H, m), 2.60 (1H, brs), 3.00 and 3.22 (total 2H, each d, J=6.1Hz), 4.54 (2H, d, J=6.2Hz), 6.29 and 6.43 (total 1H, each brs), 7.30-7.34 (4H, m), 7.68 and 7.89 (total 1H, each brs), 8.03 (1H, s); ESI+: 516 |
| 179 | 176 | NMR1: 1.06-1.91 (16H, m), 2.61 (1H, brs), 2.78 and 3.03 (total 1H, each d, J=6.6Hz), 4.66 (2H, d, J=5.7Hz), 7.19-7.47 (6H, m), 8.01 and 8.09 (total I H, each s); ESI+: 516 |
| 180 | 176 | NMR1: 1.10-2.10 (13H, m), 2.67-2.73 (1H, m), 3.00-3.05 (2H, m), 4.40-4.50 (2H, m), 7.05-7.38 (7H, m), 7.88-7.97 (1H, m), 8.11-8.22 (2H, m); ESI+: 389 |

[Table 111]

| Ex | Syn | Dat |
|---|---|---|
| 181 | 176 | NMR1: 1.17-1.30 (2H, m), 1.38-1.57 (6H,m), 1.62-1.68 (2H,m), 1.74-1.83 (2H, m), 1.94-2.08 (2H, m), 2.74-2.87 (3H, m), 3.05-3.14 (1H, m), 3.20 (2H, d, J=6.4Hz), 3.42-3.52 (2H, m), 6.98-7.49 (6H, m), 7.65-7.75 (1H,m), 8.11 (1H, s); ESI+: 403 |
| 182 | 176 | NMR1: 1.19-1.28 (2H, m), 1.40-1.51 (8H,m), 1.7-1.85 (1H, m), 1.93-2.04 (2H, m), 2.75-2.81 (1H, m), 3.18-3.25 (2H, m), 7.01 (1H, dd, J=7.3,7.3Hz), 7.30 (2H, dd, J=7.3,8.1Hz), 7.51-7.64 (1H, br), 7.77 (2H, d, J=8.114z), 9.66-9.84 (1H, br); ESI+: 375 |
| 183 | 176 | NMR1: 1.07-2.18 (17H, m), 3.22-3.34 (3H, m), 4.57 (2H, d, J=6.0Hz), 7.31-7.57 (5H, m), 7.88 and 8.09 (total 1H, each m), 8.17 (1H, s); ESI+: 487 |
| 184 | 176 | NMR1: 1.13-2.02 (25H, m), 2.77 (1H, s), 3.13-3.14 (2H, m), 3.68 (1H, brs), 6.90 and 7.28 (total 1H, each t, J=6.6Hz), 7.25 and 7.48 (total 1H, each d, J=7.9Hz), 8.09 and 8.18 (total 1H, each s); ESI+: 381 |
| 185 | 176 | NMR1: 0.87-2.03 (26H, m), 2.78 (1H, brs), 3.05-3.1.5 (4H, m), 6.94 and 7.23 (total 1H, each t, J=6.4Hz), 7.40 and 7.68 (total 1H, each t, J= 6.2Hz), 8.08 and 8.18 (total 1H, each s); ESI+: 395 |
| 186 | 186 | NMR1: 1.08-2.03 (15H, m), 2.62 and 2.80 (total 1H, each brs), 2.94 and 3.16 (total 2H, each d, J=6.3Hz), 4.49 (2H, d, J=6.0Hz), 7.18 (1H, d, J= 2.6Hz), 7.23 and 7.36 (total 1H, each m), 7.33 (1H, dd, J=2.6,8.5 Hz), 7.50 (1H, d, J=8.6Hz), 7.95 and 8.20 (totally, each m), 8.20 (1H, s); ESI+: 457, 459,461 |
| 187 | 186 | NMR1: 1.24-2.02 (16H, m), 2.80 (1H, brs), 3.23 (2H, d, J=6.2Hz), 7.18 (1H, d, J=7.9Hz), 7.25 (1H, t, J=7,9Hz), 7.53 (1H, d, J=7.9Hz), 7.66 (1H, brs), 8.36 (1H, s), 8.37 (1H, brs); ESI+: 453, 455 |
| 188 | 188 | NMR1: 1.05-1.30 (7H, m), 1.42-1.66 (6H, m), 1.78-2.01 (2H, m), 2.31-2.62 (4H, m), 2.94 and 3.15 (total 2H, each d, J=6.4Hz), 3.43-3.54 (2H, m), 3.57-3.65 (1H, m), 4.29-4.58 (3H, m), 7.15-7.38 (5H, m), 7.91 and 8.15 (total 1H, each t, J=6.4Hz), 8.18 (1H, s); ESI+: 561 |
| 189 | 188 | NMR1: 1.06-1.30 (7H, m), 1.43-1.66 (6H, m), 1.78-2.01 (2H, m), 2.31-2.55 (4H, m), 2.97 and 3.16 (total 2H, each d, J=6.2Hz), 3.45-3.53 (2H, m), 3.57-3.65 (1H, m), 3.80 and 3.83 (total 3H, each s), 4.38-4.44 (3H, m), 6.81-6.89 (1H, m), 6.94-6.99 (1H, m), 7.03-7.28 (3H, m), 7.71 and 7.97 (total 1H, each t, J=6.2Hz), 8.15 and 8.16 (total 1H, each s); ESI+: 507 |

[Table 112]

| Ex | Syn | Dat |
|---|---|---|
| 190 | 188 | NMR1: 1.04-1.31 (7H, m), 1.43-1.64 (6H, m), 1.78-2.03 (2H, m), 2.28-2.58 (7H, m), 2.96 and 3.16 (total 2H, each d, J=6.1Hz), 3.45-3.68 (3H, m), 4.33-4.72 (3H, m), 7.05-7.48 (5H, m), 7.83 and 8.11 (total 1H, each t, J=6.1Hz), 8.17 and 8.18 (total 1H, each s); ESI+: 523 |

(continued)

| Ex | Syn | Dat |
|---|---|---|
| 191 | 191 | NMR1: 1.20-1.27 (8H, m), 1.39-1.58 (1H, m), 1.74 (1H, brs), 2.05-2.10 (4H, m), 3.01 (2H, d, J=6.1Hz), 3.13 (1H, m), 3.24 (1H, brs), 3.67 (4H, d, J=5.3Hz), 4.61 (2H, d, J=6.3Hz), 7.35-7.41 (5H, m), 8.22-8.33 (3H, m), 8.50 (1H, s), 8.83 (1H, brs); ESI+: 547 |
| 192 | 191 | NMR1: 1.17-1.94 (14H, m), 2.01-2.07 (3H, m), 2.91-3.06 (2H, m), 3.47-3.56 (2H, m), 3.51-3.57 (2H, m), 3.99-4.07 (2H, m), 4.57 (3H, d), 7.25-7.44 (5H, m), 8.27-8.52 (4H, m); ESI+: 561 |
| 193 | 191 | NMR1: 1.01-2.01 (15H, m), 2.26-2.72 (3H, m), 2.94 (2H, d, J=6.0Hz), 3.09-3.40 (3H, m), 3.46-3.57 (1H, m), 4.53 (2H, d, J=6.0Hz), 4.72 (1H, brs), 4.96 (1H, brs), 7.14-7.41 (5H, m), 8.15 (1H, t, J=6.0Hz), 8.18 (1H, s); ESI+: 561 |
| 194 | 191 | NMR1: 1.05-2.00 (14H, m.), 2.29-2.69 (2H, m), 2.97 (2H, d, J-6.0Hz), 3.11-3.58 (6H, m), 3.83 (3H, s), 4.43 (2H, d, J-6.0Hz), 4.73 (1H, br), 4.96 (1H, br), 6.80-7.28 (5H, m), 7.67-8.00 (1H, m), 8.15 (1H, s); ESI+: 507 |
| 195 | 191 | NMR1: 1.05-2.00 (15H,m), 2.31-2.69 (3H, m), 2.97 (2H, d, J=6.4Hz), 3.05-3.56 (4H, m), 3.83 (3H, s), 4.23 (2H, d, J=6.4Hz), 4.75 (1H, br), 4.96 (1H, br), 6.80-7.23 (5H, m), 7.67-7.99 (1H, m), 8.15 (1H, s); ESI+: 507 |
| 196 | 191 | NMR1; 1.00-1.26 (7H, m), 1.38-2.56 (7H, m), 2.76 and 2.80 (total 4H, each s), 2.96 and 3.15 (total 2H, each d, J=6.1Hz), 3.46-3.52 (4H, m), 3.80 and 3.84 (total 3H, each s), 4.40-4.46 (2H, m), 4.49-4.55 (2H, m), 6.81-6.89 (1H, m), 6.98-7.28 (4H, m), 7.70 and 7.96 (total 1H, each t, J=6.1Hz), 8.15 and 8.16 (total 1H, each s); ESI+: 519 |
| 197 | 191 | NMR1: 1.00-2.35 (15H, m), 2.06 (3H, s), 2.94 (2H, d, J=6.0Hz), 3.13-3.46 (6H, m), 4.41-4.60 (4H,m), 7.16-7.40 (5H, m), 8.08-8.20 (2H, m); ESI+: 575 |
| 198 | 191 | NMR1: 1.11-2.68 (17H, m), 2.97 and 3.15 (total 2H, each d, J=6.3Hz), 3.25-3.36 (4H, m), 3.80-3.83 (3H, m), 4.35 (1H, brs), 4.43 (2H, d, J=6.0Hz), 6.82-6.86 (1H, m), 6.95-6.98 (1H m), 7.05-7.26 (3H, m), 7.70 and 7.97 (total 1H, each t, J=6.2Hz), 8.15 and 8.16 (total 1H, each s); ESI+: 493 |

[Table 113]

| Ex | Syn | Dat |
|---|---|---|
| 199 | 188 | NMR1: 1.04-2.43 (19H, m), 2.96 and 3.16 (total 2H, each t, J=6.2Hz), 3.37-3.46 (3H, m), 4.09-4.13 (2H, m), 4.54 (2H, d, J=6.2Hz), 7.13-7.34 (5H, m), 8.15-8.18 (2H, m); ESI+: 561 |
| 200 | 191 | NMR1: 1.02-2.03 (15H, m), 1.59 (3H, s), 2.20-2.70 (3H, m), 2.95 (2H, d, J= 6.0Hz), 3.14-3.60 (4H, m), 4.44 (2H, d, J=6.0Hz), 4.73 (1H, br), 4.98 (1H, br), 7.03-7.38 (5H, m), 8.12 (1H, t, J=6.0Hz), 8.17 (1H, s). ; ESI+: 523 |
| 201 | 191 | NMR1: 1.01-2.00 (15H, m), 2.27-2.68 (3H, m), 2.85 (2H, d, J=6.4Hz), 3.14-3.58 (4H, m), 4.51 (2H, d, J=6.4Hz), 4.73 (1H, br), 4.96 (1H, br), 7.13- 7.68 (5H, m), 8.15-8.22 (2H, m); ESI+: 511, 513 |
| 202 | 191 | NMR1: 1.02-1.98 (17H, m), 2.08-2.72 (5H, m), 2.95 (2H, d, J=6.0Hz), 3.13-3.43 (2H, m), 4.43-4.56 (6H, m), 7.16-7.45 (5H, m), 8.16-8.22 (2H, m); ESI+: 599, 601 |
| 203 | 203 | NMR1: 1.17-1.97 (13H, m), 2.88 (3H, s), 2.90 and 2.95 (total 2H, each d, J=6.3Hz), 3.16 (1H, brs), 4.52-4.56 (2H, m), 6.93 and 7.01 (total 1H, each d, J=6.5Hz), 7.28-7.35 (5H, m), 7.91 and 8.16-8.18 (total 1H, each m), 8.19 (1H, s); ESI+: 551 |
| 204 | 5 | NMR1: 1.00-2.03 (18H, m), 2.31-3.96 (10H, m), 4.48-4.58 (2H, m), 4.71-4.75 (1H, m), 7.16-7.45 (5H, m), 7.88-7.94 and 8.14-8.24 (total 2H, each m); ESI+: 564, S66 |
| 205 | 6 | NMR1: 0.78-2.06 (20H, m), 2.42-3.67 (6H, m), 4.00-4.56 (5H, m), 7.11-7.44 (5H, m), 7.84-8.22 (2H, m); ESI+: 564, 566 |
| 206 | 206 | NMR1: 0.90-2.36 (21H, m), 2.96-3.17 (2H, m), 3.37-3.42 (4H, m), 4.51-4.54 (2H, m), 7.20-7.44 (5H, m), 7.91-8.20 (2H, m); ESI+: 534, 536 |

(continued)

| Ex | Syn | Dat |
|---|---|---|
| 207 | 207 | NMR1: 1.01-3.40 (30H, m), 4.34-4.59 (3H, m), 7.14-7.46 (5H, m), 7.88-7.95 and 8.13-8.24 (total 2H, each m); ESI+: 550, 552 |
| 208 | 207 | NMR1: 1.01-3.52 (30H, m), 4.47-4.57 (3H, m), 7.13-7.45 (5H, m), 7.87-7.95 and 8.14-8.22 (total 2H, each m); ESI+: 550, 552 |
| 209 | 16 | NMR1: 0.97-2.00 (22H, m), 2.33 (2H, t, J=6.4Hz), 2.60-3.20 (4H, m), 3.38-3.51 (2H, m), 4.30 (1H, t, J=5.2Hz), 4.52 (2H, d, J=6.4Hz), 7.16-7.44 (5H, m), 7.66-8.33 (2H, m); ESI+: 550, 552 |

[Table 114]

| Ex | Syn | Dat |
|---|---|---|
| 210 | 16 | NMR1: 1.03-2.33 (22H, m), 2.41 (2H, t, J==6.0Hz), 2.65-3.20 (4H, m), 3.22 (3H, s), 3.40 (2H, t, J=6.0Hz), 4.51 (2H, d, J=6.0Hz), 7.17-7.44 (5H, m), 7.86-8.21 (2H, m); ESI+: 564, 566 |
| 211 | 16 | NMR1: 1.03-1.99 (14H, m), 2.52-2.55 (2H, m), 2.94-3.17 (2H, m), 3.40-3.42 (2H, m), 4.11-4.16 (1H, m), 4.51-4.52 (2H, m), 5.18 (1H, d, J=6.7Hz), 7.21-7.46 (5H, m), 7.92-8.21 (2H, m); ESI+: 479, 481 |
| 212 | 16 | NMR1: 1.00-2.05 (22H, m), 2.27-3.72 (6H, m), 4.40-4.59 (4H, m), 7.13-7.45 (5H, m), 7.87-8.24 (2H, m); ESI+: 552, 554 |
| 213 | 16 | NMR1: 0.96-2.08 (22H, m), 2.27-3.20 (8H, m), 4.52 (2H, d, J=6.4Hz), 7.11-7.45 (5H, m), 7.87-8.23 (2H, m); ESI+: 559, 561 |
| 214 | 16 | NMR1: 0.98-2.14 (22H, m), 2.28-3.18 (6H, m), 4.51 (2H, d, J=6.0Hz), 7.00-7.44 (7H, m), 7.88-8.22 (2H, m); ESI+: 563, 565 |
| 215 | 16 | NMR1: 0.92-2.23 (20H, m), 2.28-3.23 (6H, m), 3.67 (2H, s), 4.52 (2H, d, J= 6.0Hz), 7.11-7.46 (5H, m), 7.86-8.33 (2H, m); ESI+: 545, 547 |
| 21.6 | 16 | NMR1: 0.75-2.06 (23H, m), 2.23-3.19 (7H, m), 3.46 (2H, q, J=5.6Hz), 4.30 (1H, t, J=5.6Hz), 4.52 (2H, d, J=6.4Hz), 7.12-7.46 (5H, m), 7.87-8.32 (2H, m); ESI+: 564,566 |
| 217 | 16 | NMR1: 1.00-2.01 (21H, m), 2.23-3.19 (9H, m), 3.22 (3H, s), 3.40 (2H, t, J=6.0Hz), 4.52 (2H, d, J=6.0Hz), 7.12-7.46 (5H, m), 7.87-8.24 (2H, m); ESI+: 578, 580 |
| 218 | 16 | NMR1: 0.98-2.04 (21H, m), 2.26-3.20 (11H, m), 4.51 (2H, d, J=6.0Hz), 7.14-7.44 (5H, m), 7.88-8.23 (2H, m); ESI+: 573, 575 |
| 219 | 16 | NMR1: 0.98-2.01 (21H, m), 2.22-3.20 (9H, m), 4.10-4.60 (4H, m), 7.10-7.47 (5H, m), 7.87-8.27 (2H, m); ESI+: 566, 568 |
| 220 | 220 | NMR1: 1.08-2.00 (24H, m), 2.46-2.56 (4H, m), 2.94-3.17 (2H, m), 3.41-3.45 (2H, m), 4.29-4.44 (1H, m), 4.51-4.53 (2H, m), 7.19-7.41 (5H, m), 7.91-8.20 (2H, m); ESI+: 564, 566 |
| 221 | 220 | NMR1: 0.86-2.67 (27H, m), 2.94-3.17 (2H, m), 3.48-3.50 (2H, m), 4.10-4.14 (1H, m), 4.51-4.52 (2H, m), 5.27 (1H, brs), 7.22-7.43 (5H, m), 7.90 - 8.22 (2H, m); ESI+: 576, 577 |

[Table 115]

| Ex | Syn | Dat |
|---|---|---|
| 222 | 220 | NMR1: 1.07-3.17 (30H, m), 4.36-4.52 (4H, m), 7.21-7.41 (5H, m), 7.90-8.18 (2H, m); ESI+: 566, 568 |
| 223 | 220 | NMR1: 1.09-2.68 (30H, m), 2.85-3.17 (2H, m), 3.55-3.57 (3H, m), 4.51-4.53 (2H, m), 7.18-7.41 (5H, m), 7.90-8.20 (2H, m); ESI+: 590, 592 |
| 224 | 43 | NMR1: 0.79-2.69 (6H, m), 4.18-4.60 (3H, m), 7.17-7.71 (7H, m), 7.90-8.24 (2H, m); ESI+: 430 |

(continued)

| Ex | Syn | Dat |
|---|---|---|
| 225 | 43 | NMR1: 0.77-2.67 (7H, m), 3.11-3.23 (2H, m), 4.43-4.64 (2H, m), 7.18-7.71 (7H, m), 7.90-8.24 (2H, m); ESI+: 444 |
| 226 | 64 | NMR1: 0.85-2.17 (18H, m), 2.29-2.69 (2H, m), 2.50 (3H, s), 2.90-3.26 (3H, m), 3.34-3.42 (2H, m), 4.39-4.48 (3H, m), 7.05-7.40 (5H, m), 7.78-8.20 (2H, m); ESI+: 519 |
| 227 | 64 | NMR1: 0.75-1.91 (23H, m), 2.20-2.71 (2H, m), 2.48 (3H, s), 2.90-3.23 (4H, m), 4.29-4.36 (1H, m), 4.39-4.48 (2H, m), 7.00-7.30 (5H, m), 7.80-8.19 (2H, m); ESI+: 547 |
| 228 | 64 | NMR1: 0.74-2.62 (29H, m), 2.88-3.40 (3H, m), 4.38-4.49 (3H, m), 6.97-7.33 (5H, m), 7.78-8.19 (2H, m); ESI+: 547 |
| 229 | 64 | NMR1: 0.69-2.02 (24H, m), 2.19-2.69 (3H, m), 2.48 (3H, s), 2.87-3.25 (4H, m), 4.28-4.34 (1H, m), 4.40-4.49 (2H, m), 7.00-7.30 (5H, m), 7.78-8.18 (2H, m); ESI+: 561 |
| 230 | 64 | NMR1: 0.70-2.75 (25H, m), 2.91-3.37 (4H, m), 4.28-4.40 (1H, m), 4.48-4.57 (2H, m), 7.13-7.44 (5H, m), 7.88-8.22 (2H, m); ESI+: 535, 537 ;HPLC: rt=11.9 min, 13.3 min |
| 231 | 64 | NMR1; 0.79-2.13 (25H, m), 2.53-2.58 (3H, m), 2.90-3.24 (4H, m), 4.30-4.42 (3H, m), 7.02-7.11 (1H, m), 7.18-7.42 (2H, m), 7.83-8.22 (2H, m), 8.27-8.38 (1H, m); ESI+: 548 |
| 232 | 64 | NMR1: 0.78-2.69 (25H, m), 2.89-3.23 (4H, m), 3.87-3.94 (3H, m), 4.29-4.43 (3H, m), 6.87-6.95 (1H, m), 7.00-7.44 (2H, m), 7.74-8.09 (2H, m), 8.16 and 8.17 (total 1H, each s); ESI+: 532 |
| 233 | 64 | NMR1: 0.90-3.47 (29H, m), 4.53-4.55 (2H, m), 7.05-7.34 (5H, m), 7.92-8.18 (2H, m); ESI+: 570; HPLC: rt=10.1 min, 11.1 min |

[Table 116]

| Ex | Syn | Dat |
|---|---|---|
| 234 | 64 | NMR1: 0.88-1.83 (24H, m), 2.67-3.47 (5H, m), 4.52-4.53 (2H, m), 7.21-7.44 (5H, m), 7.91-8.19 (2H, m); ESI+: 520, 522;HPLC: rt=8.9 min, 9.9 min |
| 235 | 235 | NMR1: 1.09-2.67 (33H, m), 2.94-3.17 (2H, m), 4.51-4.53 (2H, m), 7.20-7.41 (5H, m), 7.90-8.19 (2H, m); ESI+: 624, 626;HPLC: rt=10.2 min |
| 236 | 235 | NMR1: 1.11-2.27 (26H, m), 2.66-3.16 (7H, m), 4.51-4.53 (2H, m), 7.21-7.42 (5H, m), 7.89-8.18 (2H, m); ESI+: 610, 612;HPLC: rt=10.1 min |
| 237 | 235 | NMR1: 1.03-2.67 (29H, m), 1.97-1.99 (3H, m), 2.94-3.17 (2H, m), 3.37-3.41 (4H, m), 4.51-4.53 (2H, m), 7.21-7.41 (5H, m), 7.89-8.19 (2H, m); ESI+: 631;HPLC: rt=9.2 min |
| 238 | 235 | NMR1: 1.09-1.87 (24H, m), 2.38-3.32 (12H, m), 4.32 (1H, brs), 4.51-4.53 (2H, m), 7.19-7.41 (5H, m), 7.90-8.20 (2H, m); ESI+: 604, 606;HPLC: rt= 15.4 min |
| 239 | 235 | NMR1: 0.86-1.99 (27H, m), 2.38-4.35 (10H, m), 4.51-4.54 (2H, m), 7.19-7.41 (5H, m), 7.91-8.20 (2H, m); ESI+: 604, 606;HPLC: rt=17.2 min |
| 240 | 235 | NMR1: 0.83-2.43 (29H, m), 2.60-2.82 (3H, m), 2.93-3.31 (4H, m), 4.34 (1H, brs), 4.53-4.56 (2H, m), 7.17-7.32 (5H, m), 7.90-8.18 (2H, m); ESI+: 654;HPLC: rt=7.8 min |
| 241 | 235 | NMR1: 1.08-2.55 (30H, m), 2.60-3.30 (6H, m), 4.53-4.56 (2H, m), 4.32 (1H, m), 7.16-7.32 (5H, m), 7.89-8.18 (2H,m); ESI+: 654; HPLC: rt=13.1 min |
| 242 | 68 | NMR1: 1.00-2.03 (18H, m), 2.28-2.53 (2H, m), 2.48 (3H, s), 2.90-3.18 (2H, m), 4.16-4.26 (1H, m), 4.40-4.48 (2H, m), 4.77 (1H, d, J=5.2Hz), 7.03-7.3 (5H, m), 7.78-8.18 (2H, m); ESI+: 505 |
| 243 | 68 | NMR1: 0.93-1.92 (19H, m), 1.97 (3H, s), 2.30-3.20 (5H, m), 3.65-4.21 (2H, m), 4.54 (2H, d, J=6.4Hz), 7.11-7.40 (5H, m), 7.84-8.22 (2H, m); ESI+: 598 |
| 244 | 68 | NMR1: 0.78-2.39 (22H, m), 2.11 (3H, s), 2.60-3.17 (4H, m), 4.54 (2H, d, J= 6.0Hz), 7.10-7.40 (5H, m), 7.84-8.20 (2H, m); ESI+: 570 |

(continued)

| Ex | Syn | Dat |
|---|---|---|
| 245 | 68 | NMR1: 0.97 (3H, t, J=7.2Hz), 1.02-2.03 (18H, m), 2.26 (2H, q, J=7.2Hz), 2.65-3.15 (8H, m), 4.54 (2H, d, J=6.0Hz), 7.12-7.45 (5H, m), 7.87-8.22 (2H, m); ESI+: 584 |

[Table 117]

| Ex | Syn | Dat |
|---|---|---|
| 246 | 68 | NMR1: 0.93 (6H, d, J=6.8Hz), 1.00-2.15 (18H, m), 2.21-3.17 (9H,m), 4.54 (2H, d, J=6.4Hz), 7.11-7.42 (5H, m), 7.86-8.21 (2H, m); ESI+: 598 |
| 247 | 68 | NMR1: 0.97-1.91 (19H, m), 1.93 (3H, s), 2.52-3.20 (5H, m), 3.68-4.20 (2H, m), 4,52 (2H, d, J=6.0Hz), 7.15-7.45 (5H, m), 8.12-8.22 (2H, m); ESI+:548,550 |
| 248 | 68 | NMR1: 0.77-2.38 (22H, m), 2.11 (3H, s), 2.54-3.16 (4H, m), 4.52 (2H, d, J= 5.6Hz), 7.13-7.45 (5H, m), 8.14-8.21 (1H, m), 8.31 (1H, s); ESI+: 520, 522 |
| 249 | 68 | NMR1: 0.97 (3H, t, J=7.2Hz), 1.03-2.39 (20H, m), 2.41-3.42 (8H, m), 4.52 (2H, d, J=6.0Hz), 7.16-7.44 (5H, m), 8.14-8.32 (2H, m); ESI+: 534, 536 |
| 250 | 68 | NMR1: 0.94 (6H, d, J=6.4Hz), 0.90-2.20 (18H, m), 2.21-3.17 (9H, m), 4.52 (2H, d, J=6.0Hz), 7.14-7.46 (5H, m), 7.88-8.12 (2H, m); ESI+: 548, 550 |
| 251 | 68 | NMR1: 0.91-2.69 (24H, m), 2.90-3.19 (2H, m), 3.31-3.64 (1H, m), 4.22-4.44 (1H, m), 4.52-4.64 (2H, m), 7.17-7.38 (1H, m), 7.50-7.54 (1H, m), 7.91-8.25 (2H, m), 8.35-8.45 (2H, m); ESI+: 522, 524 |
| 252 | 68 | NMR1: 0.91-2.71 (24H, m), 2.91-3.18 (2H, m), 3.30-3.64 (1H, m), 3.89 and 3.92 (total 3H, each s), 4.22-4.44 (3H, m), 6.86-6.95 (1H, m), 7.09-7.31 (1H, m), 7.35-7.44 (1H, m), 7.76-8.08 (2H, m), 8.16 (1H, s); ESI+: 518 |
| 253 | 68 | NMR1: 0.92-2.69 (24H, m), 2.96-3.16 (2H, m), 3.28-3.63 (1H, m), 3.86 and 3.88 (total 3H, each s), 4.23-4.46 (3H, m), 7.00-7.04 (1H, m), 7.09-7.31 (1H, m), 7.70-8.02 (1H, m), 8.12-8.20 (2H, m), 8.29-8.36 (1H, m); ESI+: 518 |
| 254 | 68 | NMR1: 0.78-2.35 (26H, m), 2.59-3.03 (5H, m), 4.51 (2H, d, J=6.4Hz), 7.07-7.44 (5H, m), 8.14-8.31 (2H, m); ESI+: 546, 548 |
| 255 | 68 | NMR1: 1.01-2.02 (20H, m), 2.52-3.67 (5H, m), 4.53 (2H, d, J=6.0Hz), 6.67-7.45 (11H, m), 7.87-8.30 (2H, m); ESI+: 582, 584 |
| 256 | 68 | NMR1: 1.02-2.07 (21H, m),2,57-3.49 (5H, m), 2.83 (3H, s), 4.52 (2H, d, J= 6.0Hz), 7.11-7.45 (5H, m), 7.86-8.34 (2H, m); ESI+: 584, 586 |
| 257 | 68 | NMR1: 0.75-2.04 (21H, m), 2.42-3.20 (5H, m), 4.52 (2H, d, J=6.0Hz), 5.83 (2H, s), 7.11-7.46 (5H, m), 7.87-8.24 (2H, m); ESI+: 549, 551 |

[Table 118]

| Ex | Syn | Dat |
|---|---|---|
| 258 | 68 | NMR1: 0.90-2.70 (27H, m), 2.89-3.19 (2H, m), 3.27-3.64 (1H, m), 4.23-4.48 (3H, m), 7.04-7.42 (3H, m), 7.84-8.21 (2H, m), 8.26-8.37 (1H, m); ESI+: 534 |
| 259 | 68 | NMR1: 0.91-2.69 (27H, m), 2.91-3.18 (2H, m), 3.28-3.64 (1H, m), 4.22-4.47 (3H, m), 7.14-7.35 (2H, m), 7.80-8.21 (3H, m), 8.26-8.36 (1H, m); ESI+: 534 |
| 260 | 68 | NMR1: 0.97 (3H, t, J=7.2Hz), 1.00-2.40 (21H, m), 2.26 (2H, q, J=7.2Hz), 2.31-3.19 (5H, m), 2.47 (3H, s), 4.44 (2H, d, J=6.0Hz), 7.03-7.34 (5H, m), 7.77-8.19 (2H, m); ESI+: 546 |
| 261 | 68 | NMR1: 0.90-2.69 (24H, m), 2.91-3.17 (2H, m), 3.29-3.63 (1H, m), 4.23-4.44 (1H, m), 4.61-4.70 (2H, m), 7.15-7.46 (3H, m), 7.60-7.68 (1H, m), 7.77-7.84 (111, m), 8.00-8.35 (2H, m); ESI+: 512 |

(continued)

| Ex | Syn | Dat |
|---|---|---|
| 262 | 68 | NMR1: 0.90-2.69 (24H, m), 2.88-3.19 (2H, m), 3.29-3.64 (1H, m), 4.23-4.53 (3H, m), 7.17-7.42 (2H, m), 7.57-7.67 (1H, m), 7.92-8.31 (3H, m); ESI+: 522, 524 |
| 263 | 68 | NMR1: 0.79-2.03 (23H, m), 2.21-2.62 (6H, m), 2.90-3.19 (2H, m), 3.24-3.39 (1H, m), 4.38-4.50 (3H, m), 7.04-7.31 (5H, m), 7.78-8.22 (2H, m); ESI+: 547; TLC3: Rf=0.44 |
| 264 | 68 | NMR1: 1.00-2.69 (29H, m), 2.91-3.19 (2H, m), 3.64-3.75 (1H, s), 4.19-4.25 (1H, m), 4.40-4.49 (2H, m), 7.04-7.32 (5H, m), 7.79-8.22 (2H, m); ESI+: 547; TLC3: Rf=0.50 |
| 265 | 68 | NMR1: 0.94 (6H, d, J=6.8Hz), 1.00-2.37 (22H, m), 2.49 (3H, s), 2.56-3.19 (5H, m), 4.44 (2H, d, J=6.0Hz), 7.03-7.32 (5H, m), 7.78-8.20 (2H, m); ESI+: 560 |
| 266 | 68 | NMR1: 0.92-2.13 (26H, m), 2.26-3.04 (7H, m), 3.28-3.90 (2H, m), 4.52 (2H, d, J=6.0Hz), 7.09-7.52 (5H, m), 7.80-8.10 (2H, m); ESI+: 590, 592 |
| 267 | 68 | NMR1: 0.99-2.04 (22H, m), 2.45-3.53 (12H, m), 4.52 (2H, d, J=6.0Hz), 7.08-7.48 (5H, m), 7.84-8.32 (2H, m); ESI+: 619, 621 |
| 268 | 68 | NMR1: 0.77-2.02 (20H, m), 2.39-3.87 (6H, m), 4.54 (2H, d, J=6.0Hz), 5.84 (2H, s), 7.09-7.42 (5H, m), 7.85-8.32 (2H, m); ESI+: 599 |

[Table 119]

| Ex | Syn | Dat |
|---|---|---|
| 269 | 68 | NMR1: 0,76-2.04 (20H, m), 2.49 (3H, s), 2.62-3.18 (4H, m), 3.76-3.87 (2H, m), 4.44 (2H, d, J=6.0Hz), 5.83 (2H, s), 7.02-8.37 (5H, m), 7.77-8.32 (2H, m); ESI+: 561 |
| 270 | 68 | NMR1: 1.11-2.57 (24H, m), 1.78 (3H, s), 2.94-3.17 (2H, m), 3.62-3.64 (1H, m), 4.51-4.53 (2H, m), 7.19-7.67 (6H, m), 7.89-8.18 (2H,m); ESI+: 562, 564;HPLC: rt=11.7 min |
| 271 | 68 | NMR1: 0.97-2.02 (20H, m), 1.17 (3H, t, J=7.2Hz), 2.29-3.17 (4H, m), 3.70-3.94 (2H, m), 4.01 (2H, q, J=7.2Hz), 4.51 (2H, d, J=6.0Hz), 7.13-7.46 (5H, m), 7.87-8.23 (2H, m); ESI+: 578, 580 |
| 272 | 68 | NMR1: 0.70-2.03 (21H, m), 1.00 (6H, s), 1.09 (6H, s), 2.29-3.39 (4H, m), 4.52 (2H,d,J=6.0Hz), 7.13-7.45 (5H, m), 7.87-8.22 (2H, m); ESI+:562,564 |
| 273 | 68 | NMR1: 0.92-2.41 (24H, m), 2.64-3.91 (11H, m), 4.54 (2H, d, J=6.0Hz), 7.11-7.41 (5H, m), 7.86-8.22 (2H, m); ESI+: 640 |
| 274 | 68 | NMR1: 0.77-2.09 (21H, m), 0.94 (6H, d, J=6.8Hz), 2.20-3.22 (8H, m), 4.54 (2H, d, J=6.0Hz), 7.11-7.41 (5H, m), 7.86-8.32 (2H, m); ESI+: 612 |
| 275 | 68 | NMR1: 0.94 (6H, d, J=6.8Hz), 0.97-2.16 (21H, m), 2.22-3.20 (8H, m), 4.51 (2H, d, J=6.0Hz), 7.10-7.50 (5H, m), 7.84-8.33 (2H, m); ESI+: 562, 564 |
| 276 | 68 | NMR1: 0.77-2.10 (21H, m), 2.24-3.17 (8H, m), 4.48-4.67 (6H, m), 7.13-7.44 (5H, m), 7.87-8.23 (2H, m); ESI+: 598, 600 |
| 277 | 68 | NMR1: 0.91-2.72 (24H, m), 3.12-3.64 (3H, m), 4.22-4.66 (3H, m), 7.20-7.32 (3H, m), 7.41-7.47 (1H, m), 8.44-8.59 (1H, m), 8.83-8.92 (2H, m); ESI+: 541, 543 |
| 278 | 68 | NMR1: 0.79-2.74 (25H, m), 2.91-3.34 (4H, m), 4.28-4.35 (1H, m), 4.48-4.57 (2H, m), 7.12-7.44 (5H, m), 7.87-8.22 (2H, m); ESI+: 535,537; HPLC: rt=12.0 min, 12.2 min |
| 279 | 105 | NMR1: 1.05-2.55 (29H, m), 2.96-3.16 (2H, m), 3.77-3.78 (1H, m), 4.19-4.20 (1H, m), 4.44-4.45 (2H, m), 7.06-7.30 (5H, m), 7.83-8.11 (1H, m), 8.17 (1H, s); ESI+: 547 |
| 280 | 105 | NMR1: 1.05-2.54 (29H, m), 2.96-3.28 (3H, m),4,44-4.46 (3H, m), 7.06-7.29 (5H, m),7,81-8.11 (1H, m), 8.17 (1H, s); ESI+: 547 |

[Table 120]

| Ex | Syn | Dat |
|---|---|---|
| 281 | 281 | NMR1: 0.97-2.46 (32H, m), 2.64-2.70 (3H, m), 2.94-3.17 (3H, m), 3.69-3.73 (1H, m), 4.14-4.17 (1H, m), 4.51-4.53 (2H, m), 7.15-7.41 (5H, m), 7.90-8.20 (2H, m); ESI+: 645, 647 |
| 282 | 281 | NMR1: 1.08-2.46 (29H, m), 2.65-2.70 (2H, m), 2.94-3.17 (2H, m), 3.24-3.30 (2H, m), 3.79-3.81 (2H, m), 4.52-4.53 (2H, m), 7.19-7.41 (5H, m), 7.90-8.20 (2H, m); ESI+: 604, 606 |
| 283 | 281 | NMR1: 0.95-2.71 (35H, m), 2.94-3.15 (2H, m), 4.51-4.53 (2H, m), 7.19-7.41 (5H, m), 7.91-8.20 (2H, m); ESI+: 638, 640 |
| 284 | 281 | NMR1: 1.08-2.67 (31H, m), 2.93-3.15 (2H, m), 3.25-3.30 (2H, m), 3.79-3.82 (2H, m), 4.53-4.55 (2H, m), 7.27-7.34 (5H, m), 7.88-8.18 (2H, m); ESI+:654 |
| 285 | 123 | NMR1: 1.02-2.06 (15H, m), 2.59-2.82 (1H, m), 2.88-3.19 (2H, m), 4.45-4.55 (2H, m), 7.16-7.42 (2H, m), 7.57-7.66 (1H, m), 7.93-8.32 (3H, m); ESI+: 424, 426 |
| 286 | 123 | NMR1: 1.02-2.05 (15H, m), 2.62-2.68 and 2.76-2.81 (total 1H, each m), 2.89-3.16 (2H, m), 3.40-3.56 (2H, m), 4.50-4.57 (2H, m), 4.74-4.90 (2H, m), 5.16-5.22 (1H, m), 7.06-7.48 (5H, m), 7.71-7.78 and 7.92-7.99 (total 1H, each m), 8.16 and 8.18 (total 1H, each s); ESI+; 449 |
| 287 | 123 | NMR1: 1.14-2.05 (17H, m), 2.71-3.18 (5H, m), 5.45-5.60 (1H, m), 6.99-7.29 (5H, m), 7.70-7.77 and 7.90-7.98 (total 1H, each m), 8.15 and 8.26 (total 1H, each s); ESI+: 415 |
| 288 | 123 | NMR1: 0.71-2.06 (14H, m), 2.25-2.59 (2H, m), 2.64-3.17 (2H, m), 4.59-4.78 (2H, m), 7.10-7.81 (6H, m), 7.97-8.37 (2H, m); ESI+: 445 |
| 289 | 123 | NMR1: 0.92-2.02 (19H, m), 2.20-3.18 (8H, m), 4.54 (2H, d, J=6.0Hz), 7.10-7.45 (5H, m), 7.82-8.32 (2H, m); ESI+: 556 |
| 290 | 123 | NMR1: 0.95-2.02 (20H, m), 2.30-2.56 (3H, m), 2.49 (3H, s), 2.65-3.18 (4H, m), 4.44 (2H, d, J=6.0Hz), 7.04-7.34 (5H, m), 7.79-8.31 (2H, m); ESI+: 518 |
| 291 | 123 | NMR1: 1.16-2.05 (15H, m), 2.73-2.80 (1H, m), 2.90-2.98 (2H, m), 3.08-3.18 (2H, m), 3.47-3.56 (2H, m), 7.01-7.71 (6H, m), 8.10 and 8.20 (total 1H, each s); ESI+: 437, 439 |

[Table 121]

| Ex | Syn | Dat |
|---|---|---|
| 292 | 123 | NMR1: 1.18-2.09 (15H, m), 2.74-2.87 (3H, m), 3.07-3.21 (2H, m), 3.42-3.52 (2H, m), 7.01-7.35 (5H, m), 7.42-7.74 (1H, m), 8.11 and 8.20 (total 1H, each s); ESI+: 437, 439 |
| 293 | 123 | NMR1: 1.16-2.05 (15H, m), 2.73-2.85 (3H, m), 3.07-3.19 (2H, m), 3.41-3.50 (2H, m), 7.00-7.37 (5H, m), 7.44-7.71 (1H, m), 8.11 and 8.21 (total 1H, each s); ESI+: 437, 439 |
| 294 | 123 | NMR1: 1.02-2.05 (15H, m), 2.61-2.82 (1H, m), 2.90-3.17 (2H, m), 3.89 and 3.92 (total 3H, each s), 4.34-4.41 (2H, m), 6.87-6.95 (1H, m), 7.10-7.31 (1H, m), 7.36-7.43 (1H, m), 7.78-8.09 (2H, m), 8.16 (1H, s); ESI+: 420 |
| 295 | 123 | NMR1: 1.06-2.06 (15H, m), 2.62-2.83 (1H, m), 2.90-3.17 (2H, m), 4.59-4.67 (2H, m), 7.15-7.31 (1H, m), 7.37-7.45 (2H, m), 7.59-7.68 (1H, m), 7.78-7.85 (1H, m), 8.00-8.57 (2H, m); ESI+: 414 |
| 296 | 123 | NMR1: 1.16-2.05 (17H, m), 2.55-2.81 (3H, m), 3.09-3.30 (4H, m), 6.95-7.29 (6H, m), 7.42-7.68 (1H, m), 8.10 and 8.19 (total 1H, each s); ESI+: 417 |
| 297 | 123 | NMR1: 1.18-2.04 (15H, m), 2.74-2.80 (1H, m), 3.07-3.18 (2H, m), 4.47-4.56 (2H, m), 7.01-7.41 (4H, m), 7.70-8.06 (1H, m), 8.12 and 8.23 (total 1H, each m); ESI+: 425 |
| 298 | 123 | NMR1: 0.77-2.07 (20H, m), 2.18-2.57 (3H, m), 2.65-3.18 (4H, m), 4.60-4.76 (2H, m), 7.11-7.79 (6H, m), 7.98-8.65 (2H, m); ESI+: 528 |
| 299 | 123 | NMR1: 1.18-2.04 (15H, m), 2.74-2.79 (1H, m), 3.07-3.15 (2H,m), 4.49-4.57 (2H, m), 7.05-7.48 (3H, m), 7.77-8.26 (2H, m); ESI+: 443 |

(continued)

| Ex | Syn | Dat |
|---|---|---|
| 300 | 123 | NMR1: 1.06-2.05 (15H, m), 2.65-2.83 (1H, m), 2.95-3.17 (2H, m), 4.46-4.57 (2H, m), 6.84-6.97 (1H, m), 7.17-7.45 (2H, m), 7.89-8.24 (2H, m); ESI+: 443 |
| 301 | 123 | NMR1: 1.05-2.06 (15H, m), 2.62-2.82 (1H, m), 2.92-3.17 (2H, m), 4.52-4.60 (2H, m), 7.14-7.38 (1H, m), 7.50-7.57 (1H, m), 7.92-8.26 (2H, m), 8.36-8.45 (2H, m); ESI+: 424, 426 |
| 302 | 123 | NMR1: 1.03-2.07 (18H, m), 2.66-3.27 (3H, m), 5.25-5.47 (1H, m), 7.00-7.49 (5H, m), 8.01-8.33 (2H, m); ESI+: 437, 439 |

[Table 122]

| Ex | Syn | Dat |
|---|---|---|
| 303 | 123 | NMR1: 1.09-2.72 (27H, m), 2.93-3.16 (2H, m), 4.53-4.56 (2H, m), 7.15-7.35 (5H,m), 7.89-8.15 (1H, m), 8.18 (1H, s); ESI+: 570; HPLC: rt=10.4 min |
| 304 | 123 | NMR1: 0.94-2.67 (27H, m), 2.93-3.15 (2H, m), 4.53-4.55 (2H, m), 7.15-7.35 (5H,m), 7.89-8.15 (1H, m), 8.18 (1H, s); ESI+: 570; HPLC: rt=10.1 min |
| 305 | 123 | NMR1: 1.08-2.69 (27H, m), 2.94-3.17 (2H, m), 4.51-4.53 (2H, m), 7.19-7.41 (5H, m), 7.90-8.20 (2H, m); ESI+: 529, 522;HPLC: rt=9.1 min |
| 306 | 123 | NMR1: 0.92-2.69 (27H, m). 2.94-3.16 (2H, m), 4.51-4.53 (2H, m), 7.16-7.41 (5H, m), 7.90-8.20 (2H, m); ESI+: 520, 522;HPLC: rt=8.9 min |
| 307 | 123 | NMR1: 1.06-2.32 (15H, m), 2.92-3.14 (2H, m), 3.24-3.43 (6H, m), 4.52-4.54 (2H, m), 7.15-7.34 (5H, m), 7.90-8.18 (2H, m); ESI+: 528 |
| 308 | 123 | NMR1: 1.04-2.33 (15H, m), 2.93-3.16(2H, m), 3.25-3.51 (6H, m), 4.51-4.52 (2H, m), 7.21-7.43 (5H, m), 7.90-8.20 (2H, m); ESI+: 478, 480 |
| 309 | 123 | NMR1: 1.07-2.05 (15H, m), 2.64-2.81 (1H, m), 2.95-3.17 (2H, m), 4.38-4.47 (2H, m), 7.05-7.41 (4H, m), 7.90-8.22 (2H, m); ESI+: 425 |
| 310 | 123 | NMR1: 1.09-2.06 (15H, m), 2.65-2.81 (1H, m), 2.96-3.17 (2H, m), 3.86 and 3.88 (total 3H, each s), 4.39-4.46 (2H, m), 6.99-7.05 (1H, m), 7.08-7.29 (1H, m), 7.72-8.02 (1H, m), 8.13-8.20 (2H, m), 8.30-8.36 (1H, m); ESI+: 420 |
| 311 | 123 | NMR1: 1.01-2.07 (15H, m), 2.54 and 2.55 (total 3H, each s), 2.58-2.82 (1H, m), 2.90-3.18 (2H, m), 4.29-4.41 (2H, m), 7.02-7.11 (1H, m), 7.14-7.41 (2H, m), 7,86-8.23 (2H, m), 8.29-8.37 (1H, m); ESI+: 436 |
| 312 | 123 | NMR1: 1.10-2.00 (25H, m), 2.47 (1H, m), 2.65-2.70 (1H, m), 2.97-3.16 (2H, m), 3.80-3.82 (3H, m), 4.42-4.44 (2H, m), 6.82-7.25 (5H, m), 7.70-7.97 (1H, m), 8.14-8.16 (1H, m); ESI+: 516;HPLC: rt=10.6 min |
| 313 | 123 | NMR1: 0.97-2.67 (27H, m), 2.96-3.15 (2H, m), 3.80-3.82 (3H, m), 4.42-4.44 (2H, m), 6.82-7.24 (5H, m), 7.68-7.97 (1H, m), 8.14-8.16 (1H, m); ESI+: 516;HPLC: rt=10.3 min |

[Table 123]

| Ex | Syn | Dat |
|---|---|---|
| 314 | 123 | NMR1: 0.98-0.99 (3H, m), 1.04-2.33 (24H, m), 2.94-3.16 (2H, m), 3.43-3.45 (1H, m), 4.33-4.35 (1H, m), 4.51-4.53 (2H, m), 7.19-7.41 (5H, m), 7.91-8.20 (2H, m); ESI+: 534, 536 |
| 315 | 123 | NMR1: 1.08-2.00 (24H, m), 2.23 (3H, s), 2.36-2.66 (2H, m), 2.94-3.15 (2H, m), 4.51-4.53 (2H, m), 7.19-7.41 (5H, m), 7.90-8.20 (2H, m); ESI+: 534, 536;HPLC: rt=11.6 min |
| 316 | 123 | NMR1: 0.96-2.70 (29H, m), 2.94-3.16 (2H, m), 4.51-4.53 (2H, m), 7.19-7.41 (5H, m), 7.91-8.20 (2H, m); ESI+: 534, 536;HPLC: rt=11.2 min |

(continued)

| Ex | Syn | Dat |
|---|---|---|
| 317 | 123 | NMR1: 1.07-2.05 (15H, m), 2.52 and 2.53 (total 314, each s), 2.61-2.81 (1H, m), 2.93-3.18 (2H, m), 4.36-4.45 (2H, m), 7.13-7.35 (2H,m), 7.81-8.22 (3H, m), 8.28-8.35 (1H, m); ESI+: 436 |
| 318 | 123 | NMR1: 0.90-2.23 (23H, m), 2.86-3.18 (4H, m), 4.51-4.54 (2H, m), 7.20-7.43 (5H, m), 7.91-8.18 (2H, m); ESI+: 506, 508 |
| 319 | 123 | NMR1: 0.88-2.32 (19H,m), 2.63-2.68 (4H, m), 2.93-3.18 (2H,m), 4.51-4.53 (2H, m), 7.20-7.43 (5H, m), 7.91-8.20 (2H, m); ESI+: 492, 494 |
| 320 | 123 | NMR1: 1.10-1.79 (2H, m), 1.98-3.53 (8H, m), 4.45-4.63 (2H, m), 7.27-7.43 (4H, m), 7.48-7.65 (1H, m), 7.73-8.02 and 8.11-8.24 (total 2H, each m); ESI+: 393 |
| 321 | 123 | NMR1: 0.73-2.09 (22H, m), 2.15-3.55 (7H, m), 4.51 (2H, d, J=6.0Hz), 7.11-7.47 (5H, m), 7.87-8.32 (2H, m); ESI+: 520, 522 |
| 322 | 123 | NMR1: 1.06-2.05 (14H, m), 1.42 (6H, s), 2.63-2.80 (1H, m), 2.98 (2H, s), 3.00-3.17 (2H, m), 4.36 (2H, d, J=6.0Hz), 6.64-7.31 (5H, m), 7.62-8.22 (2H, m); ESI+: 459 |
| 323 | 123 | NMR1: 1.17-2.11 (29H, m), 2.74-2.83 (1H, m), 3.08-3.20 (2H, m), 3.96-4.04 (1H, m), 6.85-7.55 (2H, m), 8.13 and 8.20 (total 1H, each s); ESI+: 433 |
| 324 | 123 | NMR1: 1.20-2.25 (19H, m), 2.77-3.47 (5H, m), 3.95-4.15 (1H, m), 4.35-4.53 (2H, m), 6.92-7.86 (4H, m), 8.12 and 8.22 (total 1H, each s), 8.45-8.50 (1H, m); ESI+: 484 |
| 325 | 123 | NMR1: 0.99-1.97 (15H, m), 2.57-2.85 (1H, m), 2.98-3.13 (2H, m), 4.68-4.72 (2H, m), 7.11-7.47 (4H, m), 7.91-8.25 (4H, m); ESI+: 445 |

[Table 124]

| Ex | Syn | Dat |
|---|---|---|
| 326 | 123 | NMR1: 1.00-1.99 (15H, m), 2.56-2.80 (1H, m), 2.98-3.13 (2H, m), 4.65-4.68 (2H, m), 7.12-7.28 (1H, m), 7.40 (1H, dd, J=8.6,2.0Hz), 7.50-7.59 (1H, m), 8.00-8.27 (4H, m); ESI+: 479, 481 |
| 327 | 123 | NMR1: 0.97-2.05 (15H, m), 2.30-3.43 (3H, m), 4.72 (2H, d, J=6.0Hz), 7.11-7.77 (5H, m), 7.89-8.24 (2H, m); ESI+: 489 |
| 328 | 123 | NMR1: 1.06-1.97 (16H, m), 2.33-3.44 (9H, m), 4.51-4.52 (2H, m), 7.17-(5H, m), 7.92-8.21 (2H, m); ESI+: 492, 494 |
| 329 | 123 | NMR1: 0.76-2.03 (22H, m), 2.22-3.44 (7H, m), 4.54 (2H, d, J=6.4Hz), 7.10-7.43 (5H, m), 7.86-8.32 (2H, m); ESI+: 570 |
| 330 | 123 | NMR1: 0.96-2.04 (20H, m), 2.30-3.43 (8H, m), 3.67-3.78 (1H, m), 4.11-4.22 (1H, m), 4.46-4.58 (2H, m), 7.15-7.36 (4H, m), 7.39-7.45 (1H, m), 7.89-7.96 and 8.15-8.24 (total 2H, each m); ESI+: 563, 565 |
| 331 | 123 | NMR1: 1.01-2.56 (22H, m), 2.82-3.19 (6H, m), 3.56-3.68 (1H, m), 4.47-4.56 (2H, m), 7.16-7.36 (4H, m), 7.38-7.44 (1H, m), 7.62-7.70 (1H, m), 7.89-7.95 and 8.16-8.23 (total 2H, each m); ESI+: 563, 565 |
| 332 | 123 | NMR1: 1.09-2.02 (15H, m), 2.66-2.80 (1H, m), 2.97-3.14 (2H, m), 4.51-4.55 (2H, m), 7.03-7.30 (4H, m), 7.97-8.23 (2H, m); ESI+: 469 |
| 333 | 123 | NMR1: 1.00-2.05 (14H, m), 2.22-2.71 (6H, m), 2.88-3.19 (2H, m), 3.23-3.44 (6H, m), 4.48-4.56 (2H, m), 7.16-7.36 (4H, m), 7.39-7.46 (1H, m), 7.89-7.95 and 8.15-8.24 (total 2H, each m); ESI+: 549, 551 |
| 334 | 123 | NMR1: 0.95-2.09 (15H, m), 2.50-2.83 (1H, m), 2.85-3.20 (2H, m), 4.61-4.72 (2H, m), 7.14-7.76 (5H, m), 7.92-8.01 and 8.16-8.27 (total 2H, each m); ESI+: 457 |
| 335 | 123 | NMR1: 1.07-1.87 (13H, m), 1.87-2.09 (2H, m), 2.63-2.84 (1H, m), 3.12-3.33 (2H, m), 4.54-4.66 (2H, m), 7.22-7.33 (3H, m), 7.42-7.50 (1H, m), 8.43-8.60 and 8.82-8.93 (total 3H, each m); ESI+: 443, 445 |
| 336 | 123 | NMR1: 1.05-2.06 (15H, m), 2.60-2.82 (1H, m), 2.92-3.18 (2H, m), 4.46-4.55 (2H, m), 7.01-7.44 (6H, m), 7.81-8.12 (1H, m), 8.16 and 8.18 (total 1H, each s); ESI+: 455 |

(continued)

| Ex | Syn | Dat |
|---|---|---|
| 337 | 123 | NMR1: 0.57-0.68 (2H, m), 0.85-0.97 (2H, m), 1.02-2.06 (16H, m), 2.58-2.82 (1H, m), 2.93-3.19 (2H, m), 4.60-4.71 (2H, m), 6.93-7.29 (5H, m), 7.80-7.87 and 8.06-8.13 (total 1H, each m), 8.16 and 8.18 (total 1H, each s); ESI+: 429 |

[Table 125]

| Ex | Syn | Dat |
|---|---|---|
| 338 | 338 | NMR1: 0.77-2.03 (24H, m), 2.34-2.68 (3H, m), 2.90-3.23 (4H, m), 4.31 (1H, t, J=5.3Hz), 4.61-4.68 (2H, m), 7.15-7.31 (1H, m), 7.36-7.46 (2H, m), 7.60-7.68 (1H, m), 7.77-7.84 (1H, m), 8.00-8.34 (2H, m); ESI+: 540 |
| 339 | 338 | NMR1: 0.77-2.03 (24H, m), 2.24-2.68 (6H, m), 2.92-3.27 (4H, m), 4.29-4.36 (1H, m), 4.41-4.49 (2H, m), 7.05-7.33 (5H, m), 7.79-8.13 (1H, m), 8.16 (1H, s); ESI+: 561 |
| 340 | 338 | NMR1: 0.79-2.03 (23H, m), 2.20-2.61 (3H, m), 2.89-3.19 (2H, m), 3.26-3.37 (1H, m), 4.40-4.45 (1H, m), 4.48-4.56 (2H, m), 7.12-7.45 (5H, m), 7.87-8.22 (2H, m) ; ESI+: 535, 537 |
| 341 | 338 | NMR1: 0.77-2.03 (24H, m), 2.24-2.69 (3H, m), 2.92-3.23 (4H, m), 4.31 (1H, t, J=5.3Hz), 4.49-4.56 (2H, m), 7.13-7.44 (5H, m), 7.87-8.21 (2H, m); ESI+: 549, 551 |
| 342 | 338 | NMR1: 0.76-2.04 (24H, m), 2.25-2.68 (6H, m), 2.90-3.23 (4H, m), 4.29-4.40 (3H, m), 7.03-7.41 (3H, m), 7.84-8.21 (2H, m), 8.27-8.38 (1H, m); ESI+: 562 |
| 343 | 338 | NMR1: 0.76-2.03 (24H, m), 2.25-2.68 (3H, m), 2.89-3.23 (4H, m), 3.89 and 3.92 (total 3H, each s), 4.29-4.42 (3H, m), 6.86-6.96 (1H, m), 7.09-7.31 (1H, m), 7.35-7.44 (1H, m), 7.77-8.09 (2H, m), 8.16 (1H, s); ESI+: 546 |
| 344 | 338 | NMR1: 0.79-2.04 (23H, m), 2.21-2.62 (6H, m), 2.88-3.18 (2H, m), 3.24-3.38 (1H, m), 4.30-4.40 (2H, m), 4.41-4.45 (1H, m), 7.03-7.12 (1H, m), 7.13-7.41 (2H, m), 7.84-8.21 (2H, m), 8.26-8.37 (1H, m); ESI+: 548 |
| 345 | 338 | NMR1: 0.79-2.03 (23H, m), 2.22-2.61 (3H, m), 2.88-3.19 (2H, m), 3.25-3.38 (1H, m), 3.89 and 3.92 (total 3H, each s), 4.33-4.46 (3H, m), 6.86-6.96 (1H, m), 7.08-7.34 (1H, m), 7.34-7.44 (1H, m), 7.75-8.09 (2H, m), 8.16 (1H, s) ; ESI+: 532 |
| 346 | 338 | NMR1: 0.78-2.56 (26H, m), 2.89-3.18 (2H, m), 3.25-3.41 (1H, m), 4.40-4.45 (1H, m), 4.60-4.68 (2H, m), 7.14-7.33 (1H, m), 7.34-7.46 (2H, m), 7.59-7.69 (1H, m), 7.77-7.85 (1H, m), 7.99-8.35 (2H, m) ; ESI+: 526 |

[Table 126]

| Ex | Syn | Dat |
|---|---|---|
| 347 | 338 | NMR1: 0.78-2.62 (26H, m), 2.89-3.18 (2H, m), 3.25-3.37 (1H, m), 4.41-4.45 (1H, m), 4.50-4.58 (2H, m), 7.10-7.40 (5H, m), 7.85-8.21 (2H, m); ESI+: 585 |
| 348 | 338 | NMR1: 0.78-2.56 (26H, m), 2.91-3.19 (2H, m), 3.25-3.38 (1H, m), 4.42-4.55 (3H, m), 7.02-7.44 (6H, m), 7.82-7.88 and 8.05-8.13 (total 1H, each m), 8.17 and 8.18 (total 1H, each s); ESI+: 567 |
| 349 | 338 | NMR1: 0.79-2.58 (26H, m), 2.86-3.19 (2H, m), 3.26-3.38 (1H, m), 4.43-4.48 (1H, m), 4.62-4.71 (2H, m), 7.18-7.73 (5H, m), 7.93-8.01 and 8.16-8.27 (total 2H, each m); ESI+: 569 |
| 350 | 338 | NMR1: 0.58-0.68 (2H, m), 0.78-2.58 (29H, m), 2.93-3.18 (2H, m), 3.25-3.37 (1H, m), 4.42-4.69 (3H, m), 6.94-7.29 (5H, m), 7.79-7.86 and 8.05-8.13 (total 1H, m), 8.17 and 8.18 (total 1H, each s); East+: 541 |
| 351 | 351 | NMR1: 0.80-2.00 (15H, m), 2.08 (3H, s), 2.29-2.43 (3H, m), 2.49 (3H, s), 3.90-3.19 (2H, m), 3.35-3.47 (2H, m), 4.34-4.49 (3H, m), 7.05-7.32 (5H, m), 7.78-8.20 (2H, m); ESI+: 507 |

(continued)

| Ex | Syn | Dat |
|---|---|---|
| 352 | 191 | NMR1: 1.03-2.00 (16H, m), 2.30-3.34 (7H, m), 3.47-3.60 (1H, m), 3.77-3.87 (3H, m), 4.43 (2H, d, J=6.0Hz), 4.62-5.07 (2H, m), 6.80-7.29 (5H, m), 7.67-8.20 (2H, m); ESI+: 507 |
| 353 | 353 | NMR1: 1.02-2.37 (17H, m), 2.93-3.15 (2H, m), 3.27-3.41 (4H, m), 4.52-4.54 (2H, m), 7.17-7.36 (5H, m), 7.90-8.18 (2H, m); ESI+: 528 |
| 354 | 5 | NMR1: 2.16-2.69 (6H, m), 3.17-3.59 (9H, m), 4.50-4.62 (2H, m), 7.26-7.45 (5H, m), 7.97-8.24 (2H, m); ESI+: 464 |
| 355 | 5 | NMR1: 2.36 and 2.63 (total 2H, each t, J=6.8Hz), 3.13-3.62 (10H, m), 4.51-4.61 (2H, m), 7.29-7.43 (5H, m), 7.98-8.24 (2H, m); ESI+: 451 |
| 356 | 356 | NMR1: 1.01-2.38 (27H, m), 2.59-3.43 (5H, m), 4.37-4.59 (4H, m), 7.11-7.45 (5H, m), 7.86-8.22 (2H, m); ESI+: 580, 582 |
| 357 | 356 | NMR1: 1.0-2.42 (23H, m), 2.60-3.41 (7H, m), 4.47-4.60 (2H, m), 5.92-6.28 (1H, m), 7.10-7.48 (5H, m), 7.85-8.24 (2H, m); ESI+: 584, 586 |
| 358 | 68 | NMR1: 0.83-1.98 (28H, m), 2.54-2.56 (4H, m), 2.94-3.18 (2H, m), 3.91 (1H, brs), 4.34-4.38 (2H, m), 7.06-7.37 (3H, m), 7.87-8.20 (2H, m), 8.30-8.35 (1H, m); ESI+: 562 |

[Table 127]

| Ex | Syn | Dat |
|---|---|---|
| 359 | 68 | NMR1: 1.02-1.99 (28H, m), 2.54-2.56 (4H, m), 2.94-3.18 (2H, m), 4.20 (1H, brs), 4.34-4.38 (2H, m), 7.05-7.37 (3H, m), 7.87-8.20 (2H, m), 8.30-8.35 (1H, m); ESI+: 562 |
| 360 | 68 | NMR1: 0.82-2.37 (25H, m), 2.55 (3H, s), 2.90-3.35 (3H, m), 3.83 (4H, s), 4.30-4.39 (2H, m), 7.02-7.40 (3H, m), 7.84-8.32 (3H, m); ESI+: 590 |
| 361 | 68 | NMR1: 1.00-2.41 (21H, m), 2.91-3.39 (5H, m), 3.77-3.89 (2H, m), 4.40-4.59 (2H, m), 7.11-7.44 (5H, m), 7.87-8.22 (2H, m); FAB+: 521, 523 |
| 362 | 68 | NMR1: 0.85-2.67 (29H, m), 2.95-3.17 (2H, m), 394 (1H, brs), 4.53-4.57 (2H, m), 7.23-7.35 (5H, m), 7.88-8.19 (2H, m); ESI+: 599 |
| 363 | 68 | NMR1: 0.82-2.40 (29H, m), 2.94-3.16 (2H, m), 4.18 (1H, brs), 4.53-4.56 (2H, m), 7.16-7.34 (5H, m), 7.89-8.16 (2H, m); ESI+: 599 |
| 364 | 68 | NMR1: 0.82-2.55 (29H, m), 2.97-3.17 (2H, m), 3.91 (1H, brs), 4.47-4.49 (2H, m), 4.79 (2H, q, J=8.0Hz), 6.95-7.30 (5H, m), 7.67-7.97 (1H, m), 8.17 (1H, s); ESI+: 613 |
| 365 | 68 | NMR1: 0.86-2.31 (29H, m), 2.95-3.15 (2H, m), 4.15 (1H, s), 4.47-4.49 (2H, m), 4.77 (2H, q, J=8.0Hz), 6.94-7.25 (5H, m), 7.67-7.94 (1H, m), 8.16 (1H, s); ESI+: 613 |
| 366 | 68 | NMR1: 1.06-2.56 (27H, m), 1.09 (3H, s), 1.33 (6H, d, J=4.0Hz), 2.97-3.17 (2H, m), 3.93 (1H, brs), 4.34-4.39 (2H, m), 5.28-5.34 (1H, m), 6.84-7.40 (2H, m), 7.68-8.02 (2H, m), 8.18 (1H, s); ESI+: 574 |
| 367 | 68 | NMR1: 0.86-2.00 (32H, m), 2.29-2.34 (3H, m), 2.95-3.15 (2H, m), 4.15 (1H, brs), 4.35-4.39 (2H, m), 5.28-5.34 (1H, m), 6.83-7.33 (3H, m), 7.67-8.00 (2H, m), 8.17 (1H, s); ESI+: 574 |
| 368 | 68 | NMR1: 0.98-2.32 (32H, m), 2.93-3.16 (2H, m), 3.22 (2H, q, J=8.0Hz), 4.15 (1H, s), 4.31-4.35 (2H, m), 7.03-7.38 (3H, m), 7.84-8.18 (2H, m), 8.27-8.34 (1H, m); ESI+: 576 |
| 369 | 68 | NMR1: 1.08-2.40 (29H, m), 2.93-3.18 (2H, m), 3.82-3.90 (1H, m), 4.50-4.54 (2H, m), 7.18-7.44 (5H, m), 7.90-8.19 (2H, m); ES1+: 549, 551 |
| 370 | 68 | NMR1: 0.94-2.40 (29H, m), 2.93-3.17 (2H, m), 4.16 (1H, s), 4.41-4.55 (2H, m), 7.15-7.42 (5H, m), 7.89-8.19 (2H, m); ESI+: 549, 551 |

[Table 128]

| Ex | Syn | Dat |
|---|---|---|
| 371 | 68 | NMR1: 1.04-2.41 (29H, m), 2.98-3.17 (2H, m), 3.86 (1H, br), 4.49-4.56 (2H, m), 7.02-7.42 (6H, m), 7.80-8.09 (1H, m), 8.16-8.18 (1H, m); ESI+: 581 |
| 372 | 68 | NMR1: 0.95-2.39 (29H, m), 2.95-3.17 (2H, m), 4.16 (1H, s), 4.50-4.54 (2H, m), 7.02-7.43 (6H, m), 7.81-8.09 (1H, m), 8.16-8.18 (1H, m); ESI+: 581 |
| 373 | 68 | NMR1: 0.85-2.38 (29H, m), 2.93-3.16 (2H, m), 3.89 and 3.92 (total 3H, each s), 4.17 (1H, br), 4.36-4.40 (2H, m), 6.88-6.94 (1H, m), 7.13-7.43 (2H, m), 7.73-8.04 (2H, m), 8.16 (1H, s); ESI+; 546 |
| 374 | 68 | NMR1: 1.03-2.37 (33H, m), 1.08 (3H, s), 2.90-3.38 (3H, m), 3.84 (1H, br s), 4.09-4.22 (1H, m), 4.26-4.35 (2H, m), 7.00-7.09 (1H, m), 7.28-7.38 (2H, m), 8.08-8.34 (3H, m); ES1+: 616 |
| 375 | 68 | NMR1: 0.80-2.38 (33H, m), 1.07 (3H, s), 2.90-3.38 (3H, m), 4.07-4.22 (2H, m), 4.24-4.36 (2H, m), 6.99-7.40 (3H, m), 7.82-8.35 (3H, m); ESI+: 616 |
| 376 | 376 | NMR1: 1.57-1.85 (2H, m), 2.18-2.43 (2H, m), 2.95-3.15 (4H, m), 3.25-3.44 (2H, m), 3.53-3.71 (4H, m), 4.55-4.67 (2H, m), 7.31-7.50 (4H, m), 7.57-8.81 (3H, m), 9.27-9.47 (2H, m); ESI-: 462 |
| 377 | 123 | NMR1: 1.03-2.06 (15H, m), 2.59-2.81 (1H, m), 2.84 and 2.85 (total 3H, each s), 2.89-3.19 (2H, m), 4.69-4.92 (2H, m), 7.16-7.3 (1H, m), 7.49-7.56 (1H, m), 7.72-7.85 (1H, m), 7.90-8.22 (2H, m), 8.55-8.64 (1H, m); ESI+: 452 |
| 378 | 123 | NMR1: 0.96-207 (15H, m), 2.52-2.83 (1H, m), 2.88-3.20 (2H, m), 3.43 (3H, s), 4.87-4.96 (2H, m), 7.19-7.37 (1H, m), 7.62-7.70 (1H, m), 7.76-7.85 (1H, m), 7.88-8.24 (2H, m), 8.50-8.59 (1H, m); ES1+: 468 |
| 379 | 123 | NMR1: 1.11-2.02 (15H, m), 2.16-2.2 (6H, m), 2.66-2.79 (1H, m), 3.01-3.15 (2H, m), 3.71 (3H, s), 4.49-4.53 (2H, m), 7.09-7.23 (1H, m), 7.44-7.79 (1H, m), 8.11-8.23 (2H, m); ESI+: 448 |
| 380 | 123 | NMR1: 1.06-2.03 (21H, m), 2.61-2.79 (1H, m), 2.94-3.15 (2H, m), 4.34-4.38 (2H, m), 5.26-5.34 (1H, m), 6.83-8.01 (5H, m), 8.17 (1H, s); ES1+: 448 |

[Table 129]

| Ex | Syn | Dat |
|---|---|---|
| 381 | 123 | NMR1: 1.07-2.03 (15H, m), 2.62-2.80 (1H, m), 2.95-3.16 (2H, m), 4.47-4.79 (2H, m), 4.75-4.81 (2H, m), 6.94-7.26 (5H, m), 7.70-7.95 (1H, m), 8.16 (1H, s); ESI+: 487 |
| 382 | 123 | NMR1: 1.07-1.02 (21H, m), 2.62-2.80 (1H, m), 2.94-3.16 (2H, m), 4.07-4.14 (1H, m), 4.29-4.33 (2H, m), 7.03-7.39 (3H, m), 7.82-8.11 (1H, m), 8.17 (1H, s), 8.30-8.33 (1H, m); ESI+: 464 |
| 383 | 123 | NMR1: 0.96-2.10 (17H, m), 2.26-3.39 (8H, m), 4.38-4.73 (3H, m), 7.11-7.48 (5H, m), 7.82-8.35 (2H, m); ESI+: 524, 526 |
| 384 | 123 | NMR1: 1.23-1.62 (4H, m), 2.05-2.43 (3H, m), 2.51-2.69 (4H, m), 3.12-3.43 (6H, m), 4.48-4.61 (2H, m), 7.28-7.56 (5H, m), 7.91-8.22 (2H, m); ESI+: 478 |
| 385 | 123 | NMR1: 1.06-2.02 (15H, m), 1.32 (3H, t, J=8.0Hz), 2.61-2.79 (1H, m), 2.93 -3.17 (2H, m), 3.22 (2H, q, J=8.0H.z), 4.31-4.34 (2H, m), 7.04-7.35 (3H, m), 7.87-8.17 (2H, m), 8.29-8.33 (1H, m); ESI+: 450 |
| 386 | 123 | NMR1: 1.31-1.48 (2H, m), 1.70-1.83 (2H, m), 2.24 and 2.40 (total 2H, each t, J=6.8Hz), 2.68-2.82 (2H, m), 3.02-3.14 (2H, m), 3.38-3.61 (4H, m), 3.64-3.79 (1H, m), 4.51-4.61 (2H, m), 7.26-7.47 (4H, m), 7.78-7.97 (1H, m), 7.98-8.23 (2H, m); ESI+: 464 |
| 387 | 123 | NMR1: 1.59-1.83 (2H, m), 2.22-2.94 (6H, m), 3.15-3.64 (7H, m), 4.49-4.61 (2H, m), 7.25-7.43 (5H, m), 7.97-8.23 (2H, m); ESI+: 464 |
| 388 | 123 | NMR1: 1.07-1.85 (13H, m), 1.89-2.08 (2H, m), 2.63-2.83 (1H, m), 3.10-3.40 (2H, m), 4.54-4.68 (2H, m), 7.29-7.42 (4H, m), 8.42-8.90 (3H, m); ESI+: 493 |

(continued)

| Ex | Syn | Dat |
|---|---|---|
| 389 | 123 | NMR1: 0.99-2.26 (23H, m), 2.57-3.61 (3H, m), 4.07-4.19 (1H, m), 4.23-4.38 (2H, m), 6.98-7.40 (3H, m), 7.69-8.35 (3H, m); ESI+: 490 |
| 390 | 123 | NMR1: 1.04-1.74 (13H, m), 1.86-2.01 (2H, m), 2.62-2.72 (1H, m), 2.85-3.03 (2H, m), 4.36-4.50 (2H, m), 6.93-7.09 (2H, m), 7.23-7.39 (4H, m), 7.66 (1H, d, J=4.0Hz); ESI+: 466 |
| 391 | 123 | NMR1: 0.97-1.74 (13H, m), 1.85-2.00 (2H, m), 2.61-2.74 (1H, m), 2.86-3.04 (2H, m), 4.36-4.49 (2H, m), 6.58-7.13 (2H, m), 7.16-7.47 (4H, m), 7.66 (1H, d, J=4.4Hz); ESI+: 416, 418 |

[Table 130]

| Ex | Syn | Dat |
|---|---|---|
| 392 | 338 | NMR1: 0.80-2.58 (26H, m), 2.84 and 2.86 (total 3H, each s), 2.88-3.17 (2H, m), 3.25-3.38 (1H, m), 4.42-4.45 (1H, m), 4.69-4.93 (2H, m), 7.16-7.37 (1H, m), 7.48-7.57 (1H, m), 7.71-7.86 (1H, m), 7.89-8.23 (2H, m), 8.52-8.64 (1H, m); ESI+: 564 |
| 393 | 338 | NMR1: 0.78-2.55 (26H, m), 2.88-3.20 (2H, m), 3.25-3.37 (1H, m), 3.43 (3H, s), 4.41-4.47 (1H, m), 4.86-4.97 (2H, m), 7.19-7.39 (1H, m), 7.61-7.71 (1H, m), 7.75-7.86 (1H, m), 7.86-8.18 (1H, m), 8.18 and 8.21 (total 1H, each s), 8.45-8.60 (1H, m); ESI+: 580 |
| 394 | 338 | NMR1: 0.78-2.00 (23H, m), 1.42 (6H, s), 2.21-2.70 (3H, m), 2.98 (2H, s), 3.00-3.37 (3H, m), 4.36 (2H, d, J=4.0Hz), 4.43 (1H, d, J=4.0Hz), 6.65-6.74 (1H, m), 6.82-6.91 (1H, m), 6.95-7.05 (1H, m), 7.06-7.29 (1H, m), 7.62-7.97 (1H, m), 8.13-8.20 (1H, m); ESI+: 571 |
| 395 | 338 | NMR1: 0.82-2.67 (33H, m), 2.95-3.18 (2H, m), 4.32-4.42 (3H, m), 5.28-5.34 (1H, m), 6.83-7.40 (3H, m), 7.65-8.00 (2H, m), 8.16 (1H, s); ESI+: 560 |
| 396 | 338 | NMR1: 0.82-2.31 (27H, m), 2.96-3.17 (2H, m), 4.41 (1H, d, J=4.0Hz), 4.48-4.49 (2H, m), 4.77 (2H, q, J=8.0Hz), 6.94-7.23 (5H, m), 7.64-7.91 (1H, m), 8.16 (1H, s); ESI+: 599 |
| 397 | 338 | NMR1: 0.82-2.32 (33H, m), 2.94-3.17 (2H, m), 4.08-4.15 (1H, m), 4.29-4.33 (2H, m), 4.42-4.43 (1H, m), 7.03-7.08 (1H, m), 7.16-7.39 (2H, m), 7.84-8.17 (1H, m), 8.17 (1H, s), 8.27-8.33 (1H, m); ESI+: 576 |
| 398 | 338 | NMR1: 0.81-2.04 (23H, m), 2.20-2.69 (3H, m), 2.54-2.56 (3H, each s), 2.88-3.42 (2H, m), 3.67-3.76 (1H, m), 4.18-4.25 (1H, m), 4.30-4.44 (2H, m), 7.00-7.22 (1H, m), 7.26-7.44 (2H, m), 7.82-8.24 (2H, m), 8.25-8.38 (1H, m); ESI+: 547 |
| 399 | 338 | NMR1: 0.81-2.31 (27H, m), 1.32 (3H, t, J=8.0Hz), 2.93-3.17 (2H, m), 3.22 (2H, q, J=8.0Hz), 4.31-4.36 (2H, m), 4.43 (1H, d, J=4.0Hz), 7.03-7.38 (3H, m), 7.85-8.17 (2H, m), 8.26-8.33 (1H, m); ESI+: 562 |
| 400 | 338 | NMR1: 1.08-2.36 (29H, m), 2.94-3.17 (2H, m), 3.22 (2H, q, J=8.0Hz), 3.72 (1H, brs), 4.22-4.33 (1H, m), 4.31-4.36 (2H, m), 7.04-7.37 (3H, m), 7.86 -8.28 (2H, m), 8.27-8.33 (1H, m); ESI+: 562 |

[Table 131]

| Ex | Syn | Dat |
|---|---|---|
| 401 | 338 | NMR1: 1.04-2.01 (23H, m), 2.28-2.38 (3H, m), 2.92-3.18 (2H, m), 3.68-3.72 (1H, m), 4.19-4.23 (1H, m), 4.50-4.54 (2H, m), 7.15-7.46 (5H, m), 7.90-8.19 (2H, m); ESI+: 535, 537 |
| 402 | 338 | NMR1: 1.06-2.02 (23H, m), 2.30-2.36 (3H, m), 2.93-3.18 (2H, m), 3.68-3.74 (1H, m), 3.89 and 3.92 (total 3H, each s), 4.20-4.40 (3H, m), 6.88-6.94 (1H, m), 7.06-7.44 (2H, m), 7.76-8.06 (2H, m), 8.15-8.16 (1H, m); ESI+: 532 |
| 403 | 338 | NMR1: 0.78-2.39 (26H, m), 3.09-3.36 (3H, m), 4.42 (1H, d, J=4.4Hz), 4.54-4.68 (2H, m), 7.29-7.43 (4H, m), 8.43-8.84 (2H, m), 8.90 (1H, s); ESI+: 605 |

(continued)

| Ex | Syn | Dat |
|---|---|---|
| 404 | 338 | NMR1: 0.79-2.39 (26H, m), 3.11-3.36 (3H, m), 4.42 (1H, d, J=4.4Hz), 4.54-4.66 (2H, m), 7.20-7.32 (3H, m), 7.42-7.47 (1H, m), 8.42-8.59 (1H, m), 8.81-8.92 (1H, m), 8.89 and 8.90 (total 1H, each s); ESI+: 555, 557 |
| 405 | 338 | NMR1: 0.82-2.55 (27H, m), 2.98-3.13 (2H, m), 4.43 (1H, d, J=4.0Hz), 4.65-4.68 (2H, m), 7.11-7.59 (3H, m), 7.98-8.31 (4H, m); ESI+: 591, 593 |
| 406 | 338 | NMR1: 0.82-2.36 (27H, m), 2.97-3.15 (2H, m), 4.43 (1H, d, J=4.0Hz), 4.52-4.55 (2H, m), 7.02-7.30 (4H, m), 7.95-8.22 (2H, m); ESI+: 581 |
| 407 | 338 | NMR1: 0.80-1.32 (15H, m), 1.66-1.91 (9H, m), 2.26-2.28 (2H, m), 2.36 (1H, brs), 2.93-2.94 (2H, m), 4.43 (1H, d, J=4.0Hz), 4.45 (2H, d, J=8.0Hz), 6.99-7.04 (2H, m), 7.29-7.35 (4H, m), 7.67 (1H, d, J=4.0Hz); ESI+: 578 |
| 408 | 338 | NMR1: 0.81-1.31 (15H, m), 1.65-1.91 (9H, m), 2.26-2.28 (2H, m), 2.37 (1H, brs), 2.95 (2H, brs), 4.42-4.43 (3H, m), 6.99-7.07 (2H, m), 7.16- 7.28 (3H, m), 7.37-7.39 (1H, m), 7.66 (1H, d, J=4.0Hz); ESI+: 528, 530 |
| 409 | 338 | NMR1: 0.81-2.48 (28H, m), 2.68-2.72 (2H, m), 3.10-3.19 (2H, m), 3.37-3.51 (2H, m), 4.41 (1H, brs), 6.84-7.03 (2H, m), 7.13-7.19 (1H, m), 7.25 -7.68 (2H, m), 8.10-8.20 (1H, m); ESI+: 565, 567 |
| 410 | 338 | NMR1: 1.06-2.31 (26H, m), 2.93-3.16 (2H, m), 3.71 (1H, brs), 4.21 (1H, d, J=4.0Hz), 4.53-4.55 (2H, m), 7.14-7.33 (5H, m), 7.89-8.14 (1H, m), 8.18 (1H, s); ESI+: 585 |

[Table 132]

| Ex | Syn | Dat |
|---|---|---|
| 411 | 338 | NMR1: 1.07-2.35 (26H, m), 2.96-3.17 (2H, m), 3.71 (1H, brs), 4.20-4.21 (1H, m), 4.47-4.49 (2H, m), 4.77 (2H, q, J=8.8Hz), 6.94-7.24 (5H, m), 7.67-7.94 (1H, m), 8.16 (1H, s); ESI+: 599 |
| 412 | 412 | NMR1: 1.00-2.46 (25H, m), 2.54-2.56 (3H, each s), 2.90-3.37 (3H, m), 4.31-4.40 (2H, m), 7.02-7.40 (3H, m), 8.11-8.22 (2H, m), 8.27-8.37 (1H, m); ESI+: 546 |

The compounds shown in Tables below can be prepared using each of the corresponding starting materials, in the same manner as the methods of Preparation Examples and Examples above.

[Table 133]

| No | Str | No | Str |
|---|---|---|---|
| A1 rel | 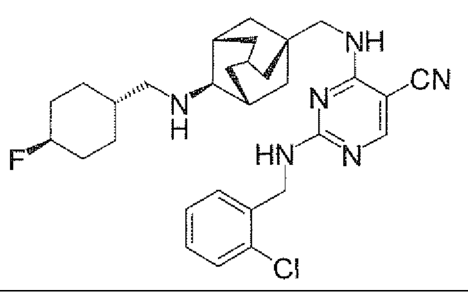 | A2 rel | 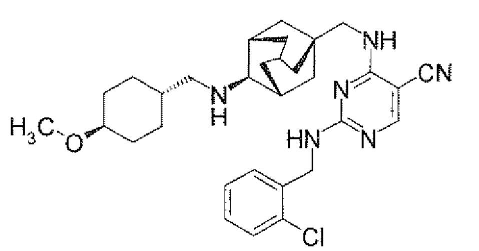 |

(continued)

| No | Str | No | Str |
|---|---|---|---|
| A3 rel | 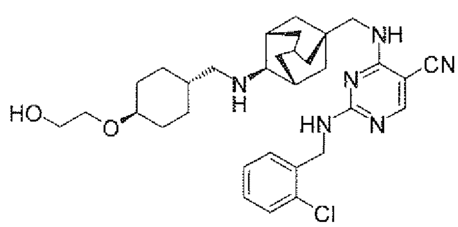 | A4 rel | 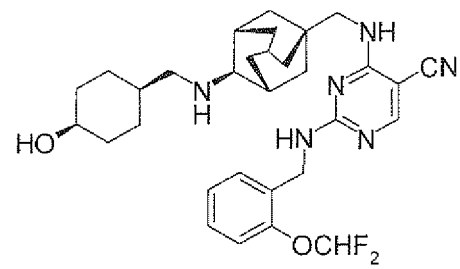 |
| A5 rel | 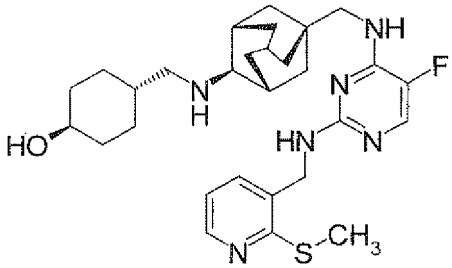 | A6 rel | 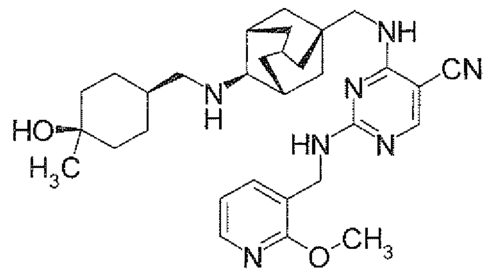 |
| A7 rel | 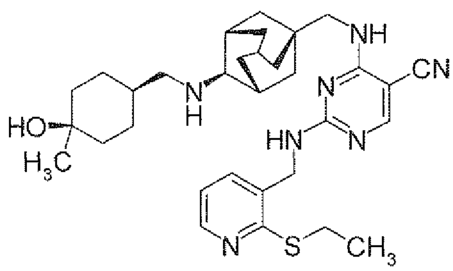 | A8 rel | 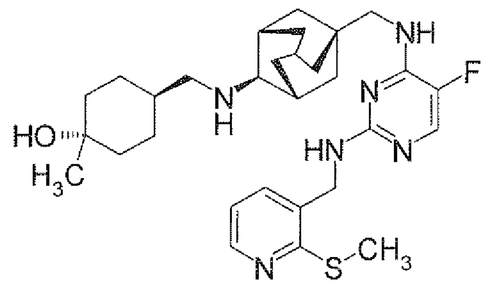 |
| A9 rel | 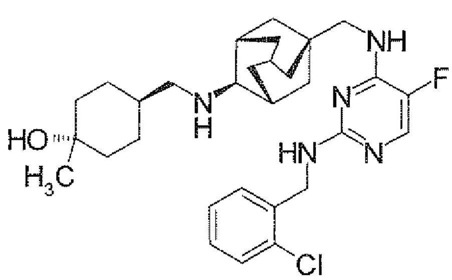 | A10 rel | 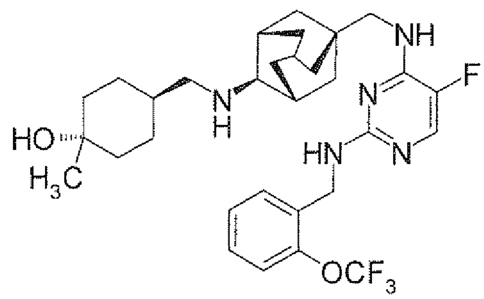 |

[Table 134]

| No | Str | No | Str |
|---|---|---|---|
| A11 rel | 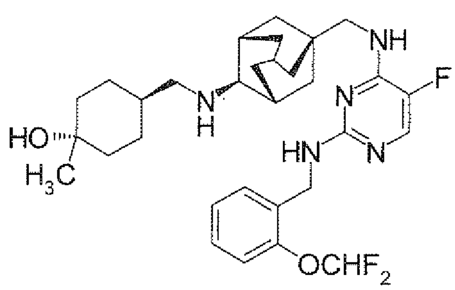 | A12 rel | 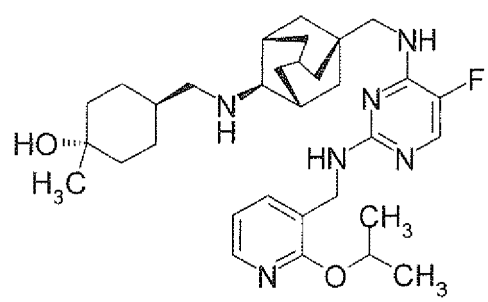 |

(continued)

| No | Str | No | Str |
|---|---|---|---|
| A13 rel | 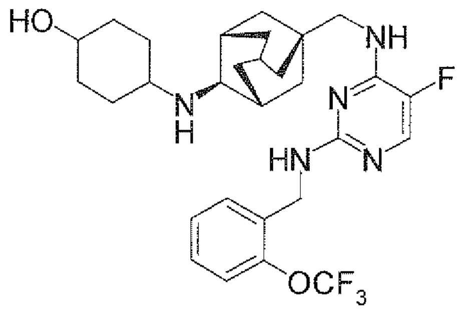 | A14 rel | 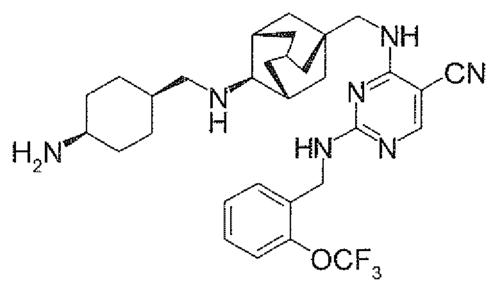 |
| A15 rel | 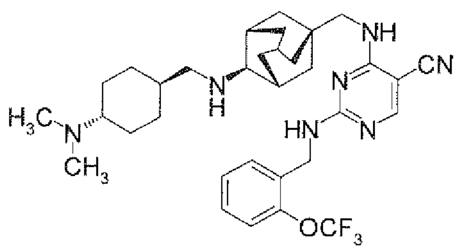 | A16 rel | 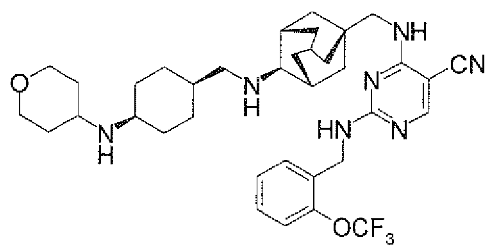 |
| A17 rel | 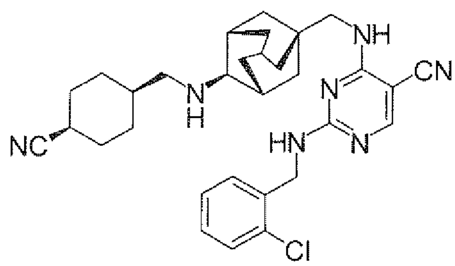 | A18 rel | 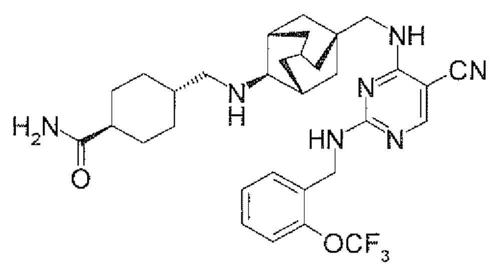 |
| A19 rel | 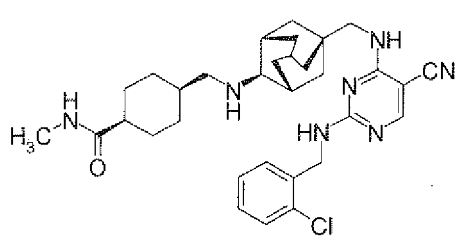 | A20 rel | 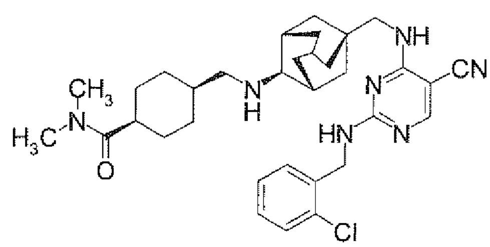 |

[Table 135]

| No | Str | No | Str |
|---|---|---|---|
| A21 rel | 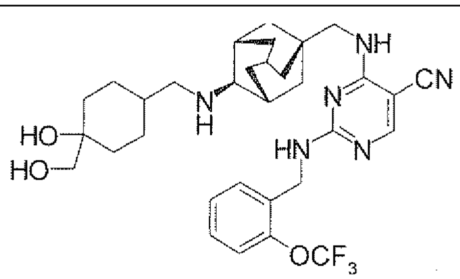 | A22 rel | 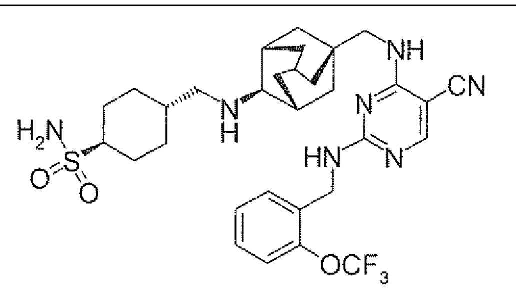 |

(continued)

| No | Str | No | Str |
|---|---|---|---|
| A23 rel | 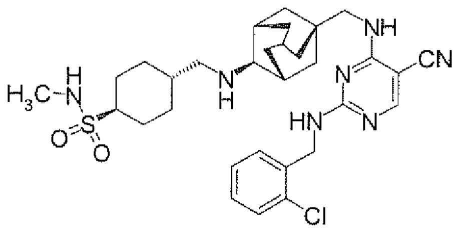 | A24 rel | 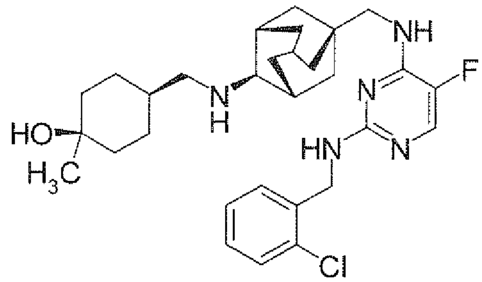 |
| A25 rel | 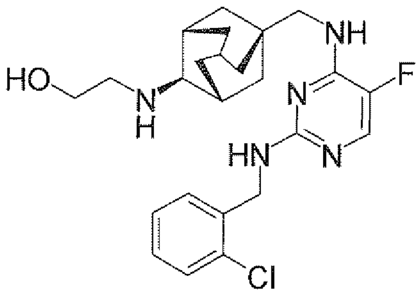 | A26 rel | 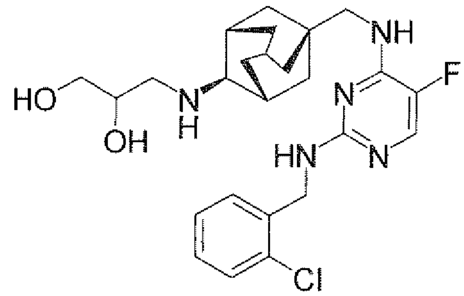 |
| A27 rel | 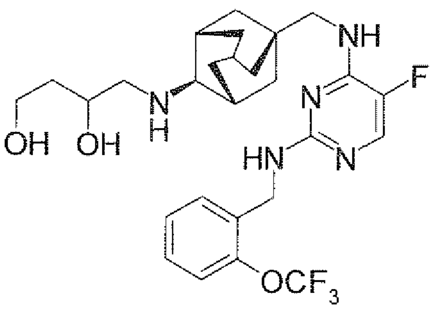 | A28 rel | 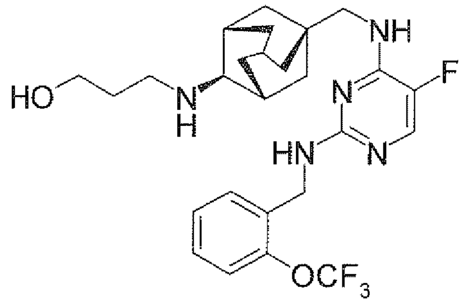 |
| A29 rel | 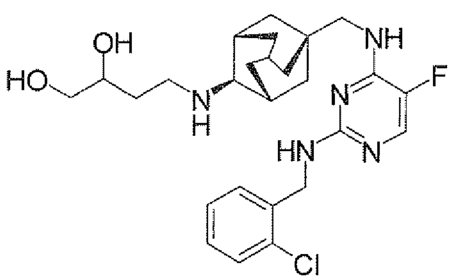 | A30 rel | 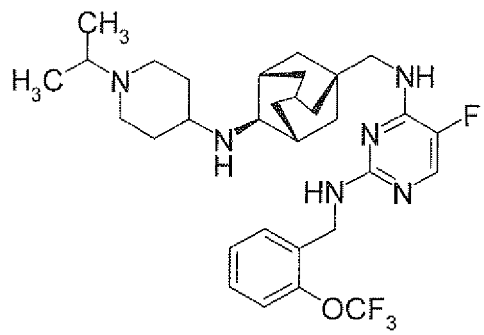 |

[Table 136]

| No | Str | No | Str |
|---|---|---|---|
| A31 rel | 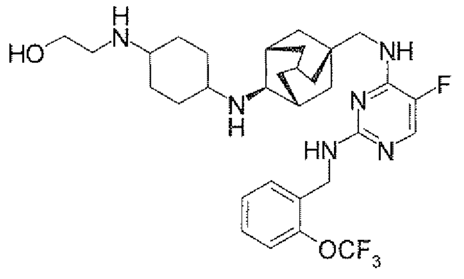 | A32 rel | 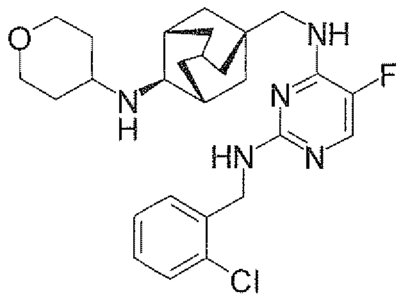 |
| A33 rel | 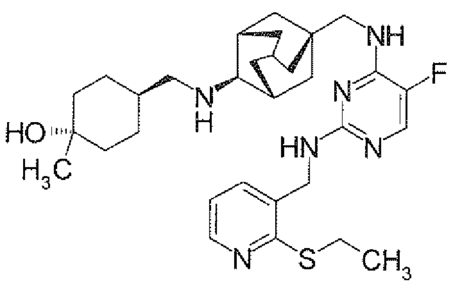 | A34 rel | 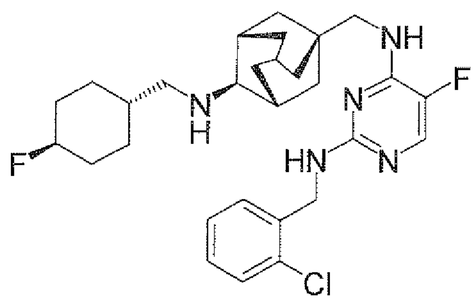 |
| A35 rel | 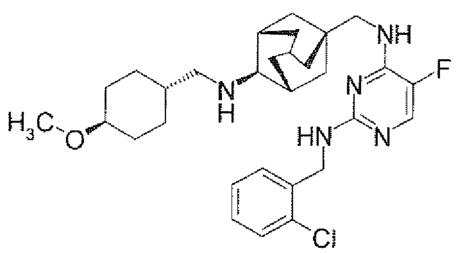 | A36 rel | 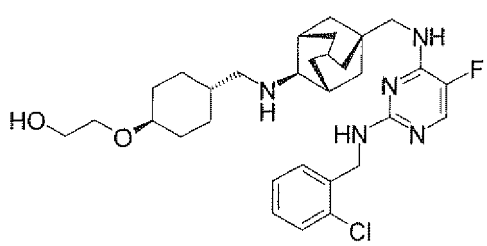 |
| A37 rel | 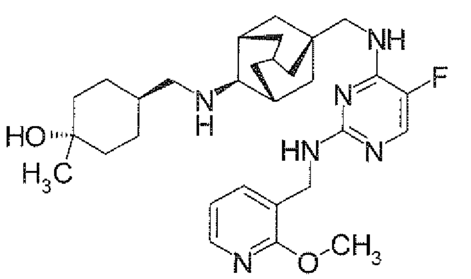 | A38 rel | 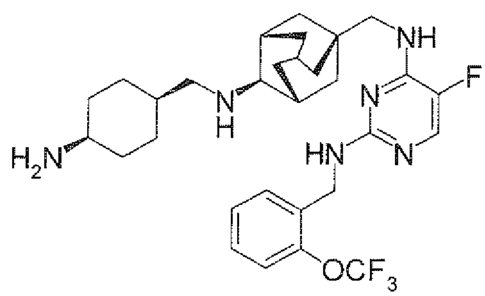 |
| A39 rel | 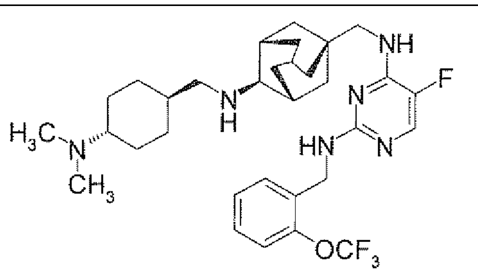 | A40 rel | 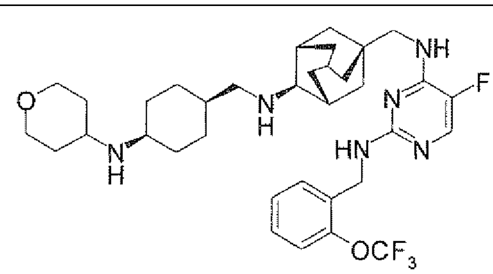 |

[Table 137]

| No | Str | No | Str |
|---|---|---|---|
| A41 rel | 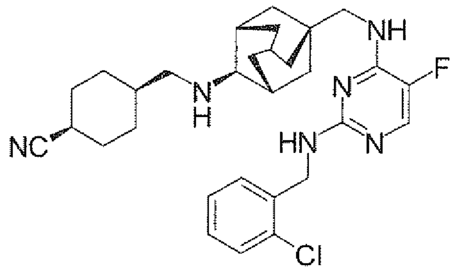 | A42 rel | 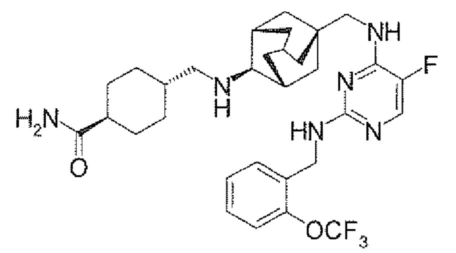 |
| A43 rel | 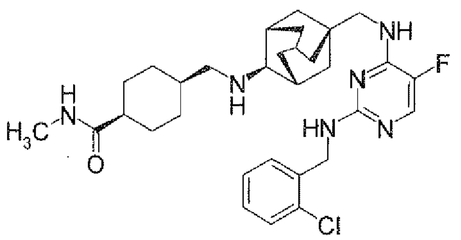 | A44 rel | 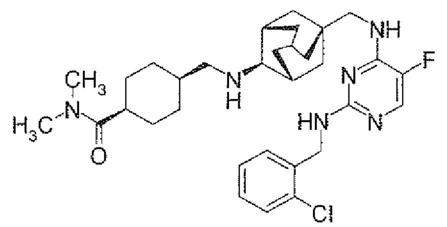 |
| A45 rel | 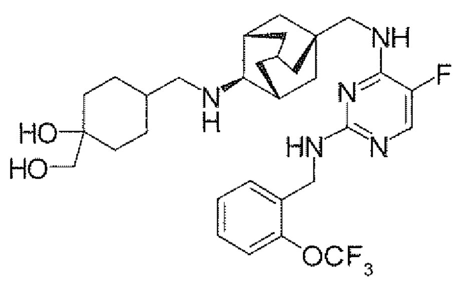 | A46 rel | 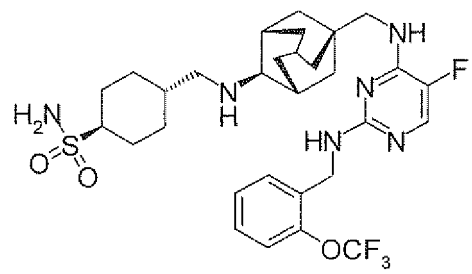 |
| A47 rel | 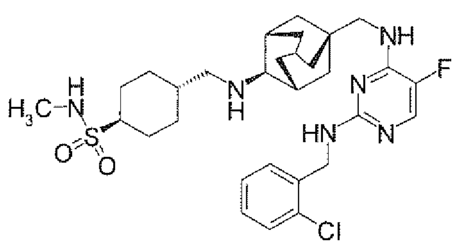 | | |

Industrial Applicability

The compound of the formula (I) or a pharmaceutically acceptable salt has a PKCθ inhibition action and can be used as an inhibitor of acute rejection occurring in transplantation.

## Claims

**1.** A compound of the formula (I) or a pharmaceutically acceptable salt thereof:

[Chem. 21]

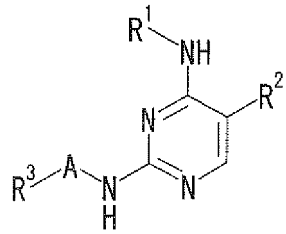

(I)

(the symbols in the formula have the following meanings:

$R^1$ represents any one group selected from the group consisting of:

[Chem. 22]

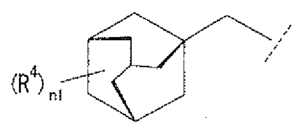, 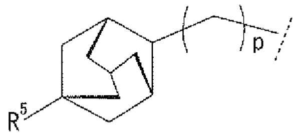, 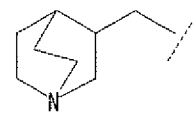,

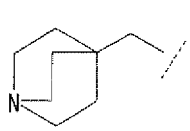, 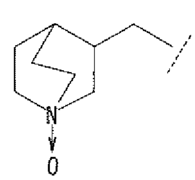, 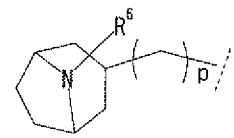,

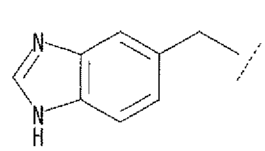, 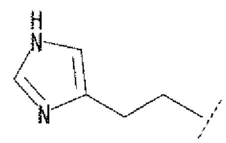, 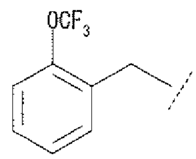,

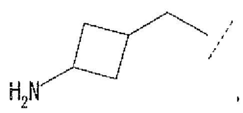, 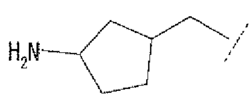, 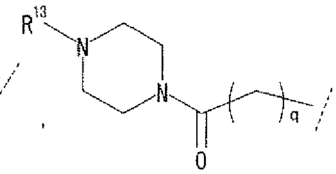,

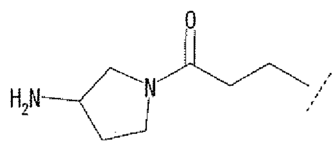, 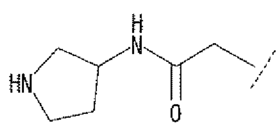, 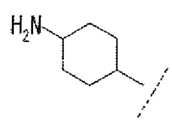,

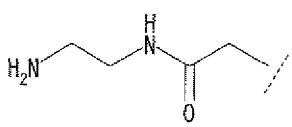, 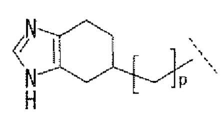, 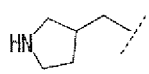,

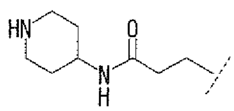, 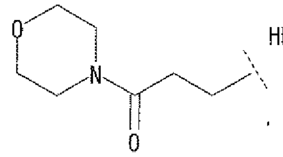, 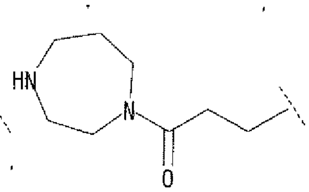;

$R^4$ represents -OH, amine which may be substituted, or $-CH_2NH_2$;

n1 represents 0 or 1;

$R^5$ represents -OH, ($C_{1-6}$ alkyl which may be substituted with -OH or $-NH_2$), or -CN;

$R^6$ represents -H or $C_{1-6}$ alkyl which may be substituted with aryl;

p represents 0 or 1;
q represents 1, 2, 3, or 4;
$R^{13}$ represents -H or $C_{1-6}$ alkyl;
$R^2$ represents -CN, $-CF_3$, $-NO_2$, or halogen;
A represents a single bond or $C_{1-6}$ alkylene;
$R^3$ represents any one group selected from the group consisting of:

[Chem. 23]

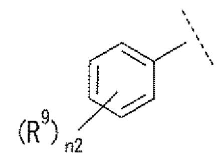,
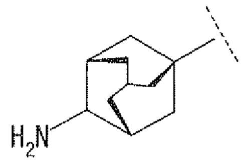,
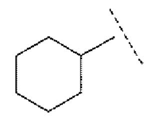,
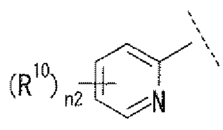,
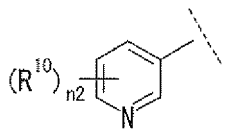,
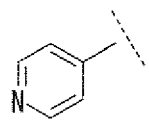,
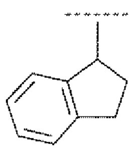,
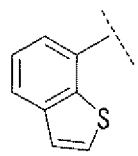,
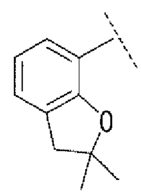,
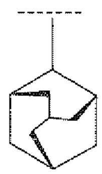,
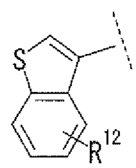,
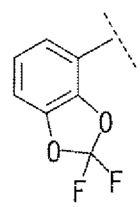,
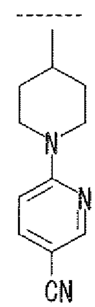
;

$R^9$s are the same as or different from each other and represent halogen, $C_{1-6}$ alkyl which may be substituted, -OH, -CN, cycloalkyl, -Q-($C_{1-6}$ alkyl which may be substituted), or aryl which may be substituted;
Q represents -O-, -S-, -SO-, $-SO_2-$, or $-NHSO_2-$;
n2 represents 0, 1, 2, or 3;
$R^{10}$ represents halogen, $C_{1-6}$ alkyl, -CN, -O-($C_{1-6}$ alkyl), -S-($C_{1-6}$ alkyl), -SO-($C_{1-6}$ alkyl), $-SO_2-$($C_{1-6}$ alkyl), -S-(cycloalkyl), or $-OCF_3$; and
$R^{12}$ represents -H or halogen).

**2.** The compound or a pharmaceutically acceptable salt thereof described in claim 1, wherein
$R^4$ is -OH, $-NR^7R^8$, or $-CH_2NH_2$;
$R^7$ and $R^8$ are the same as or different from each other and represent:

(a) -H;
(b) $C_{1-6}$ alkyl, in which the $C_{1-6}$ alkyl may be substituted with at least one group selected from the group consisting of the following 1) to 12):

1) -OH
2) protected -OH
3) halogen
4) -COOH
5) $-CONH_2$
6) oxo
7) aryl

8) heteroaryl
9) cycloalkyl which may be substituted with at least one group selected from the group consisting of -OH, protected -OH, ($C_{1-6}$ alkyl which may be substituted with -OH), halogen, -CN, $-NR_{14}R_{15}$, $-CONR_{14}R_{15}$, $-SO_2NR_{14}R_{15}$, ($C_{1-6}$ alkyl which may be substituted with -OH)-O-, and oxo
10) heterocycloalkyl which may be substituted with -OH or ($C_{1-6}$ alkyl which may be substituted with -OH, $-OCH_3$, -CN, or halogen)
11) (heterocycloalkyl which may be substituted with -OH or $-NH_2$)-CO-, and
12) (heterocycloalkyl)-NH-CO-;

(c) cycloalkyl, in which the cycloalkyl may be substituted with at least one group selected from the group consisting of the following 1) to 6):

1) -OH
2) $-NHR^{11}$
3) halogen
4) oxo
5) $C_{1-6}$ alkyl which may be substituted with -OH, and
6) heterocyloalkyl which may be substituted with (halogen, -OH, $-CH_2OH$, or $-COCH_3$);

(d) heterocycloalkyl, in which the heterocycloalkyl may be substituted with at least one group selected from the group consisting of the following 1) to 11):

1) $C_{1-6}$ alkyl which may be substituted with (-OH, $-OCH_3$, -CN, halogen, or $-CONH_2$)
2) cycloalkyl
3) aryl
4) heterocycloalkyl
5) heterocycloalkyl-CO-
6) $-COCH_3$
7) $-CONH_2$
8)$-COCH_2OH$
9) $-COOCH_2CH_3$
10) $-SO_2CH_3$
11) oxo, and
12) halogen;

(e) aryl;
(f) nicotinoyl; and
(g) $-SO_2CH_3$; or
(h) $R^7$ and $R^8$, together with a nitrogen atom to which they bind, are a nitrogen-containing a heterocycloalkyl which may be substituted with at least one group selected from the group consisting of (-OH, $-NH_2$, -COOH, $-COCH_3$, $-CONH_2$ and $-CH_2OH$);

$R^{11}$ is -H, $C_{1-6}$ alkyl which may be substituted with (halogen or -OH), cycloalkyl which may be substituted with halogen, heterocycloalkyl which may be substituted with $-COCH_3$, or $-COCH_3$; and
$R^{14}$ and $R^{15}$ are the same as or different from each other and are -H, $C_{1-6}$ alkyl, or heterocycloalkyl.

**3.** The compound or a pharmaceutically acceptable salt thereof described in claim 2,
wherein
$R^1$ is

[Chem. 24]

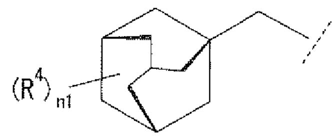

;

and
$R^3$ is

[Chem. 25]

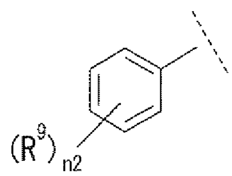

or

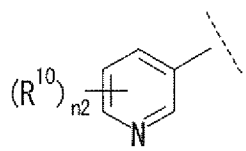

.

**4.** The compound or a pharmaceutically acceptable salt thereof described in claim 3, wherein
$R^4$ is $-NR^7R^8$;
$R^7$ and $R^8$ are the same as or different from each other and are (b) $C_{1-6}$ alkyl, in which the $C_{1-6}$ alkyl may be substituted with at least one group selected from the group consisting of the following 1) to 12):

1) -OH
2) -OH protected with methyl group, or when having two OH groups adjacent to each other, -OH protected with a dimethylmethylene group or a benzylidene group
3) -F
4) -COOH
5) $-CONH_2$
6) oxo
7) phenyl
8) pyridyl
9) cyclohexyl which may be substituted with at least one group selected from the group consisting of -OH and ($C_{1-6}$ alkyl which may be substituted with -OH)
10) (piperidinyl or pyrrolidinyl) which may be substituted with -OH or ($C_{1-6}$ alkyl which may be substituted with -OH, $-OCH_3$, -CN, or -F)
11) (piperazinyl)-CO- or (piperidinyl which may be substituted with -OH or $-NH_2$)-CO-, and
12) (piperidinyl)-NH-CO-; or

(c) cycloalkyl, in which the cycloalkyl may be substituted with at least one group selected from the group consisting of the following 1) to 6):

1) -OH
2) $-NHR^{11}$
3) -F
4) oxo
5) $C_{1-6}$ alkyl which may be substituted with -OH, and
6) (azetidinyl, pyrrolidinyl, piperidinyl, piperazinyl or morpholinyl) which may be substituted with (halogen, -OH, $-CH_2OH$, or $-COCH_3$);

$R^{11}$ is -H;
n1 is 1;
$R^2$ is -CN, $-CF_3$, $-NO_2$, or -F;
A is $C_{1-6}$ alkylene;
$R^9$ is
(i) -F, -Cl, or -Br
(j) $C_{1-6}$ alkyl which may be substituted with -OH or halogen,
(k) -OH,
(l) -CN,
(m) cyclopropyl,
(n) -Q-($C_{1-6}$ alkyl which may be substituted with halogen, -OH, $-OCH_3$, -CN, or $-CONH_2$), or
(o) phenyl which may be substituted with $-CH_2NH_2$; and

n2 is 1.

**5.** The compound or a pharmaceutically acceptable salt thereof described in claim 4, wherein
$R^7$ and $R^8$ are the same as or different from each other and are

(b) $C_{1-6}$ alkyl, in which the $C_{1-6}$ alkyl may be substituted with the following groups:

9) cyclohexyl substituted with at least one group selected from the group consisting of -OH, $-CH_3$, and $-CH_2OH$, and
10) piperidinyl which may be substituted with -OH or ($C_{1-6}$ alkyl which may be substituted with -OH, $-OCH_3$, -CN, or -F); or

(c) cycloalkyl, in which the cycloalkyl may be substituted with at least one group selected from the group consisting of the following 1), 2), and 5):

1) -OH
2) $-NHR^{11}$, and
5) $C_{1-6}$ alkyl which may be substituted with -OH;

$R^{11}$ is -H;
$R^2$ is -CN;
A is methylene;
$R^9$ is

(i) -F, -Cl, or -Br
(j) $C_{1-6}$ alkyl which may be substituted with -OH or -F,
(k) -OH,
(l) -CN,
(m) cyclopropyl,
(n) -Q-($C_{1-6}$ alkyl which may be substituted with halogen, -OH, $-OCH_3$, -CN, or $-CONH_2$), or
(o) phenyl which may be substituted with $-CH_2NH_2$; and

$R^{10}$ is -Cl, $-CH_3$, $-OCH_3$, $-OCH_2CH_3$, $-OCH(CH_3)_2$, $-SCH_3$, $-SCH_2CH_3$, $-SCH(CH_3)_2$, $-SOCH_3$, $-SO_2CH_3$, -S-(cylopentane), or $-OCF_3$.

**6.** A pharmaceutical composition comprising the compound or a pharmaceutically acceptable salt thereof described in claim 1, and a pharmaceutically acceptable excipient.

**7.** A PKCθ inhibitor comprising the compound or a pharmaceutically acceptable salt thereof described in claim 1.

**8.** A pharmaceutical composition for inhibiting acute rejection occurring in transplantation, comprising the compound or a pharmaceutically acceptable salt thereof described in claim 1.

**9.** Use of the compound or a pharmaceutically acceptable salt thereof described in claim 1 for the manufacture of an

inhibitor of acute rejection occurring in transplantation.

**10.** A method for inhibiting acute rejection occurring in transplantation, comprising administering to a patient an effective amount of the compound or a pharmaceutically acceptable salt thereof described in claim 1.

**INTERNATIONAL SEARCH REPORT**

International application No.
PCT/JP2009/065149

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D239/48, A61K31/505, A61K31/506, A61K31/5377, A61K31/5513, A61P37/06, C07D401/12, C07D403/12, C07D405/12, C07D405/14, C07D409/12, C07D453/02, C07D471/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAPlus/REGISTRY(STN), JSTPlus/JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | WO 2004/048343 A1 (SCHERING AG.),<br>10 June, 2004 (10.06.04),<br>Abstract; Claims; page 145; examples 227, 229 etc.<br>& JP 2006-508997 A & US 2004/0186118 A1<br>& EP 1565446 A & CA 2502970 A<br>& BR 316680 A & NO 20053144 A<br>& HR 20050601 A & IS 7881 A<br>& NZ 539823 A & PL 377795 A<br>& KR 10-2005-0084027 A & CN 1717396 A<br>& ZA 200505184 A | 1,6<br>2-5,7-9 |

☒ Further documents are listed in the continuation of Box C.

☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 16 September, 2009 (16.09.09) | 06 October, 2009 (06.10.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/065149

C (Continuation). DOCUMENTS CONSIDERED TO BE RELEVANT

| Category* | Citation of document, with indication, where appropriate, of the relevant passages | Relevant to claim No. |
|---|---|---|
| X<br>A | WO 03/032997 A1 (BOERINGER INGELHEIM PHARMA KG.),<br>24 April, 2003 (24.04.03),<br>Bezeichnung; patentanspruche; page 87, (68); page 91, (109); page 95, (153); page 97, (170); page 99, (192)<br>& JP 2005-509624 A & US 2003/0171359 A1<br>& US 2006/0100211 A1 & EP 1438053 A<br>& DE 50212771 D & CA 2463989 A<br>& UY 27487 A & AT 407678 T<br>& DK 1438053 T & ES 2314106 T | 1,6<br>2-5,7-9 |
| X<br>A | VERMA, S. et al, Substituted aminobenzimidazole pyrimidines as cyclin-dependent kinase inhibitors, Bioorganic & Medicinal Chemistry Letters, 2005, Vol.15, No.8, p.1973-1977, p.1974, Table 1. | 1,6<br>2-5,7-9 |
| X<br>A | WO 2004/067516 A1 (BOERINGER INGELHEIM PHARMACEUTICALS, INC.),<br>12 August, 2004 (12.08.04),<br>Abstract; Claims<br>& JP 2006-515014 A & US 2004/0242613 A1<br>& US 2005/0124640 A1 & EP 1590334 A<br>& CA 2514612 A | 1,6-9<br>2-5 |
| X<br>A | WO 2006/014482 A1 (BOERINGER INGELHEIM PHARMACEUTICALS, INC.),<br>09 February, 2006 (09.02.06),<br>Abstract; Claims<br>& JP 2008-505910 A & US 2006/0025433 A1<br>& US 2008/0287410 A1 & EP 1765791 A<br>& CA 2571937 A | 1,6-9<br>2-5 |
| X<br>A | WO 2007/076247 A1 (BOERINGER INGELHEIM PHARMA GMBH & CO., KG.),<br>05 July, 2007 (05.07.07),<br>Abstract; Claims<br>& JP 2009-521488 A & US 2008/0318929 A1<br>& EP 1966163 A & CA 2633992 A | 1,6-9<br>2-5 |
| P,X<br>P,A | WO 2009/012421 A1 (RIGEL PHARMACEUTICALS, INC.),<br>22 January, 2009 (22.01.09),<br>Abstract; Claims<br>(Family: none) | 1,6-9<br>2-5 |
| A | CYWIN, C.L. et al, Discovery of potent and selective PKC-θ inhibitors, Bioorganic & Medicinal Chemistry Letters, 2007, Vol.17, No.1, p.225-230 | 1-9 |

Form PCT/ISA/210 (continuation of second sheet) (April 2007)

**INTERNATIONAL SEARCH REPORT**

International application No.
PCT/JP2009/065149

C (Continuation). DOCUMENTS CONSIDERED TO BE RELEVANT

| Category* | Citation of document, with indication, where appropriate, of the relevant passages | Relevant to claim No. |
|---|---|---|
| A | WO 2005/118544 A2 (RIGEL PHARMACEUTICALS, INC.), 15 December, 2005 (15.12.05), & JP 2007-538092 A & JP 2007-501793 A & JP 2007-514775 A & JP 2008-520580 A & US 2005/0113398 A1 & US 2006/0035891 A1 & US 2006/0167249 A1 & US 2006/0228272 A1 & US 2007/0010217 A1 & US 2007/0032912 A1 & US 2008/0009494 A1 & US 2008/0027045 A1 & US 2005/0192301 A1 & US 2006/0232899 A1 & US 2008/0051412 A1 & US 2009/0137589 A & US 2009/0176981 A & GB 2420559 A & EP 1763514 A & EP 1663242 A & EP 1694652 A & EP 1824489 A & WO 2005/013996 A2 & WO 2005/063722 A1 & WO 2006/055561 A2 & DE 102005054092 A & FR 2877944 A & CA 2566531 A & CA 2580150 A & NO 20073068 A & KR 10-2007-0085571 A & ES 2296477 A & CN 101171012 A & BRA PI0516795 & ZA 200702180 A | 1-9 |

Form PCT/ISA/210 (continuation of second sheet) (April 2007)

**INTERNATIONAL SEARCH REPORT**

International application No.
PCT/JP2009/065149

Continuation of A. CLASSIFICATION OF SUBJECT MATTER
(International Patent Classification (IPC))

C07D239/48(2006.01)i, A61K31/505(2006.01)i, A61K31/506(2006.01)i, A61K31/5377(2006.01)i, A61K31/5513(2006.01)i, A61P37/06(2006.01)i, C07D401/12(2006.01)i, C07D403/12(2006.01)i, C07D405/12(2006.01)i, C07D405/14(2006.01)i, C07D409/12(2006.01)i, C07D453/02(2006.01)i, C07D471/08(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/065149

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 10
because they relate to subject matter not required to be searched by this Authority, namely:
Claim 10 pertains to methods for treatment of the human body by therapy and thus relates to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004067516 A
- WO 2006014482 A
- WO 2007076247 A
- WO 2003032997 A
- WO 2004043936 A
- WO 2004054617 A


### Non-patent literature cited in the description


- *Prog. Med.,* 1985, vol. 5, 2157-2161
- Iyakuhin no Kaihatsu. Bunshi Sekkei. Hirokawa Shoten, 1990, vol. 7, 163-198
- Green's Protective Groups in Organic Synthesis. 2006
- Organic Functional Group Preparations. Academic Press Inc, 1991, vol. 1
- Jikken Kagaku Koza. Maruzen, 2005, vol. 14
- Organic Functional Group Preparations. Academic Press Inc, 1991
- Jikken Kagaku Koza. Maruzen, 2005, vol. 16
- Comprehensive Organic Functional Group Transformations II. Elsevier Pergamon, 2005, vol. 2
- Reductions in Organic Chemistry. ACS, 1996
- Comprehensive Organic Transformations. VCH Publishers, Inc, 1999
- Oxidation and Reduction in Organic Synthesis. Oxford Science Publications, 2000
- Jikken Kagaku. Maruzen, 2005, vol. 14